# EUROPEAN PATENT APPLICATION

(11) **EP 1 321 521 A1**
(43) Date of publication of application: **25.06.2003**
(21) Application number: 03002270.1
(22) Date of filing: 05.05.1998
(51) Int. Cl.: C12N 15/10

(54) **Ribozymes directed against flt-1**

(30) Priority: 09.05.1997 US 46059 P; 09.06.1997 US 49002 P; 03.07.1997 US 51718 P; 22.08.1997 US 56808 P; 02.10.1997 US 61324 P; 02.10.1997 US 61321 P; 05.11.1997 US 64866 P; 19.12.1997 US 68212 P
(62) Divisional of application: 98920299.9
(71) Applicant: RIBOZYME PHARMACEUTICALS, INC., Boulder, CO 80301 (US)
(72) Inventor: Jarvis, Thale, Boulder, CO 80301 (US); Matulic-Adamic, Jasenka, Boulder, CO 80303 (US); Reynolds, Mark, Lafayette, CO 80026 (US); Kisich, Kevin, Lafayette, CO 80026 (US); Bellon, Laurent, Boulder, CO 80301 (US); Parry, Tom, Broomfield, CO 80020 (US); Beigelman, Leonid, Longmont, CO 80503 (US); McSwiggen, James A., Boulder, CO 80301 (US); Karpeisky, Alexander, Lafayette, CO 80024 (US); Burgin, Alex, Chula Vista, CA 91910 (US); Thompson, James, Boulder, CO 80301 (US); Workman, Christopher, Boulder, CO 80303 (US); Beaudry, Amber, Lafayette, CO 80026 (US); Sweedler, David, Louisville, CO 80027 (US)
(74) Representative: Viering, Jentschura & Partner

(57) **Abstract**

Nucleic acid catalysts which modulate the expression of flt-1 gene; methods of delivery, screening, identification, synthesis, deprotection, purification of nucleic acid catalysts and process for identification of nucleic acid molecules is described.

## Description

### Background Of The Invention

This invention relates to methods and reagents for the treatment of diseases or conditions relating to the levels of expression of *raf* genes.

The following is a discussion of relevant art, none of which is admitted to be prior art to the present invention.

The Raf family of serine/threonine kinases function as cytoplasmic signaling proteins that transduce mitogenic signals in response to activation of various growth factor receptors (for reviews, see Daum, 1994 *Trends in Biochem. Sci.* 19, 474; Katz, 1997, *Curr. Opin. Genet. Devel.* 7, 75; Marais, 1996, *Cancer Surveys* 27; Naumann, 1997, *Cancer Res.* 143, 237). c-Raf is the cellular homolog of v-Raf, the transforming element of the murine sarcoma virus 3611. The Raf family consists of three highly conserved isozymes in vertebrates: c-Raf-1, which is constitutively expressed in all tissues, A-Raf, which is expressed in urogenital tissue and B-Raf which is expressed in and cerebrum and testes (Storm, 1990, *Oncogene 5,* 345). Inappropriate expression of these key genes involved in cell growth and differentiation can result in uncontrolled cell proliferation and/or propagation of damaged DNA, leading to hyperproliferative disorders such as cancer, restenosis, psoriasis and rheumatoid arthritis.

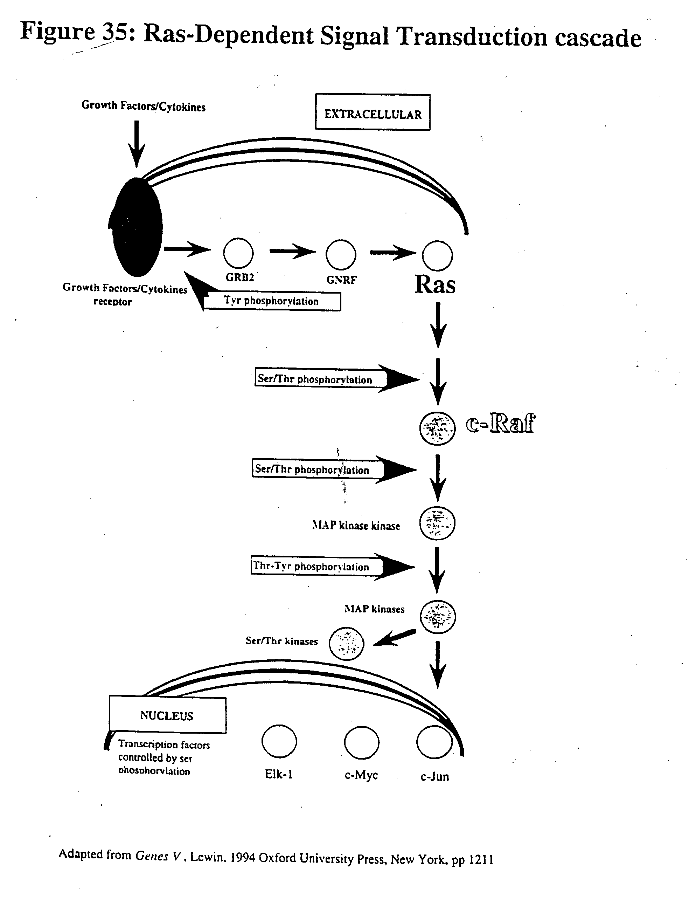

Raf is one of the major downstream effectors of Ras, a member of the class of small GDP/GTP-binding proteins involved in cellular signal transduction pathways (figure 35; Marshall, 1995, *Molec. Reprod. Devel., 42, 493*). Appropriate mitogenic signals cause an increase in levels of the GTP-bound Ras. In its GTP-bound active state, Ras binds Raf and localizes it to the plasma membrane. This results in activation of the Raf kinase activity. Activated Raf in turn phosphorylates MEK, thereby activating the MAP kinase signaling cascade leading to cell cycle progression. Amino terminal truncation of Raf leads to constitutively active protein. Expression of either constitutively active Raf or constitutively active MEK is sufficient for oncogenic transformation of fibroblasts (Cowley, 1994, *Cell* 77, 81; Mansour, 1994, *Science* 265, 966; Kolch, 1991, *Nature* 349, 426). In normal cells, the expression level of Raf is limiting in cellular transformation (Cuadrado, 1993, *Oncogene* 8, 2443). The pivotal position that the Ras and Raf family of proteins occupy in cellular signal transduction pathways emphasizes their importance in the control of normal cellular growth.

Activation of Raf in mammalian cells is triggered by a variety of growth factors and cytokines. Raf activation has been observed in cardiac myocyte cultures stimulated by fibroblast growth factor (FGF), endothelin or phorbol ester (Bogoyevitch, 1995, *J. Biol. Chem.* 270, 1). Activation has also been seen in Swiss 3T3 cells treated with bombesin and platelet derived growth factor (Mitchell, 1995, *J. Biol. Chem.* 270, 8623) or with colony stimulating factor or lipopolysacchride (Reimann, 1994, *J. Immun.* 153, 398), in L6 myoblasts stimulated with insulin-like growth factor (Cross, 1994, *Biochem J.* 303, 21), as well as in B cells stimulated via the immunoglobulin receptor (Kumar, 1995, *Biochem J.* 307, 215).

There is growing evidence from a number of laboratories that suggests that the Ras/Raf pathway may also be involved in cell motility (Bar-Sagi and Feramisco, 1986 *Science* 233, 1061; Partin *et al.,* 1988 *Cancer Res.* 48-6050; Fox *et al.,* 1994 *Oncogene* 9, 3519). These studies show that cell lines transfected with activated Ras show an increase in ruffling, pseudopod extension and chemotactic response, all of which are cell-motility-related processes. Uncontrolled cell motility has been implicated in several pathological processes such as restenosis, angiogenesis and wound healing.

Raf activation leads to induction of several immediate early transcription factors including NF-kB and AP-1 (Bruder, 1992, *Genes Devel. 6,* 545; Finco, 1993, *J. Biol. Chem.* 268, 17676). AP-1 regulates expression of a variety of proteases (Sato, 1994 *Oncogene* 8, 395; Gaire, 1994, *J Biol Chem* 269, 2032; Lauricell-Lefebvre, 1993, *Invasion Metastasis* 13, 289; Troen, 1991, *Cell Growth Differ* 2, 23). A cascade of MMP and serine proteinase expression is implicated in the acquisition of an invasive phenotype as well as in angiogenesis in tumors (MacDougall, 1995, *Cancer and Metastasis Reviews* 14, 351). Thus, Raf signaling is expected to contribute to increased invasiveness in tumor cells, leading to metastasis.

Coexpression studies of Raf-1 and Bcl-2 have shown that these proteins bind and interact to synergistically suppress apoptosis (Wang, 1994, *Oncogene* 9, 2751). Thus, overexpression of Raf-1 in tumor cells is likely to contribute to malignant transformation and increased resistance to chemotherapeutic agents. Overexpression of c-Raf-1 is observed in squamous cell carcinomas of the head and neck taken from patients resistant to radiation therapy (Riva, 1995, *Oral Oncol., Eur. J. Cancer* 31B, 384) and in lung carcinomas (Rapp, 1988, *The Oncogene Handbook,* 213). Activated (truncated) Raf has been detected in a number of human cancers including small-cell lung, stomach, renal, breast and laryngeal cancer (Rapp, 1988, *The Oncogene Handbook,* 213).

Therapeutic intervention in down-regulating Raf expression have focused on antisense oligonucleotide approaches:

Antisense oligonucleotides targeting c-Raf-1 were used to demonstrate that IL-2 stimulated growth of T cells requires c-raf (Riedel, 1993, *Eur. J. Immunol.* 23, 3146). Antisense oligonucleotides targeting c-Raf-1 in SQ-20B cells showed reduced Raf expression and increased radiation sensitivity (Soldatenkov, 1997, *The Cancer J. from Scientific American* 3, 13). Rapp et al. have disclosed a method for inhibiting c-Raf-1 gene expression using a vector expressing the gene in the antisense orientation (International PCT Publication No. WO 93/04170). Antisense oligonucleotides targeting c-Raf-1 in SQ-20B cells showed reduced DNA synthesis in response to insulin stimulation in rat hepatoma cells (Tornkvist, 1994, *J*. *Biol. Chem.* 269, 13919). Monia *et al*. have disclosed a method for inhibiting Raf expression using antisense oligonucleotides (U.S. Patent No. 5,563,255) and shown that antisense oligonucleotides targeting c-Raf-1 can inhibit Raf mRNA expression in cell culture, and inhibit growth of a variety of tumor types in human tumor xenograft models (Monia *et al*., 1996, *Proc. Natl. Acad. Sci.*93, 15481; Monia *et al.,* 1996, *Nature Med.* 2, 668). No toxicity was observed in these studies following systemic administration of c-Raf antisense oligonucleotides, suggesting that at least partial down regulation of Raf in normal tissues is not overtly toxic.

It has been proposed that synthetic ribozymes can be delivered to target cells exogenously in the presence or absence of lipid delivery vehicles (Thompson *et al*., International PCT Publication No. WO 93/23057; Sullivan *et al*., International PCT Publication No. WO 94/02595).

Recently Sandberg *et al*., 1996, Abstract, IBC USA Conferences on Angiogenesis Inhibitors and other novel therapeutics for Ocular Diseases of Neovascularization, reported pharmacokinetics of a chemically modifies hammerhead ribozyme targeted against a vascular endothelial growth factor (VEGF) receptor RNA in normal and tumor bearing mice after daily bolus or continuous infusion.

Desjardins *et al.,* 1996, *J. Pharmacol. Exptl. Therapeutic,* 27, 8, 1419, reported pharmacokinetics of a synthetic, chemically modified hammerhead ribozyme against the rat cytochrome P-450 3A2 mRNA after single intravenous injection.

The references cited above are distinct from the presently claimed invention since they do not disclose and/or contemplate the use of ribozymes to cleave Raf RNA. Furthermore, Applicant believes that the references do not disclose and/or enable the use of ribozymes to down regulate normal Raf gene expression in mammalian cells and/or whole animal.

### Summary Of The Invention

This invention relates to identification, synthesis and use of nucleic acid catalysts to cleave RNA species that are required for cellular growth responses. In particular, applicant describes the selection and function of ribozymes capable of cleaving RNA encoded by *c-raf* gene. Such ribozymes may be used to inhibit the hyper-proliferation of tumor cells in one or more cancers, restenosis, psoriasis, fibrosis and rheumatoid arthritis.

In the present invention, ribozymes that cleave *c-raf* RNA are described. Moreover, applicant shows that these ribozymes are able to inhibit gene expression and cell proliferation *in vitro* and *in vivo,* and that the catalytic activity of the ribozymes is required for their inhibitory effect. From those of ordinary skill in the art, it is clear from the examples described herein, that other ribozymes that cleave target RNAs required for cell proliferation may be readily designed and are within the invention.

By "inhibit" is meant that the activity of *c-raf* or level of RNAs encoded by *c-raf* is reduced below that observed in the absence of the nucleic acid, particularly, inhibition with ribozymes is preferably below that level observed in the presence of an inactive RNA molecule able to bind to the same site on the mRNA, but unable to cleave that RNA.

By "nucleic acid catalyst" is meant a nucleic acid molecule capable of catalyzing (altering the velocity and/or rate of) a variety of reactions including the ability to repeatedly cleave other separate nucleic acid molecules (endonuclease activity) in a nucleotide base sequence-specific manner. Such a molecule with endonuclease activity may have complementarity in a substrate binding region to a specified gene target, and also has an enzymatic activity that specifically cleaves RNA or DNA in that target. That is, the nucleic acid molecule with endonuclease activity is able to intramolecularly or intermolecularly cleave RNA or DNA and thereby inactivate a target RNA or DNA molecule. This complementarity functions to allow sufficient hybridization of the enzymatic RNA molecule to the target RNA or DNA to allow the cleavage to occur. 100% complementarity is preferred, but complementarity as low as 50-75% may also be useful in this invention. The nucleic acids may be modified at the base, sugar, and/or phosphate groups. The term enzymatic nucleic acid is used interchangeably with phrases such as ribozymes, catalytic RNA, enzymatic RNA, catalytic DNA, catalytic oligonucleotides, nucleozyme, DNAzyme, RNA enzyme, endoribonuclease, endonuclease, minizyme, leadzyme, oligozyme or DNA enzyme. All of these terminologies describe nucleic acid molecules with enzymatic activity. The specific nucleic acid catalysts described in the instant application are not limiting in the invention and those skilled in the art will recognize that all that is important in a nucleic acid catalyst of this invention is that it has a specific substrate binding site which is complementary to one or more of the target nucleic acid regions, and that it have nucleotide sequences within or surrounding that substrate binding site which impart a nucleic acid cleaving activity to the molecule.

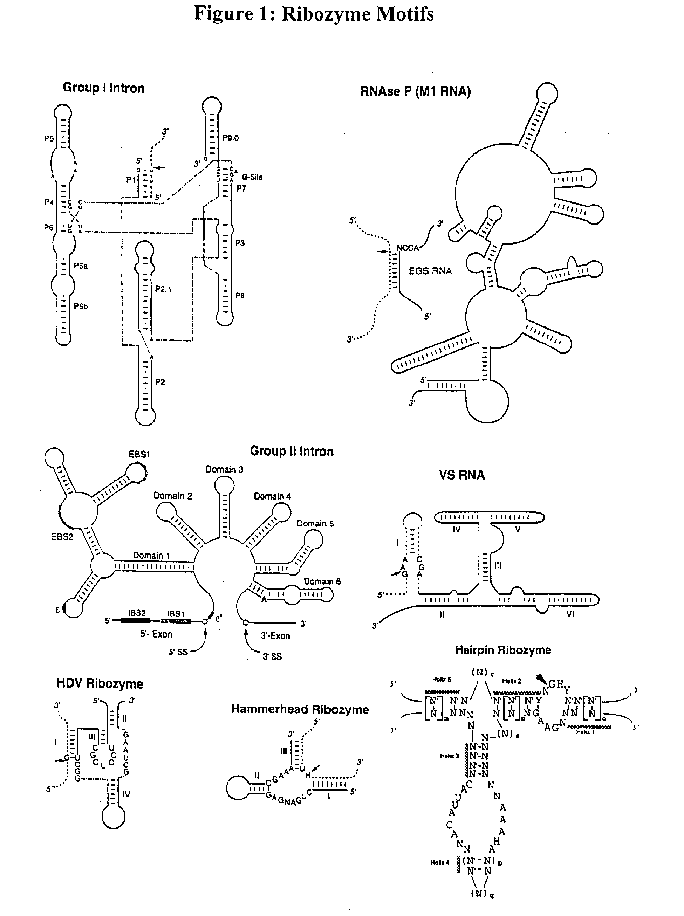

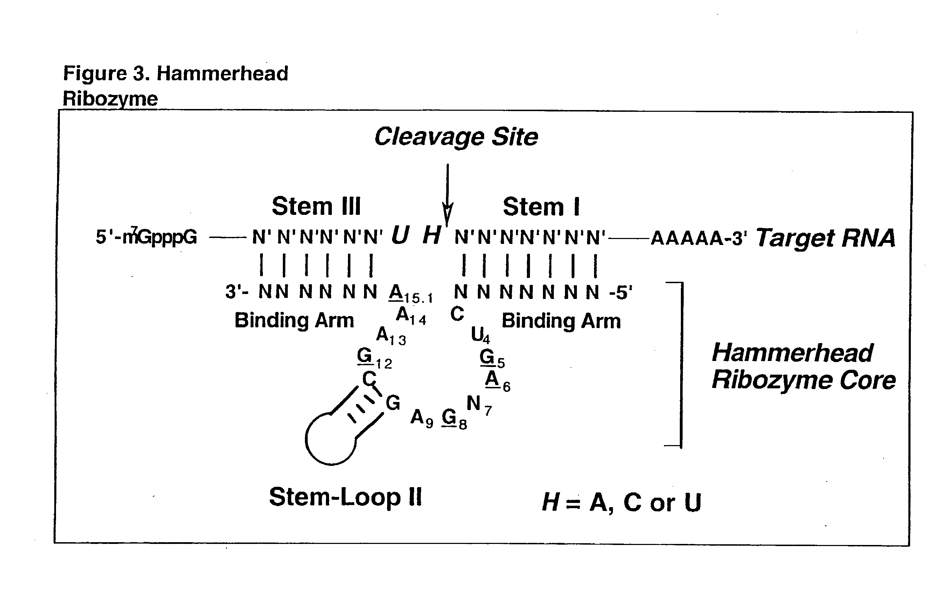

By "substrate binding arm" or "substrate binding domain" is meant that portion/region of a ribozyme which is complementary to (*i.e.,* able to base-pair with) a portion of its substrate. Generally, such complementarity is 100%, but can be less if desired. For example, as few as 10 bases out of 14 may be base-paired. Such arms are shown generally in Figure 1 and 3. That is, these arms contain sequences within a ribozyme which are intended to bring ribozyme and target RNA together through complementary base-pairing interactions. The ribozyme of the invention may have binding arms that are contiguous or non-contiguous and may be of varying lengths. The length of the binding arm(s) are preferably greater than or equal to four nucleotides; specifically 12-100 nucleotides; more specifically 14-24 nucleotides long. If two binding arms are chosen, the design is such that the length of the binding arms are symmetrical (*i.e*., each of the binding arms is of the same length; *e.g.,* five and five nucleotides, six and six nucleotides or seven and seven nucleotides long) or asymmetrical (*i*.*e*., the binding arms are of different length; *e.g*., six and three nucleotides; three and six nucleotides long; four and five nucleotides long; four and six nucleotides long; four and seven nucleotides long; and the like).

In one of the preferred embodiments of the inventions herein, the nucleic acid catalyst is formed in a hammerhead or hairpin motif, but may also be formed in the motif of a hepatitis d virus, group I intron, group II intron or RNaseP RNA (in association with an RNA guide sequence) or *Neurospora* VS RNA. Examples of such hammerhead motifs are described by Dreyfus, *supra,* Rossi *et al.,* 1992, *AIDS Research and Human Retroviruses* 8, 183; of hairpin motifs by Hampel *et al.,* EP0360257, Hampel and Tritz, 1989 *Biochemistry* 28, 4929, Feldstein *et al.,* 1989, *Gene* 82, 53, Haseloff and Gerlach, 1989, *Gene,* 82, 43, and Hampel *et al.,* 1990 *Nucleic Acids Res.* 18, 299; of the hepatitis d virus motif is described by Perrotta and Been, 1992 *Biochemistry* 31, 16; of the RNaseP motif by Guerrier-Takada *et al.,* 1983 *Cell* 35, 849; Forster and Altman, 1990, *Science* 249, 783; Li and Altman, 1996, *Nucleic Acids Res.* 24, 835; *Neurospora* VS RNA ribozyme motif is described by Collins (Saville and Collins, 1990 *Cell* 61, 685-696; Saville and Collins, 1991 *Proc. Natl. Acad. Sci. USA* 88, 8826-8830; Collins and Olive, 1993 *Biochemistry* 32, 2795-2799; Guo and Collins, 1995, *EMBO. J*. 14, 363); Group II introns are described by Griffin *et al.,* 1995, *Chem. Biol.* 2, 761; Michels and Pyle, 1995, *Biochemistry* 34, 2965; Pyle *et al.,* International PCT Publication No. WO 96/22689; and of the Group I intron by Cech *et al.,* U.S. Patent 4,987,071. These specific motifs are not limiting in the invention and those skilled in the art will recognize that all that is important in a nucleic acid catalyst of this invention is that it has a specific substrate binding site which is complementary to one or more of the target gene RNA regions, and that it have nucleotide sequences within or surrounding that substrate binding site which impart an RNA cleaving activity to the molecule.

By "equivalent" RNA to *c-raf* is meant to include those naturally occurring RNA molecules associated with cancer in various animals, including human, rodent, primate, rabbit and pig. The equivalent RNA sequence also includes in addition to the coding region, regions such as 5'-untranslated region, 3'-untranslated region, introns, intron-exon junction and the like.

By "complementarity" is meant a nucleic acid that can form hydrogen bond(s) with another RNA sequence by either traditional Watson-Crick or other non-traditional types (for example, Hoogsteen type) of base-paired interactions.

In a preferred embodiment the invention provides a method for producing a class of enzymatic cleaving agents which exhibit a high degree of specificity for the RNA of a desired target. The nucleic acid catalyst is preferably targeted to a highly conserved sequence region of a target mRNAs encoding c-raf proteins such that specific treatment of a disease or condition can be provided with either one or several enzymatic nucleic acids. Such nucleic acid catalysts can be delivered exogenously to specific cells as required. Alternatively, the ribozymes can be expressed from DNA/RNA vectors that are delivered to specific cells.

Such ribozymes are useful for the prevention of the diseases and conditions discussed above, and any other diseases or conditions that are related to the levels of c-Raf activity in a cell or tissue.

By "related" is meant that the inhibition of c-raf RNAs and thus reduction in the level of respective protein activity will relieve to some extent the symptoms of the disease or condition.

In preferred embodiments, the ribozymes have binding arms which are complementary to the target sequences in **TablesXII-XIX.** Examples of such ribozymes are also shown in **Tables XII-XIX.** Examples of such ribozymes consist essentially of sequences defined in these Tables.

By "consists essentially of" is meant that the active ribozyme contains an enzymatic center or core equivalent to those in the examples, and binding arms able to bind mRNA such that cleavage at the target site occurs. Other sequences may be present which do not interfere with such cleavage.

Thus, in a first aspect, the invention features ribozymes that inhibit gene expression and/or cell proliferation. These chemically or enzymatically synthesized RNA molecules contain substrate binding domains that bind to accessible regions of their target mRNAs. The RNA molecules also contain domains that catalyze the cleavage of RNA. The RNA molecules are preferably ribozymes of the hammerhead or hairpin motif. Upon binding, the ribozymes cleave the target mRNAs, preventing translation and protein accumulation. In the absence of the expression of the target gene, cell proliferation is inhibited.

In a preferred embodiment, ribozymes are added directly, or can be complexed with cationic lipids, packaged within liposomes, or otherwise delivered to target cells. The nucleic acid or nucleic acid complexes can be locally administered to relevant tissues ex vivo, or in vivo through injection, infusion pump or stent, with or without their incorporation in biopolymers. In another preferred embodiment, the ribozyme is administered to the site of *c-raf* expression (e.g., tumor cells) in an appropriate liposomal vehicle.

In another aspect of the invention, ribozymes that cleave target molecules and inhibit *c-raf* activity are expressed from transcription units inserted into DNA or RNA vectors. The recombinant vectors are preferably DNA plasmids or viral vectors. Ribozyme expressing viral vectors could be constructed based on, but not limited to, adeno-associated virus, retrovirus, adenovirus, or alphavirus. Preferably, the recombinant vectors capable of expressing the ribozymes are delivered as described above, and persist in target cells. Alternatively, viral vectors may be used that provide for transient expression of ribozymes. Such vectors might be repeatedly administered as necessary. Once expressed, the ribozymes cleave the target mRNA. Delivery of ribozyme expressing vectors could be systemic, such as by intravenous or intramuscular administration, by administration to target cells ex-planted from the patient followed by reintroduction into the patient, or by any other means that would allow for introduction into the desired target cell (for a review see Couture and Stinchcomb, 1996, *TIG*., 12, 510). In another aspect of the invention, ribozymes that cleave target molecules and inhibit cell proliferation are expressed from transcription units inserted into DNA, RNA, or viral vectors. Preferably, the recombinant vectors capable of expressing the ribozymes are locally delivered as described above, and transiently persist in smooth muscle cells. However, other mammalian cell vectors that direct the expression of RNA may be used for this purpose.

By "patient" is meant an organism which is a donor or recipient of explanted cells or the cells themselves. "Patient" also refers to an organism to which nucleic acid catalysts can be administered. Preferably, a patient is a mammal or mammalian cells. More preferably, a patient is a human or human cells.

By "vectors" is meant any nucleic acid- and/or viral-based technique used to deliver a desired nucleic acid.

These ribozymes, individually, or in combination or in conjunction with other drugs, can be used to treat diseases or conditions discussed above. For example, to treat a disease or condition associated with c-raf levels, the patient may be treated, or other appropriate cells may be treated, as is evident to those skilled in the art.

In a further embodiment, the described ribozymes can be used in combination with other known treatments to treat conditions or diseases discussed above. For example, the described ribozymes could be used in combination with one or more known therapeutic agents to treat cancer.

In preferred embodiments, the ribozymes have binding arms which are complementary to the sequences in the tables, shown as **Seq. I.D. Nos. 1-501, 1078-1152, 1461-1768, 1841-1912, 2354-2794 and 2846-2956.** Examples of such ribozymes are shown as **Seq. I.D. Nos. 502-1002, 1003-1077, 1153-1460, 1769-1840, 1913-2353 and 2795-2845**. Other sequences may be present which do not interfere with such cleavage.

Ribozymes that cleave the specified sites in Raf mRNAs represent a novel therapeutic approach to treat tumor angiogenesis, ocular diseases, rhuematoid arthritis, psoriasis and others. Applicant indicates that ribozymes are able to inhibit the activity of Raf and that the catalytic activity of the ribozymes is required for their inhibitory effect. Those of ordinary skill in the art will find that it is clear from the examples described that other ribozymes that cleave Raf mRNAs may be readily designed and are within the invention.

Other features and advantages of the invention will be apparent from the following description of the preferred embodiments thereof, and from the claims.

### Description Of The Preferred Embodiments

The drawings will first briefly be described.

### Drawings:

Figure 1 shows the secondary structure model for seven different classes of nucleic acid catalysts. Arrow indicates the site of cleavage. --------- indicate the target sequence. Lines interspersed with dots are meant to indicate tertiary interactions. - is meant to indicate base-paired interaction. **Group I Intron**: P1-P9.0 represent various stem-loop structures (Cech *et al*., 1994, *Nature Struc. Bio.,* 1, 273). **RNase P (M1RNA):** EGS represents external guide sequence (Forster *et al*., 1990, *Science,* 249, 783; Pace *et al*., 1990, *J. Biol. Chem.,* 265, 3587). **Group II Intron:** 5'SS means 5' splice site; 3'SS means 3'-splice site; IBS means intron binding site; EBS means exon binding site (Pyle *et al*., 1994, *Biochemistry,* 33, 2716). **VS RNA:** I-VI are meant to indicate six stem-loop structures; shaded regions are meant to indicate tertiary interaction (Collins, International PCT Publication No. WO 96/19577). **HDV Ribozyme: :** I-IV are meant to indicate four stem-loop structures (Been *et al*., US Patent No. 5,625,047). **Hammerhead Ribozyme: :** I-III are meant to indicate three stem-loop structures; stems I-III can be of any length and may be symmetrical or asymmetrical (Usman *et al*., 1996, *Curr. Op. Struct. Bio.,* 1, 527). **Hairpin Ribozyme:** Helix 1, 4 and 5 can be of any length; Helix 2 is between 3 and 8 base-pairs long; Y is a pyrimidine; Helix 2 (H2) is provided with a least 4 base pairs (*i.e*., n is 1, 2, 3 or 4) and helix 5 can be optionally provided of length 2 or more bases (preferably 3 - 20 bases, *i.e.,* m is from 1 - 20 or more). Helix 2 and helix 5 may be covalently linked by one or more bases (*i.e*., r is 1 base). Helix 1, 4 or 5 may also be extended by 2 or more base pairs (*e.g*., 4 - 20 base pairs) to stabilize the ribozyme structure, and preferably is a protein binding site. In each instance, each N and N' independently is any normal or modified base and each dash represents a potential base-pairing interaction. These nucleotides may be modified at the sugar, base or phosphate. Complete base-pairing is not required in the helices, but is preferred. Helix 1 and 4 can be of any size (*i.e*., o and p is each independently from 0 to any number, *e.g.,* 20) as long as some base-pairing is maintained. Essential bases are shown as specific bases in the structure, but those in the art will recognize that one or more may be modified chemically (abasic, base, sugar and/or phosphate modifications) or replaced with another base without significant effect. Helix 4 can be formed from two separate molecules, *i.e.,* without a connecting loop. The connecting loop when present may be a ribonucleotide with or without modifications to its base, sugar or phosphate. "q" is 2 bases. The connecting loop can also be replaced with a non-nucleotide linker molecule. H refers to bases A, U, or C. Y refers to pyrimidine bases. " ―" refers to a covalent bond. (Burke *et al.,* 1996, *Nucleic Acids & Mol. Biol.,* 10, 129; Chowrira *et al.,* US Patent No. 5,631,359).
Figure 2 shows a general approach to accessible site and target discovery using nucleic acid catalysts.
Figure 3 is a diagram of a hammerhead ribozyme. The consensus hammerhead cleavage site in a target RNA is a "U" followed by "H" (anything but "G"). The hammerhead ribozyme cleaves after the "H." This simple di-nucleotide sequence occurs, on average, every 5 nt in a target RNA. Thus, there are approximately 400 potential hammerhead cleavage sites in a 2-Kb mRNA. Stems I and III are formed by hybridization of the hammerhead binding arms with the complementary sequence in target RNA; it is these binding arms that confer specificity to the hammerhead ribozyme for its target. The binding arms of the hammerhead are interrupted by the catalytic domain that forms part of the structure responsible for cleavage.

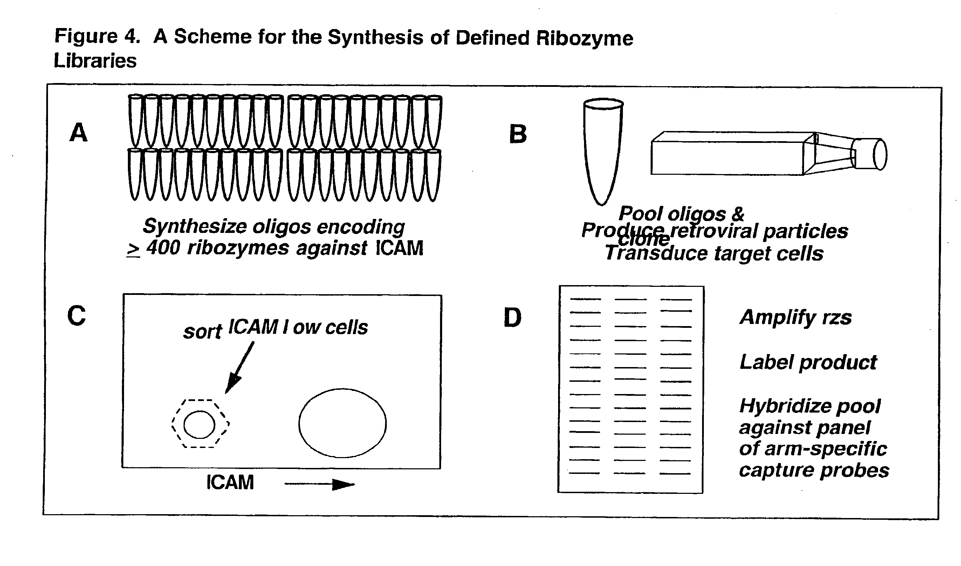

Figure 4 shows a scheme for the design and synthesis of a Defined Library: simultaneous screen of 400 different ICAM-targeted ribozymes is used as an example. DNA oligonucleotides encoding each ICAM-targeted ribozyme are synthesized individually (A), pooled (B), then cloned and converted to retroviral vectors as a pool. The resulting retroviral vector particles are used to transduce a target cell line that expresses ICAM (B). Cells expressing ribozymes that inhibit ICAM expression (ICAM-low) are sorted from cells expressing ineffective ribozymes by FACS sorting (C), effective ribozymes enriched in the ICAM-low population of cells are identified by filter hybridization (D).

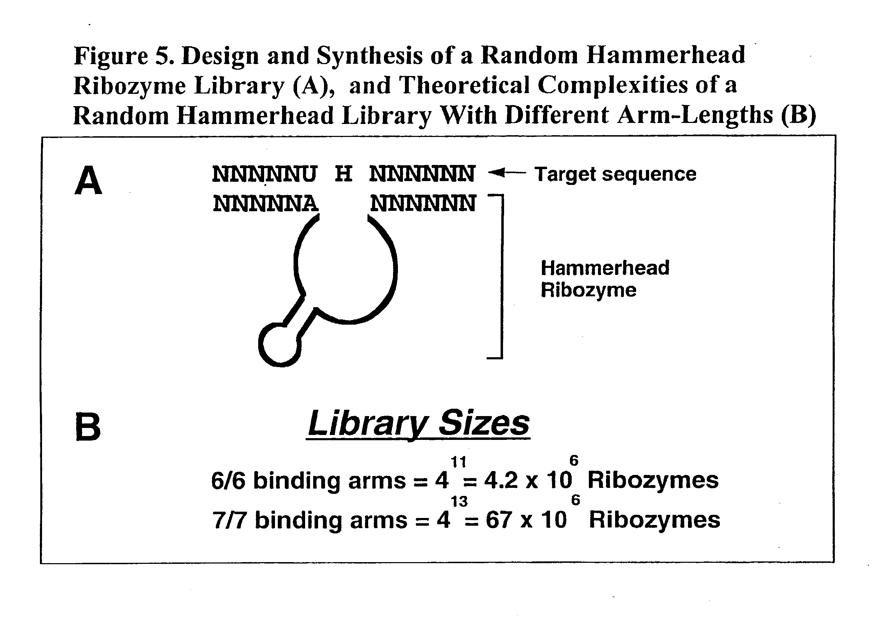

Figure 5 A) shows randomization of the binding arms of a hammerhead ribozyme to produce a Random Library. The binding arms can be of any length and any symmetry, *i.e.*, symmetrical or assymmetrical. B) shows complexities of hammerhead Random Ribozyme Libraries comprising a 6-nt or a 7-nt long binding arms.

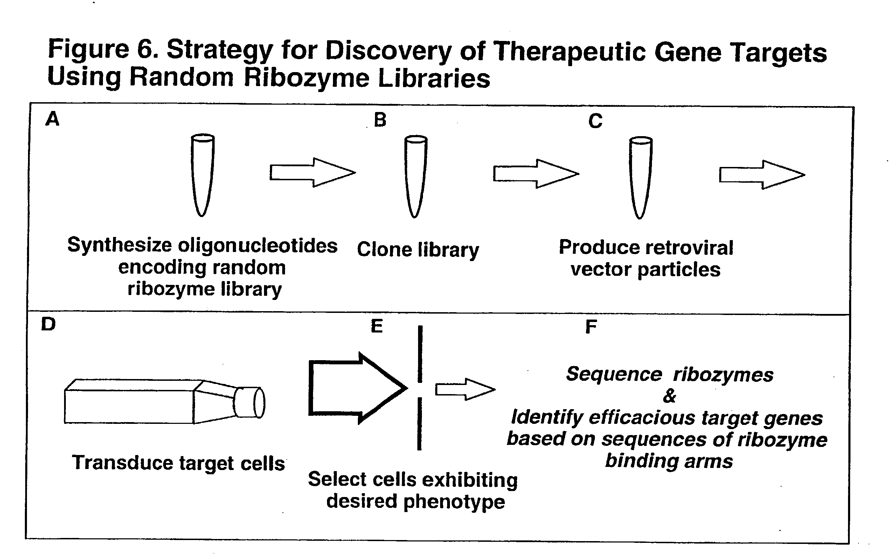

Figure 6 is a schematic overview of Target Discovery strategy. An oligonucleotide is prepared in a single reaction vessel in which all 4 standard nucleotides are incorporated in a random fashion in the target binding arm(s) of the ribozyme to produce a pool of all possible ribozymes (A). This pool is cloned into an appropriate vector in a single tube to produce the Random Library expression vector (B) and retroviral vector particles are produced from this pool in a single tube (C). The resulting Random Ribozyme Library retroviral expression vector pool is then used to transduce a cell type of interest (D). Cells exhibiting the desired phenotype are then separated from the rest of the population using a number of possible selection strategies (E and see text). Genes that are critical for expression of the selected phenotype can then be identified by sequencing the target binding arms of ribozymes contained in the selected population (F).

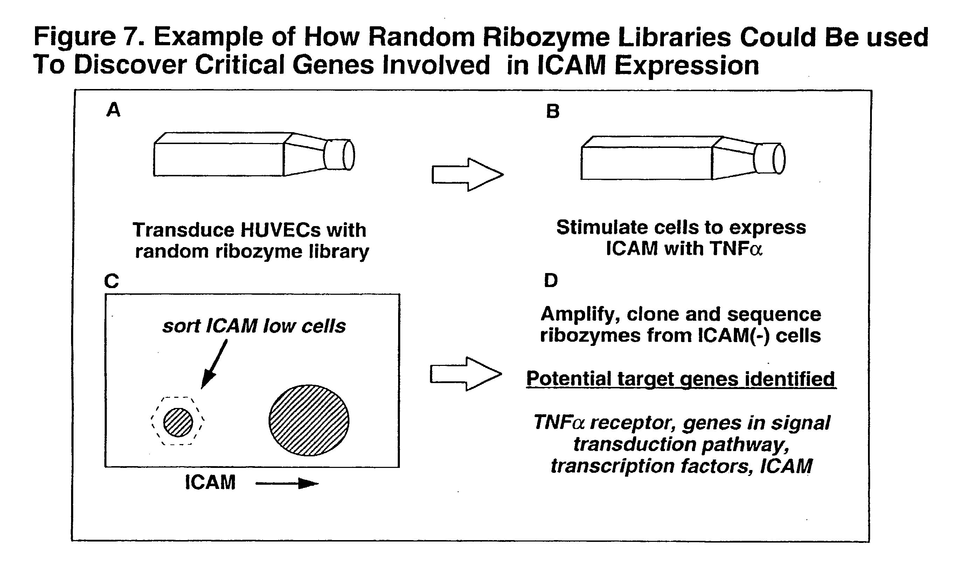

Figure 7 shows an example of application of Random Ribozyme Libraries to identify genes critical for the induction of ICAM expression. Human Umbilical Vein Endothelial Cells (HUVECs) are transduced with a Random Ribozyme Library (A), ICAM expression is induced using TNF-alpha (B), and cells expressing ribozymes that inhibit ICAM induction are selected from cells expressing ineffective ribozymes by sorting ICAM-low cells (C). Genes critical for ICAM induction are identified by sequencing the binding arms of the ribozymes that inhibit ICAM expression in the ICAM-low cells.
Figure 8 is an example of an efficient cloning strategy for producing a Defined or Random Ribozyme Libraries. DNA oligos encoding ribozyme coding regions and restriction sites for cloning are designed to also contain a stem-loop structure on the 3' ends (**1**). This stem loop forms an intramolecular primer site for extension to form a double-stranded molecule by DNA polymerase (**2**). The double-stranded fragment is cleaved with appropriate restriction endonucleases to produce suitable ends for subsequent cloning (**3**).

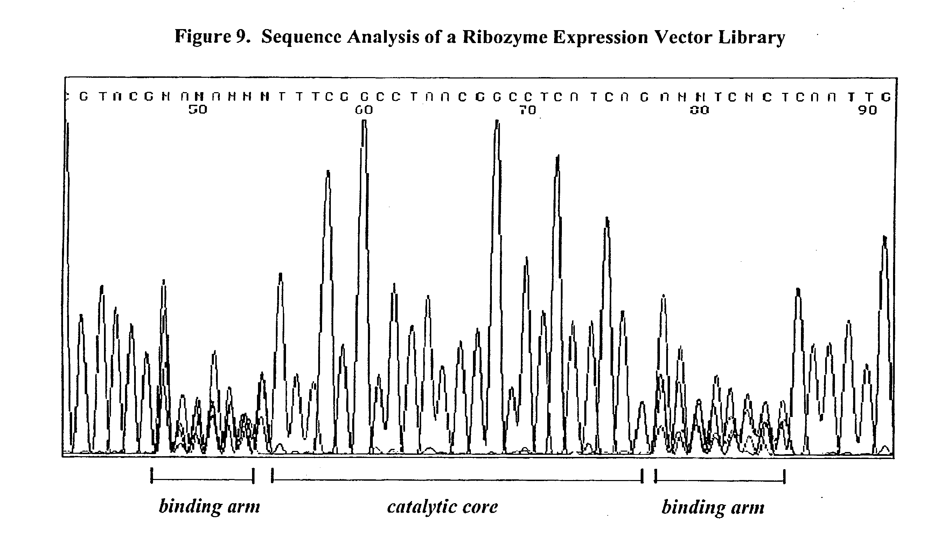

Figure 9 shows molecular analysis of the PNP-targeted Defined Ribozyme Library: sequence analysis. Plasmid DNA from the PNP-targeted Defined Ribozyme Library was prepared and sequenced as a pool. The sequencing primer used reads the non-coding strand of the region encoding the ribozymes. Note that the sequence diverges at the binding arm, converges at the catalytic domain (5' TTTCGGCCTAACGGCCTCATCAG-3'), and then diverges at the other binding arm. These results are consistent with those expected for a sequence of a heterogeneous pool of clones containing different sequences at the ribozyme binding arms.

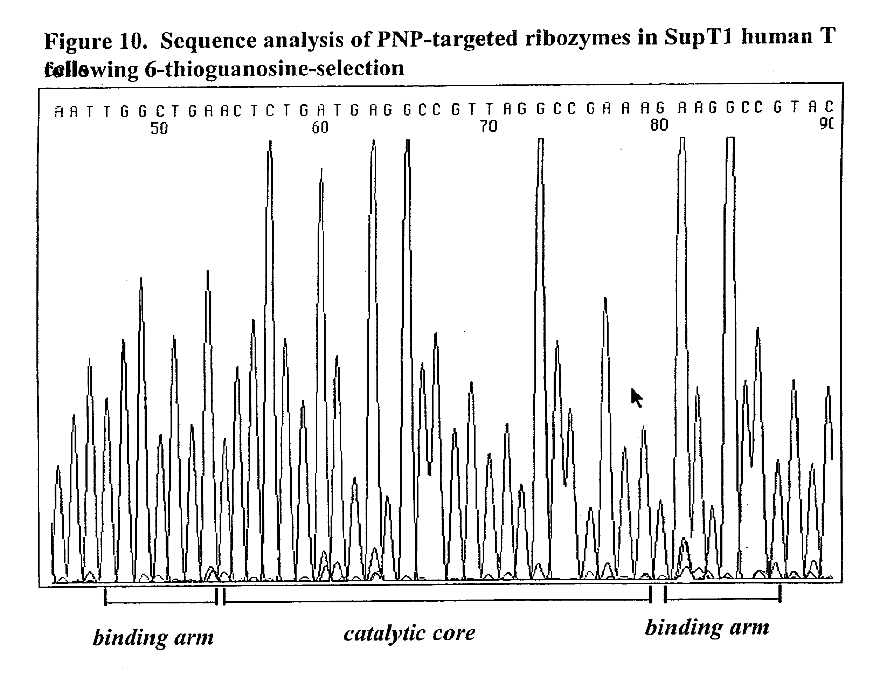

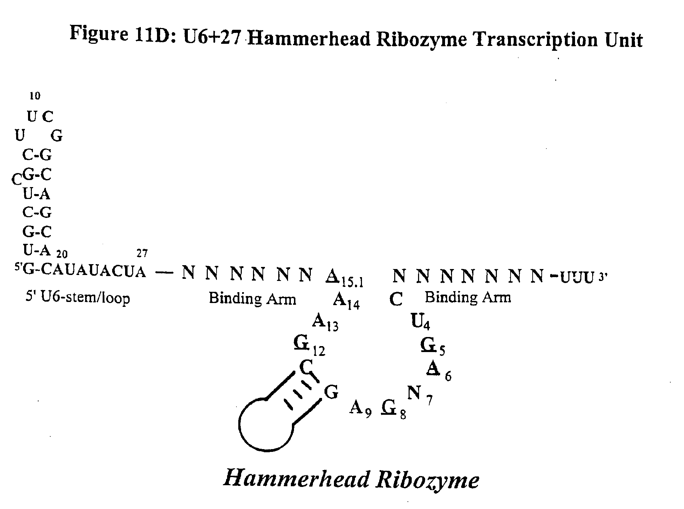

Figure 10 shows molecular analysis of the PNP-targeted Defined Ribozyme Library: sequence analysis after propagation in Sup T1 human T cells and selection in 10 mmol 6-thioguanosine. Sup T1 cells were transduced with retroviral vector-based Defined Ribozyme Library comprised of 40 different PNP-targeted ribozyme oligos cloned into the U6+27 transcription unit (Figure 11D). The cells were propagated for 2 weeks following transduction, then subjected to 16 days of selection in 10 mmol 6-thioguanosine. Surviving cells were harvested, and ribozyme sequences present in the selected population of cells were amplified and sequenced. Note that, relative to the original Library where sequences of the binding arms were ambiguous due to the presence of 40 different ribozymes (Figure 9), the sequence of the binding arms in the selected population corresponded to only 1 of the 40 ribozymes included in the Library. These results suggest that this ribozyme was the most-potent ribozyme of 40 ribozymes tested.

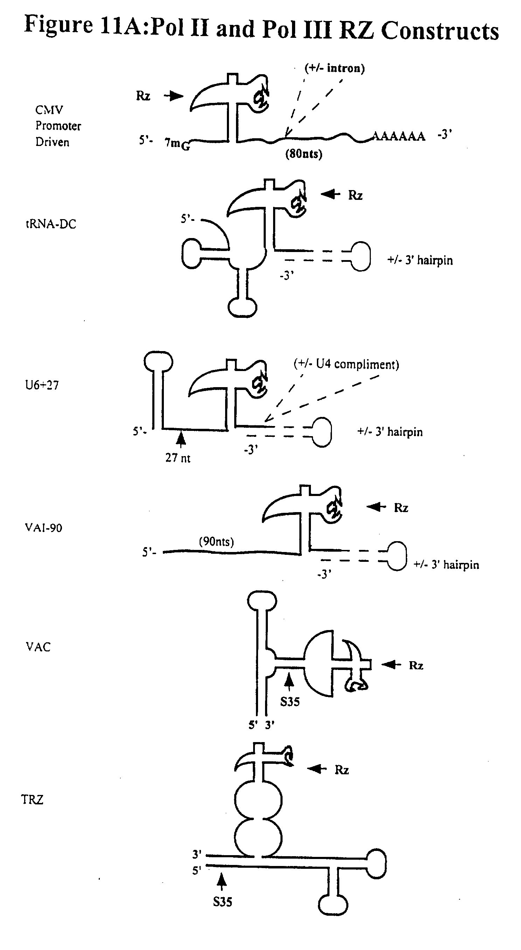

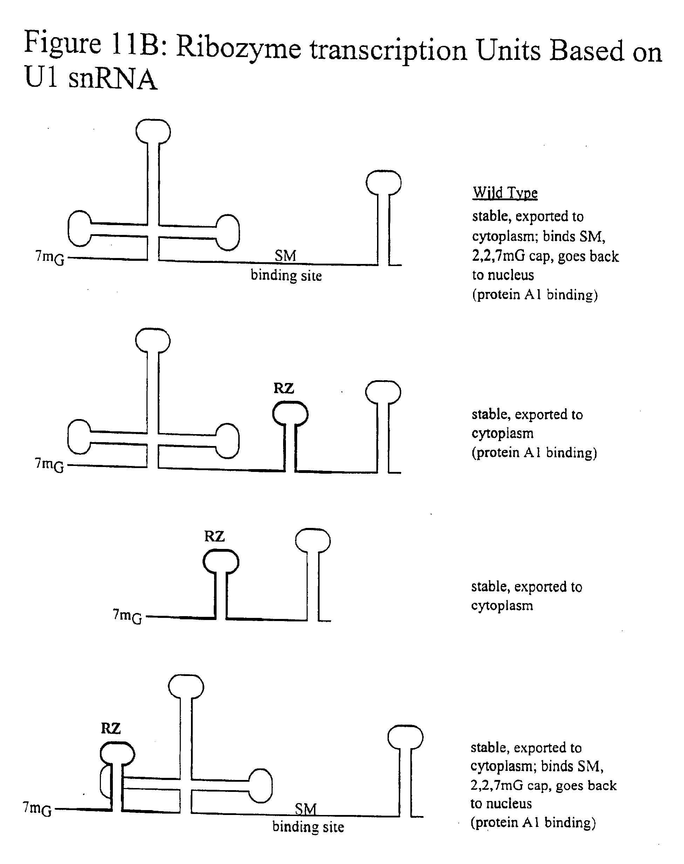

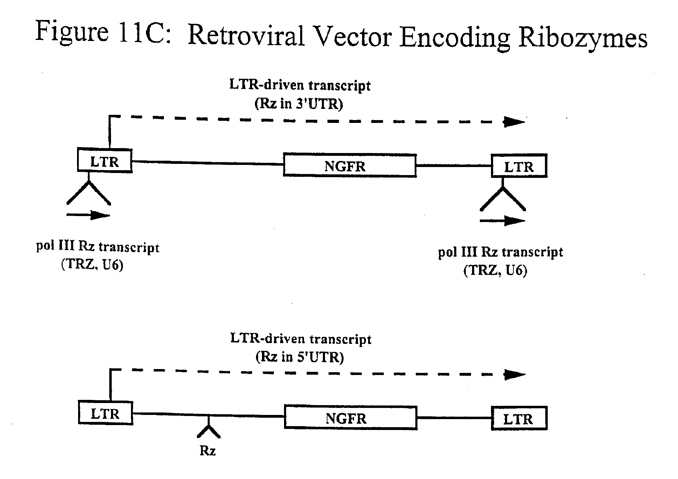

Figure 11 is a schematic representation of transcription units suitable for expression ribozyme library of the instant invention. **A**) is a diagrammatic representation of some RNA polymerase (Pol) II and III ribozyme (RZ) transcription units. CMV Promoter Driven is a Pol II transcript driven by a cytomegalovirus promoter; the transcript can designed such that the ribozyme is at the 5'- region, 3'-region or some where in between and the transcript optionally comprises an intron. tRNA-DC is a Pol III transcript driven by a transfer RNA (tRNA) promoter, wherein the ribozyme is at the 3'-end of the transcript; the transcript optionally comprises a stem-loop structure 3' of the ribozyme. U6+27 is a Pol III transcript driven by a U6 small nuclear (snRNA) promoter; ribozyme is 3' of a sequence that is homologous to 27 nucleotides at the 5'-end of a U6 snRNA; the transcript optionally comprise a stem-loop structure at the 3'-end of the ribozyme. VAI-90 is a Pol III transcript driven by an adenovirus VA promoter; ribozyme is 3' of a sequence homologous to 90 nucleotides at the 5'-end of a VAI RNA; the transcript optionally comprises a stem-loop structure at the 3'-end of the ribozyme. VAC is a Pol III transcript driven by an adenovirus VAI promoter; the ribozyme is inserted towards the 3'- region of the VA RNA and into a S35 motif, which is a stable greater than or equal to 8 bp long intramolecular stem formed by base-paired inteaction between sequences in the 5'-region and the 3'-region flanking the ribozyme (see Beigelman *et al.,* International PCT Application No. WO 96/18736); the S35 domain positions the ribozyme away from the main transcript as an independent domain. TRZ is a Pol III transcript diven by a tRNA promoter; ribozyme is inserted in the S35 domain and is positioned away from the main transcript (see Beigelman *et al.,* International PCT Application No. WO 96/18736). **B**) shows various transcription units based on the U1 small nuclear RNA (snRNA) system. **C**) is a schematic representation of a retroviral vectors encoding ribozyme genes. NGFR, nerve growth factor receptor is used as a selectable marker, LTR, long terminal repeat of a retrovirus, UTR, untranslated region. **D**) shows a U6+27 hammerhead ribozyme transcription unit based on the U6 snRNA. The ribozyme transcript comprises the first 27 nt from the U6 snRNA which is reported to be necessary for the stability of the transcript. The transcript terminates with a stretch of uridine residues. The hammerhead ribozyme shown in the figure has random (N) binding arm sequence.
Figure 12 is a schematic representation of a combinatorial approach to the screening of ribozyme variants.

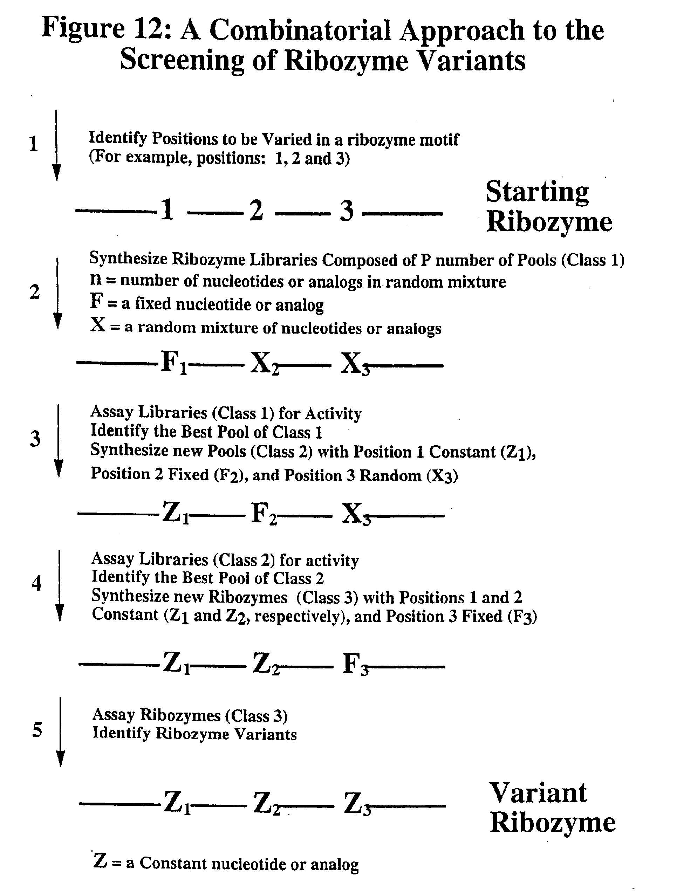

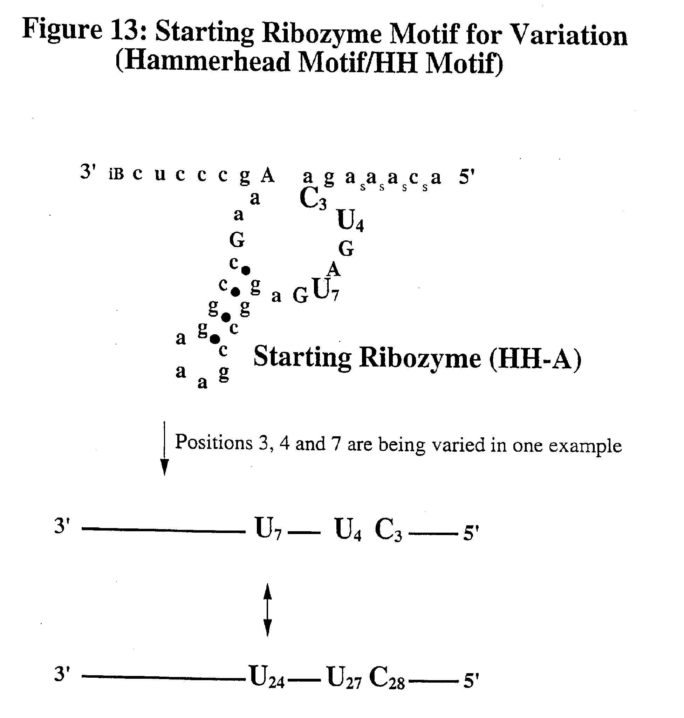

Figure 13 shows the sequence of a Starting Ribozyme to be used in the screening approach described in Figure 12. The Starting Ribozyme is a hammerhead (HH) ribozyme designed to cleave target RNA A (HH-A). Position 7 in HH-A is also referred to in this application as position 24 to indicate that U24 is the 24th nucleotide incorporated into the HH-A ribozyme during chemical synthesis. Similarly, positions 4 and 3 are also referred to as positions 27 and 28, respectively. s indicates phosphorothioate substitution. Lower case alphabets in the HH-A sequence indicate 2'-*O*-methyl nucleotides; uppercase alphabets in the sequence of HH-A at positions 5, 6, 8, 12 and 15.1 indicate ribonucleotides. Positions 3, 4 and 7 are shown as uppercase, large alphabets to indicate the positions selected for screening using the method shown in Figure 12. ● indicates base-paired interaction. iB represents abasic inverted deoxy ribose moiety.

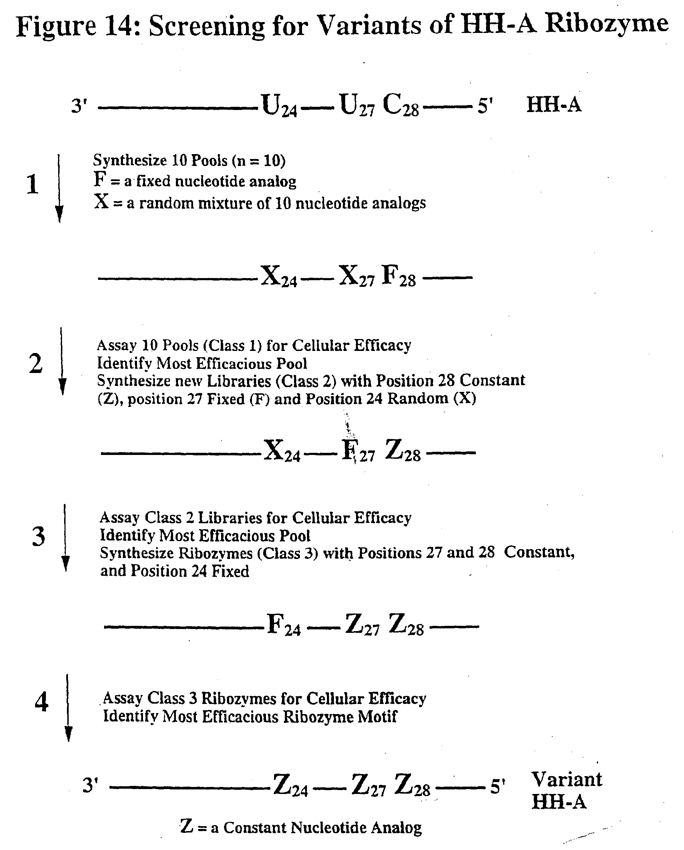

Figure 14 shows a scheme for screening variants of HH-A ribozyme. Positions 24, 27 and 28 are selected for analysis in this scheme.
Figure 15 shows non-limiting examples of some of the nucleotide analogs that can be used to construct ribozyme libraries. 2'-O-MTM-U represents 2'-O-methylthiomethyl uridine; 2'-O-MTM-C represents 2'-O-methylthiomethyl cytidine; 6-Me-U represents 6-methyl uridine (Beigelman *et al*., International PCT Publication No. WO 96/18736 which is incorporated by reference herein).
Figure 16 shows activity of HH-A variant ribozymes as determined in a cell-based assay. * indicates the substitution that provided the most desirable attribute in a ribozyme.

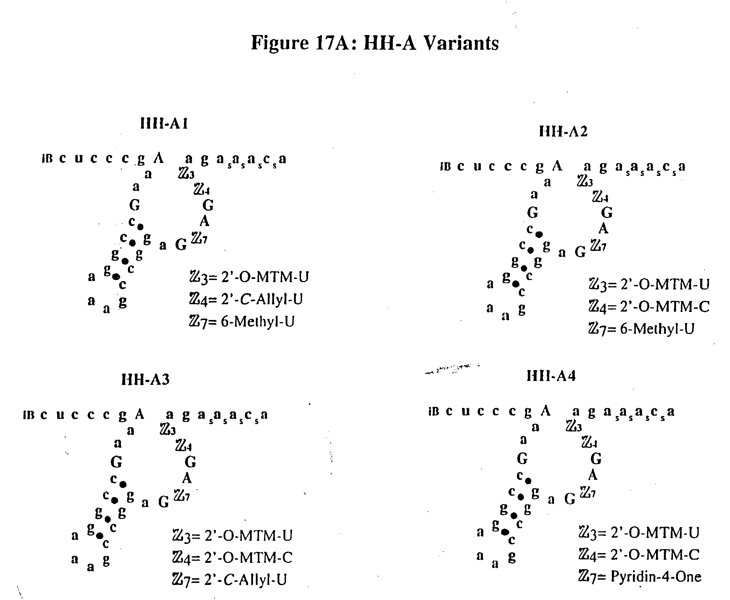

Figure 17A shows the sequence and chemical composition of ribozymes that showed the most desirable attribute in a cell.

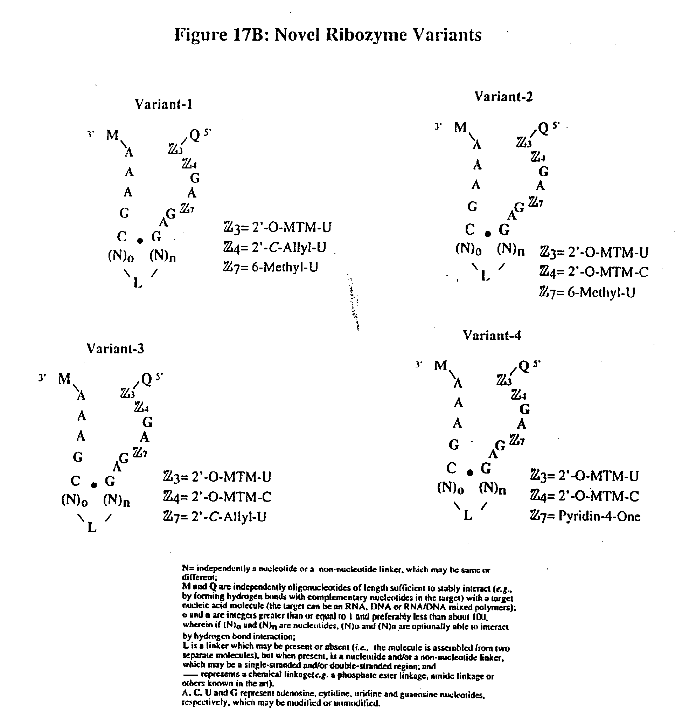

Figure 17B shows formulae for four different novel ribozyme motifs.
Figure 18 shows the formula foe a novel ribozyme motif.

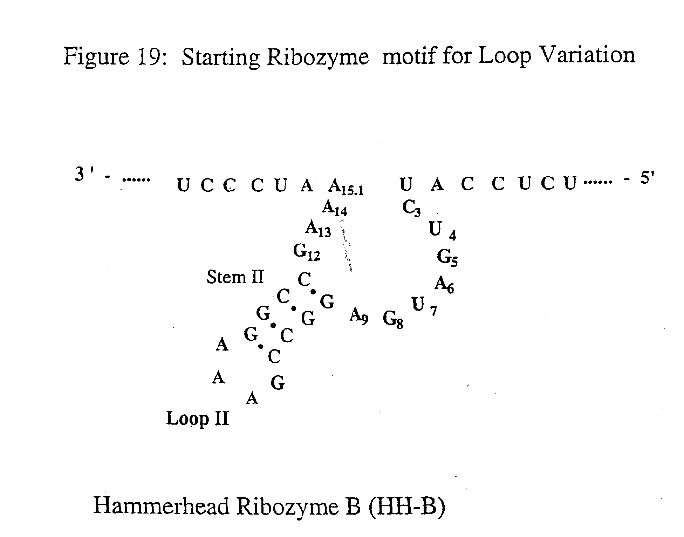

Figure 19 shows the sequence of a Starting Ribozyme to be used in the screening approach described in Figure 14. A HH ribozyme targeted against RNA B (HH-B) was chosen for analysis of the loop II sequence variants.
Figure 20 shows a scheme for screening loop-II sequence variants of HH-B ribozyme.
Figure 21 shows the relative catalytic rates (kᵣₑₗ) for RNA cleavage reactions catalyzed by HH-B loop-II variant ribozymes.
Figure 22 is a schematic representation of HH-B ribozyme-substrate complex and the activity of HH-B ribozyme with either the 5'-GAAA-3' or the 5'-GUUA-3' loop-II sequence.
Figure 23 shows a scheme for using a combinatorial approach to identify potential ribozyme targets by varying the binding arms.
Figure 24 shows a scheme for using a combinatorial approach to identify novel ribozymes by the varying putative catalytic domain sequence.
Figure 25 shows a table of accessible sites within a Bcl-2 transcript ((975 nucleotides) which were found using the combinatorial in vitro screening process.
Figure 26 shows a table of accessible sites with a Kras transcript (796 nucleotides) which were found using the combinatorial in vitro screening process as well as a graphic depiction of relative activity of ribozymes to those sites.
Figure 27 shows a table of accessible sites with a UPA transcript (400 nucleotides) which were found using the combinatorial in vitro screening process as well as a graphic depiction of relative activity of ribozymes to those sites.
Figure 28 shows a graph displaying data from a ribonuclease protection assay (RPA) after treatment of MCF-7 cells with ribozymes to targeted to site 549 of the transcript (Seq.ID #9). The Bcl-2 mRNA isolated from MCF-7 cells is normalized to GAPDH which was also probed in the RPA. The graph includes an untreated control and an irrelevant ribozyme (no complementarity with Bcl-2 mRNA).
Figure 29 displays a schematic representation of NTP synthesis using nucleoside substrates.
Figure 30 depicts a scheme for the synthesis of a xylo ribonucleoside phosphoramidite.

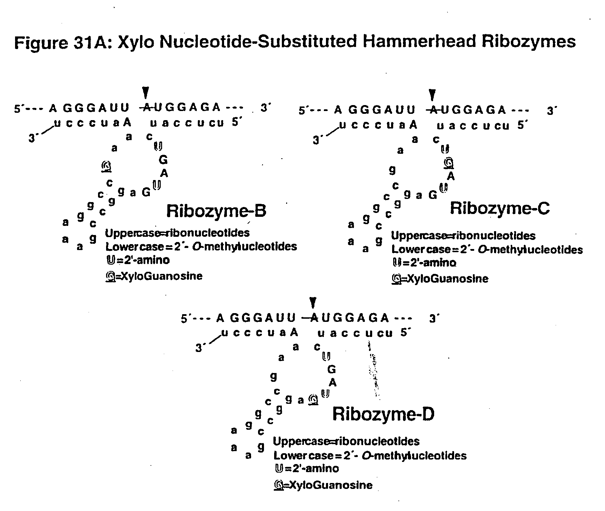

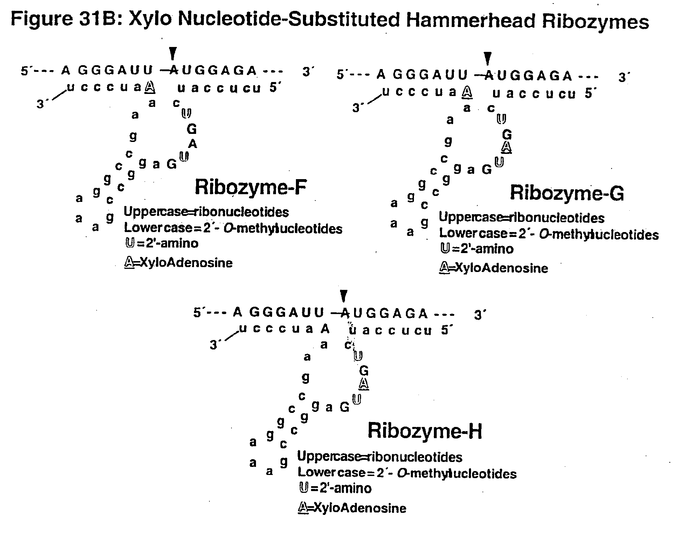

Figure 31 is a diagrammatic representation of hammerhead (HH) ribozyme targeted against stromelysin RNA (site 617) with various modifications.
Figure 32 is a is a schematic representation of a one pot deprotection of RNA synthesized using RNA phosphoramidite chemistry.
Figure 33 is a comparison of a one-pot and a two-pot process for deprotection of RNA.
Figure 34 shows the results of a one-pot deprotection with different polar organic reagents.
Figure 35 is a diagrammatic represention of ras signal transduction pathway.
Figure 36 is a diagrammatic representation of hammerhead ribozymes targeted against c-raf RNA.
Figure 37 is a graphical representation of c-raf 2'-*C*-allyl 1120 hammerhead (HH) ribozyme-mediated inhibition of cell proliferation.
Figure 38 is a graphical representation of inhibition of cell proliferation mediated by c-raf 2'-C-allyl 1120 and 1251 hammerhead (HH) ribozymes.
Figure 39 shows the effects of *flt-1* ribozymes (active/inactive) on LLC-HM primary tumor growth in mice.
Figure 40 shows the effects of *flt-1* ribozymes on LLC-HM primary tumor volume immediately following the cessation of treatment.
Figure 41 shows the effects of *flt-1* ribozymes on lung metastatic indices (number of metastases and lung mass).
Figure 42 shows the effects of *flk-1* ribozymes (active/inactive) on LLC-HM primary tumor growth in mice.
Figure 43 shows the effects of *flk-1* ribozymes on LLC-HM primary tumor volume immediately following the cessation of treatment.
Figure 44 shows the effects of *flk-1* ribozymes on lung metastatic indices (number of metastases and lung mass).

### Nucleic Acid Catalysts:

Catalytic nucleic acid molecules (ribozymes) are nucleic acid molecules capable of catalyzing one or more of a variety of reactions, including the ability to repeatedly cleave other separate nucleic acid molecules in a nucleotide base sequence-specific manner. Such nucleic acid catalysts can be used, for example, to target cleavage of virtually any RNA transcript (Zaug *et al.,* 324, *Nature* 429 1986 ; Cech, 260 *JAMA* 3030, 1988; and Jefferies *et al.,* 17 *Nucleic Acids Research* 1371, 1989). Catalytic nucleic acid molecules mean any nucleotide base-comprising molecule having the ability to repeatedly act on one or more types of molecules, including but not limited to nucleic acid catalysts. By way of example but not limitation, such molecules include those that are able to repeatedly cleave nucleic acid molecules, peptides, or other polymers, and those that are able to cause the polymerization of such nucleic acids and other polymers. Specifically, such molecules include ribozymes, DNAzymes, external guide sequences and the like. It is expected that such molecules will also include modified nucleotides compared to standard nucleotides found in DNA and RNA.

Because of their sequence-specificity, *trans*-cleaving nucleic acid catalysts show promise as therapeutic agents for human disease (Usman & McSwiggen, 1995 *Ann. Rep. Med. Chem.* **30,** 285-294; Christoffersen and Marr, 1995 *J. Med. Chem.* **38**, 2023-2037). Nucleic acid catalysts can be designed to cleave specific RNA targets within the background of cellular RNA. Such a cleavage event renders the RNA non-functional and abrogates protein expression from that RNA. In this manner, synthesis of a protein associated with a disease state can be selectively inhibited. In addition, nucleic acid catalysts can be used to validate a therapeutic gene target and/or to determine the function of a gene in a biological system (Christoffersen, 1997, *Nature Biotech.* 15, 483).

There are at least seven basic varieties of enzymatic RNA molecules derived from naturally occurring self-cleaving RNAs (see Table I). Each can catalyze the hydrolysis of RNA phosphodiester bonds in *trans* (and thus can cleave other RNA molecules) under physiological conditions. In general, enzymatic nucleic acids act by first binding to a substrate/target RNA. Such binding occurs through the substrate/target binding portion of an nucleic acid catalyst which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic and selective cleavage of such a target RNA will destroy its ability to direct synthesis of an encoded protein. After an enzymatic nucleic acid has bound and cleaved its RNA target, it is released from that RNA to search for another target and thus can repeatedly bind and cleave new targets.

In addition, several *in vitro* selection (evolution) strategies (Orgel, 1979, *Proc. R. Soc. London,* B 205, 435) have been used to evolve new nucleic acid catalysts capable of catalyzing a variety of reactions, such as cleavage and ligation of phosphodiester linkages and amide linkages, (Joyce, 1989, *Gene,* 82, 83-87; Beaudry *et al.,* 1992, *Science* 257, 635-641; Joyce, 1992, *Scientific American 267,* 90-97; Breaker *et al.,* 1994, *TIBTECH* 12, 268; Bartel *et al.,* 1993, *Science* 261:1411-1418; Szostak, 1993, *TIBS* 17, 89-93; Kumar *et al.,* 1995, *FASEB J., 9,* 1183; Breaker, 1996, *Curr. Op. Biotech.,* 7, 442; Breaker, 1997, *Nature Biotech.* 15, 427).

There are several reports that describe the use of a variety of *in vitro* and *in vivo* selection strategies to study structure and function of catalytic nucleic acid molecules (Campbell *et al.,* 1995, *RNA* 1, 598; Joyce 1989, *Gene,* 82,83; Lieber *et al.,* 1995, *Mol Cell Biol.* 15, 540; Lieber *et al.,* International PCT Publication No. WO 96/01314; Szostak 1988, in *Redesigning the Molecules of Life,* Ed. S. A. Benner, pp 87, Springer-Verlag, Germany; Kramer *et al.,* U.S. Patent No. 5,616,459; Draper *et al.,* US Patent No. 5,496,698; Joyce, U.S. Patent No. 5,595,873; Szostak *et al.,* U.S. Patent No. 5,631,146).

The enzymatic nature of a ribozyme is advantageous over other technologies, since the effective concentration of ribozyme sufficient to effect a therapeutic treatment is generally lower than that of an antisense oligonucleotide. This advantage reflects the ability of the ribozyme to act enzymatically. Thus, a single ribozyme (enzymatic nucleic acid) molecule is able to cleave many molecules of target RNA. In addition, the ribozyme is a highly specific inhibitor, with the specificity of inhibition depending not only on the base-pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base-pairing. Thus, it is thought that the specificity of action of a ribozyme is greater than that of antisense oligonucleotide binding the same RNA site.

The development of ribozymes that are optimal for catalytic activity would contribute significantly to any strategy that employs RNA-cleaving ribozymes for the purpose of regulating gene expression. The hammerhead ribozyme, for example, functions with a catalytic rate (*k*_{cat}) of ∼1 min⁻¹ in the presence of saturating (10 mM) concentrations of Mg²⁺ cofactor. However, the rate for this ribozyme in Mg²⁺ concentrations that are closer to those found inside cells (0.5 - 2 mM) can be 10- to 100-fold slower. In contrast, the RNase P holoenzyme can catalyze pre-tRNA cleavage with a *k*_{cat} of ∼30 min⁻¹ under optimal assay conditions. An artificial 'RNA ligase' ribozyme (Bartel *et al., supra)* has been shown to catalyze the corresponding self-modification reaction with a rate of ∼100 min⁻¹. In addition, it is known that certain modified hammerhead ribozymes that have substrate binding arms made of DNA catalyze RNA cleavage with multiple turn-over rates that approach 100 min⁻¹. Finally, replacement of a specific residue within the catalytic core of the hammerhead with certain nucleotide analogues gives modified ribozymes that show as much as a 10-fold improvement in catalytic rate. These findings demonstrate that ribozymes can promote chemical transformations with catalytic rates that are significantly greater than those displayed *in vitro* by most natural self-cleaving ribozymes. It is then possible that the structures of certain self-cleaving ribozymes may not be optimized to give maximal catalytic activity, or that entirely new RNA motifs could be made that display significantly faster rates for RNA phosphoester cleavage.

By "nucleotide" as used herein is as recognized in the art to include natural bases (standard), and modified bases well known in the art. Such bases are generally located at the 1' position of a sugar moiety. Nucleotide generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other ; see for example, Usman and McSwiggen, *supra;* Eckstein *et al*., International PCT Publication No. WO 92/07065; Usman *et al*., International PCT Publication No. WO 93/15187; all hereby incorporated by reference herein). There are several examples of modified nucleic acid bases known in the art and has recently been summarized by Limbach *et al*., 1994, *Nucleic Acids Res.* 22, 2183. Some of the non-limiting examples of base modifications that can be introduced into enzymatic nucleic acids without significantly effecting their catalytic activity include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (*e.g*., 5-methylcytidine), 5-alkyluridines *(e.g.,* ribothymidine), 5-halouridine (*e.g.,* 5-bromouridine) or 6-azapyrimidines or 6-alkylpyrimidines (*e.g.,* 6-methyluridine) and others (Burgin *et al*., 1996, *Biochemistry,* 35, 14090). By "modified bases" in this aspect is meant nucleotide bases other than adenine, guanine, cytosine and uracil at 1' position or their equivalents; such bases may be used within the catalytic core of the enzyme and/or in the substrate-binding regions.

In another preferred embodiment, catalytic activity of the molecules described in the instant invention can be optimized as described by Draper et al., *supra.* The details will not be repeated here, but include altering the length of the ribozyme binding arms, or chemically synthesizing ribozymes with modifications (base, sugar and/or phosphate) that prevent their degradation by serum ribonucleases and/or enhance their enzymatic activity (see *e.g.,* Eckstein *et al.,* International Publication No. WO 92/07065; Perrault *et al.,* 1990 *Nature* 344, 565; Pieken et al., 1991 *Science* 253, 314; Usman and Cedergren, 1992 *Trends in Biochem. Sci.* 17, 334; Usman *et al.,* International Publication No. WO 93/15187; and Rossi *et al.,* International Publication No. WO 91/03162; Sproat, US Patent No. 5,334,711; and Burgin *et al., supra;* all of these describe various chemical modifications that can be made to the base, phosphate and/or sugar moieties of enzymatic RNA molecules). Modifications which enhance their efficacy in cells, and removal of bases from stem loop structures to shorten RNA synthesis times and reduce chemical requirements are desired. (All of these publications are hereby incorporated by reference herein).

There are several examples in the art describing sugar and phosphate modifications that can be introduced into nucleic acid catalysts without significantly effecting catalysis and with significant enhancement in their nuclease stability and efficacy. Ribozymes are modified to enhance stability and/or enhance catalytic activity by modification with nuclease resistant groups, for example, 2'-amino, 2'-*C*-allyl, 2'-flouro, 2'-*O*-methyl, 2'-H, nucleotide base modifications (for a review see Usman and Cedergren, 1992 *TIBS* 17, 34; Usman *et al.,* 1994 *Nucleic Acids Symp. Ser.* 31, 163; Burgin *et al.,* 1996 *Biochemistry* 35, 14090). Sugar modification of nucleic acid catalysts has been extensively described in the art (see Eckstein *et al., International Publication* PCT No. WO 92/07065; Perrault *et al. Nature* 1990, 344, 565-568; Pieken *et al. Science* 1991, 253, 314-317; Usman and Cedergren, *Trends in Biochem. Sci.* 1992, *17,* 334-339; Usman *et al. International Publication* PCT No. WO 93/15187; Sproat, *US Patent* No. 5,334,711 and Beigelman *et al.,* 1995 *J. Biol. Chem.* 270, 25702; all of the references are hereby incorporated in their totality by reference herein).

Such publications describe general methods and strategies to determine the location of incorporation of sugar, base and/or phosphate modifications and the like into ribozymes without inhibiting catalysis, and are incorporated by reference herein. In view of such teachings, similar modifications can be used as described herein to modify the nucleic acid catalysts of the instant invention.

In yet another preferred embodiment, nucleic acid catalysts having chemical modifications which maintain or enhance enzymatic activity is provided. Such a nucleic acid is also, generally, more resistant to nucleases than the corresponding unmodified nucleic acid. Thus, in a cell and/or *in vivo* the activity may not be significantly lowered. As exemplified herein such ribozymes are useful in a cell and/or *in vivo* even if activity over all is reduced 10 fold (Burgin *et al*., 1996, *Biochemistry,* 35, 14090). Such modifications herein are said to "maintain" the enzymatic activity on all RNA ribozyme.

In a preferred embodiment, the nucleic acid catalysts of the invention are added directly, or can be complexed with cationic lipids, packaged within liposomes, or otherwise delivered to smooth muscle cells. The RNA or RNA complexes can be locally administered to relevant tissues through the use of a catheter, infusion pump or stent, with or without their incorporation in biopolymers. Using the methods described herein, other nucleic acid catalysts that cleave target nucleic acid may be derived and used as described above. Specific examples of nucleic acid catalysts of the instant invention are provided below in the Tables and figures.

Sullivan, et al., WO 94/02595, describes the general methods for delivery of nucleic acid catalysts. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Sullivan *et al.,* supra and Draper *et al., WO 93*/*23569* which have been incorporated by reference herein.

Such nucleic acid catalysts can be delivered exogenously to specific cells as required. In the preferred hammerhead motif the small size (less than 60 nucleotides, preferably between 30-40 nucleotides in length) of the molecule allows the cost of treatment to be reduced.

Therapeutic ribozymes delivered exogenously must remain stable within cells until translation of the target RNA has been inhibited long enough to reduce the levels of the undesirable protein. This period of time varies between hours to days depending upon the disease state. Clearly, ribozymes must be resistant to nucleases in order to function as effective intracellular therapeutic agents. Improvements in the chemical synthesis of RNA (Wincott *et al*., 1995 *Nucleic Acids Res.* 23, 2677; incorporated by reference herein) have expanded the ability to modify ribozymes by introducing nucleotide modifications to enhance their nuclease stability as described above.

### Synthesis, Deprotection, and Purification of Nucleic Acid Catalysts:

Generally, RNA molecules are chemically synthesized and purified by methodologies based on the use of tetrazole to activate the RNA phosphoramidite, ethanolic-NH₄OH to remove the exocyclic amino protecting groups, tetra-n-butylammonium fluoride (TBAF) to remove the *2'-OH* alkylsilyl protecting groups, and gel purification and analysis of the deprotected RNA. Examples of chemical synthesis, deprotection, purification and analysis procedures for RNA are provided by Usman et al., 1987 *J. Am. Chem. Soc*., 109, 7845; Scaringe *et al. Nucleic Acids Res.* 1990, *18,* 5433-5341; Perreault *et al. Biochemistry* 1991, *30* 4020-4025; Slim and Gait *Nucleic Acids Res.* 1991, *19,* 1183-1188. All the above noted references are all hereby incorporated by reference herein.

Synthesis of nucleic acids greater than 100 nucleotides in length is difficult using automated methods, and the therapeutic cost of such molecules is prohibitive. In this invention, small nucleic acid motifs (*e.g*., antisense oligonucleotides, hammerhead or the hairpin ribozymes) are used for exogenous delivery. The simple structure of these molecules increases the ability of the nucleic acid to invade targeted regions of the mRNA structure, However, these nucleic acid molecules can also be expressed within cells from eukaryotic promoters (*e.g.,* Izant and Weintraub, 1985 *Science* 229, 345; McGarry and Lindquist, 1986 *Proc. Natl. Acad. Sci.*USA 83, 399; SullengerScanlon *et al.,* 1991, *Proc. Natl. Acad. Sci.USA,* 88, 10591-5; Kashani-Sabet *et al.,* 1992 *Antisense Res. Dev.,* 2, 3-15; Dropulic *et al*., 1992 *J. Virol,* 66, 1432-41; Weerasinghe *et al.,* 1991 *J. Virol,* **65,** 5531-4; Ojwang *et al.,* 1992 *Proc. Natl. Acad. Sci.USA* 89, 10802-6; Chen *et al.,* 1992 *Nucleic Acids Res.,* 20, 4581-9; Sarver *et al.*, 1990 *Science* 247, 1222-1225; Thompson *et al*., 1995 *Nucleic Acids Res.* 23, 2259). Those skilled in the art realize that any nucleic acid can be expressed in eukaryotic cells from the appropriate DNA/RNA vector. The activity of such nucleic acids can be augmented by their release from the primary transcript by a ribozyme (Draper *et al.,* PCT WO93/23569, and Sullivan *et al.,* PCT WO94/02595, both hereby incorporated in their totality by reference herein; Ohkawa *et al.*, 1992 *Nucleic Acids Symp. Ser., 27, 15-6;* Taira *et al.,* 1991, *Nucleic Acids Res.,* 19, 5125-30; Ventura *et al.,* 1993 *Nucleic Acids Res.,* 21, 3249-55; Chowrira *et al*., 1994 *J. Biol. Chem.* 269, 25856).

The ribozymes were chemically synthesized. The method of synthesis used follows the procedure for normal RNA synthesis as described in Usman *et al*., 1987 *J. Am. Chem. Soc.,* 109, 7845; Scaringe *et al*., 1990 *Nucleic Acids Res.,* 18, 5433; and Wincott *et al*., 1995 *Nucleic Acids Res.* 23, 2677-2684 and makes use of common nucleic acid protecting and coupling groups, such as dimethoxytrityl at the 5'-end, and phosphoramidites at the 3'-end. Small scale synthesis were conducted on a 394 Applied Biosystems, Inc. synthesizer using a modified 2.5 µmol scale protocol with a 5 min coupling step for alkylsilyl protected nucleotides and 2.5 min coupling step for 2'-O-methylated nucleotides. Table **II** outlines the amounts, and the contact times, of the reagents used in the synthesis cycle. A 6.5-fold excess (163 µL of 0.1 M = 16.3 µmol) of phosphoramidite and a 24-fold excess of S-ethyl tetrazole (238 µL of 0.25 M = 59.5 µmol) relative to polymer-bound 5'-hydroxyl was used in each coupling cycle. Average coupling yields on the 394 Applied Biosystems, Inc. synthesizer, determined by colorimetric quantitation of the trityl fractions, were 97.5-99%. Other oligonucleotide synthesis reagents for the 394 Applied Biosystems, Inc. synthesizer:detritylation solution was 2% TCA in methylene chloride (ABI); capping was performed with 16% *N*-methyl imidazole in THF (ABI) and 10% acetic anhydride/10% 2,6-lutidine in THF (ABI); oxidation solution was 16.9 mM I₂, 49 mM pyridine, 9% water in THF (Millipore). B & J Synthesis Grade acetonitrile was used directly from the reagent bottle. *S*-Ethyl tetrazole solution (0.25 M in acetonitrile) was made up from the solid obtained from American International Chemical, Inc.

Deprotection of the RNA was performed as follows. The polymer-bound oligoribonucleotide, trityl-off, was transferred from the synthesis column to a 4mL glass screw top vial and suspended in a solution of methylamine (MA) at 65 °C for 10 min. After cooling to -20 °C, the supernatant was removed from the polymer support. The support was washed three times with 1.0 mL of EtOH:MeCN:H₂O/3:1:1, vortexed and the supernatant was then added to the first supernatant. The combined supernatants, containing the oligoribonucleotide, were dried to a white powder.

The base-deprotected oligoribonucleotide was resuspended in anhydrous TEA·HF/NMP solution (250 µL of a solution of 1.5mL *N*-methylpyrrolidinone, 750 µL TEA and 1.0 mL TEA•3HF to provide a 1.4M HF concentration) and heated to 65°C for 1.5 h. The resulting, fully deprotected, oligomer was quenched with 50 mM TEAB (9 mL) prior to anion exchange desalting.

For anion exchange desalting of the deprotected oligomer, the TEAB solution was loaded onto a Qiagen 500® anion exchange cartridge (Qiagen Inc.) that was prewashed with 50 mM TEAB (10 mL). After washing the loaded cartridge with 50 mM TEAB (10 mL), the RNA was eluted with 2 M TEAB (10 mL) and dried down to a white powder.

### Deprotection of RNA:

For high throughput chemical synthesis of oligoribonucleotides, it is important that the two main steps involved in the deprotection of oligoribonucleotides (i.e., aqueous basic treatment to remove exocyclic amino protecting groups and phosphate protecting groups and fluoride treatment to remove the 2'*-OH* alkylsilyl protecting groups such as the tButylDiMethylSilyl) are condensed.

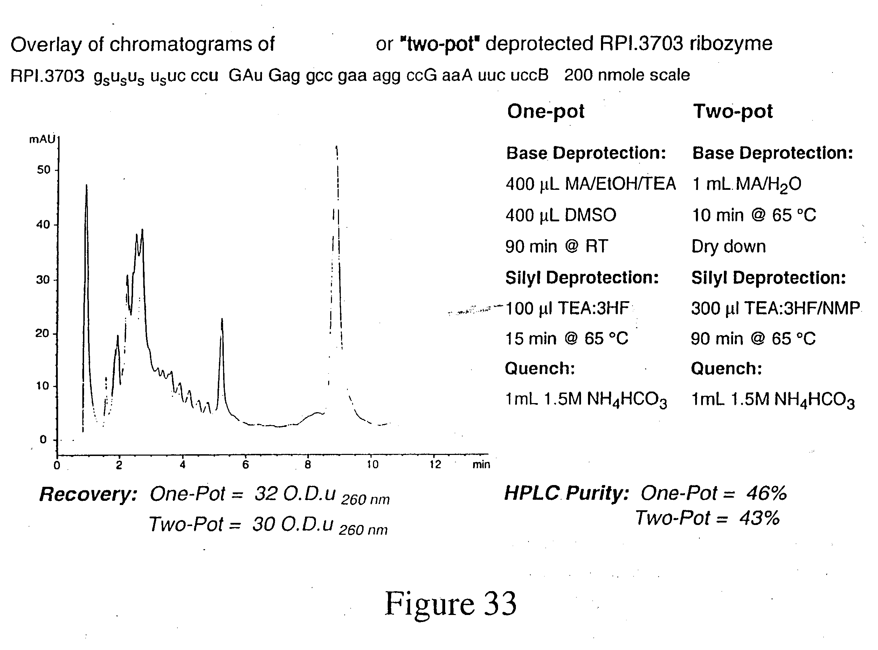

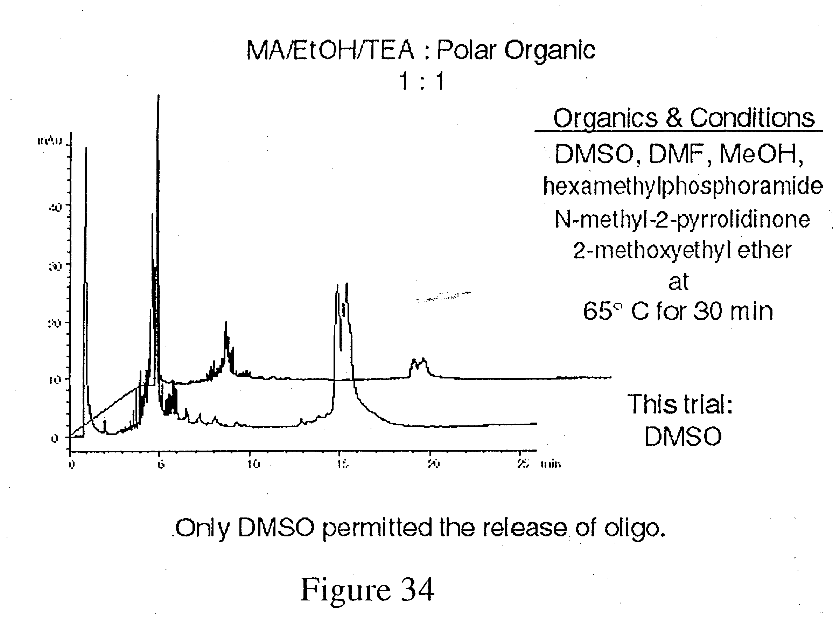

Stinchcomb *et al., supra* describe a time-efficient (∼ 2 hrs) one-pot deprotection protocol based on anhydrous methylamine and triethylamine trihydrogen fluoride. Since it has recently been reported that water contamination during fluoride treatment may be detrimental to the efficiency of the desilylation reaction (Hogrefe et al, Nucleic Acids Res. (1993), 21 4739-4741), it is necessary to use an anhydrous solution of base such as a 33% methylamine in absolute ethanol followed by neat triethylamine trihydrofluoride to effectively deprotect oligoribonucleotides in a one-pot fashion. However it may be cumbersome to apply such a protocol to plate format deprotection where the solid-support is preferentially separated from the partially deprotected oligoribonucleotides prior to the 2'-hydroxyl deprotection. Indeed, because the methylamine solution used is anhydrous, it may not be suitable to solubilize the negatively charged oligoribonucleotides obtained after basic treatment. Therefore, applicant investigated a 1:1 mixture of the ethanolic methylamine solution and different polar additives such as dimethylsulfoxide (DMSO), N,N-dimethylformamide (DMF), methanol, hexamethylphosphoramide (HMPA), 1-methyl-2-pyrrolidinone (NMP) or 2-methoxyethyl ether (glyme). Of all these additives, dimethylsufoxide is capable of efficiently solubilizing partially deprotected oligoribonucleotides (figure 34). A comparison of the one pot and two pot deprotection methods are outlined and demonstrated in figure 33.

The deprotection process commonly involves the deprotection of the exocyclic amino protecting groups by NH₄OH, which is time consuming (6-24 h) and inefficient. This step is then followed by treatment with TBAF to facilitate the removal of alkylsilyl protecting groups, which again is time consuming and not very effective in achieving efficient deprotection.

A recent modification of this two-step strategy for oligoribonucleotide deprotection has been reported by Wincott *et al., (Nucleic Acids Res.,* 1995, 23, 2677-2784) and by Vinayak *et al., (Nucleic Acids Symposium series,* 1995. 33, 123-125). The optimized conditions make use of aqueous methylamine at 65°C for 15 minutes in place of the ammonium hydroxide cocktail to remove exocyclic amino protecting groups while the desilylation treatment needed to remove the *2'-OH* alkylsilyl protecting groups utilizes a mixture of triethylamine trihydrogen fluoride (TEA.3HF), N-methylpyrrolidinone and triethylamine at 65°C for 90 minutes, thereby replacing tetrabutyl ammonium fluoride.

Stinchcomb *et al.,* International PCT Publication No. *WO 95*/*23225* describe a process for one pot deprotection of RNA. On page 73, it states that:
"In an attempt to minimize the time required for deprotection and to simplify the process of deprotection of RNA synthesized on a large scale, applicant describes a one pot deprotection protocol... According to this protocol, anhydrous methylamine is used in place of aqueous methyl amine. Base deprotection is carried out at 65 °C for 15 minutes and the reaction is allowed to cool for 10 min. Deprotection of 2'-hydroxyl groups is then carried out in the same container for 90 minutes in a TEA•3HF reagent. The reaction is quenched with 16 mM TEAB solution."

This invention concerns a one-pot process for the deprotection of RNA molecules. This invention features a novel method for the removal of protecting groups from the nucleic acid base and *2'-OH* groups, which accelerates the process for generating synthetic RNA in a high throughput manner (*e.g*., in a 96 well format).

Chemical synthesis of RNA is generally accomplished using a traditional column format on a RNA synthesizer where only one oligoribonucleotide is synthesized at a time. Simultaneous synthesis of more than one RNA molecule in a time efficient manner requires alternate methods to the traditional column format, such as synthesis in a 96 well plate format where up to 96 RNA molecules can be synthesized at the same time. To expedite this process of simultaneous synthesis of multiple RNA molecules, it is important to accelerate some of the time consuming processes such as the deprotection of RNA following synthesis (*i.e*., removal of base protecting group, such as the exocyclic amino protecting group and the phosphate protecting groups and the removal of 2'*-OH* protecting groups, such as the tButyIDiMethylSilyl). In a preferred embodiment, the invention features a one-pot process for rapid deprotection of RNA.

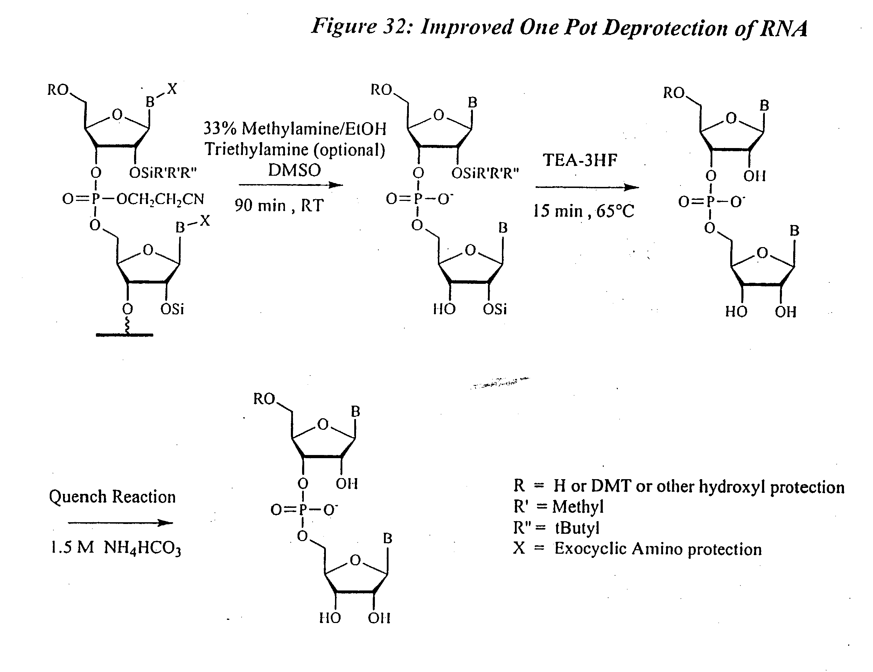

Stinchcomb *et al., supra* described a one-pot protocol for RNA deprotection using anhydrous methylamine and triethylamine trihydrogen fluoride. This procedure involves the use of an anhydrous solution of base such as a 33% methylamine in absolute ethanol followed by neat triethylamine trihydrofluoride to effectively deprotect oligoribonucleotides in a one-pot fashion. However such a protocol may be cumbersome for deprotection of RNA synthesized on a plate format, such as a 96 well plate, because it may be necessary to separate the solid-support from the partially deprotected RNA prior to the 2'-hydroxyl deprotection. Also, since the methylamine solution used is anhydrous, it may be difficult to solubilize the negatively charged oligoribonucleotides obtained after basic treatment. So, in a first aspect the invention features the use of a 1:1 mixture of the ethanolic methylamine solution and a polar additive, such as dimethylsulfoxide (DMSO), N,N-dimethylformamide (DMF), methanol, hexamethylphosphoramide (HMPA), 1-methyl-2-pyrrolidinone (NMP), 2-methoxyethyl ether (glyme) or the like. More specifically, dimethylsufoxide is used to partially deprotect oligoribonucleotides (Figure 32). A comparison of the one pot and two pot deprotection methods are outlined and demonstrated in Figure 33.

This invention also concerns a rapid (high through-put) deprotection of RNA in a 96-well plate format. More specifically rapid deprotection of enzymatic RNA molecules in greater than microgram quantities with high biological activity is featured. It has been determined that the recovery of enzymatically active RNA in high yield and quantity is dependent upon certain critical steps used during its deprotection.

In a preferred embodiment, the invention features a process for one-pot deprotection of RNA molecules comprising protecting groups, comprising the steps of: a) contacting the RNA with a mixture of anhydrous alkylamine (where alkyl can be branched or unbranched, ethyl, propyl or butyl and is preferably methyl, *e.g*., methylamine), trialkylamine (where alkyl can be branched or unbranched, methyl, propyl or butyl and is preferably ethyl, *e.g*., ethylamine) and dimethylsulfoxide, preferably in a 10:3:13, or 1:0.3:1 proportion at temperature 20-30 °C for about 30-100 minutes, preferably 90 minutes, to remove the exocyclic amino (base) protecting groups and the phosphate protecting group *(e.g.,* 2-cyanoethyl) (*vs* 4-20 h at 55-65 °C using NH₄OH/EtOH or NH₃/EtOH, or 10-15 min at 65°C using 40% aqueous methylamine) under conditions suitable for partial deprotection of the RNA; b) contacting the partially deprotected RNA with anhydrous triethylamine•hydrogen fluoride (3HF•TEA) and heating at about 50-70 °C, preferably at 65 °C, for about 5-30 min, preferably 15 min to remove the 2'-hydroxyl protecting group (*vs* 8 - 24 h using TBAF, or TEA•3HF for 24 h (Gasparutto *et al. Nucleic Acids Res.* **1992,** 20, 5159-5166) (Other alkylamine·HF complexes may also be used, e.g., trimethylamine or diisopropylethylamine) under conditions suitable for the complete deprotection of the RNA. The reaction can then be quenched by using aqueous ammonium bicarbonate (1.4 M). Although some other buffers can be used to quench the desilylation reaction (*i.e.,* triethylammonium bicarbonate, ammonium acetate), the ammonium bicarbonate buffer is perfectly suited to retain the 5'-*O*-dimethoxytrityl group at the 5'-end of the oligoribonucleotide thereby facilitating a reverse phase-based solid-phase extraction purification protocol.

By "one-pot" deprotection is meant that the process of deprotection RNA is carried out in one container instead of multiple containers as in two-pot deprotection.

In another preferred embodiment, the invention features a process for one pot deprotection of RNA molecules comprising protecting groups, comprising the steps of: a) contacting the RNA with a mixture of anhydrous alkylamine (where alkyl can be branched or unbranched, ethyl, propyl or butyl and is preferably methyl, *e.g.,* methylamine), and dimethylsulfoxide, preferably in a 1:1 proportion at 20-30 °C temperature for about 30-100 minutes, preferably 90 minutes, to remove the exocyclic amino (base) protecting groups and the phosphate protecting group *(e.g.,* 2-cyanoethyl) (ν*s* 4-20 h at 55-65 °C using NH₄OH/EtOH or NH₃/EtOH, or 10-15 min at 65°C using 40% aqueous methylamine) under conditions suitable for partial deprotection of the RNA; b) contacting the partially deprotected RNA with anhydrous triethylamine•hydrogen fluoride (3HF•TEA) and heating at about 50-70 °C, preferably at 65 °C, for about 5-30 min, preferably 15 min to remove the 2'-hydroxyl protecting group (Other alkylamine•HF complexes may also be used, *e.g*., trimethylamine or diisopropylethylamine) under conditions suitable for the complete deprotection of the RNA. The reaction can then be quenched by using aqueous ammonium bicarbonate (1.4 M). Although some other buffers can be used to quench the desilylation reaction (*i.e*., triethylammonium bicarbonate, ammonium acetate), the ammonium bicarbonate buffer is perfectly suited to retain the 5'-*O*-dimethoxytrityl group at the 5'-end of the oligoribonucleotide thereby facilitating a reverse phase-based solid-phase extraction purification protocol.

In another aspect the invention features a process for RNA deprotection where the exocyclic amino and phosphate deprotection reaction is performed with the ethanolic methylamine solution at room temperature for about 90 min or at 65°C for 15 min or at 45°C for 30 min or at 35°C for 60 min.

In a preferred embodiment, the process for deprotection of RNA of the present invention is used to deprotect a ribozyme synthesized using a column format as described in (Scaringe *et al*., supra; Wicott *et al., supra).*

Inactive hammerhead ribozymes were synthesized by substituting a U for G₅ and a U for A₁₄ (numbering from Hertel, K. J., *et al*., 1992, *Nucleic Acids Res*., 20, 3252).

The average stepwise coupling yields were >98% (Wincott *et al*., 1995 *Nucleic Acids Res.* 23, 2677-2684).

Hairpin ribozymes are synthesized in two parts and annealed to reconstruct the active ribozyme (Chowrira and Burke, 1992 *Nucleic Acids Res.,* 20, 2835-2840). Ribozymes are also synthesized from DNA templates using bacteriophage T7 RNA polymerase (Milligan and Uhlenbeck, 1989, *Methods Enzymol.* 180, 51).

Ribozymes are modified to enhance stability and/or enhance catalytic activity by modification with nuclease resistant groups, for example, 2'-amino, 2'-C-allyl, 2'-flouro, 2'-O-methyl, 2'-H, nucleotide base modifications (for a review see Usman and Cedergren, 1992 *TIBS* 17, 34; Usman *et al*., 1994 *Nucleic Acids Symp. Ser.* 31, 163; Burgin *et al*., 1996 *Biochemistry* 6, 14090).

Ribozymes were purified by gel electrophoresis using general methods or are purified by high pressure liquid chromatography (HPLC; See Stinchcomb *et al*., International PCT Publication No. WO 95/23225, the totality of which is hereby incorporated herein by reference) and are resuspended in water.

The sequences of the ribozymes that are chemically synthesized, useful in this study, are shown in Tables XII-XIX. Those in the art will recognize that these sequences are representative only of many more such sequences where the enzymatic portion of the ribozyme (all but the binding arms) is altered to affect activity. For example, stem-loop II sequence of hammerhead ribozymes can be altered (substitution, deletion, and/or insertion) to contain any sequences provided a minimum of two base-paired stem structure can form. Similarly, stem-loop IV sequence of hairpin ribozymes, can be altered (substitution, deletion, and/or insertion) to contain any sequence, provided a minimum of two base-paired stem structure can form. Preferably, no more than 200 bases are inserted at these locations. The sequences listed in Tables XII-XIX may be formed of ribonucleotides or other nucleotides or non-nucleotides. Such ribozymes (which have enzymatic activity) are equivalent to the ribozymes described specifically in the Tables.

### Nucleotide Triphosphates:

The use of modified nucleotide triphosphates would greatly assist in the combinatorial chemistry. The synthesis of nucleoside triphosphates and their incorporation into nucleic acids using polymerase enzymes has greatly assisted in the advancement of nucleic acid research. The polymerase enzyme utilizes nucleoside triphosphates as precursor molecules to assemble oligonucleotides. Each nucleotide is attached by a phosphodiester bond formed through nucleophilic attack by the 3' hydroxyl group of the oligonucleotide's last nucleotide onto the 5' triphosphate of the next nucleotide. Nucleotides are incorporated one at a time into the oligonucleotide in a 5' to 3' direction. This process allows RNA to be produced and amplified from virtually any DNA or RNA templates.

Most natural polymerase enzymes incorporate standard nucleoside triphosphates into nucleic acid. For example, a DNA polymerase incorporates dATP, dTTP, dCTP, and dGTP into DNA and an RNA polymerase generally incorporates ATP, CTP, UTP, and GTP into RNA. There are however, certain polymerases that are capable of incorporating non-standard nucleoside triphosphates into nucleic acids (Joyce, 1997, *PNAS* 94, 1619-1622, Huang et al., *Biochemistry* 36, 8231-8242).

Before nucleosides can be incorporated into RNA transcripts using polymerase enzymes they must first be converted into nucleoside triphosphates which can be recognized by these enzymes. Phosphorylation of unblocked nucleosides by treatment with POCl₃ and trialkyl phosphates was shown to yield nucleoside 5'-phosphorodichloridates (Yoshikawa *et al.,* 1969, *Bull. Chem. Soc. (Japan)* 42, 3505). Adenosine or 2'-deoxyadenosine 5'-triphosphate was synthesized by adding an additional step consisting of treatment with excess tri-n-butylammonium pyrophosphate in DMF followed by hydrolysis (Ludwig, 1981, *Acta Biochim. et Biophys. Acad. Sci. Hung.* 16, 131-133).

Non-standard nucleoside triphosphates are not readily incorporated into RNA transcripts by traditional RNA polymerases. Mutations have been introduced into RNA polymerase to facilitate incorporation of deoxyribonucleotides into RNA (Sousa & Padilla, 1995, *EMBO J.* 14,4609-4621 , Bonner *et al.,* 1992, *EMBO J.* 11, 3767-3775, Bonner et al., 1994, *J. Biol. Chem.* 42, 25120-25128, Aurup *et al.,* 1992, *Biochemistry* 31, 9636-9641).

McGee *et al.,* International PCT Publication No. WO 95/35102, describes the incorporation of 2'-NH2-NTP's, 2'-F-NTP's, and 2'-deoxy-2'-benzyloxyamino UTP into RNA using bacteriophage T7 polymerase.

Wieczorek *et al.,* 1994, *Bioorganic & Medicinal Chemistry Letters* 4, 987-994, describes the incorporation of 7-deaza-adenosine triphosphate into an RNA transcript using bacteriophage T7 RNA polymerase.

Lin *et al.,* 1994, *Nucleic Acids Research* 22, 5229-5234, reports the incorporation of 2'-NH₂-CTP and 2'-NH₂-UTP into RNA using bacteriophage T7 RNA polymerase and polyethylene glycol containing buffer. The article describes the use of the polymerase synthesized RNA for *in vitro* selection of aptamers to human neutrophil elastase (HNE).

The invention features NTP's having the formula triphosphate-OR, for example the following formula I: 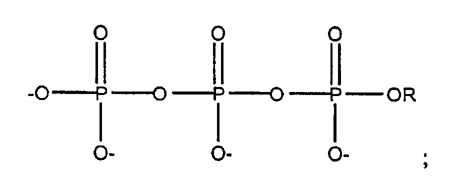 where R is any nucleoside; specifically the nucleosides 2'-*O*-methyl-2,6-diaminopurine riboside; 2'-deoxy-2'amino-2,6-diaminopurine riboside; 2'-(*N*-alanyl) amino-2'-deoxy-uridine; 2'-(*N*-phenylalanyl)amino-2'-deoxy-uridine; 2'-deoxy -2'-(*N*-β-alanyl) amino ; 2'-deoxy-2'-(lysiyl) amino uridine; 2'-*C*-allyl uridine; 2'-*O*-amino-uridine; 2'-*O*-methylthiomethyl adenosine; 2'-*O*-methylthiomethyl cytidine ; 2'-*O*-methylthiomethyl guanosine; 2'-*O*-methylthiomethyl-uridine; 2'-Deoxy-2'-(*N*-histidyl) amino uridine; 2'-deoxy-2'-amino-5-methyl cytidine; 2'-(*N*-β-carboxamidine-β-alanyl)amino-2'-deoxy-uridine; 2'-deoxy-2'-(N-β-alanyl)-guanosine; and 2'-*O*-amino-adenosine.

In a second aspect, the invention features a process for the synthesis of pyrimidine nucleotide triphosphate (such as UTP, 2'-O-MTM-UTP, dUTP and the like) including the steps of monophosphorylation where the pyrimidine nucleoside is contacted with a mixture having a phosphorylating agent (such as phosphorus oxychloride, phospho-tris-triazolides, phospho-tris-triimidazolides and the like), trialkyl phosphate (such as triethylphosphate or trimethylphosphate or the like) and dimethylaminopyridine (DMAP) under conditions suitable for the formation of pyrimidine monophosphate; and pyrophosphorylation where the pyrimidine monophosphate is contacted with a pyrophosphorylating reagent (such as tributylammonium pyrophosphate) under conditions suitable for the formation of pyrimidine triphosphates.

By "pyrimidines" is meant nucleotides comprising modified or unmodified derivatives of a six membered pyrimidine ring. An example of a pyrimidine is modified or unmodified uridine.

By "nucleotide triphosphate" or "NTP" is meant a nucleoside bound to three inorganic phosphate groups at the 5' hydroxyl group of the modified or unmodified ribose or deoxyribose sugar where the 1' position of the sugar may comprise a nucleic acid base or hydrogen. The triphosphate portion may be modified to include chemical moieties which do not destroy the functionality of the group (*i.e*., allow incorporation into an RNA molecule).

In another preferred embodiment, nucleoside triphosphates (NTP's) of the instant invention are incorporated into an oligonucleotide using an RNA polymerase enzyme. RNA polymerases include but are not limited to mutated and wild type versions of bacteriophage T7, SP6, or T3 RNA polymerases.

In yet another preferred embodiment, the invention features a process for incorporating modified NTP's into an oligonucleotide including the step of incubating a mixture having a DNA template, RNA polymerase, NTP, and an enhancer of modified NTP incorporation under conditions suitable for the incorporation of the modified NTP into the oligonucleotide.

By "enhancer of modified NTP incorporation" is meant a reagent which facilitates the incorporation of modified nucleotides into a nucleic acid transcript by an RNA polymerase. Such reagents include but are not limited to methanol; LiCl; polyethylene glycol (PEG); diethyl ether; propanol; methyl amine; ethanol and the like.

In another preferred embodiment, the modified nucleoside triphosphates can be incorporated by transcription into a nucleic acid molecules including enzymatic nucleic acid, antisense, 2-5A antisense chimera, oligonucleotides, triplex forming oligonucleotide (TFO), aptamers and the like (Stull *et al.,* 1995 *Pharmaceutical Res.* 12, 465).

By "antisense" it is meant a non-nucleic acid catalyst that binds to target RNA by means of RNA-RNA or RNA-DNA or RNA-PNA (protein nucleic acid; Egholm *et al.,* 1993 *Nature* 365, 566) interactions and alters the activity of the target RNA (for a review see Stein and Cheng, 1993 *Science* 261, 1004; Agrawal *et al.,* U.S. Patent No. 5,591,721; Agrawal, U.S. Patent No. 5,652,356).

By "2-5A antisense chimera" it is meant, an antisense oligonucleotide containing a 5' phosphorylated 2'-5'-linked adenylate residues. These chimeras bind to target RNA in a sequence-specific manner and activate a cellular 2-5A-dependent ribonuclease which, in turn, cleaves the target RNA (Torrence *et al.,* 1993 *Proc. Natl. Acad. Sci. USA* 90, 1300).

By "triplex forming oligonucleotides (TFO)" it is meant an oligonucleotide that can bind to a double-stranded DNA in a sequence-specific manner to form a triple-strand helix. Formation of such triple helix structure has been shown to inhibit transcription of the targeted gene (Duval-Valentin *et al.,* 1992 *Proc. Natl. Acad. Sci. USA* 89, *504).*

By "oligonucleotide" as used herein is meant a molecule having two or more nucleotides. The polynucleotide can be single, double or multiple stranded and may have modified or unmodified nucleotides or non-nucleotides or various mixtures and combinations thereof.

In yet another preferred embodiment, the modified nucleoside triphosphates of the instant invention can be used for combinatorial chemistry or *in vitro* selection of nucleic acid molecules with novel function. Modified oligonucleotides can be enzymatically synthesized to generate libraries for screening.

Nucleoside modifications of bases and sugars, have been discovered in a variety of naturally occurring RNA (*e.g.,* tRNA, mRNA, rRNA; reviewed by Hall, 1971 *The Modified Nucleosides in Nucleic Acids,* Columbia University Press, New York; Limbach *et al.,* 1994 *Nucleic Acids Res.* 22, 2183). In an attempt to understand the biological significance, structural and thermodynamic properties, and nuclease resistance of these nucleoside modifications in nucleic acids, several investigators have chemically synthesized nucleosides, nucleotides and phosphoramidites with various base and sugar modifications and incorporated them into oligonucleotides.

Uhlmann and Peyman, 1990, *Chem. Reviews* 90, 543, review the use of certain nucleoside modifications to stabilize antisense oligonucleotides.

Usman *et al.*, International PCT Publication Nos. WO/93/15187; and *WO 95*/*13378;* describe the use of sugar, base and backbone modifications to enhance the nuclease stability of nucleic acid catalysts.

Eckstein *et al*., International PCT Publication No. WO 92/07065 describe the use of sugar, base and backbone modifications to enhance the nuclease stability of nucleic acid catalysts.

Grasby *et al*., 1994, *Proc. Indian Acad. Sci.,* 106, 1003, review the "applications of synthetic oligoribonucleotide analogues in studies of RNA structure and function".

Eaton and Pieken, 1995, *Annu. Rev. Biochem.,* 64, 837, review sugar, base and backbone modifications that enhance the nuclease stability of RNA molecules.

Rosemeyer *et al*., 1991, *Helvetica Chem. Acta*, 74, 748, describe the synthesis of 1-(2'-deoxy-β-D-xylofuranosyl) thymine-containing oligodeoxynucleotides.

Seela *et al*., 1994, *Helvetica Chem. Acta*, 77, 883, describe the synthesis of 1-(2'-deoxy-β-D-xylofuranosyl) cytosine-containing oligodeoxynucleotides.

Seela *et al*., 1996, *Helvetica Chem. Acta*, 79, 1451, describe the synthesis xylose-DNA containing the four natural bases.

In another preferred embodiment, catalytic activity of the molecules described in the instant invention can be optimized as described by Draper et al., *supra.* The details will not be repeated here, but include altering the length of the ribozyme binding arms, or chemically synthesizing ribozymes with modifications (base, sugar and/or phosphate) that prevent their degradation by serum ribonucleases and/or enhance their enzymatic activity (see *e.g.,* Eckstein *et al.,* International Publication No. WO 92/07065; Perrault *et al.,* 1990 *Nature* 344, 565; Pieken et al., 1991 *Science* 253, 314; Usman and Cedergren, 1992 *Trends in Biochem. Sci.* 17, 334; Usman *et al.,* International Publication No. WO 93/15187; and Rossi *et al.*, International Publication No. WO 91/03162; Sproat, US Patent No. 5,334,711; and Burgin *et al., supra;* all of these describe various chemical modifications that can be made to the base, phosphate and/or sugar moieties of enzymatic RNA molecules). Modifications which enhance their efficacy in cells, and removal of bases from stem loop structures to shorten RNA synthesis times and reduce chemical requirements are desired. (All these publications are hereby incorporated by reference herein.).

By "enhanced enzymatic activity" is meant to include activity measured in cells and/or *in vivo* where the activity is a reflection of both catalytic activity and ribozyme stability. In this invention, the product of these properties is increased or not significantly (less that 10 fold) decreased *in vivo* compared to an all RNA ribozyme.

In yet another preferred embodiment, nucleic acid catalysts having chemical modifications which maintain or enhance enzymatic activity is provided. Such nucleic acid is also generally more resistant to nucleases than unmodified nucleic acid. Thus, in a cell and/or *in vivo* the activity may not be significantly lowered. As exemplified herein such ribozymes are useful in a cell and/or *in vivo* even if activity over all is reduced 10 fold (Burgin *et al.,* 1996, *Biochemistry,* 35, 14090). Such ribozymes herein are said to "maintain" the enzymatic activity on all RNA ribozyme.

In a most preferred embodiment the invention features a method of synthesizing ribozyme libraries of various sizes. This invention describes methods to chemically synthesize ribozyme libraries of various sizes from suitable nucleoside analogs.

*Considerations for the selection of nucleotide building blocks and determination of coupling efficiency:* In addition to structural considerations (hydrogen bond donors and acceptors, stacking properties, pucker orientation of sugars, hydrophobicity or hydrophilicity of some subgroups constitutive of the nucleotides) that may lead to the selection of a specific nucleotide to be included in the design of a ribozyme library, one of the important features that needs to be considered when selecting nucleotide building blocks is the chemical compatibility of such building blocks with ribozyme synthesis. A "nucleotide building block" is a nucleoside or nucleoside analog that possess a suitably protected phosphorus atom at the oxidation state V reacting readily, upon activation, to give a P^{V}-containing internucleoside linkage. A suitable nucleoside building block may also contain a phosphorus atom at the oxidation state III reacting readily, upon activation, to give a P^{III}-containing internucleoside linkage that can be oxidized to the desired P^{V}-containing internucleoside linkage. Applicant has found that the phosphoramidite chemistry (P^{III}) is a preferred coupling method for ribozyme library synthesis. There are several other considerations while designing and synthesizing certain ribozyme libraries, such as: a) the coupling efficiencies of the nucleotide building blocks considered for a ribozyme library should not fall below 90% to provide a majority of full-length ribozyme; b) the nucleotide building blocks should be chemically stable to the selected synthesis and deprotection conditions of the particular ribozyme library; c) the deprotection schemes for the nucleotide building blocks incorporated into a ribozyme library, should be relatively similar and be fully compatible with ribozyme deprotection protocols. In particular, nucleoside building blocks requiring extended deprotection or that cannot sustain harsh treatment should be avoided in the synthesis of a ribozyme library. Typically, the reactivity of the nucleotide building blocks should be optimum when diluted to 100 mM to 200 mM in non-protic and relatively polar solvent. Also the deprotection condition using 3:1 mixture of ethanol and concentrated aqueous ammonia at 65 degrees C. for 4 hours followed by a fluoride treatment as exemplified in Wincott *et al. supra,* is particularly useful for ribozyme synthesis and is a preferred deprotection pathway for such nucleotide building blocks.

In one preferred embodiment, a "nucleotide building block mixing" approach to generate ribozyme libraries is described. This method involves mixing various nucleotide building blocks together in proportions necessary to ensure equal representation of each of the nucleotide building blocks in the mixture. This mixture is incorporated into the ribozyme at position(s) selected for randomization.

The nucleotide building blocks selected for incorporation into a ribozyme library, are typically mixed together in appropriate concentrations, in reagents, such as anhydrous acetonitrile, to form a mixture with a desired phosphoramidite concentration. This approach for combinatorial synthesis of a ribozyme library with one or more random positions within the ribozyme (X as described above) is particularly useful since a standard DNA synthesizer can handle a building block mixture similar to a building block solution containing a single building block. Such a nucleotide building block mixture is coupled to a solid support or to a growing ribozyme sequence attached to a solid-support. To ensure that the ribozyme library synthesized achieves the desired complexity, the scale of the synthesis is increased substantially above that of the total complexity of the library. For example, a 2.5 µmole ribozyme synthesis provides ∼ 3x10¹⁷ ribozyme molecules corresponding to sub-nanomolar amounts of each member of a billion compounds ribozyme library.

Divinylbenzene highly cross-linked polystyrene solid-support constitutes the preferred stationary phase for ribozyme library synthesis. However, other solid-support systems utilized in DNA or RNA synthesis can also be used for ribozyme library synthesis. This includes silica-based solid-supports such as controlled-pore glass (CPG) or polymeric solid-supports such as all types of derivatized polystyrene resins, grafted polymers of chloromethylated polystyrene crosslinked with ethylene glycol, oligoethylene glycol.

Because of different coupling kinetics of the nucleotide building blocks present in a mixture, it is necessary to evaluate the relative incorporation of each of the members of the mixture and to adjust, if needed, the relative concentration of the building blocks in the mixture to get equimolar representation, compensating thereby the kinetic parameter. Typically a building block that presents a slow coupling kinetic will be over-represented in the mixture and vice versa for a building block that presents a fast coupling kinetic. When equimolar incorporation is sought, acceptable limits for unequal incorporation may generally be +/-10%.

Synthesis of a random ribozyme library can be performed either with the mixture of desired nucleotide building blocks, or with a combination of certain random positions (obtained by using one or more building block mixtures) and one or more fixed positions that can be introduced through the incorporation of a single nucleotide building block reagent. For instance, in the oligonucleotide model 5'-TT XXXX TT**B**-3' used in example 2 *infra*, the positions from 3'-end 1 is fixed as 2'-deoxy-inverted abasic ribose (**B**), positions 2, 3, 8 and 9 have been fixed as 2'-deoxy-thymidine (T) while the X positions 4-7 correspond to an approximately equimolar distribution of all the nucleotide building blocks that make up the X mixture.

In another preferred embodiment, a "mix and split" approach to generate ribozyme libraries is described. This method is particularly useful when the number of selected nucleotide building blocks to be included in the library is large and diverse (greater than 5 nucleotide building blocks) and/or when the coupling kinetics of the selected nucleotide building blocks do not allow competitive coupling even after relative concentration adjustments and optimization. This method involves a multi-step process wherein the solid support used for ribozyme library synthesis is "split" (divided) into equal portions, (the number of portions is equal to the number of different nucleotide building blocks (n) chosen for incorporation at one or more random positions within the ribozyme). For example, if there are 10 different nucleotide building blocks chosen for incorporation at one or more positions in the ribozyme library, then the solid support is divided into 10 different portions. Each portion is independently coupled to one of the selected nucleotide building blocks followed by mixing of all the portions of solid support. The ribozyme synthesis is then resumed as before the division of the building blocks. This enables the synthesis of a ribozyme library wherein one or more positions within the ribozyme is random. The number of "splitting" and "mixing" steps is dependent on the number of positions that are random within the ribozyme. For example if three positions are desired to be random then three different splitting and mixing steps are necessary to synthesize the ribozyme library.

Random ribozyme libraries are synthesized using a non-competitive coupling procedure where each of the selected nucleotide analogs "n" separately couple to an inverse "n" (1/n) number of aliquots of solid-support or of a growing ribozyme chain on the solid-support. A very convenient way to verify completeness of the coupling reaction is the use of a standard spectrophotometric DMT assay (Oligonucleotide Synthesis, A Practical Approach, ed. M. Gait, pp 48, IRC Press, Oxford, UK; incorporated by reference herein). These aliquots may be subsequently combined, mixed and split into one new aliquot. A similar approach to making oligonucleotide libraries has recently been described by Cook *et al.,* (US Patent No. 5,587,471) and is incorporated by reference herein.

### Nucleotide Synthesis

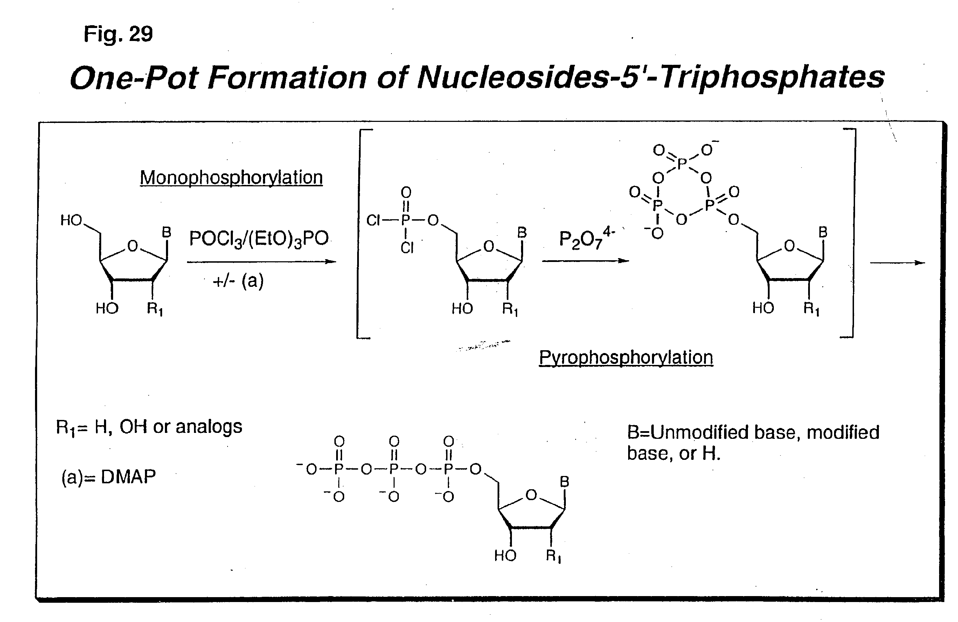

Addition of dimethylaminopyridine (DMAP) to the phosphorylation protocols known in the art can greatly increase the yield of nucleoside monophosphates while decreasing the reaction time (Fig. 29). Synthesis of the nucleosides of the invention have been described in several publications and Applicants previous applications (Beigelman *et al.,* International PCT publication No. WO 96/18736; Dudzcy *et al*., Int. PCT Pub. No. WO 95/11910; Usman *et al*., Int. PCT Pub. No. WO 95/13378; Matulic-Adamic *et al*., 1997, Tetrahedron Lett. 38, 203; Matulic-Adamic *et al*., 1997, *Tetrahedron Lett.* 38, 1669; all of which are incorporated herein by reference). These nucleosides are dissolved in triethyl phosphate and chilled in an ice bath. Phosphorus oxychloride (POCl₃) is then added followed by the introduction of DMAP. The reaction is then warmed to room temperature and allowed to proceed for 5 hours. This reaction allows the formation of nucleoside monophosphates which can then be used in the formation of nucleoside triphosphates. Tributylamine is added followed by the addition of anhydrous acetonitrile and tributylammonium pyrophosphate. The reaction is then quenched with TEAB and stirred overnight at room temperature (about 20C). The triphosphate is purified using column purification and HPLC and the chemical structure is confirmed using NMR analysis. Those skilled in the art will recognize that the reagents, temperatures of the reaction, and purification methods can easily be alternated with substitutes and equivalents and still obtain the desired product.

The invention provides nucleoside triphosphates which can be used for a number of different functions. The nucleoside triphosphates formed from nucleosides found in table III are unique and distinct from other nucleoside triphosphates known in the art. Incorporation of modified nucleotides into DNA or RNA oligonucleotides can alter the properties of the molecule. For example, modified nucleotides can hinder binding of nucleases, thus increasing the chemical half-life of the molecule. This is especially important if the molecule is to be used for cell culture or *in vivo.* It is known in the art that the introduction of modified nucleotides into these molecules can greatly increase the stability and thereby the effectiveness of the molecules (Burgin *et al.,* 1996, *Biochemistry* 35, 14090-14097; Usman *et al.,* 1996, *Curr. Opin. Struct. Biol.* 6, 527-533).

Modified nucleotides are incorporated using either wild type and mutant polymerases. For example, mutant T7 polymerase is used in the presence of modified nucleotide triphosphate(s), DNA template and suitable buffers. Those skilled in the art will recognize that other polymerases and their respective mutant versions can also be utilized for the incorporation of NTP's of the invention. Nucleic acid transcripts were detected by incorporating radiolabelled nucleotides (α-³²P NTP). The radiolabeled NTP contained the same base as the modified triphosphate being tested. The effects of methanol, PEG and LiCl were tested by adding these compounds independently or in combination. Detection and quantitation of the nucleic acid transcripts was performed using a Molecular Dynamics PhosphorImager. Efficiency of transcription was assessed by comparing modified nucleotide triphosphate incorporation with all-ribonucleotide incorporation control. Wild type polymerase was used to incorporate NTP's using the manufacturers buffers and instructions (Boehringer Mannheim).

### Transcription Conditions

Incorporation rates of modified nucleoside triphosphates into oligonucleotides can be increased by adding to traditional buffer conditions, several different enhancers of modified NTP incorporation. Applicant has utilized methanol and LiCl in an attempt to increase incorporation rates of dNTP using RNA polymerase. These enhancers of modified NTP incorporation can be used in different combinations and ratios to optimize transcription. Optimal reaction conditions differ between nucleoside triphosphates and can readily be determined by standard experimentation. Overall however, inclusion of enhancers of modified NTP incorporation such as methanol or inorganic compound such as lithium chloride, have been shown by the applicant to increase the mean transcription rates.

### Administration of Nucleoside mono, di or triphosphates

The nucleotide monophosphates, diphosphates, or triphosphates can be used as a therapeutic agent either independently or in combination with other pharmaceutical components. These molecules of the inventions can be administered to patients using the methods of Sullivan *et al*., PCT WO 94/02595. Molecules of the invention may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the modified nucleotide triphosphate, diphosphate or monophosphate/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of delivery and administration are provided in Sullivan *et al.,* supra and Draper *et al.,* PCT WO93/23569 which have been incorporated by reference herein.

This invention further relates to a compound having the Formula II: 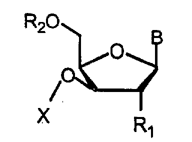 wherein, R₁ is OH, O-R₃, where R₃ is independently a moiety selected from a group consisting of alkyl, alkenyl, alkynyl, aryl, alkylaryl, carbocyclic aryl, heterocyclic aryl, amide and ester; C-R₃, where R₃ is independently a moiety selected from a group consisting of alkyl, alkenyl, alkynyl, aryl, alkylaryl, carbocyclic aryl, heterocyclic aryl, amide and ester; halo, NHR₄ (R₄=alkyl (C1-22), acyl (C1-22), substituted or unsubstituted aryl), or OCH₂SCH₃ (methylthiomethyl), ONHR₅ where R₅ is independently H, aminoacyl group, peptidyl group, biotinyl group, cholesteryl group, lipoic acid residue, retinoic acid residue, folic acid residue, ascorbic acid residue, nicotinic acid residue, 6-aminopenicillanic acid residue, 7-aminocephalosporanic acid residue, alkyl, alkenyl, alkynyl, aryl, alkylaryl, carbocyclic aryl, heterocyclic aryl, amide or ester, ON=R₆, where R₆ is independently pyridoxal residue, pyridoxal-5-phosphate residue, 13-cis-retinal residue, 9-cis-retinal residue, alkyl, alkenyl , alkynyl, alkylaryl, carbocyclic alkylaryl, or heterocyclic alkylaryl; B is independently a nucleotide base or its analog or hydrogen; X is independently a phosphorus-containing group; and R₂ is independently blocking group or a phosphorus-containing group.

Specifically, an "alkyl" group refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxy, cyano, alkoxy, NO₂ or N(CH₃)₂, amino, or SH.

The term "alkenyl" group refers to unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, NO₂, halogen, N(CH₃)₂, amino, or SH.

The term "alkynyl" refers to an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, NO₂ or N(CH₃)₂, amino or SH.

An "aryl" group refers to an aromatic group which has at least one ring having a conjugated p electron system and includes carbocyclic aryl, heterocyclic aryl and biaryl groups, all of which may be optionally substituted. The preferred substituent(s) on aryl groups are halogen, trihalomethyl, hydroxyl, SH, cyano, alkoxy, alkyl, alkenyl, alkynyl, and amino groups.

An "alkylaryl" group refers to an alkyl group (as described above) covalently joined to an aryl group (as described above).

"Carbocyclic aryl" groups are groups wherein the ring atoms on the aromatic ring are all carbon atoms. The carbon atoms are optionally substituted.

"Heterocyclic aryl" groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, pyrrolo, pyrimidyl, pyrazinyl, imidazolyl and the like, all optionally substituted.

An "amide" refers to an -C(O)-NH-R, where R is either alkyl, aryl, alkylaryl or hydrogen.

An "ester" refers to an -C(O)-OR', where R is either alkyl, aryl, or alkylaryl.

A "blocking group" is a group which is able to be removed after polynucleotide synthesis and/or which is compatible with solid phase polynucleotide synthesis.

A "phosphorus containing group" can include phosphorus in forms such as dithioates, phosphoramidites and/or as part of an oligonucleotide.

In a preferred embodiment, the invention features a process for synthesis of the compounds of formula **II**.

In a preferred embodiment the invention features a process for the synthesis of a xylofuranosyl nucleoside phosphoramidite comprising the steps of: a) oxidation of a 2' and 5'-protected ribonucleoside with a an oxidant such as chromium oxide/pyridine/aceticanhydride, dimethylsulfoxide/aceticanhydride, or Dess-Martin reagent (periodinane) followed by reduction with a reducing agent such as, triacetoxy sodium borohydride, sodium borohydride, or lithium borohydride, under conditions suitable for the formation of 2' and 5'-protected xylofuranosyl nucleoside; b) phosphitylation under conditions suitable for the formation of xylofuranosyl nucleoside phosphoramidite.

In yet another preferred embodiment, the invention features the incorporation of the compounds of Formula **II** into polynucleotides. These compounds can be incorporated into polynucleotides enzymatically. For example by using bacteriophage T7 RNA polymerase, these novel nucleotide analogs can be incorporated into RNA at one or more positions (Milligan *et al.,* 1989, *Methods Enzymol.,* 180, 51). Alternatively, novel nucleoside analogs can be incorporated into polynucleotides using solid phase synthesis (Brown and Brown, 1991, in *Oligonucleotides and Analogues: A Practical Approach, p.* 1, ed. F. Eckstein, Oxford University Press, New York; Wincott *et al.,* 1995, *Nucleic Acids Res.,* 23, 2677; Beaucage & Caruthers, 1996, in *Bioorganic Chemistry: Nucleic Acids,* p *36,* ed. S. M. Hecht, Oxford University Press, New York).

The compounds of Formula **II** can be used for chemical synthesis of nucleotide-tri-phosphates and/or phosphoramidites as building blocks for selective incorporation into oligonucleotides. These oligonucleotides can be used as an antisense molecule, 2-5A antisense chimera, triplex forming oligonucleotides (TFO) or as an nucleic acid catalyst. The oligonucleotides can also be used as probes or primers for synthesis and/or sequencing of RNA or DNA.

The compounds of the instant invention can be readily converted into nucleotide diphosphate and nucleotide triphosphates using standard protocols (for a review see Hutchinson, 1991, in *Chemistty of Nucleosides and Nucleotides,* v.2, pp 81-160, Ed. L. B. Townsend, Plenum Press, New York, USA; incorporated by reference herein).

The compounds of Formula **II** can also be independently or in combination used as an antiviral, anticancer or an antitumor agent. These compounds can also be independently or in combination used with other antiviral, anticancer or an antitumor agents.

In one of the preferred embodiments of the inventions herein, the nucleic acid catalyst is formed in a hammerhead or hairpin motif, but may also be formed in the motif of a hepatitis d virus, group I intron, group II intron or RNaseP RNA (in association with an RNA guide sequence) or *Neurospora* VS RNA. Examples of such hammerhead motifs are described by Dreyfus, *supra,* Rossi *et al*., 1992, *AIDS Research and Human Retroviruses* 8, 183; of hairpin motifs by Hampel *et al*., EP0360257, Hampel and Tritz, 1989 *Biochemistry* 28, 4929, Feldstein *et al*., 1989, *Gene* 82, 53, Haseloff and Gerlach, 1989, *Gene,* 82, 43, and Hampel *et al*., 1990 *Nucleic Acids Res.* 18, 299; of the hepatitis d virus motif is described by Perrotta and Been, 1992 *Biochemistry* 31, 16; of the RNaseP motif by Guerrier-Takada *et al*., 1983 *Cell* 35, 849; Forster and Altman, 1990, *Science* 249, 783; Li and Altman, 1996, *Nucleic Acids Res.* 24, 835; *Neurospora* VS RNA ribozyme motif is described by Collins (Saville and Collins, 1990 *Cell* 61, 685-696; Saville and Collins, 1991 *Proc. Natl. Acad. Sci. USA* 88, 8826-8830; Collins and Olive, 1993 *Biochemistry* 32, 2795-2799; Guo and Collins, 1995, *EMBO. J.* 14, 363); Group II introns are described by Griffin *et al*., 1995, *Chem. Biol.* 2, 761; Michels and Pyle, 1995, *Biochemistry* 34, 2965; Pyle *et al*., International PCT Publication No. WO 96/22689; and of the Group I intron by Cech *et al*., U.S. Patent 4,987,071. These specific motifs are not limiting in the invention and those skilled in the art will recognize that all that is important in a nucleic acid catalyst of this invention is that it has a specific substrate binding site which is complementary to one or more of the target gene RNA regions, and that it have nucleotide sequences within or surrounding that substrate binding site which impart an RNA cleaving activity to the molecule.

In a preferred embodiment, a polynucleotide of the invention would bear one or more 2'-hydroxylamino functionalities attached directly to the monomeric unit or through the use of an appropriate spacer. Since oligonucleotides have neither aldehyde nor hydroxylamino groups, the formation of an oxime would occur selectively using oligo as a polymeric template. This approach would facilitate the attachment of practically any molecule of interest (peptides, polyamines, coenzymes, oligosaccharides, lipids, etc.) directly to the oligonucleotide using either aldehyde or carboxylic function in the molecule of interest.

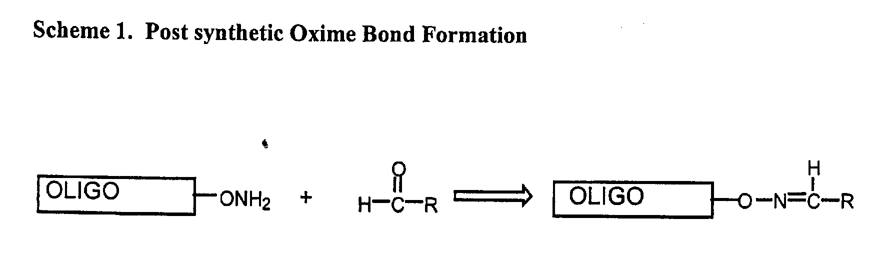

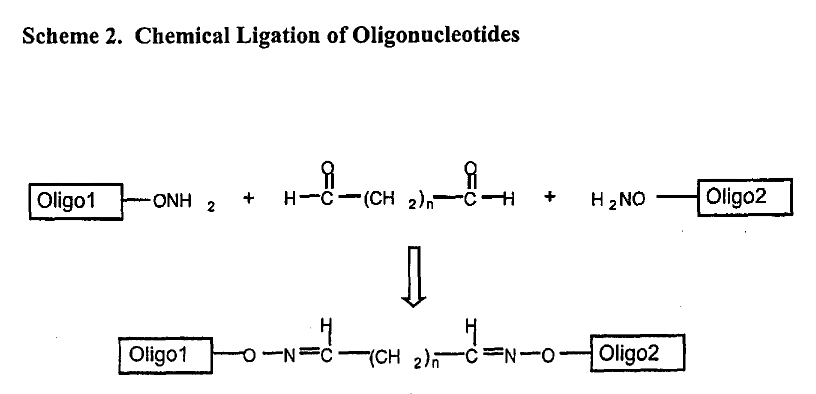

### Advantages of oxime bond formation:

- The oximation reaction proceeds in water
- Quantitative yields
- Hydrolytic stability in a wide pH range (5 - 8)
- The amphoteric nature of oximes allows them to act either as weak acids or weak bases.
- Oximes exhibit a great tendency to complex with metal ions

In yet another preferred embodiment, the aminooxy "tether" in oligonucleotides, such as a ribozyme, is reacted with different compounds bearing carboxylic groups (e.g., aminoacids, peptides, "cap" structures ,etc.) resulting in the formation of oxyamides as shown below.

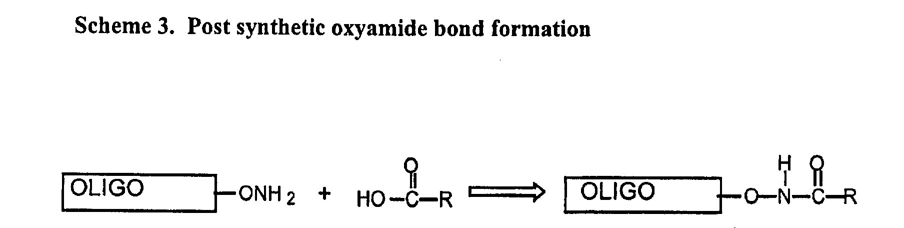

### Target Discovery:

Applicant has developed an efficient and rapid method for screening libraries of catalytic nucleic acid molecules capable of performing a desired function in a cell. The invention also features the use of a catalytic nucleic acid library to modulate certain attributes or processes in a biological system, such as a mammalian cell, and to identify and isolate a) nucleic acid catalysts from the library involved in modulating the cellular process/attribute of interest; and b) modulators of the desired cellular process/attribute using the sequence of the nucleic acid catalyst.

More specifically, the method of the instant invention involves designing and constructing a catalytic nucleic acid library, where the catalytic nucleic acid includes a catalytic and a substrate binding domain, and the substrate binding domain (arms) are randomized. This library of catalytic nucleic acid molecules with randomized binding arm(s) are used to modulate certain processes/attributes in a biological system. The method described in this application involves simultaneous screening of a library or pool of catalytic nucleic acid molecules with various substitutions at one or more positions and selecting for ribozymes with desired function or characteristics or attributes. This invention also features a method for constructing and selecting for catalytic nucleic acid molecules for their ability to cleave a given target nucleic acid molecule or an unknown target nucleic acid molecule (e.g., RNA), and to inhibit the biological function of that target molecule or any protein encoded by it.

It is not necessary to know either the sequence or the structure of the target nucleic acid molecule in order to select for catalytic nucleic acid molecules capable of cleaving the target in this cellular system. The cell-based screening protocol described in the instant invention (*i.e.,* one which takes place inside a cell) offers many advantages over extracellular systems, because the synthesis of large quantities of RNA by enzymatic or chemical methods prior to assessing the efficacy of the catalytic nucleic acid molecules is not necessary. The invention further describes a rapid method of using catalytic nucleic acid molecule libraries to identify the biological function of a gene sequence inside a cell. Applicant describes a method of using catalytic nucleic acid molecule libraries to identify a nucleic acid molecule, such as a gene, involved in a biological process; this nucleic acid molecule may be a known molecule with a known function, or a known molecule with a previously undefined function or an entirely novel molecule. This is a rapid means for identifying, for example, genes involved in a cellular pathway, such as cell proliferation, cell migration, cell death, and others. This method of gene discovery is not only a novel approach to studying a desired biological process but also a means to identify active reagents that can modulate this cellular process in a precise manner.

Applicant describes herein, a general approach for simultaneously assaying the ability of one or more members of a catalytic nucleic acid molecule library to modulate certain attributes/process(es) in a biological system, such as plants, animals or bacteria, involving introduction of the library into a desired cell and assaying for changes in a specific "attribute," "characteristic" or "process." The specific attributes may include cell proliferation, cell survival, cell death, cell migration, angiogenesis, tumor volume, tumor metastasis, levels of a specific mRNA(s) in a cell, levels of a specific protein(s) in a cell, levels of a specific protein secreted, cell surface markers, cell morphology, cell differentiation pattern, cartilage degradation, transplantation, restenosis, viral replication, viral load, and the like. By modulating a specific biological pathway using a catalytic nucleic acid molecule library, it is possible to identify the gene(s) involved in that pathway, which may lead to the discovery of novel genes, or genes with novel function. This method provides a powerful tool to study gene function inside a cell. This approach also offers the potential for designing novel catalytic oligonucleotides, identifying ribozyme accessible sites within a target, and for identifying new nucleic acid targets for ribozyme-mediated modulation of gene expression.

In another aspect the invention involves synthesizing a Random Binding Arm Nucleic Acid Catalyst Library (Random Library) and simultaneously testing all members of the Random Library in cells. This library has ribozymes with random substrate binding arm(s) and a defined catalytic domain. Cells with an altered attribute (such as inhibition of cell proliferation) as a result of interaction with the members of the Random Library are selected and the sequences of the ribozymes from these cells are determined. Sequence information from the binding arm(s) of these ribozymes can be used to isolate nucleic acid molecules that are likely to be involved in the pathway responsible for the desired cellular attribute using standard technology known in the art, *e.g*., nucleic acid amplification using techniques such as polymerase chain reaction (PCR). This method is a powerful means to isolate new genes or genes with new function.

By "Random Library" as used herein is meant ribozyme libraries comprising all possible variants in the binding arm (s) of a given ribozyme motif. Here the complexity and the content of the library is not defined. The Random Library is expected to comprise sequences complementary to every potential target sequence, for the ribozyme motif chosen, in the genome of an organism. This Random Library can be used to screen for ribozyme cleavage sites in a known target sequence or in a unknown target. In the first instance, the Random Library is introduced into the cell of choice and the expression of the known target gene is assayed. Cells with an altered expression of the target will yield the most effective ribozyme against the known target. In the second instance, the Random Library is introduced into the cell of choice and the cells are assayed for a specific attribute, for example, survival of cells. Cells that survive the interaction with the Random Library are isolated and the ribozyme sequence from these cells is determined. The sequence of the binding arm of the ribozyme can then be used as probes to isolate the gene(s) involved in cell death. Because, the ribozyme(s) from the Random Library is able to modulate (e.g., down regulate) the expression of the gene(s) involved in cell death, the cells are able to survive under conditions where they would have otherwise died. This is a novel method of gene discovery. This approach not only provides the information about mediators of certain cellular processes, but also provides a means to modulate the expression of these modulators. This method can be used to identify modulators of any cell process in any organism, including but not limited to mammals, plants and bacteria.

The invention provides a method for producing a class of enzymatic cleaving agents which exhibit a high degree of specificity for the nucleic acid sequence of a desired target. The nucleic acid catalyst is preferably targeted to a highly conserved sequence region of a target such that specific diagnosis and/or treatment of a disease or condition can be provided with a single enzymatic nucleic acid.

In a first aspect the invention features a method for identifying one or more nucleic acid molecules, such as gene(s), involved in a process (such as, cell growth, proliferation, apoptosis, morphology, angiogenesis, differentiation, migration, viral multiplication, drug resistance, signal transduction, cell cycle regulation, temperature sensitivity, chemical sensitivity and others) in a biological system, such as a cell. The method involves the steps of: a) providing a random library of nucleic acid catalysts, with a substrate binding domain and a catalytic domain, where the substrate binding domain has a random sequence, to the biological system under conditions suitable for the process to be altered; b) identifying any nucleic acid catalyst present in that biological system where the process has been altered by any nucleic acid catalyst; and c) determining the nucleotide sequence of at least a portion of the binding arm of such a nucleic acid catalyst to allow identification of the nucleic acid molecule involved in the process in that biological system.

In a related aspect the invention features a method for identification of a nucleic acid molecule capable of modulating a process in a biological system. The method includes: a) introducing a library of nucleic acid catalysts with a substrate binding domain and a catalytic domain, where the substrate binding domain has a random sequence, into the biological system under conditions suitable for modulating the process; and b) determining the nucleotide sequence of at least a portion of the substrate binding domain of any nucleic acid catalyst from a biological system where the process has been modulated to allow said identification of the nucleic acid molecule capable of modulating said process in that biological system.

In a second aspect, the invention the invention further concerns a method for identification of a nucleic acid catalyst capable of modulating a process in a biological system. This involves: a) introducing a library of nucleic acid catalysts with a substrate binding domain and a catalytic domain, where the substrate binding domain has a random sequence, into the biological system under conditions suitable for modulating the process; and b) identifying any nucleic acid catalyst from a biological system where the process has been modulated.

By "enzymatic portion" or "catalytic domain" is meant that portion/region of the ribozyme essential for cleavage of a nucleic acid substrate (for example see Figure 3).

By "nucleic acid molecule" as used herein is meant a molecule having nucleotides. The nucleic acid can be single, double or multiple stranded and may comprise modified or unmodified nucleotides or non-nucleotides or various mixtures and combinations thereof. An example of a nucleic acid molecule according to the invention is a gene which encodes for macromolecule such as a protein.

The "biological system" as used herein may be a eukaryotic system or a prokaryotic system, may be a bacterial cell, plant cell or a mammalian cell, or may be of plant origin, mammalian origin, yeast origin, Drosophila origin, or archebacterial origin.

This invention further relates to novel nucleic acid molecules with catalytic activity, which are particularly useful for cleavage of RNA or DNA. The nucleic acid catalysts of the instant invention are distinct from other nucleic acid catalysts known in the art. This invention also relates to a method of screening variants of nucleic acid catalysts using standard nucleotides or modified nucleotides. Applicant has determined an efficient method for screening libraries of catalytic nucleic acid molecules, particularly those with chemical modifications at one or more positions. The method described in this application involves systematic screening of a library or pool of ribozymes with various substitutions at one or more positions and selecting for ribozymes with desired function or characteristic or attribute.

In one preferred embodiment, a method for identifying a nucleic acid molecule involved in a process in a cell is described, including the steps of: a) synthesizing a library of nucleic acid catalysts, having a substrate binding domain and a catalytic domain, where the substrate binding domain has a random sequence; b) testing the library in the cell under conditions suitable to cause the process in the cell to be altered (such as: inhibition of cell proliferation, inhibition of angiogenesis, modulation of growth and /or differentiation, and others); c) isolating and enriching the cell with the altered process; d) identifying and isolating the nucleic acid catalyst in the altered cell; e) using an oligonucleotide, having the sequence homologous to the sequence of the substrate binding domain of the nucleic acid catalyst isolated from the altered cell, as a probe to isolate the nucleic acid molecule from the cell or the altered cell. Those nucleic acid molecules identified using the selection/screening method described above are likely to be involved in the process that was being assayed for alteration by the member(s) of the ribozyme library. These nucleic acid molecules may be new gene sequences, or known gene sequences, with a novel function. One of the advantages of this method is that nucleic acid sequences, such as genes, involved in a biological process, such as differentiation, cell growth, disease processes including cancer, tumor angiogenesis, arthritis, cardiovascular disease, inflammation, restenosis, vascular disease and the like, can be readily identified using the Random Library approach. Thus theoretically, one Random Library for a given ribozyme motif can be used to assay any process in any biological system.

In another preferred embodiment the invention involves synthesizing a Defined Arm Nucleic Acid Catalyst Library (Defined Library) and simultaneously testing it against known targets in a cell. The library includes ribozymes with binding arm(s) of known complexity (Defined) and a defined catalytic domain. Modulation of expression of the target gene by ribozymes in the library will cause the cells to have an altered phenotype. Such cells are isolated and the ribozymes in these cells are the ones most suited for modulating the expression of the desired gene in the cell.

By "Defined Library" as used herein is meant a library of nucleic acid catalysts, wherein each member nucleic acid catalyst is designed and produced independently, then added to the library. Thus, the content, complexity (number of different ribozymes contained in the library) and ratios of library members are defined at the outset. Defined Library comprises ≥ 2 ribozymes. The process involves screening the sequence of the known target RNA for all possible sites that can be cleaved by a given ribozyme motif and as described, for example in McSwiggen, US Patent No. 5,525,468, incorporated by reference herein. Synthesizing a representative number of different ribozymes aginst the target sequence. Combining the ribozymes and introducing the pooled ribozymes into a biological system comprising the target RNA under conditions suitable to facilitate modulation of the expression of the target RNA in said biological system.

### Screening of Nucleic Acid Catalysts

Applicant describes herein, a general combinatorial approach for assaying ribozyme variants based on ribozyme activity and/or a specific "attribute" of a ribozyme, such as the cleavage rate, cellular efficacy, stability, delivery, localization and the like. Variations of this approach also offer the potential for designing novel catalytic oligonucleotides, identifying ribozyme accessible sites within a target, and for identifying new nucleic acid targets for ribozyme-mediated modulation of gene expression.

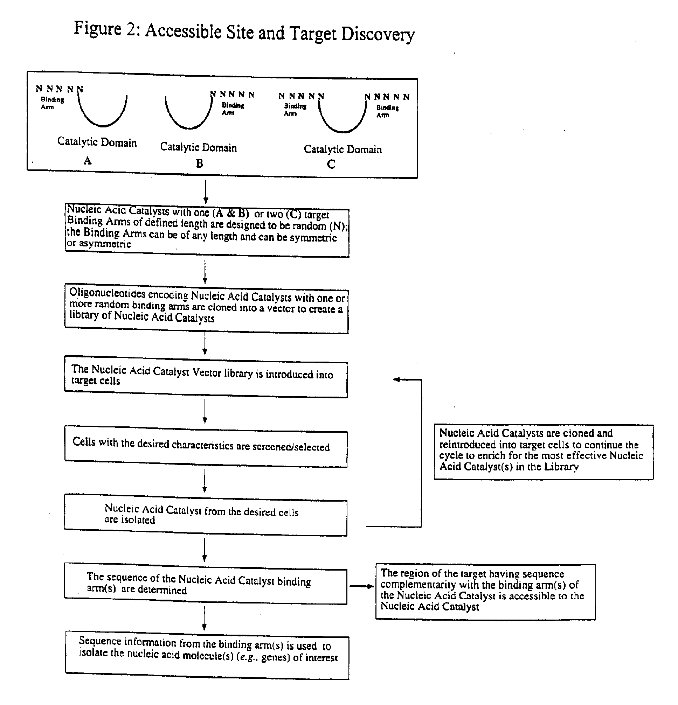

In one preferred embodiment, the method relies upon testing mixtures (libraries) of ribozymes with various nucleotides, nucleotide analogs, or other analog substitutions, rather than individual ribozymes, to rapidly identify the nucleotide, nucleotide analog, or other analog that is variable at one or more positions within a ribozyme. In the first step (step 1, Figure 2), a desired number of positions (for example, 3 positions as shown in Figure 2) are chosen for variation in a first ribozyme motif (Starting Ribozyme); there is no requirement on the number of positions that can be varied and these positions may or may not be phylogenetically conserved for the ribozyme. In addition, these position may reside within the catalytic core, binding arms, or accessory domains. The number of positions that are chosen to be varied defines the number of "Classes" of ribozyme libraries that will be synthesized. In the example illustrated in Figure 2, three positions (designated positions 1, 2 and 3) are varied, so three different Classes of ribozyme pool are synthesized. In the next step (step 2), ribozyme pools are synthesized containing a random mixture of different nucleotides, nucleotide analogs, or other analogs at all of the desired positions (designated "X") to be varied except one, which is the "fixed" position (designated "F"). The fixed position contains a specific nucleotide, nucleotide analog or other analog. There is no requirement for the number of nucleotides, or analogs be used. The number of nucleotides or analogs defines the number of pools (designated n) in each Class. For example if ten different nucleotides or analogs are chosen, ten different pools (n=10) will be synthesized for each Class; each of the pools will contain a specific modification at one fixed position (designated F) but will contain an equal mixture of all ten modifications at the other positions (designated X). In a subsequent step (step 3), the different pools of ribozymes are tested for desired activity, phenotype, characteristic or attribute. For example, the testing may be determining *in vitro* rates of target nucleic acid cleavage for each pool, testing ribozyme-substrate binding affinities, testing nuclease resistance, determining pharmacodynamic properties, or determining which pool is most efficacious in a cellular or animal model system. Following testing, a particular pool is identified as a desired variant (designated " Desired Variant-1") and the nucleotide or the analog present at the fixed position within the Desired Variant-1 is made constant (designated "Z") for all subsequent experiments; a single position within a ribozyme is therefore varied, *i.e.,* the variable nucleotide or analog at a single position, when all other X positions are random, is identified within a ribozyme motif. Subsequently, new ribozyme pools (Classes 2, 3 *etc*.) are synthesized containing an equal mixture of all nucleotides or analogs at the remaining positions to be optimized except one fixed position and one or more constant positions. Again, a specific nucleotide or analog is "fixed" at a single position that is not randomized and the pools are assayed for a particular phenotype or attribute (step 4). This process is repeated until all desired positions have been varied and screened. For example if three positions are chosen for optimization, the synthesis and testing will need to be repeated three times (3 Classes). In the first two Classes, pools will be synthesized; in the final Class, specific ribozymes will be synthesized and tested. When the final position is analyzed (step 5), no random positions will remain and therefore only individual ribozymes are synthesized and tested. The resulting ribozyme or ribozymes (designated "second ribozyme motif') will have a defined chemical composition which will likely be distinct from the Starting Ribozyme motif (first ribozyme motif). This is a rapid method of screening for variability of one or more positions within a ribozyme motif.

In another preferred embodiment, the invention involves screening of chemical modifications at one or more positions within a hammerhead ribozyme motif. More specifically, the invention involves variability in the catalytic core sequence of a hammerhead ribozyme. Particularly, the invention describes screening for variability of positions 3, 4 and 7 within a hammerhead ribozyme. The invention also features screening for optimal loop II sequence in a hammerhead ribozyme.

In yet another preferred embodiment, the invention features a rapid method for screening accessible ribozyme cleavage sites within a target sequence. This method involves screening of all possible sequences in the binding arm of a ribozyme. The sequence of the binding arms determines the site of action of certain ribozymes. The combinatorial approach can be used to identify desirable and/or accessible sites within a target sequence by essentially testing all possible arm sequences. The difficulty with this approach is that ribozymes require a certain number of base pairs (for example, for hammerhead ribozymes the binding arm length is approximately 12-16 nucleotides) in order to bind functionally and sequence-specifically. This would require, for example 12-16 different groups of hammerhead ribozyme pools; 12-16 positions would have to be optimized which would require 12-16 different groups being synthesized and tested. Each pool would contain the four different nucleotides (A, C, U and G) or nucleotide analogs (p = 4 for nucleotides). It would be very time consuming to test each group, identify the best pool, synthesize another group of ribozyme pools with one additional position constant, and then repeat the procedure until all 12-16 groups had been tested. However it is possible to decrease the number of Classes by testing multiple positions within a single Class. In this case, the number of pools within a Class equals the number of nucleotides or analogs in the random mixture (i.e., n) to the w power, where w equals the number of positions fixed in each Class. The number of Classes that need to be synthesized to optimize the final ribozyme equals the total number of positions to be optimized divided by the number of positions (w) tested within each Class. The number of pools in each Class = n^{w}. The number of Class = total number of positions /w.

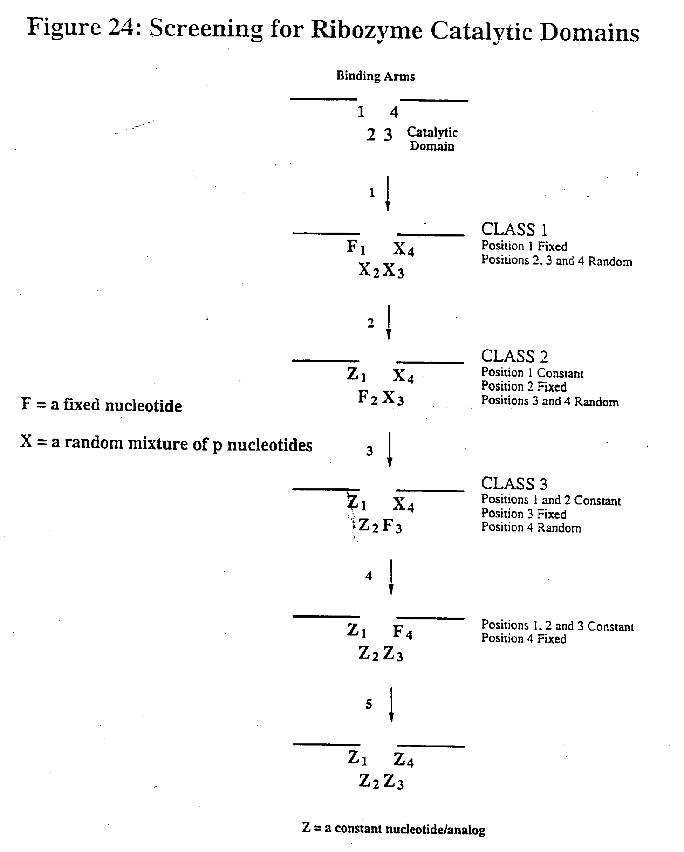

In another preferred embodiment, the invention features a rapid method of screening for new catalytic nucleic acid motifs by keeping the binding arms constant and varying one or more positions in a putative catalytic domain. Applicant describes a method to vary positions within the catalytic domain, without changing positions within the binding arms, in order to identify new catalytic motifs. An example is illustrated in Figure 24. It is unclear how many positions are required to obtain a functional catalytic domain in a nucleic acid molecule, however it is reasonable to presume that if a large number of functionally diverse nucleotide analogs can be used to construct the pools, a relatively small number of positions could constitute a functional catalytic domain. This may especially be true if analogs are chosen that one would expect to participate in catalysis (e.g., acid/base catalysts, metal binding, etc.). In the example illustrated, four positions (designated 1, 2, 3 and 4) are chosen. In the first step, ribozyme libraries (Class 1) are constructed: position 1 is fixed (F₁) and positions 2, 3 and 4 are random (X₂, X₃ and X₄, respectively). In step 2, the pools (the number of pools tested depends on the number of analogs used; n) are assayed for activity. This testing may be performed *in vitro* or in a cellular or animal model. Whatever assay that is used, the pool with the desired characteristic is identified and libraries (class 2) are again synthesized with position 1 now constant (Z₁) with the analog that was identified in class 1. In class 2, position 2 is fixed (F₂) and positions 3 and 4 are random (X₃ and X₄). This process is repeated until every position has been made constant and the chemical composition of the catalytic domain is determined. If the number of positions in the catalytic domain to be varied are large, then it is possible to decrease the number of Classes by testing multiple positions within a single Class. The number of pools within a Class equals the number of nucleotides or analogs in the random mixture (i.e., n) to the w power, where w equals the number of positions fixed in each Class. The number of Classes that need to be synthesized to optimize the final ribozyme equals the total number of positions to be optimized divided by the number of positions (w) tested within each Class. The number of pools in each Class= n^{w}. The number of Classes= total number of positions /w.

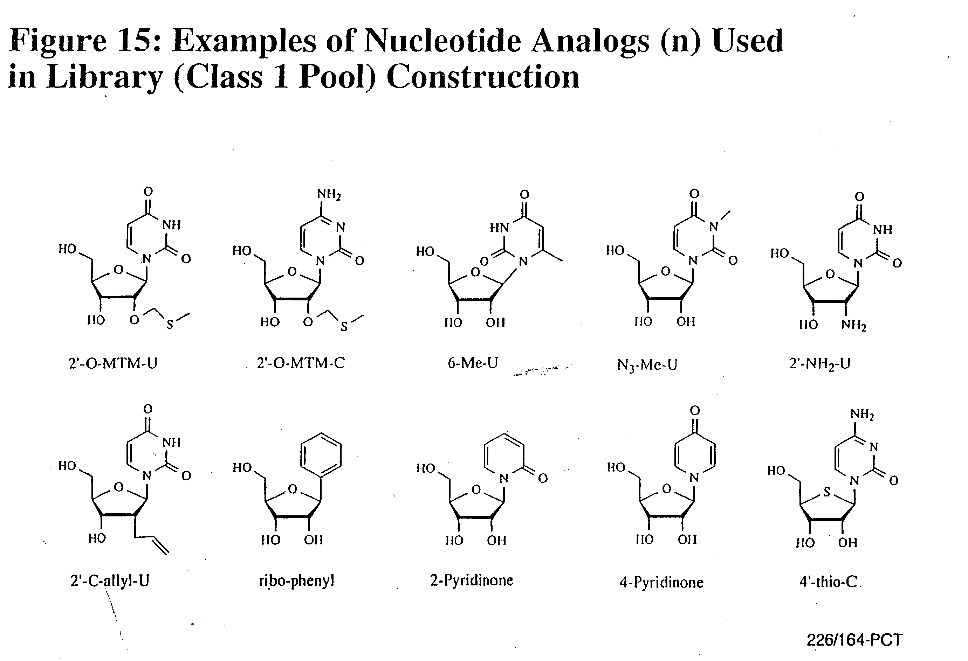

In a preferred embodiment a method for identifying variants of a nucleic acid catalyst is described comprising the steps of: a) selecting at least three (3) positions, preferably 3-12, specifically 4-10, within said nucleic acid catalyst to be varied with a predetermined group of different nucleotides, these nucleotides are modified or unmodified (non-limiting examples of nucleotides that can used in this method are shown in Figure 15); b) synthesizing a first class of different pools of said nucleic acid catalyst, wherein the number of pools synthesized is equal to the number of nucleotides in the predetermined group of different nucleotides (for example if 10 different nucleotides are selected to be in the group of predetermined nucleotides then 10 different pools of nucleic acid catalysts have to be synthesized), wherein at least one of the positions to be varied in each pool comprises a defined nucleotide (fixed position; F) selected from the predetermined group of different nucleotides and the remaining positions to be varied comprise a random mixture of nucleotides (X positions) selected from the predetermined group of different nucleotides; c) testing the different pools of said nucleic acid catalyst under conditions suitable for said pools to show a desired attribute (including but not limited to improved cleavage rate, cellular and animal efficacy, nuclease stability, enhanced delivery, desirable localization) and identifying the pool with said desired attribute and wherein the position with the defined nucleotide (F) in the pool with the desired attribute is made constant (Z position) in subsequent steps; d) synthesizing a second class of different pools of nucleic acid catalyst, wherein at least one of the positions to be varied in each of the second class of different pools comprises a defined nucleotide (F) selected from the predetermined group of different nucleotides and the remaining positions to be varied comprise a random mixture (X) of nucleotides selected from the predetermined group of different nucleotides (this second class of pools therefore has F, X and Z positions); e) testing the second class of different pools of said nucleic acid catalyst under conditions suitable for showing desired attribute and identifying the pool with said desired attribute and wherein the position with the defined nucleotide in the pool with the desired attribute is made constant (Z) in subsequent steps; and f) this process is repeated until every position selected in said nucleic acid catalyst to be varied is made constant.

In yet another preferred embodiment, a method for identifying novel nucleic acid molecules in a biological system is described, comprising the steps of:
a) synthesizing a pool of nucleic acid catalyst with a substrate binding domain and a catalytic domain, wherein said substrate binding domain comprises a random sequence;
b) testing the pools of nucleic acid catalyst under conditions suitable for showing a desired effect (such as inhibition of cell proliferation, inhibition of angiogenesis, modulation of growth and /or differentiation, and others) and identifying the catalyst with said desired attribute;
c) using an oligonucleotide, comprising the sequence of the substrate binding domain of the nucleic acid catalyst showing said desired effect, as a probe, screening said biological system for nucleic acid molecules complementary to said probe ; and
d) isolating and sequencing said complementary nucleic acid molecules. These nucleic acid molecules identified using a nucleic acid screening method described above may be new gene sequences, or known gene sequences. The advantage of this method is that nucleic acid sequences, such as genes, involved in a biological process, such as differentiation, cell growth, disease processes including cancer, tumor angiogenesis, arthritis, cardiovascular disease, inflammation, restenosis, vascular disease and the like, can be readily identified.

In a preferred embodiment, the invention features a nucleic acid molecule with catalytic activity having one of the formulae **III-VII**:

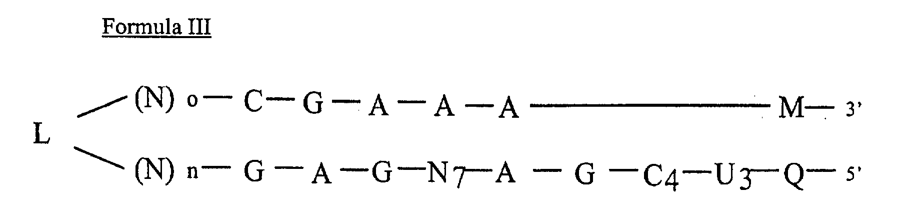

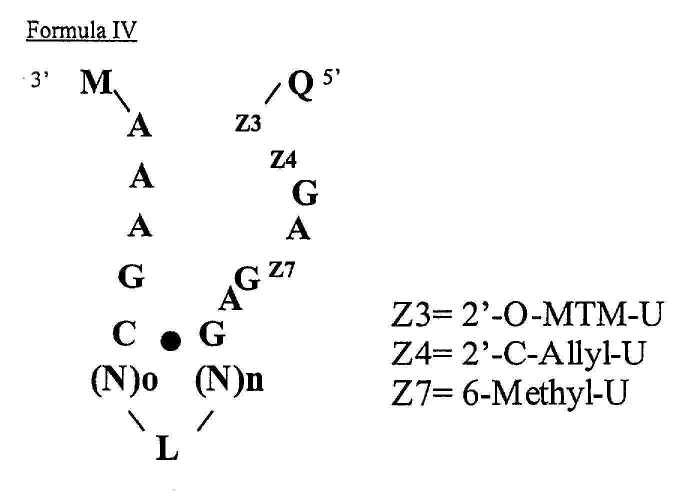

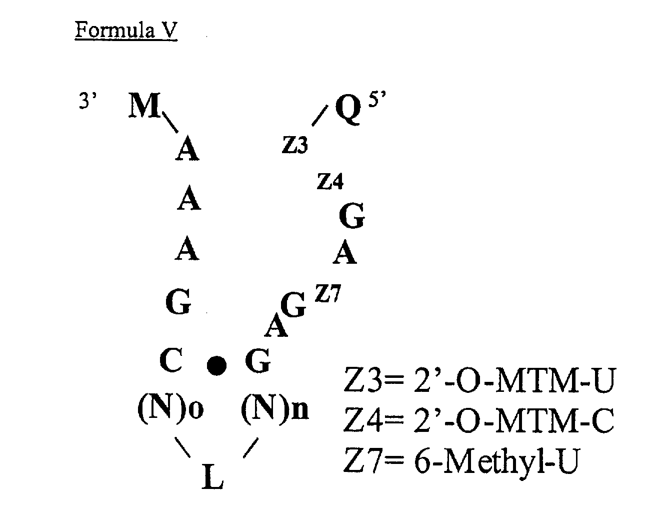

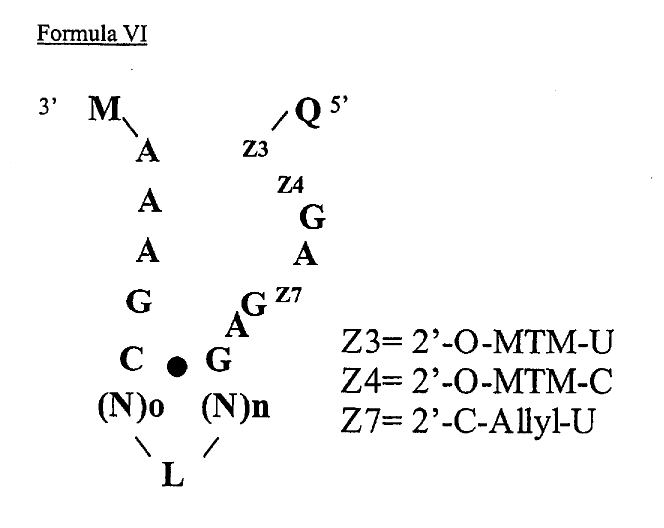

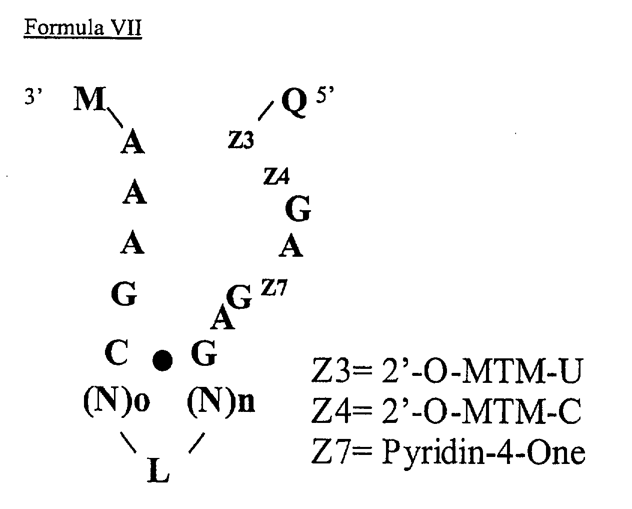

In each of the above formulae, N represents independently a nucleotide or a non-nucleotide linker, which may be same or different; M and Q are independently oligonucleotides of length sufficient to stably interact (*e.g.,* by forming hydrogen bonds with complementary nucleotides in the target) with a target nucleic acid molecule (the target can be an RNA, DNA or RNA/DNA mixed polymers); preferably the length of Q is greater than or equal to 3 nucleotides and the length of M is preferably greater than or equal to 5 nucleotides; o and n are integers greater than or equal to 1 and preferably less than about 100, wherein if (N)ₒ and (N)ₙ are nucleotides, (N)o and (N)n are optionally able to interact by hydrogen bond interaction; L is a linker which may be present or absent (*i.e.,* the molecule is assembled from two separate molecules), but when present, is a nucleotide and/or a non-nucleotide linker, which may be a single-stranded and/or double-stranded region; and _ represents a chemical linkage (*e.g*., a phosphate ester linkage, amide linkage or others known in the art). 2'-O-MTM-U and 2'-O-MTM-C refers to 2'-O-methylthiomethyl uridine and 2'-O-methylthiomethyl-cytidine, respectively. A, C, U and G represent adenosine, cytidine, uridine and guanosine nucleotides, respectively. The nucleotides in the formulae are unmodified or modified at the sugar, base, and/or phosphate portions as known in the art.

In yet another embodiment, the nucleotide linker (L) is a nucleic acid aptamer, such as an ATP aptamer, HIV Rev aptamer (RRE), HIV Tat aptamer (TAR) and others (for a review see Gold *et al.,* 1995, *Annu. Rev. Biochem.,* 64, 763; and Szostak & Ellington, 1993, in *The RNA World,* ed. Gesteland and Atkins, pp 511, CSH Laboratory Press). A "nucleic acid aptamer" as used herein is meant to indicate nucleic acid sequence capable of interacting with a ligand. The ligand can be any natural or a synthetic molecule, including but not limited to a resin, metabolites, nucleosides, nucleotides, drugs, toxins, transition state analogs, peptides, lipids, proteins, aminoacids, nucleic acid molecules, hormones, carbohydrates, receptors, cells, viruses, bacteria and others.

In yet another embodiment, the non-nucleotide linker (L) is as defined herein.

In particular, the invention features modified ribozymes having a base substitution selected from pyridin-4-one, pyridin-2-one, phenyl, pseudouracil, 2, 4, 6-trimethoxy benzene, 3-methyluracil, dihydrouracil, naphthyl, 6-methyl-uracil and aminophenyl.

In yet another embodiment, the non-nucleotide linker (L) is as defined herein. The term "non-nucleotide" as used herein include either abasic nucleotide, polyether, polyamine, polyamide, peptide, carbohydrate, lipid, or polyhydrocarbon compounds. Specific examples include those described by Seela and Kaiser, *Nucleic Acids Res.* 1990, *18*:6353 and *Nucleic Acids Res.* 1987, *15*:3113; Cload and Schepartz, *J. Am. Chem. Soc.* 1991, *113*:6324; Richardson and Schepartz, *J. Am. Chem. Soc.* 1991, *113*:5109; Ma et al., *Nucleic Acids Res.* 1993, *21*:2585 and *Biochemistry* 1993, *32:1751;* Durand et al., *Nucleic Acids Res.* 1990, *18*:6353; McCurdy et al., *Nucleosides & Nucleotides* 1991, *10*:287; Jschke et al., *Tetrahedron Lett.* 1993, 34:301; Ono et al., *Biochemistry* 1991, *30*:9914; Arnold *et al.,* International Publication No. WO 89/02439; Usman *et al.,* International Publication No. WO 95/06731; Dudycz *et al.,* International Publication No. WO 95/11910 and Ferentz and Verdine, *J*. *Am. Chem. Soc.* 1991, *113*:4000, all hereby incorporated by reference herein. Thus, in a preferred embodiment, the invention features a nucleic acid catalyst having one or more non-nucleotide moieties, and having enzymatic activity to cleave an RNA or DNA molecule. By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine. The terms "abasic" or "abasic nucleotide" as used herein encompass sugar moieties lacking a base or having other chemical groups in place of base at the 1' position.

In preferred embodiments, the enzymatic nucleic acid includes one or more stretches of RNA, which provide the enzymatic activity of the molecule, linked to the non-nucleotide moiety. The necessary RNA components are known in the art, see, *e.g.,* Usman, *supra.* By RNA is meant a molecule comprising at least one ribonucleotide residue.

As the term is used in this application, non-nucleotide-containing enzymatic nucleic acid means a nucleic acid molecule that contains at least one non-nucleotide component which replaces a portion of a ribozyme, *e.g.,* but not limited to, a double-stranded stem, a single-stranded "catalytic core" sequence, a single-stranded loop or a single-stranded recognition sequence. These molecules are able to cleave (preferably, repeatedly cleave) separate RNA or DNA molecules in a nucleotide base sequence specific manner. Such molecules can also act to cleave intramolecularly if that is desired. Such enzymatic molecules can be targeted to virtually any RNA transcript.

The specific nucleic acid catalysts described in the instant application are not limiting in the invention and those skilled in the art will recognize that all that is important in a nucleic acid catalyst of this invention is that it has a specific substrate binding site (*e.g.,* M and/or Q of Formulae III-VII above) which is complementary to one or more of the target nucleic acid regions, and that it have nucleotide sequences within or surrounding that substrate binding site which impart a nucleic acid cleaving activity to the molecule.

### Vector Expression of Enzymatic Nucleic Acid

The nucleic acid catalysts of the instant invention can be expressed within cells from eukaryotic promoters (*e.g.,* Izant and Weintraub, 1985 *Science* 229, 345; McGarry and Lindquist, 1986 *Proc. Natl. Acad. Sci.*USA 83, 399; Scanlon *et al.,* 1991, *Proc. Natl. Acad. Sci.USA,* 88, 10591-5; Kashani-Sabet *et al.,* 1992 *Antisense Res. Dev.,* 2, 3-15; Dropulic *et al.,* 1992 *J. Virol,* 66, 1432-41; Weerasinghe *et al.,* 1991 *J. Virol,* **65,** 5531-4; Ojwang *et al.,* 1992 *Proc. Natl. Acad. Sci.USA* 89, 10802-6; Chen *et al.,* 1992 *Nucleic Acids Res.,* 20, 4581-9; Sarver *et al.,* 1990 *Science* 247, 1222-1225; Thompson *et al.,* 1995 *Nucleic Acids Res.* 23, 2259; Good *et al.,* 1997, *Gene* Therapy, 4, 45; all of the references are hereby incorporated in their totality by reference herein). Those skilled in the art realize that any nucleic acid can be expressed in eukaryotic cells from the appropriate DNA/RNA vector. The activity of such nucleic acids can be augmented by their release from the primary transcript by a ribozyme (Draper *et al.,* PCT WO 93/23569, and Sullivan *et al.,* PCT WO 94/02595; Ohkawa *et al.,* 1992 *Nucleic Acids Symp. Ser.,* 27, 15-6; Taira *et al.,* 1991, *Nucleic Acids Res.,* 19, 5125-30; Ventura *et al.,* 1993 *Nucleic Acids Res.,* 21, 3249-55; Chowrira *et al.,* 1994 *J. Biol. Chem.* 269, 25856; all of the references are hereby incorporated in their totality by reference herein).

In another aspect of the invention, nucleic acid catalysts that cleave target molecules are expressed from transcription units (see for example Figure 11) inserted into DNA or RNA vectors. The recombinant vectors are preferably DNA plasmids or viral vectors. Ribozyme expressing viral vectors could be constructed based on, but not limited to, adeno-associated virus, retrovirus, adenovirus, or alphavirus. Preferably, the recombinant vectors capable of expressing the ribozymes are delivered as described above, and persist in target cells. Alternatively, viral vectors may be used that provide for transient expression of ribozymes. Such vectors might be repeatedly administered as necessary. Once expressed, the ribozymes cleave the target mRNA. The active ribozyme contains an enzymatic center or core equivalent to those in the examples, and binding arms able to bind target nucleic acid molecules such that cleavage at the target site occurs. Other sequences may be present which do not interfere with such cleavage. Delivery of ribozyme expressing vectors could be systemic, such as by intravenous or intramuscular administration, by administration to target cells ex-planted from the patient followed by reintroduction into the patient, or by any other means that would allow for introduction into the desired target cell (for a review see Couture and Stinchcomb, 1996, *TIG.,* 12, 510).

In a preferred embodiment, an expression vector comprising nucleic acid sequence encoding at least one of the nucleic acid catalyst of the instant invention is disclosed. The nucleic acid sequence encoding the nucleic acid catalyst of the instant invention is operable linked in a manner which allows expression of that nucleic acid molecule.

In one embodiment, the expression vector comprises: a transcription initiation region (*e.g*., eukaryotic pol I, II or III initiation region); b) a transcription termination region (*e.g*., eukaryotic pol I, II or III termination region); c) a gene encoding at least one of the nucleic acid catalyst of the instant invention; and wherein said gene is operably linked to said initiation region and said termination region, in a manner which allows expression and/or delivery of said nucleic acid molecule. The vector may optionally include an open reading frame (ORF) for a protein operably linked on the 5' side or the 3'-side of the gene encoding the nucleic acid catalyst of the invention; and/or an intron (intervening sequences).

Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990 *Proc. Natl. Acad. Sci.US A,* 87, 6743-7; Gao and Huang 1993 *Nucleic Acids Res.,* 21, 2867-72; Lieber et al., 1993 *Methods Enzymol.,* 217, 47-66; Zhou et al., 1990 *Mol. Cell. Biol.,* 10, 4529-37). Several investigators have demonstrated that ribozymes expressed from such promoters can function in mammalian cells (e.g., Kashani-Sabet et al., 1992 *Antisense Res. Dev.,* 2, 3-15; Ojwang et al., 1992 *Proc. Natl. Acad. Sci.U S A,* 89, 10802-6; Chen *et al.,* 1992 *Nucleic Acids Res.,* 20, 4581-9; Yu et al., 1993 *Proc. Natl. Acad. Sci.U S A,* 90, 6340-4; L'Huillier *et al.,* 1992 *EMBO J.* 11, 4411-8; Lisziewicz *et al.,* 1993 *Proc. Natl. Acad. Sci.U. S. A.,* 90, 8000-4; Thompson *et al.,* 1995 *Nucleic Acids Res.* 23, 2259; Sullenger & Cech, 1993, *Science,* 262, 1566). More specifically, transcription units such as the ones derived from genes encoding U6 small nuclear (snRNA), transfer RNA (tRNA) and adenovirus VA RNA are useful in generating high concentrations of desired RNA molecules such as ribozymes in cells (Thompson *et al., supra;* Couture and Stinchcomb, *1996, supra;* Noonberg *et al*., 1994, *Nucleic Acid Res.,* 22, 2830; Noonberg *et al*., US Patent No. 5,624,803; Good *et al*., 1997, *Gene Ther.* 4, 45; Beigelman *et al*., International PCT Publication No. *WO 96*/*18736*; all of these publications are incorporated by reference herein. Examples of transcription units suitable for expression of ribozymes of the instant invention are shown in Figure 11. The above ribozyme transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture and Stinchcomb, *1996*, *supra).*

In a preferred embodiment an expression vector comprising nucleic acid sequence encoding at least one of the catalytic nucleic acid molecule of the invention, in a manner which allows expression of that nucleic acid molecule. The expression vector comprises in one embodiment; a) a transcription initiation region; b) a transcription termination region; c) a gene encoding at least one said nucleic acid molecule; and wherein said gene is operably linked to said initiation region and said termination region, in a manner which allows expression and/or delivery of said nucleic acid molecule. In another preferred embodiment the expression vector comprises: a) a transcription initiation region; b) a transcription termination region; c) an open reading frame; d) a gene encoding at least one said nucleic acid molecule, wherein said gene is operably linked to the 3'-end of said open reading frame; and wherein said gene is operably linked to said initiation region, said open reading frame and said termination region, in a manner which allows expression and/or delivery of said nucleic acid molecule. In yet another embodiment the expression vector comprises: a) a transcription initiation region; b) a transcription termination region; c) an intron; d) a gene encoding at least one said nucleic acid molecule; and wherein said gene is operably linked to said initiation region, said intron and said termination region, in a manner which allows expression and/or delivery of said nucleic acid molecule. In another embodiment, the expression vector comprises: a) a transcription initiation region; b) a transcription termination region; c) an intron; d) an open reading frame; e) a gene encoding at least one said nucleic acid molecule, wherein said gene is operably linked to the 3'-end of said open reading frame; and wherein said gene is operably linked to said initiation region, said intron, said open reading frame and said termination region, in a manner which allows expression and/or delivery of said nucleic acid molecule.

### Delivery of Nucleic Acid Catalysts:

In a preferred embodiment, the nucleic acid catalysts are added directly, or can be complexed with cationic lipids, packaged within liposomes, or otherwise delivered to smooth muscle cells. The RNA or RNA complexes can be locally administered to relevant tissues through the use of a catheter, infusion pump or stent, with or without their incorporation in biopolymers. Using the methods described herein, other nucleic acid catalysts that cleave target nucleic acid may be derived and used as described above. Specific examples of nucleic acid catalysts of the instant invention are provided below in the Tables and figures.

Sullivan, *et al., supra,* describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination is locally delivered by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Sullivan *et al*., supra and Draper *et al., supra* which have been incorporated by reference herein.

The present invention also includes pharmaceutically acceptable formulations of the compounds described. These formulations include salts of the above compounds, *e*.*g*., ammonium, sodium, calcium, magnesium, lithium, and potassium salts.

A pharmacological composition or formulation refers to a composition or formulation in a form suitable for administration, *e.g*., systemic administration, into a cell or patient, preferably a human. Suitable forms, in part, depend upon the use or the route of entry, for example oral, transdermal, or by injection. Such forms should not prevent the composition or formulation to reach a target cell (*i.e*., a cell to which the negatively charged polymer is desired to be delivered to). For example, pharmacological compositions injected into the blood stream should be soluble. Other factors are known in the art, and include considerations such as toxicity and forms which prevent the composition or formulation from exerting its effect.

By "systemic administration" is meant in vivo systemic absorption or accumulation of drugs in the blood stream followed by distribution throughout the entire body. Administration routes which lead to systemic absorption include, without limitations: intravenous, subcutaneous, intraperitoneal, inhalation, oral, intrapulmonary and intramuscular. Each of these administration routes expose the desired negatively charged polymers, *e.g*., NTP's, to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation which can facilitate the association of drug with the surface of cells, such as, lymphocytes and macrophages is also useful. This approach may provide enhanced delivery of the drug to target cells by taking advantage of the specificity of macrophage and lymphocyte immune recognition of abnormal cells, such as the cancer cells.

The invention also features the use of the a composition comprising surface-modified liposomes containing poly (ethylene glycol) lipids (PEG-modified, or long-circulating liposomes or stealth liposomes). These formulations offer an method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic *et al, Chem. Rev. 1995,* **95,** 2601-2627; Ishiwataet *al., Chem. Pharm. Bull. 1995,* **43,** 1005-1011). Such liposomes have been shown to accumulate selectively in tumors, presumably by extravasation and capture in the neovascularized target tissues (Lasic *et al., Science* 1995, **267,** 1275-1276; Oku *et al.,* 1995, *Biochim. Biophys. Acta,* **1238,** 86-90). The long-circulating liposomes enhance the pharmacokinetics and pharmacodynamics of drugs, particularly compared to conventional cationic liposomes which are known to accumulate in tissues of the MPS (Liu *et al., J. Biol. Chem.* 1995, **42,** 24864-24870; Choi *et al.,* International PCT Publication No. WO 96/10391; Ansell *et al.,* International PCT Publication No. WO 96/10390; Holland *et al.,* International PCT Publication No. WO 96/10392; all of these are incorporated by reference herein). Long-circulating liposomes are also likely to protect drugs from nuclease degradation to a greater extent compared to cationic liposomes, based on their ability to avoid accumulation in metabolically aggressive MPS tissues such as the liver and spleen. All of these references are incorporated by reference herein.

The present invention also includes compositions prepared for storage or administration which include a pharmaceutically effective amount of the desired compounds in a pharmaceutically acceptable carrier or diluent. Acceptable carriers or diluents for therapeutic use are well known in the pharmaceutical art, and are described, for example, in *Remington's Pharmaceutical Sciences,* Mack Publishing Co. (A.R. Gennaro edit. 1985) hereby incorporated by reference herein. For example, preservatives, stabilizers, dyes and flavoring agents may be provided. *Id.* at 1449. These include sodium benzoate, sorbic acid and esters of *p*-hydroxybenzoic acid. In addition, antioxidants and suspending agents may be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors which those skilled in the medical arts will recognize. Generally, an amount between 0.1 mg/kg and 100 mg/kg body weight/day of active ingredients is administered dependent upon potency of the negatively charged polymer. In a one aspect, the invention provides nucleic acid catalysts that can be delivered exogenously to specific cells as required.

Local ribozyme administration offers the advantages of achieving high tissue concentrations of ribozymes and limiting their exposure to catabolic and excretory mechanisms. Although local routes of administration provide access to pathologies involving a number of organ systems, systemic administration would make ribozyme treatment of several other major human diseases feasible.

It has been demonstrated that certain tissues accumulate oligonucleotides and/or oligonucleotide formulations following systemic administration. These tissues include sites of inflammation (Wu *et al*. 1993, *Cancer Res.* **53:** 3765-3767), solid tumors (Yuan *et al.* 1994, *Cancer Res.* **54:** 3352-3356), kidney (Cossum *et al.* 1993, *J. Pharmaco. and Exp. Ther.* **267:** 1181-1190), brain (Wu *et al.* 1996, *J. Pharmacol. Exp. Ther.* **276:** 206-11) and those rich in reticulo-endothelial cells (liver, spleen, lymphatics; Litzinger *et al.* 1994, *Biochim. Biophys. Acta* **1190**: 99-107; Agrawal *et al.* 1991, *Proc. Natl. Acad. Sci.USA* **88:** 7595-7599; Agrawal *et al.* 1995, *Clin. Pharmacology* **28:** 7-16; Sands *et al.* 1994, *Molecular Pharmacol.* **45:** 932-943; Saijo *et al.* 1994, *Oncology Research* **6:** 243-249).

The kidney, as well as organs of the reticulo-endothelial system (RES), are mainly responsible for clearance of ribozymes following intravenous (i.v.) administration. Diseases involving these tissues are good candidates for systemic ribozyme therapy by virtue of their tendency to accumulate ribozymes.

In one preferred embodiment, the invention features method of treating inflammation using ribozymes. Inflammatory processes underlie the pathology of a large number of human diseases. Many of these processes can be blocked by inhibiting the expression of inflammatory mediators and/or their receptors (Cohen *et al.* 1995, *Am. J. Med.* **99:** 45S-52S). Systemic administration of monoclonal antibodies specific to these mediators have been shown to be efficacious in animal models of rheumatoid arthritis, inflammatory bowel disease, and acute respiratory distress syndrome (Arend *et al.* 1990, *Arthritis and Rheumatism* **33:** 305-315). One potential way for systemic administration of ribozymes to impact systemic inflammatory disease is through inhibition of TNF-a production by macrophages. TNF-a has been shown participate in a variety of inflammatory processes and is produced mainly by macrophages which are known to accumulate cationic lipid-formulated ribozymes (Masahiro *et al.* 1990, *J*. *Immunology.* **144:** 1425-1431). Antimouse TNF- a ribozymes were effective in cell culture, thus, it may be possible that systemic delivery of ribozymes by a liposome formulation could be an effective therapeutic in the above mentioned inflammatory disease states.

In another preferred embodiment, the invention features methods of treating diseases involving RES using ribozymes. A number of studies have shown that systemically administered oligonucleotides distribute to RES tissues (liver, spleen and lymphatics). Several studies with cationic lipid complexed oligonucleotides have also shown specific biodistribution to these. Pathology involving the RES includes a number of infectious diseases of major importance, such as human immunodeficiency virus (HIV), mycobacterium infections including tuberculosis (TB), avium, and leprae (leprosy). These diseases are all associated with, for example, overproduction of interleukin-10 (IL-10), a potent immunosuppressive cytokine (Barnes *et al.* 1993, *Infect. Immun.* **61:** 3482-9). Some of these infections can potentially be ameliorated by administration of neutralizing antibodies to IL-10.

In yet another preferred embodiment, the invention features method of treating cancer using ribozymes. As evidence of the potential use of systemic oligonucleotides as anticancer agents, antisense phosphorothioates have been have been reported to exhibit antitumor efficacy in a murine model of Burkitt's lymphoma (Huang *et al*. 1995, *Mol. Med.* **1**: 647-658). The molecular targets of systemic antineoplastic ribozymes could include oncogenes, protooncogenes, or angiogenic factors and receptors. Although the link between oncogenes and tumorigenesis is now well established, the specific mutations that lead to activation of a proto-oncogene can be widely diverse. Upregulation of protooncogene products is also common in human cancer. Reducing the levels of these gene products may be beneficial in treatment of cancer. In addition, since many tumors are highly vascularized, angiogenic factors or receptors may provide good alternate or adjunct targets to oncogenes for the therapy of solid tumors and their metastases. Applicant, in a non-limiting example *infra,* show ribozymes targeting angiogenic mediators.

The potential number of molecular targets in cancer is quite large. Among these targets are oncogenes, protooncogenes, metalloproteinases, growth factors, and angiogenic factors. However, a common denominator in many forms of metastatic solid tumors is extensive vascularization of the tumor. As tumors exceed about 1 mm in diameter, they require neovascularization for continued growth (Gimbrone *et al*., 1972, *J. Exp. Med.,* 136, 261). In addition, the appearance of new blood vessels within a tumor correlates with the initiation of the process of metastasis (Martiny-Baron and Marmé, 1995). It is possible that by using a systemically administered ribozyme targeting a key player in the process of angiogenesis would reduce both primary tumor growth, tumor progression and tumor metastasis.

"Angiogenesis" refers to formation of new blood vessels from existing blood vessels which is an essential process in reproduction, development and wound repair.

"Tumor angiogenesis" refers to the induction of the growth of blood vessels from surrounding tissue into a solid tumor. Tumor growth and tumor metastasis are dependent on angiogenesis (for a review see Folkman, 1985, *Nature Med.* **1**: 27-31; Folkman 1990 *J. Natl. Cancer Inst.,* 82, 4; Folkman and Shing, 1992 *J. Biol. Chem.* 267, 10931).

"Tumor metastasis" refers to the transfer and/or migration of tumor cells, originating from a primary tumor, from one part of the body or organ to another. Most malignant tumors have the capacity to metastasize.

"Tumor" refers to a new growth of tissue wherein the cells multiply, divide and grow uncontrolled.

In a preferred embodiment, the invention features a method of treating non-hepatic ascites using ribozymes. Nonhepatic ascites or peritoneal fluid accumulation resulting from abdominal cancer and ovarian hyperstimulation syndrome (OHSS) can result in significant fluid loss from the intravascular space and hypovolemia. If ascites volumes are large, abdominal pain, hypovolemic hypotension, electrolyte abnormalities and respiratory difficulties can ensue. Thus, if ascites is left untreated, it can be life threatening. Evidence is now accumulating that nonhepatic ascites may be induced, at least in part, by vascular endothelial growth factor (VEGF). For this reason, nonhepatic ascites may be a potential therapeutic indication for ribozymes directed against vascular endothelial growth factor (VEGF) receptors delivered either systemically or regionally to the peritoneum.

Ovaries can be overstimulated by hormonal therapy during fertility treatment. As a result, women can experience ovarian hyperstimulation syndrome which is associated with grossly enlarged ovaries and extreme ascites fluid accumulation. This fluid accumulation is thought to be induced by the release of a vascular permeability agent which may interact with vessels of the peritoneal cavity leading to plasma extravasation. Abramov and co-workers (1997, *Fertil. Steril.* 67: 261) have shown that plasma VEGF levels are elevated in OHSS and return to normal upon resolution of the syndrome. An earlier study has shown that VEGF is elevated in the serum and follicular fluid of OHSS patients and that the source of this VEGF may be the luteinizing granulosa cells of the ovary (Krasnow et al., 1996, Fertil. Steril. 65: 552). McClure et al. (1994, Lancet 344, 235) concluded that VEGF is the key mediator of OHSS ascites production since rhVEGF increases OHSS ascites but not liver ascites and that this increase is reversible by rhVEGF antiserum. Thus, reducing the expression of VEGF receptors in the vasculature of the peritoneum may have a therapeutic benefit in OHSS by substantially reducing OHSS-stimulated ascites production. Since VEGF can interact with VEGF receptors on vessels throughout the peritoneum from ovarian release of VEGF into systemic circulation, systemic treatment may represent the best option for treating this syndrome.

Malignant ascites: Another form of ascites can be induced by malignancies of the peritoneum including breast, pancreatic, uterine and colorectal cancers. It is thought that certain cancers produce factors which influence peritoneal vascular permeability leading to plasma extravasation (Garrison et al., 1986; Ann. Surg. 203: 644; Garrison et al., 1987, J. Surg. Res. 42: 126; Nagy et al., 1993, Cancer Res. 53: 2631). Several solid tumors including some colorectal and breast carcinomas are known to secrete VEGF to recruit blood vessels for sustained growth and metastasis. This secreted VEGF may also serve to increase local vasculature permeability. In support of this hypothesis, Nagy et al. (*supra*) showed in mice that peritoneal fluid resulting from MOT and TTA3/St carcinomas contained elevated levels of VEGF whose concentration correlated directly with fluid accumulation and development of hyperpermeable microvessels. Therefore, ribozymes directed against VEGF receptors administered systemically may impact both the tumor growth and metastases of VEGF secreting tumors as well as ascites induced by VEGF interacting with the vasculature of the peritoneum.

### Strategies for Systemic Delivery

### Methods to enhance tissue accumulation

Tissue accumulation of ribozymes can be improved by formulation, conjugation, or further chemical stabilization of the ribozyme. Elimination due to glomerular filtration can be slowed by increasing the apparent molecular weight of the ribozyme, *e*.*g*., by liposome encapsulation or bioconjugation to PEG. Applicant has observed that the rate of catabolism can be slowed by a factor of 100 and lung accumulation increased 500 fold by formulation with DMRIE/DOPE reagents. Liposomal encapsulation is likely to have a similar effect on the rate of catabolism. The rate of clearance into non-target tissues could also be reduced by encapsulation into liposomes, provided that the liposomes were surface modified with PEG such that RES clearance were avoided. Increasing the rate of uptake by target tissues can also be enhanced, for example, by conjugation of cholesterol to the ribozymes. Applicant has also observed that in tissues of the RES, accumulation has been increased several hundred fold by complexation with a cationic lipid carrier.

### Sustained release as a means to increase exposure

Sustained or continuous delivery devices, such as ALZET® osmotic mini-pumps, may also enhance accumulation in target tissues by increasing exposure relative to bolus *i.v.* administration. Sustained delivery from ALZET® pumps has been shown to be an effective way of administering a phosphorothioate antisense molecule for inhibition of tumor growth in mice (Huang *et al.* 1995, *supra*). Applicant has observed that the rate of ribozyme catabolism in and rate of clearance from the circulation is concentration dependent and may relate to the equilibrium plasma protein binding of the ribozyme. Phosphorothioate DNA is rapidly cleared from circulation when its concentration exceeds the plasma protein binding constant, as is the case after *i.v.* bolus administration. Osmotic pumps administer oligonucleotides at a slower and constant rate, and therefore may maintain plasma levels near the equilibrium binding capacity. This would result in less of the administered dose being lost to glomerular filtration (elimination) and hepatic extraction (catabolism); more of the administered dose may be available for uptake into target tissues.

### Animal Models

### Use of murine models

For a typical systemic study involving 10 mice (20 g each) per dose group, 5 doses (1, 3, 10, 30 and 100 mg/kg daily over 14 days continuous administration), approximately 400 mg of ribozyme, formulated in saline would be used. A similar study in young adult rats (200 g) would require over 4 g. Parallel pharmacokinetic studies may involve the use of similar quantities of ribozymes further justifying the use of murine models.

### Ribozymes and Lewis lung carcinoma and B-16 melanoma murine models

Identifying a common animal model for systemic efficacy testing of ribozymes is an efficient way of screening ribozymes for systemic efficacy.

The Lewis lung carcinoma and B-16 murine melanoma models are well accepted models of primary and metastatic cancer and are used for initial screening of anti-cancer. These murine models are not dependent upon the use of immunodeficient mice, are relatively inexpensive, and minimize housing concerns. Both the Lewis lung and B-16 melanoma models involve subcutaneous implantation of approximately 10⁶ tumor cells from metastatically aggressive tumor cell lines (Lewis lung lines 3LL or D122, LLc-LN7; B-16-BL6 melanoma) in C57BL/6J mice. Alternatively, the Lewis lung model can be produced by the surgical implantation of tumor spheres (approximately 0.8 mm in diameter). Metastasis also may be modeled by injecting the tumor cells directly *i.v.*. In the Lewis lung model, microscopic metastases can be observed approximately 14 days following implantation with quantifiable macroscopic metastatic tumors developing within 21-25 days. The B-16 melanoma exhibits a similar time course with tumor neovascularization beginning 4 days following implantation. Since both primary and metastatic tumors exist in these models after 21-25 days in the same animal, multiple measurements can be taken as indices of efficacy. Primary tumor volume and growth latency as well as the number of micro- and macroscopic metastatic lung foci or number of animals exhibiting metastases can be quantitated. The percent increase in lifespan can also be measured. Thus, these models would provide suitable primary efficacy assays for screening systemically administered ribozymes/ribozyme formulations.

In the Lewis lung and B-16 melanoma models, systemic pharmacotherapy with a wide variety of agents usually begins 1-7 days following tumor implantation/inoculation with either continuous or multiple administration regimens. Concurrent pharmacokinetic studies can be performed to determine whether sufficient tissue levels of ribozymes can be achieved for pharmacodynamic effect to be expected. Furthermore, primary tumors and secondary lung metastases can be removed and subjected to a variety of *in vitro* studies (*i*.*e*., target mRNA reduction).

### Anti- VEGF receptor ribozymes

Sustained tumor growth and metastasis depend upon angiogenesis. In fact, the appearance of vessels in a growing tumor is correlated with the beginning of metastatic potential. Several studies have shown that antiangiogenic agents alone or in combination with cytotoxic agents reduce lung metastases and/or primary tumor volume in the Lewis lung and B-16 melanoma models (Borgstrom *et al.* 1995, *Anticancer Res.* **15:** 719-728; Kato *et al.* 1994, *Cancer Res.* **54**: 5143-5147; O'Reilly *et al.* 1994, *Cell* **79**: 315-328; Sato *et al.* 1995, *Jpn*. *J. Cancer Res.* **86:** 374-382).

A major factor implicated in the induction of solid tumor angiogenesis is vascular endothelial growth factor (VEGF; Folkman, 1995, *supra*). Several human tumors have been shown to synthesize and secrete. With regard to treating lung metastasis, VEGF and VEGF receptors of both subtypes and their expression are upregulated in the lung under conditions of hypoxia (Tuder *et al.* 1994, *J. Clin. Invest.* **95:** 1798-1807). This may lead to neovascularization which provides the means by which tumor cells gain access to circulation (Mariny-Baron and Marmé, 1995). Thus, VEGF and its receptors may be important targets in the treatment of metastatic disease.

Applicant has shown that a catalytically active ribozyme targeting *flt-1* RNA inhibits VEGF-induced neovascularization in a dose-dependent manner in a rat corneal model of angiogenesis. Testing with cytotoxic agents in combination with antiangiogenic ribozymes may also prove useful.

### Anti-K- and H-ras ribozymes

Mutations involving *ras* underlie a number of human cancers. *Ras* also plays a role in metastatic potential (Shekhar and Miller, 1994, *Invasion Metastasis* **14:** 27-37) and may do so, in part, by influencing endothelial cell migration (Fox *et al.* 1994, *Oncogene* **9:** 3519-26). With regard to lung cancer, *ras* has been shown to induce abnormal mitoses in lung fibroblasts (Lyubuski *et al.* 1994, *Cytobios* **80:** 161-178) and is a clinical marker in non-small cell lung tumors (Niklinski and Furman, 1995, *Eur. J*. *Cancer Prev.* **4**: 129-138). Studies in cells cultured from human small cell lung tumor xenografts demonstrated overexpression of K-*ras* (Arvelo *et al.* 1994, *Anticancer Res.* **14:** 1893-1901). This evidence provides ample support for the systemic testing of ribozymes directed against H- and K*-ras* in the murine cancer models (primary and secondary metastasis) discussed above.

Four of the current synthetic ribozymes directed against human K- *ras* will cleave homologous mouse K*-ras* targets at four sites and inhibit cultured rat aortic smooth muscle cell proliferation.

### Anti-c-fos ribozymes

The protein product of the proto-oncogene c-*fos* is a nuclear transcription factor which is involved in tumorigenesis. In support of the possible use of systemically administered ribozymes directed against c-*fos,* null mouse mutations of *c-fos* have been shown to result in viable mice. Using this mouse model, it has been shown that c-*fos* is important in malignant conversion of papillomas. Additionally, c-*fos* has been shown to up-regulate tumor metalloproteinases (Schonthal *et al.* 1988, *Cell* **54:** 325-334). It is possible that c-*fos* may play a role in tumor angiogenesis as evidenced by VEGF mRNA levels being significantly reduced in c-*fos* deficient tumors. It has also been shown that c-*fos* is highly expressed in some B-16 cell and human melanoma cell lines (Kroumpouzos *et al.* 1994, *Pigment Cell Res.* 7: 348-353; Nakayama *et al.* 1995, *J. Dermatol.* **22:** 549-559; Peris *et al.* 1991, *Arch. Dermatol. Res.* **283:** 500-505). The expression of c-*fos* may be directly proportional to metastatic potential in B-16 melanoma cell lines. With this evidence, it is reasonable to conclude that c-*fos* represents a suitable systemic ribozyme target in either the Lewis lung, B-16 melanoma, or human melanoma models.

### Delivery of ribozymes and ribozyme formulations in the Lewis lung model

Several ribozyme formulations, including cationic lipid complexes which may be useful for inflammatory diseases (*e.g.,* DIMRIE/DOPE, *etc.*) and RES evading liposomes which may be used to enhance vascular exposure of the ribozymes, are of interest in cancer models due to their presumed biodistribution to the lung. Thus, liposome formulations can be used for delivering ribozymes to sites of pathology linked to an angiogenic response.

The sequences of the ribozymes that are chemically synthesized, useful in this study, are non-limiting examples. Those in the art will recognize that these sequences are representative only of many more such sequences where the enzymatic portion of the ribozyme (all but the binding arms) is altered to affect activity. For example, stem-loop II sequence of hammerhead ribozymes can be altered (substitution, deletion, and/or insertion) to contain any sequences provided a minimum of two base-paired stem structure can form. Similarly, stem-loop IV sequence of hairpin ribozymes listed in Tables IV (5'-CACGUUGUG-3') can be altered (substitution, deletion, and/or insertion) to contain any sequence, provided a minimum of two base-paired stem structure can form. Preferably, no more than 200 bases are inserted at these locations. The sequences listed in Tables III and IV may be formed of ribonucleotides or other nucleotides or non-nucleotides. Such ribozymes (which have enzymatic activity) are equivalent to the ribozymes described specifically in the Tables.

### Target sites

Targets for useful ribozymes can be determined as disclosed in Draper *et al*., WO 93/23569; Sullivan *et al*., WO 93/23057; Thompson *et al*., WO 94/02595; Draper *et al*., WO 95/04818; McSwiggen *et al*., US Patent No. 5,525,468 and hereby incorporated by reference herein in totality. Rather than repeat the guidance provided in those documents here, below are provided specific examples of such methods, not limiting to those in the art. Ribozymes to such targets are designed as described in those applications and synthesized to be tested *in vitro* and *in vivo,* as also described. Such ribozymes can also be optimized and delivered as described therein.

The sequence of human *c-raf* mRNAs were screened for optimal ribozyme target sites using a computer folding algorithm. Hammerhead or hairpin ribozyme cleavage sites were identified. These sites are shown in Tables XII-XIX (All sequences are 5' to 3' in the tables) The nucleotide base position is noted in the Tables as that site to be cleaved by the designated type of ribozyme. The nucleotide base position is noted in the Tables as that site to be cleaved by the designated type of ribozyme.

Because Raf RNAs are highly homologous in certain regions, some ribozyme target sites are also homologous **(see Table XVIII and XIX).** In this case, a single ribozyme will target different classes of Raf RNA. The advantage of one ribozyme that targets several classes of Raf RNA is clear, especially in cases where one or more of these RNAs may contribute to the disease state.

Hammerhead or hairpin ribozymes were designed that could bind and were individually analyzed by computer folding (Jaeger *et al.,* 1989 *Proc. Natl. Acad. Sci. USA,* 86, 7706) to assess whether the ribozyme sequences fold into the appropriate secondary structure. Those ribozymes with unfavorable intramolecular interactions between the binding arms and the catalytic core are eliminated from consideration. Varying binding arm lengths can be chosen to optimize activity. Generally, at least 5 bases on each arm are able to bind to, or otherwise interact with, the target RNA. Ribozymes of the hammerhead or hairpin motif were designed to anneal to various sites in the mRNA message. The binding arms are complementary to the target site sequences described above.

### Examples

The following are non-limiting examples showing the selection, isolation, synthesis and activity of enzymatic nucleic acids of the instant invention.

The following examples demonstrate the selection of ribozymes that cleave c-raf RNA. The methods described herein represent a scheme by which ribozymes may be derived that cleave other RNA targets required for cell division. Also provided is a description of how such ribozymes may be delivered to cells. The examples demonstrate that upon delivery, the ribozymes inhibit cell proliferation in culture and modulate gene expression *in vivo.* Moreover, significantly reduced inhibition is observed if mutated ribozymes that are catalytically inactive are applied to the cells. Thus, inhibition requires the catalytic activity of the ribozymes.

### Example 1: Identification of Potential Ribozyme Cleavage Sites in Human c-raf RNA

The sequence of human c-raf RNA was screened for accessible sites using a computer folding algorithm. Regions of the mRNA that did not form secondary folding structures and contained potential hammerhead and/or hairpin ribozyme cleavage sites were identified. The sequences of these cleavage sites are shown in **tables XII-XIX.**

### Example 2: Selection of Ribozyme Cleavage Sites in Human c-raf RNA

To test whether the sites predicted by the computer-based RNA folding algorithm corresponded to accessible sites in c-raf RNA, 20 hammerhead sites were selected for analysis, Ribozyme target sites were chosen by analyzing genomic sequences of human c-raf (GenBank Accession No. X03484; Bonner *et al.,* 1986, *Nucleic Acids Research, 14,* 1009-1015) and prioritizing the sites on the basis of folding. Hammerhead ribozymes were designed that could bind each target (see Figure 1) and were individually analyzed by computer folding (Christoffersen *et al*., 1994 *J. Mol. Struc. Theochem,* 311, 273; Jaeger *et al*., 1989, *Proc. Natl. Acad. Sci. USA*, **86**, 7706) to assess whether the ribozyme sequences fold into the appropriate secondary structure. Those ribozymes with unfavorable intramolecular interactions between the binding arms and the catalytic core were eliminated from consideration. As noted below, varying binding arm lengths can be chosen to optimize activity. Generally, at least 5 bases on each arm are able to bind to, or otherwise interact with, the target RNA. Ribozyme target sites within A-Raf were chosen by analyzing genomic sequences of human A-raf-1 (GenBank Accession No. X04790; Beck *et al.,* 1987, *Nucleic Acids Research,* 115, 595-609). Ribozyme target sites within B-Raf were chosen by analyzing genomic sequences of human B-raf-1 (GenBank Accession No. M95712 M95720 X54072; Sitanandam *et al.,* 1990, *Oncogene, 5,* 1775-1780).

### Example 3: Chemical Synthesis and Purification of Ribozymes for Efficient Cleavage of c-raf RNA

Ribozymes of the hammerhead or hairpin motif were designed to anneal to various sites in the RNA message. The binding arms are complementary to the target site sequences described above. The ribozymes were chemically synthesized. The method of synthesis used followed the procedure for normal RNA synthesis as described in Usman et al., (1987 J. Am. Chem. Soc., 109, 7845), Scaringe et al., (1990 Nucleic Acids Res., 18, 5433) and Wincott et al., supra, and made use of common nucleic acid protecting and coupling groups, such as dimethoxytrityl at the 5'-end, and phosphoramidites at the 3'-end. The average stepwise coupling yields were >98%.

Inactive ribozymes were synthesized by substituting a U for G5 and a U for A14 (numbering from Hertel et al., 1992 Nucleic Acids Res., 20, 3252). Hairpin ribozymes were synthesized in two parts and annealed to reconstruct the active ribozyme (Chowrira and Burke, 1992 Nucleic Acids Res., 20, 2835-2840). Ribozymes were also synthesized from DNA templates using bacteriophage T7 RNA polymerase (Milligan and Uhlenbeck, 1989, Methods Enzymol. 180, 51). Ribozymes were modified to enhance stability by modification with nuclease resistant groups, for example, 2'-amino, 2'-C-allyl, 2'-flouro, 2'-O-methyl, 2'-H (for a review see Usman and Cedergren, 1992 TIBS 17, 34). Ribozymes were purified by gel electrophoresis using general methods or were purified by high pressure liquid chromatography (HPLC; See Wincott et al., supra; the totality of which is hereby incorporated herein by reference) and were resuspended in water. The sequences of the chemically synthesized ribozymes used in this study are shown below in **Table XII-XIX.**

### Example 4: Ribozyme Cleavage of c-raf RNA Target in vitro

Ribozymes targeted to the human c-raf RNA are designed and synthesized as described above. These ribozymes can be tested for cleavage activity *in vitro,* for example using the following procedure. The target sequences and the nucleotide location within the c-raf mRNA are given in Table XII.

*Cleavage Reactions:* Full-length or partially full-length, internally-labeled target RNA for ribozyme cleavage assay is prepared by *in vitro* transcription in the presence of [a-³²P] CTP, passed over a G 50 Sephadex column by spin chromatography and used as substrate RNA without further purification. Alternately, substrates are 5'-³²P-end labeled using T4 polynucleotide kinase enzyme. Assays are performed by pre-warming a 2X concentration of purified ribozyme in ribozyme cleavage buffer (50 mM Tris-HCl, pH 7.5 at 37°C, 10 mM MgCl₂) and the cleavage reaction was initiated by adding the 2X ribozyme mix to an equal volume of substrate RNA (maximum of 1-5 nM) that was also pre-warmed in cleavage buffer. As an initial screen, assays are carried out for 1 hour at 37°C using a final concentration of either 40 nM or 1 mM ribozyme, *i.e.,* ribozyme excess. The reaction is quenched by the addition of an equal volume of 95% formamide, 20 mM EDTA, 0.05% bromophenol blue and 0.05% xylene cyanol after which the sample is heated to 95°C for 2 minutes, quick chilled and loaded onto a denaturing polyacrylamide gel. Substrate RNA and the specific RNA cleavage products generated by ribozyme cleavage are visualized on an autoradiograph of the gel. The percentage of cleavage is determined by Phosphor Imager® quantitation of bands representing the intact substrate and the cleavage products.

### Example 5: Ability of c-raf Ribozymes to Inhibit Smooth Muscle Cell Proliferation.

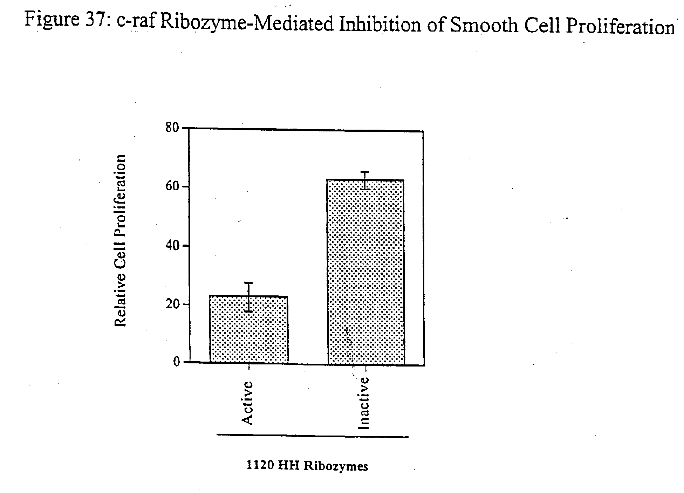

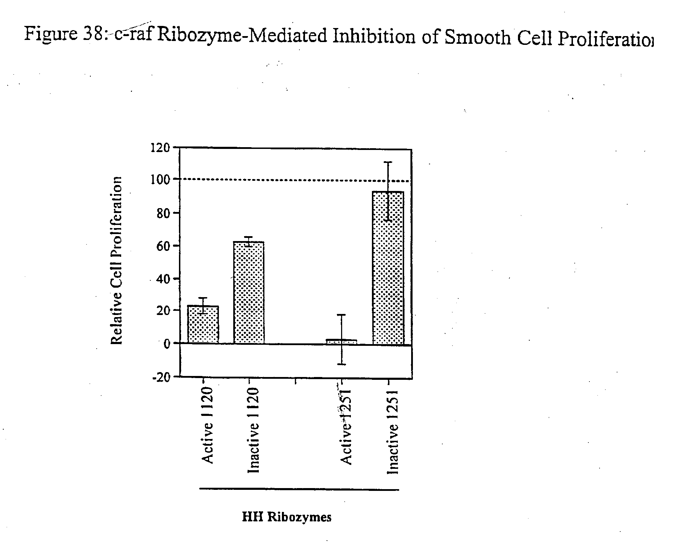

Ribozymes targeting sites in c-Raf mRNA were synthesized using modifications that confer nuclease resistance (Beigelman, 1995, *J. Biol. Chem.* 270, 25702). The ribozymes were screened for their ability to inhibit cell proliferation in serum-starved primary rat aortic smooth muscle cells as described by Jarvis et al. (1996, *RNA* 2, 419; incorporated by reference herein). The ribozyme targeting site represented by Seq ID Nos **175** and **198** showed particularly high activity in inhibiting cell proliferation. An inactive control ribozyme was synthesized which had identical substrate binding arms but contained mutations in the catalytic core that eliminate cleavage activity. Inhibition of cell proliferation by active versus inactive c-Raf ribozymes is shown in **Figures 37 and 38**. The data are presented as proliferation relative to the serum-stimulated untreated control cells. Clearly the active ribozyme is showing substantial inhibition relative to both the untreated control and its corresponding inactive control, thus indicating that the inhibition of proliferation is mediated by ribozyme-mediated cleavage of c-Raf.

In several other systems, cationic lipids have been shown to enhance the bioavailability of oligonucleotides to cells in culture (Bennet, C. F., et al., 1992, *Mol. Pharmacology*, **41**, 1023-1033). In many of the following experiments, ribozymes were complexed with cationic lipids. The cationic lipid, Lipofectamine (a 3:1 (w/w) formulation of DOSPA (2,3-dioleyloxy-N-[2(sperminecarboxamido)ethyl]-N,N-dimethyl-1-propanaminium trifluoroacetate) and dioleoyl phosphatidylethanolamine (DOPE)), was purchased from Life Technologies, Inc. DMRIE (N-[1-(2,3-ditetradecyloxy)propyl]-*N,N*-dimethyl-*N*-hydroxyethylammonium bromide) was obtained from VICAL. DMRIE was resuspended in CHCl₃ and mixed at a 1:1 molar ratio with dioleoyl phosphatidylethanolamine (DOPE). The CHCl₃ was evaporated, the lipid was resuspended in water, vortexed for 1 minute and bath sonicated for 5 minutes. Ribozyme and cationic lipid mixtures were prepared in serum-free DMEM immediately prior to addition to the cells. DMEM plus additives was warmed to room temperature (about 20-25°C), cationic lipid was added to the final desired concentration and the solution was vortexed briefly. RNA oligonucleotides were added to the final desired concentration and the solution was again vortexed briefly and incubated for 10 minutes at room temperature. In dose response experiments, the RNA/lipid complex was serially diluted into DMEM following the 10 minute incubation.

Serum-starved smooth muscle cells were washed twice with PBS, and the RNA/lipid complex was added. The plates were incubated for 4 hours at 37°C. The medium was then removed and DMEM containing 10% FBS, additives and 10 µM bromodeoxyuridine (BrdU) was added. In some wells, FBS was omitted to determine the baseline of unstimulated proliferation.

The plates were incubated at 37°C for 20-24 hours, fixed with 0.3% H₂O₂ in 100% methanol, and stained for BrdU incorporation by standard methods. In this procedure, cells that have proliferated and incorporated BrdU stain brown; non-proliferating cells are counter-stained a light purple. Both BrdU positive and BrdU negative cells were counted under the microscope. 300-600 total cells per well were counted. In the following experiments, the percentage of the total cells that have incorporated BrdU (% cell proliferation) is presented. Errors represent the range of duplicate wells. Percent inhibition then is calculated from the % cell proliferation values as follows: % inhibition = 100 - 100((Ribozyme - 0% serum)/(Control - 0% serum)).

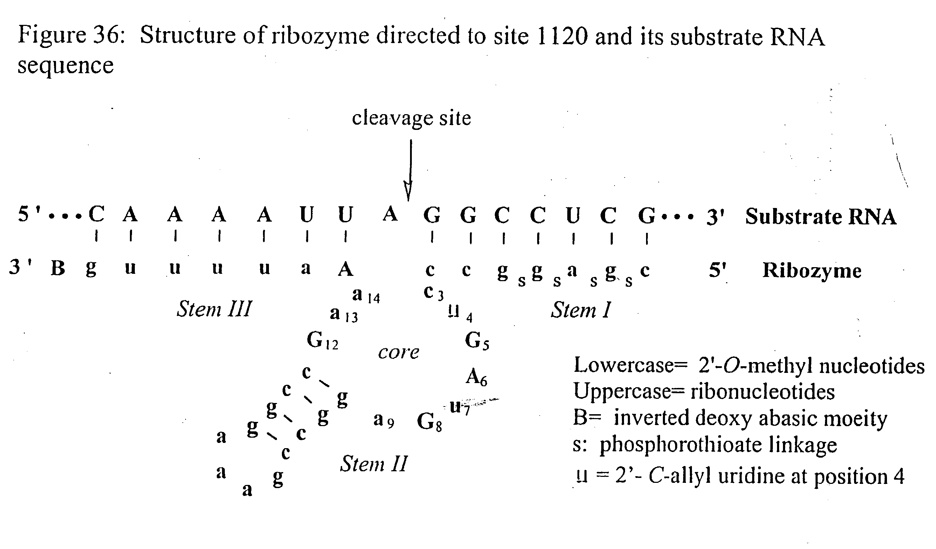

From this initial screen, hammerhead ribozyme targeted against c-raf site 1120 (Figure 36) was further tested. The active ribozyme was able to inhibit proliferation of smooth muscle cell, whereas, the control inactive ribozyme, that cannot cleave c-raf RNA due to alterations in their catalytic core sequence, fails to inhibit smooth muscle cell proliferation (Figure 37). Thus, inhibition of cell proliferation by these hammerhead sequences is due to their ability to cleave c-raf RNA, and not because of any non-ribozyme activity.

### Example 6: Oligonucleotide design and preparation for cloning Defined and Random Libraries

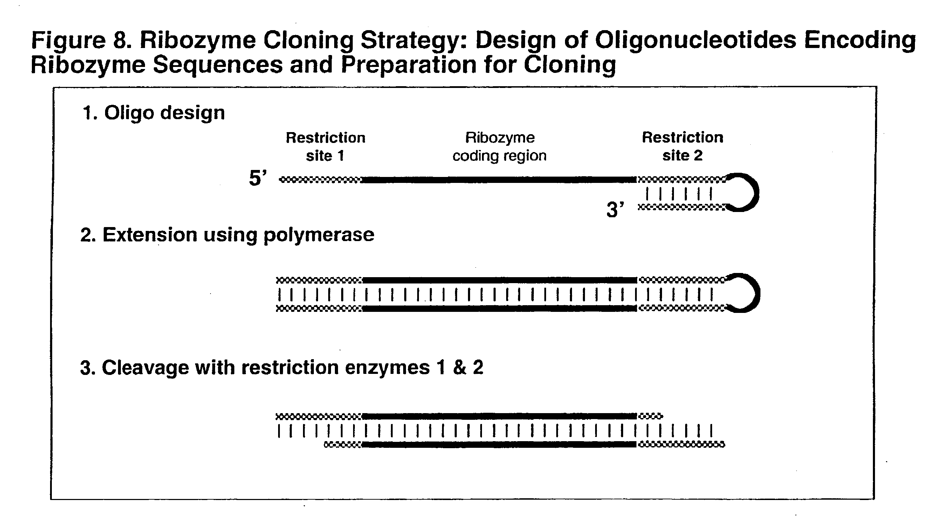

The DNA oligonucleotides used in this study to construct Defined and Random Ribozyme Libraries were purchased from Life Technologies (BRL). A schematic of the oligonucleotide design used to construct said Defined or Comprehensive Ribozyme Libraries is shown in Figure 8. This example is meant to illustrate one possible means to construct such libraries. The methods described herein are not meant to be inclusive of all possible methods for constructing such libraries. The oligonucleotides used to construct the hammerhead ribozyme libraries were designed as follows:

5'-CGAAATCAATTG-(N1)X-{CatalyticCore}-(N2)X-CGTACGACACGAAAGTATCG-3'

Where N1 = the Stem I target-specific binding arm of length x, Catalytic Core = the hammerhead catalytic domain 5'-CTGATGAGGCCGUUAGGCCGAAA-3', and N2 = the Stem III target specific binding arm of length x. The oligonucleotides were designed to self-prime via formation of a stem-loop structure encoded at the 3' ends of the oligos (Figure 8A). This intramolecular interaction favored an unbiased extension of complex pools of ribozyme-encoding oligonucleotides. In the case of Defined Ribozyme Library described below (Figures 9-10), N1 and N2 were 8 nt each and were designed to be complimentary to the RNA encoded by the purine nucleoside phosphorylase (PNP) gene. In the case of Random Ribozyme Libraries, N1 and N2 were randomized during synthesis to produce a single pool of all possible hammerhead ribozymes.

In the example shown (Figures 9-10), oligonucleotides encoding 40 different PNP-specific hammerhead ribozymes (greater than 40 ribozymes can be used) were pooled to a final concentration of 1 µM total oligonucleotides (2.5 nM each individual oligo). Oligos were heated to 68°C for 30 min and then cooled to ambient temperature to promote formation of the 3' stem-loop for self-priming (Figure 8A). The 3' stem loop was extended (Figure 8B) using Klenow DNA polymerase (1 µM total oligonucleotides in 1 ml of 50 mM Tris pH 7.5, 10mM MgCl2, 100 µg/ml BSA. 25 µg M dNTP mix, and 200 U Klenow) by incubating for 30 min at 37°C. The reaction mixtures were then heated to 65°C for 15 min to inactivate the polymerase. The double-stranded oligos (approximately 30 µg) were digested with the 100 U of the 5' restriction endonuclease Mfe I (NEB) as described by the manufacturer, then similarly digested with the 3' restriction endonuclease BsiWI (Figure 8C). To reduce the incidence of multiple ribozyme inserts during the cloning steps, the cleaved products were treated with Calf Intestinal Phosphatase (CIP, Boehringer Mannheim) as described by the manufacturer to remove the phosphate groups at the 5' ends. This step inhibits intra- and intermolecular ligation of the ribozyme-encoding fragments. Full-length product corresponding to the double-stranded, restriction digested and phosphatase-treated products was gel-purified following electrophoresis through 10% non-denaturing acrylamide gels prior to cloning to enrich for full-length material.

### Example 7: Cloning of Defined and Random Libraries

The cloning vectors used contained the following cloning sites: 5'- MfeI - Cla I - BsiWI -3'. Vectors were digested with Mfe I and BsiWI prior to use. Thus, vectors cleaved with both enzymes should lack the Cla I site present between the sites, while vectors cleaved with only one of the enzymes should still retain the Cla I site. Pooled oligos were ligated to vector using a 2:1 or 5:1 molar ratio of double-stranded oligo to vector in 50-mL reactions containing 500 ng vector and 5 U ligase in 1x ligase buffer (Boehringer Mannheim). Ligation reactions were incubated over night at 16°C, then heated to 65°C 10 min to inactivate the ligase enzyme. The desired products contain a single ribozyme insert and lack the original Cla I site included between the Mfe I and BsiWI cloning sites. Any unwanted, background vector lacking ribozyme inserts and thus still containing the Cla I sites were inactivated by cleaving the product with 5 U of the restriction endonuclease Cla I for 1 h at 37°C. Approximately 150 ng of ligated vector was used to transform 100 µl XL-2 Blue competent bacteria as described by the supplier (Stratagene).

### Example 8: Simultaneous screening of 40 different ribozymes targeting PNP using Defined Ribozyme Libraries.

A Defined Ribozyme Library containing 40 different hammerhead ribozymes targeting PNP was constructed as described above (Figures 8-10). PNP is an enzyme that plays a critical role in the purine metabolic/salvage pathways. PNP was chosen as a target because cells with reduced PNP activity can be readily selected from cells with wild-type activity levels using the drug 6-thioguanosine. This agent is not toxic to cells until it is converted to 6-thioguanine by PNP. Thus, cells with reduced PNP activity are more resistant to this drug and can be selectively grown in concentrations of 6-thioguanosine that are toxic to cells with wild-type activity levels.

The PNP-targeted Defined Ribozyme Library expression vectors were converted into retroviral vector particles, and the resulting particles were used to transduce the Sup T1 human T cell line. A T-cell line was chosen for study because T lymphocytes are more dependent on the purine salvage pathway and thus are highly susceptible to 6-thioguanosine killing. Two weeks after transduction, the cells were challenged with 10 mmol 6-thioguanosine. Resistant cells began to emerge two weeks after initiation of selection. 6-Thioguanosine-resistant cells were harvested, and the ribozyme-encoding region of the expression vector was amplified using PCR and sequenced. The sequence pattern of the ribozyme region in the selected cells was significantly different from that produced from the starting library shown in Figure 9. In the original library, sequences of the binding arms were ambiguous due to the presence of all 40 PNP-targeted ribozymes (Figure 9). However, the sequence of the ribozyme-encoding regions from the 6-thioguanosine selected cells was clearly weighted towards one of the ribozymes contained in the original pool - the ribozyme designed to cleave at nucleotide #32 of PNP mRNA. These data suggests that the ribozyme targeting position 32 of the PNP mRNA appears to be more active than the other 39 PNP-targeted ribozymes included in the pool.

### Example 9: Optimizing Loop II sequence of a Hammerhead Ribozyme (HH-B) for Enhanced Catalytic Rates

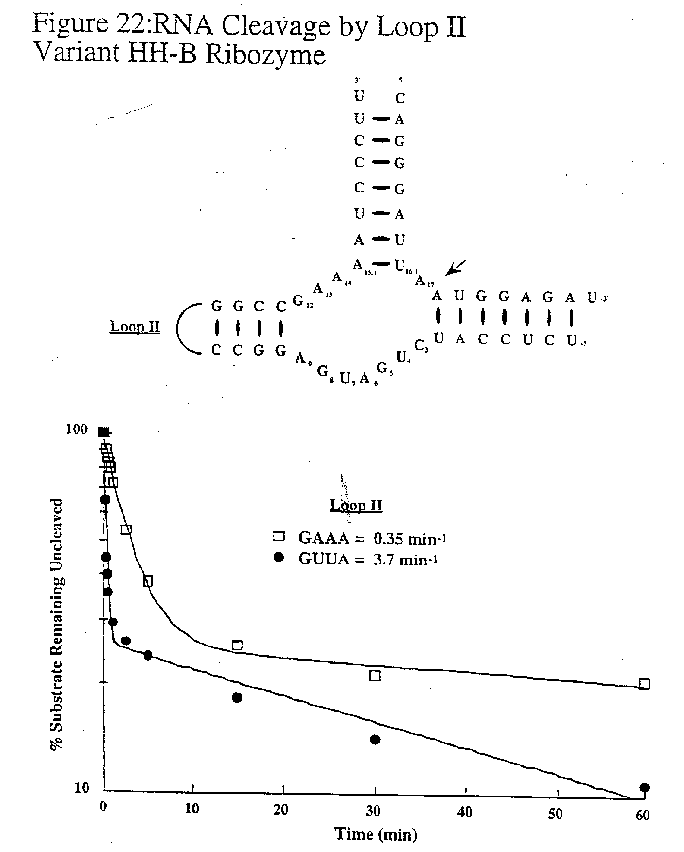

To test the feasibility of the combinatorial approach described in Figure 12 approach, Applicant chose to optimize the sequence of loop-II of a hammerhead ribozyme (HH-B) (see Figure 22). Previous studies had demonstrated that a variety of chemical modifications and different sequences within loop-II may have significant effects on the rate of cleavage *in vitro,* despite the fact that this sequence is not phylogenetically conserved and can in fact be deleted completely. According to the standard numbering system for the hammerhead ribozyme, the four positions within loop II are numbered 12.1, 12.2, 12.3, and 12.4. The Starting Ribozyme (HH-B) contained the sequence G_{12.1} A_{12.2} A _{12.3} A _{12.4}. For simplicity, the four positions will be numbered 5' to 3': G_{12.1}= 1; A_{12.2}= 2; A _{12.3}=3; A _{12.4}= 4. The remainder of the hammerhead ribozyme "template" remained constant and is based on a previously described hammerhead motif (Draper *et al*., International PCT Publication No. WO 95/13380, incorporated by reference herein).

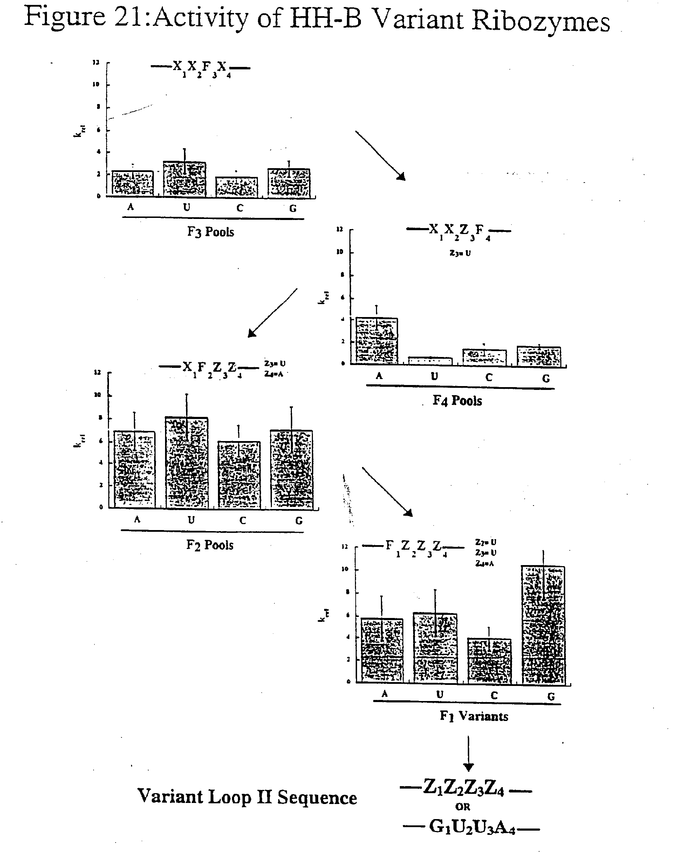

A strategy for optimizing the four (number of Classes = 4) loop-II positions is illustrated in Figure 180. The four standard ribose nucleotides (A, C, U and G) were chosen to construct the ribozyme pools (n = 4). In the first step, four different pools were synthesized by the nucleotide building block mixing approach described herein. Applicant first chose to "fix" (designated F) position 3 because preliminary experiments indicated that the identity of the base at this position had the most profound effects on activity; positions 1, 2 and 4 are random. The four pools were assayed under stoichiometric conditions (1µM ribozyme; 1µM substrate), to help ensure that the entire population of ribozymes in each pool was assayed. Substrate and ribozyme were pre-annealed and the reactions were initiated with the addition of 10mM MgCl₂. The rate of cleavage for each library was derived from plots of fraction of substrate cleaved as a function of time. Reactions were also performed simultaneously with the starting ribozyme (i.e., homogenous, loop-II = GAAA). The relative rate of cleavage for each library (kᵣₑₗ) was calculated by dividing the observed rate of the library by the rate of the control/starting ribozyme and is plotted in Figure 21. The error bars indicate the standard error derived from the curve fits. The results show that all four pools had similar rates (kᵣₑₗ); however, the library possessing "U" at position 3 was slightly faster.

Ribozyme pools were again synthesized (Class 2) with position 3 being made constant (U₃), position 4 was fixed (F₄) and positions 1 and 2 were random (X). The four pools were assayed as before; the pool containing "A" at position 4 was identified as the most desirable pool. Therefore, during the synthesis of the next pool (Class 3), positions 3 and 4 were constant with U₃ and A₄, position 2 was fixed (F₂) and position 1 was random (X). The four pools were again assayed; all four pools showed very similar, but substantially elevated rates of cleavage. The pool containing U at position 2 was identified as the fastest. Therefore, during the synthesis of the final four ribozymes (Class 4), position 3, 4 and 2 were made constant with U₃, A₄ and U₂; position 1 was fixed with A, U, C or G. The final ribozyme containing G at position 4 was clearly identified as the fastest ribozyme, allowing the identification of G_{12.1} U_{12.2} U_{12.3} A_{12.4} as the optimized ribozyme motif.

To confirm that the final ribozyme (G_{12.1} U_{12.2} U_{12.3} A_{12.4}) was indeed faster that the starting ribozyme (G_{12.1} A_{12.2} A_{12.3} A_{12.4}), we compared the two ribozymes (illustrated in Figure 22) under single-turnover conditions at saturating ribozyme concentrations. The observed rates should therefore measure the rate of the chemical step, k₂. The fraction of substrate remaining uncleaved as a function of time is shown in Figure 22 (lower panel), and the derived rate contents are shown. The results show that the optimized ribozyme cleaves >10 times faster (3.7 min⁻¹ vs. 0.35 min⁻¹) than the starting ribozyme.

### Example 10: Optimizing Core Chemistry of a Hammerhead Ribozyme (HH-A)

To further test the feasibility of the approach described in Figure 12, we chose to optimize the three pyrimidine residues within the core of a hammerhead ribozyme (HH-A). These three positions (shown in Figure 13 as U7, U4 and C3) were chosen because previous studies indicated that these positions are critical for both stability (Beigelman *et al*., 1995, *supra*) and activity (Ruffner *et al*.,, 1990, *supra;* Burgin *et al*., 1996, *supra*) of the ribozyme. According to the standard numbering system for the hammerhead ribozyme, the three pyrimidine positions are 7, 4 and 3. For construction of the libraries, the ribozyme positions are numbered 3' to 5': position 24 = 7, position 27 = 4, and position 28 = 3 (see Figure 13). The remainder of the hammerhead ribozyme "template" remained constant and is based on a previously described hammerhead motif (Thompson *et al*., US Patent No. 5,610,052, incorporated by reference herein). The starting ribozyme template is targeted against nucleotide position 823 of k-ras mRNA (Site A). Down regulation of this message, as a result of ribozyme action, results in the inability of the cells to proliferate. Therefore in order to optimize a ribozyme, we chose to identify "variants" which were successful in inhibiting cell proliferation.

### Cell Culture Assay:

### Ribozyme:lipid complex formation

Ribozymes and LipofectAMINE were combined DMEM at final concentrations of 100 nM and 3.6 µM, respectively. Complexes were allowed to form for 15 min at 37 C in the absence of serum and antibiotics.

### Proliferation Assay

Primary rat aortic smooth muscle cells (RASMC) were seeded at a density of 2500 cells/well in 48 well plates. Cells were incubated overnight in DMEM, supplemented with 20% fetal bovine serum (FBS), Na-pyruvate, penicillin (50 U/ml), and streptomycin (50 µg/ml). Subsequently cells were rendered quiescent by a 48 h incubation in DMEM with 0.5% FBS.

Cells were incubated for 1.5 h with serum-free DMEM ribozyme:lipid complexes. The medium was replaced and cells were incubated for 24 h in DMEM with 0.25% FCS.

Cells were then stimulated with 10% FBS for 24 h. ³H-thymidine (0.3 µCi//well) was present for the last 12 h of serum stimulation.

At the end of the stimulation period the medium was aspirated and cells were fixed in icecold TCA (10%) for 15 min. The TCA solution was removed and wells were washed once with water. DNA was extracted by incubation with 0.1 N NaOH at RT for 15 min. Solubilized DNA was quantitatively transferred to minivials. Plates were washed once with water. Finally, ³H-thymidine incorporation was determined by liquid scintillation counting.

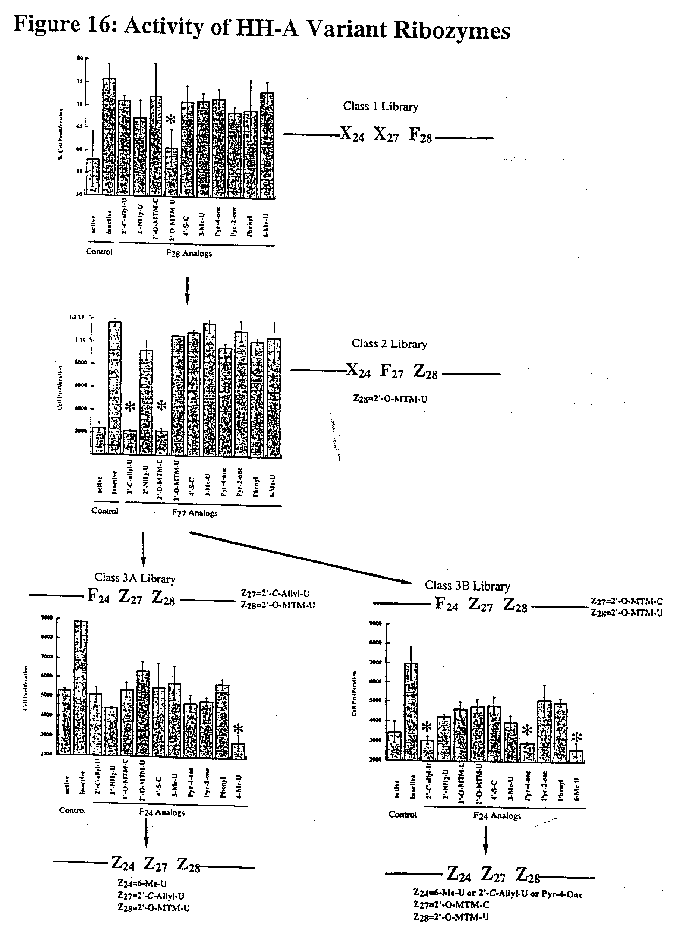

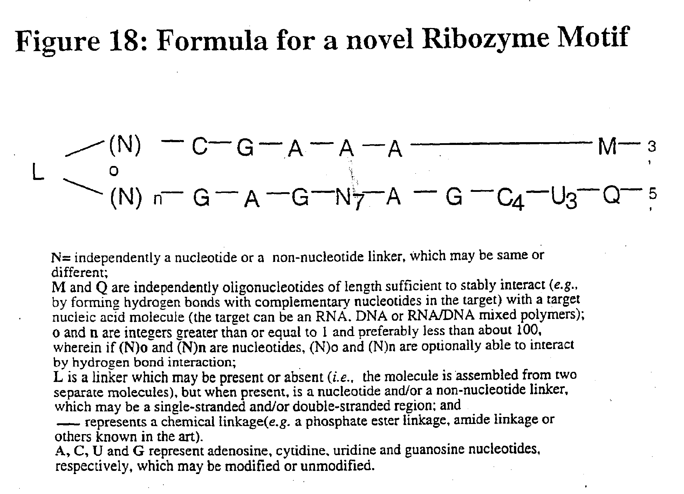

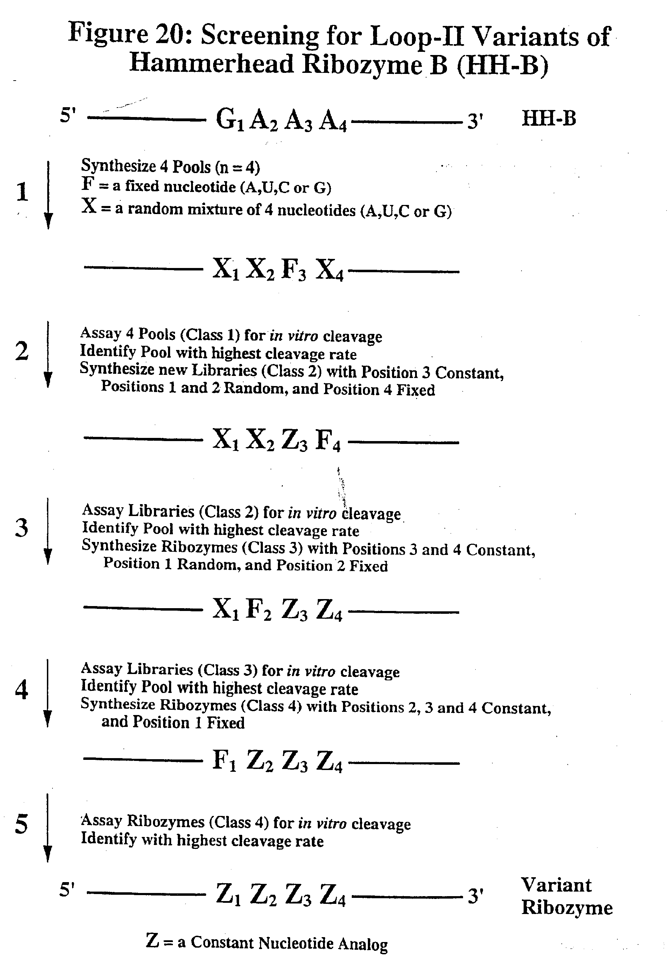

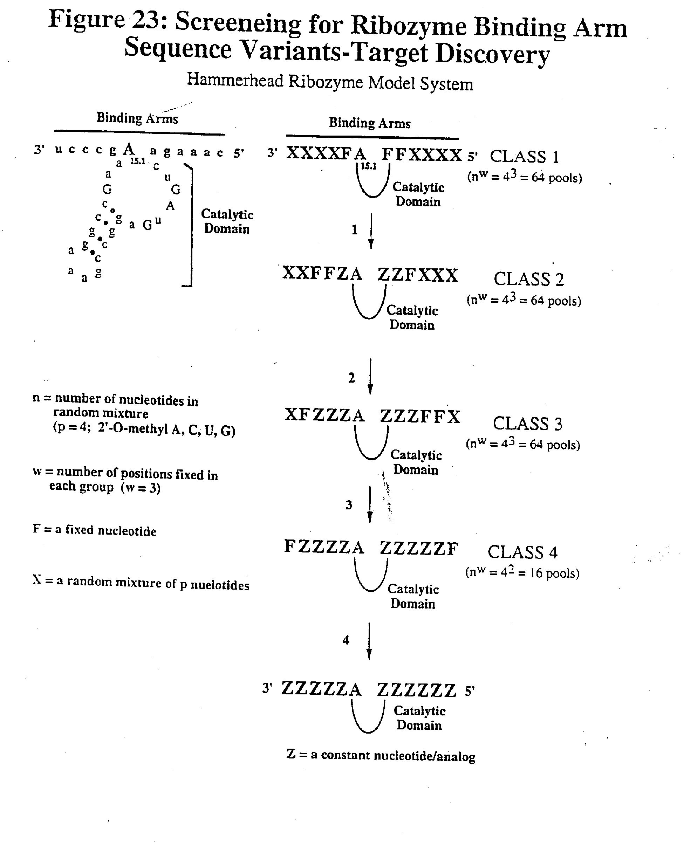

A strategy for optimizing the three (number of Class = 3) pyrimidine residues is illustrated in Figure 20. Ten different nucleotide analogs (illustrated in Figure 15) were chosen to construct the ribozyme library (n = 10). In the first step, ten different pools (Class 1) were synthesized by the mix and split approach described herein. Positions 24 and 27 were random and position 28 was fixed with each of the ten different analogs. The ten different pools were formulated with a cationic lipid (Jarvis *et al.,* 1996, *RNA,* 2,419; incorporated by reference herein), delivered to cells *in vitro,* and cell proliferation was subsequently assayed (see Figure 16). A positive control (active ribozyme) inhibited cell proliferation by ∼50% and an inactive control (inactive) resulted in a less than 25% reduction in cell proliferation. The ten ribozyme pools resulted in intermediate levels of reduction. However, the best pool could be identified as X₂₄ X₂₇ 2'-MTM-U₂₈ (positions 24 and 27 random; 2'-O-MTM-U at position 28). Therefore, a second ribozyme library (Class 2) was synthesized with position 28 constant (2'-O-MTM-U); position 24 was random (X₂₄) and position 27 was fixed with each of the ten different analogs (F₂₇). Again, the ten pools were assayed for their ability to inhibit cell proliferation. Among Class 2, two pools inhibited proliferation equally well: X₂₄ 2'-C-allyl-U₂₇ 2'-O-MTM-U₂₈ and X₂₄ 2'-O-MTM-C₂₇ 2'-O-MTM-U₂₈. Because a single "winner" could not be identified in Class 2, position 27 was made constant with either 2'-C-allyl-U or with 2'-O-MTM-C and the ten analogs were placed individually at position 24 (Class 3). Therefore in Class 3, twenty different ribozymes were assayed for their ability to inhibit cell proliferation. Because both positions 27 and 28 are constant, the final twenty ribozymes contain no random positions. Thus in the final group (Class 3), pure ribozymes and not pools were assayed. Among the final groups four ribozymes inhibited cell proliferation to a greater extent than the control ribozyme (Figure 22). These four winners are illustrated in Figure 23A. Figure 23B shows general formula for four different motifs. A formula for a novel ribozyme motif is shown in Figure 18.

### Example 11: Identifying Accessible Sites for Ribozyme Action in a target

In the previous two examples (9 and 10), positions within the catalytic domain of the hammerhead ribozyme were optimized. The number of groups that needed to be tested equals = the total number of positions within the ribozyme that were chosen to be tested. A similar procedure can be used on the binding arms of the ribozyme. The sequence of the binding arms determines the site of action of the ribozyme. The combinatorial approach can be used to identify those sites by essentially testing all possible arm sequences. The difficulty with this approach is that ribozymes require a certain number of base pairs (12-16) in order bind tightly and specifically. According to the procedure outlined above, this would require 12-16 different groups of ribozyme pools; 12-16 positions would have to be optimized which would require 12-16 different groups being synthesized and tested. Each pool would contain the four different nucleotides (A, C, U and G) or nucleotide analogs (n = 4). It would be very time consuming to test each group, identify the best pool, synthesize another group of ribozyme pools with one additional position constant, and then repeat the procedure until all 12-16 groups had been tested. However it is possible to decrease the number of groups by testing multiple positions within a single group. In this case, the number of pools within a group equals the number of nucleotides or analogs in the random mixture (i.e., n) to the w power, where w equals the number of positions fixed in each group. The number of groups that need to be synthesized to optimize the final ribozyme equals the total number of positions to be optimized divided by the number of positions (w) tested within each group. The number of pools in each group = n^{w}. The number of groups = total number of positions / w.

For example, Figure 23 illustrates this concept on a hammerhead ribozyme containing 12 base pair binding arms. Each of the two binding arms form 6 base pairs with it's corresponding RNA target. It is important to note that for the hammerhead ribozyme one residue (A15.1) must remain constant; A15.1 forms a base pair with a substrate nucleotide (U16.1) but is also absolutely required for ribozyme activity. It is the only residue within the hammerhead ribozyme that is part of both the catalytic domain, and the binding domain (arms). In the example this position is not optimized. In the first Group, three positions are fixed (designated F) with the four different 2'-*O*-methyl nucleotides (A, C, U and G). The 2'-*O*-methyl modification stabilizes the ribozyme against nuclease degradation and increases the binding affinity with it's substrate. The total number of pools in each group does not equal n, as in the previous examples. The number of pools in each group equals 4³ = (four analogs)^(number of positions fixed; 3) = 64. In all 64 pools, all other positions in the arm are made random (designated X) by the nucleotide mixing building block approach. The catalytic domain is not considered in this example and therefore remains part of the ribozyme template (i.e., constant).

In the first step, all 64 ribozyme pools are tested. This test may be cleavage *in vitro* (see Example 9), or efficacy in a cellular (see Example 10) or animal model, or any other assayable end-point. This end-point however, should be specific to a particular RNA target. For example, if one wishes to identify accessible sites within the mRNA of GeneB, a suitable end-point would be to look for decreased levels of GeneB mRNA after ribozyme treatment. After a winning pool is identified, since each pool specifies the identity of three positions (w), three positions can be made constant for the next group (Class 2). Class 2 is synthesized containing 64 different pools; three positions that were fixed in Class 1 are now constant (designated Z), three more positions are fixed (F), and the remaining positions (X) are a random mix of the four nucleotides. The 64 pools are assayed as before, a winning pool is identified, allowing three more positions to be constant in the next Class of ribozyme pools (Class 3) and the process is repeated again. In the final Class of ribozymes (Class 4), only two positions are fixed, all other positions have been previously fixed. The total number of ribozymes is therefore n^{w} = 4² = 16; these ribozymes also contain no random positions. In the final step (step 4), the 16 ribozymes are tested; the winning ribozyme defines the sequence of the binding arms for a particular target.

Fixing multiple positions within a single group it is possible to decrease the overall number of groups that need to be tested. As mentioned, this is particularly useful when a large number of different positions need to be optimized. A second advantage to this approach is that it decreases the complexity of molecules in each pool. If one would expect that many combinations within a given pool will be inactive, by decreasing the number of different ribozymes in each pool, it will be easier to identify the "winning" pool. In this approach, a larger number of pools have to be tested in each group, however, the number of groups is smaller and the complexity of each ribozyme pool is smaller. Finally, it should be emphasized there is not a restriction on the number of positions or analogs that can be tested. There is also no restriction on how many positions are tested in each group.

### Example 12: Identifying new RNA targets for Ribozymes

As described above for identifying ribozyme-accessible sites, the assayed used to identify the "winning" pool of ribozymes is not defined and may be cleavage *in vitro* (see Example 8), or efficacy in a cellular (see Example 9) or animal model, or any other assayable end-point. For identifying accessible sites, this end-point should be specific to a particular RNA target (e.g., mRNA levels). However, the end-point could also be nonspecific. For example, one could choose a disease model and simply identify the winning ribozyme pool based on the ability to provide a desired effect. In this case, it is not even necessary to know what the cellular target that is being acted upon by the ribozyme is. One can simply identify a ribozyme that has a desired effect. The advantage to this approach is that the sequence of the binding arms will be complementary to the RNA target. It is therefore possible to identify gene products that are involved in a disease process or any other assayable phenotype. One does not have to know what the target is prior to starting the study. The process of identifying an optimized ribozyme (arm combinatorial) identifies both the drug (ribozyme) and the RNA target, which may be a known RNA sequence or a novel sequence leading to the discovery of new genes.

### Example 13: Identifying New Ribozyme Catalytic Domains

In the previous two examples, positions within the binding domain of the hammerhead ribozyme were varied and positions within the catalytic domain were not changed. Conversely, it is possible to vary positions within the catalytic domain, without changing positions within the binding arms, in order to identify new catalytic motifs. An example is illustrated in Figure 24. The hammerhead ribozyme, for example comprises about 23 residues within the catalytic domain. It is unclear how many of these 23 positions are required to obtain a functional catalytic domain, however it is reasonable to presume that if a large number of functionally diverse nucleotide analogs can be used to construct the pools, a relatively small number of positions could constitute a functional catalytic domain. This may especially be true if analogs are chosen that one would expect to participate in catalysis (e.g., acid/base catalysts, metal binding, etc.). In the example illustrated in Figure 24, four positions (designated 1, 2, 3 and 4) are chosen. In the first step, ribozyme libraries (Class 1) are constructed: position 1 is fixed (F,) and positions 2, 3 and 4 are random (X₂, X₃ and X₄, respectively). In step 2, the pools (the number of pools tested depends on the number of analogs used; n) are assayed for activity. This testing may be performed *in vitro* or in a cellular or animal model. Whatever assay that is used, the pool with the most activity is identified and libraries (class 2) are again synthesized with position 1 now constant (Z₁) with the analog that was identified in class 1. In class 2, position 2 is fixed (F₂) and positions 3 and 4 are random (X₃ and X₄). This process is repeated until every position has been made constant, thus identifying the catalytic domain or a new motif.

### EXAMPLE 14: Determination of Coupling Efficiency of the phosphoramidite derivatives of 2'-C-allyl-uridine, 1; 4'-thio-cytidine, 2; 2'-methylthiomethyl-uridine, 3; 2'-methylthiomethyl-cytidine, 4; 2'-amino-uridine, 5; N3-methyl-uridine, 6; 1-b-D-(ribofuranosyl)-pyridin-4-one, 7; 1-b-D-(ribofuranosyl)-pyridin-2-one, 8; 1-b-D-(ribofuranosyl)-phenyl, 9; 6-methyl-uridine, 10 to be used in a split and mix approach.

The determination of the coupling efficiency of amidites **1** to **10** was assessed using ten model sequences agacXGAuGa (where upper case represents ribonucleotide residues, lower case represents 2'-*O*-methyl ribonucleotide residues and X is amidites **1** to **10,** to be used in the construction of a hammerhead ribozyme library wherein the modified amidites 1 to 10 would be incorporated. Ten model sequences were synthesized using ten 0.112 g aliquots of 5'-*O*-DMT-2'-*O*-Me-Adenosine Polystyrene (PS) solid-support loaded at 22.3 µmol/g and equivalent to a 2.5 µmol scale synthesis. Synthesis of these ten decamers were performed on ABI 394 DNA synthesizer (Applied Biosystems, Foster City, Calif.) using standard nucleic acid synthesis reagents and synthesis protocols, with the exception of an extended (7.5 min) coupling time for the ribonucleoside phosphoramidites and phosphoramidites **1, 2, 3, 4, 6, 7, 8, 9, 10,** 12.5 min coupling time for the 2'-amino-uridine phosphoramidite, amidite 5 and 2.5 min coupling time for the 2'-*O*-methyl nucleoside phosphoramidites.

Oligomers were cleaved from the solid support by treatment with a 3:1 mixture of ammonium hydroxide:absolute ethanol at 65 degree C for 4 hrs followed by a desilylation treatment and butanol precipitation as described in Wincott et al. (Wincott et al, *Nucleic Acids Res,* 1995, **23,** 2677-2684; incorporated by reference herein). Oligonucleotides were analyzed directly on an anion-exchange HPLC column (Dionex, Nucleopac, PA-100, 4x250 mm) using a gradient of 50% to 80% of B over 12 minutes (A = 10 mM sodium perchlorate, 1 mM Tris, pH 9.43; B = 300 mM sodium perchlorate, 1 mM Tris, pH 9.36) and a Hewlett-Packard 1090 HPLC system.

The average stepwise yield (ASWY), indicating the coupling efficiency of phosphoramidites, **1** to **10**, were calculated from peak-area percentages according to the equation ASWY = (FLP%)^{1/n} where FLP% is the percentage full-length product in the crude chromatogram and n the number of synthesis cycles. ASWY ranging from of 96.5% to 97.5% were obtained for phosphoramidites, **1** to **10.** The experimental coupling efficiencies of the phosphoramidites **1** to **10,** as determined using a standard spectrophotometric dimethoxytrityl assay were in complete agreement with the ASWY and were judged satisfactory to proceed with the X24, X27, X28 ribozyme library synthesis.

### EXAMPLE 15: Determination of optimal relative concentration of a mixture of 2'-O-methyl-guanosine, cytidine, uridine and adenosine providing equal representation of the four nucleotides.

A mixture N, composed of an equimolar mixture of the four 2'-O-Me- nucleoside phosphoramidites (mG=2'-*O*-methyl guanosine; mA=2'-*O*-methyl adenosine; mC=2'-*O*-methyl cytidine; mU=2'-*O*-methyl uridine) was used in the synthesis of a model sequence TTXXXXTTB, where T is 2'-deoxy-thymidine and B is a 2'-deoxy-inverted abasic polystyrene solid-support as described in Example 14. After standard deprotection (Wincott *et al., supra*), the crude nonamer was analyzed on an anion-exchange HPLC column (see example 6). From the HPLC analysis, an averaged stepwise yield (ASWY) of 99.3% was calculated (see example 14) indicating that the overall coupling efficiency of the mixture N was comparable to that of 2'-deoxythymidine. To further assess the relative incorporation of each of the components within the mixture, N, the full-length product TTXXXXTTB (over 94.3% at the crude stage) was further purified and subjected to base composition analysis as described herein. Purification of the FLP from the failures is desired to get accurate base composition.

### Base composition analysis summary:

A standard digestion/HPLC analysis was performed: To a dried sample containing 0.5 A.sub.260 units of TTXXXXTTB, 50 µl mixture, containing 1 mg of nuclease P1 (550 units/mg), 2.85 ml of 30 mM sodium acetate and 0.3 ml of 20 mM aqueous zinc chloride, was added. The reaction mixture was incubated at 50 degrees C overnight. Next, 50 µl of a mixture comprising 500 µl of alkaline phosphatase (1 units/µl), 312 µl of 500 mM Tris pH 7.5 and 2316 µl water was added to the reaction mixture and incubated at 37 degrees C for 4 hours. After incubation, the samples were centrifuged to remove sediments and the supernatant was analyzed by HPLC on a reversed-phase C18 column equilibrated with 25 mM KH2PO4. Samples were analyzed with a 5% acetonitrile isocratic gradient for 8 min followed by a 5% to 70% acetonitrile gradient over 8 min.

The HPLC percentage areas of the different nucleoside peaks, once corrected for the extinction coefficient of the individual nucleosides, are directly proportional to their molar ratios.
The results of these couplings are shown in Table IV.

| Nucleoside | dT 0.1 M | **2'-OMe-C** **0.025M** | **2'-OMe-U** **0.025M** | **2'-OMe-G** **0.025M** | **2'-OMe-A** **0.025M** |
|---|---|---|---|---|---|
| % area | 43.81 | 6.04 | 14.07 | 18.54 | 17.54 |
| Epsilon 260 nm | 8800 | 7400 | 10100 | 11800 | 14900 |
| moles | 0.00498 | 0.00082 | 0.00139 | 0.00157 | 0.00118 |
| equivalent | 4 | **0.656** | **1.119** | **1.262** | **0.946** |

As can be seen in Table IV, the use of an equimolar mixture of the four 2'-*O*-methyl phosphoramidites does not provide an equal incorporation of all four amidites, but favors 2'-*O*-methyl-U and G and incorporates 2'-*O*-methyl-A and C to a lower efficiency. To alleviate this, the relative concentrations of 2'-*O*-methyl-A, G, U and C amidite were adjusted using the inverse of the relative incorporation as a guide line. After several iterations, the optimized mixture providing nearly identical incorporation of all four amidites was obtained as shown in Table V below. The relative representation do not exceed 12% difference between the most and least incorporated residue corresponding to a +/- 6% deviation from equimolar incorporation.

| Nucleoside | dT 0.1M | **2'-OMe-C** **0.032M** | **2'-OMe-U** **0.022M** | **2'-OMe-G** **0.019M** | **2'-OMe-A** **0.027M** |
|---|---|---|---|---|---|
| % area | 44.47 | 8.91 | 11.81 | 15.53 | 19.28 |
| Epsilon 260 nm | 8800 | 7400 | 10100 | 11800 | 14900 |
| moles | 0.00505 | 0.00120 | 0.00117 | 0.00132 | 0.00129 |
| equivalent | 4 | **0.953** | **0.926** | **1.042** | **1.024** |

### EXAMPLE 16: A Non-competitive coupling method for the preparation of the X24, X27 and N28 ribozyme library 5'- aₛcₛaₛaₛag aFX GAX Gag gcg aaa gcc Gaa Agc ccu cB -3' wherein 2'-C-allyl-uridine, 1; 4'-thio-cytidine, 2; 2'-methylthiomethyl-uridine, 3; 2'-methylthiomethyl-cytidine, 4; 2'-amine-uridine, 5; N3-methyl-uridine, 6; 1-b-D-(ribofuranosyl)-pyrimidine-4-one, 7; 1-b-D-(ribofuranosyl)-pyrimidine-2-one, 8; 1-b-D-(ribofuranosyl)-phenyl, 9; and/or 6-methyl-uridine, 10 are incorporated at the X24, X27 and F28 positions through the mix and split approach.

The synthesis often different batches of 2,5 µmol scale Gag gcg aaa gcc Gaa Agc ccu c**B** sequence was performed on 2'-deoxy inverted abasic polystyrene solid support **B** on a 394 ABI DNA synthesizer (Applied Biosystems, Foster City, CA). These ten aliquots were then separately reacted with phosphoramidite building blocks **1** to **10** according to the conditions described in example 11. After completion of the individual incorporation of amidites **1** to **10**, their coupling efficiencies were determined to be above 95 % as judged by trityl monitoring. The 10 different aliquots bearing the ten different sequences were mixed thoroughly and divided into ten equal subsets. Each of these aliquots were then successively reacted with ribo-A, ribo-G amidites and one of the amidites **1** to **10**. The ten aliquots were combined, mixed and split again in 10 subsets. At that point, the 10 different polystyrene aliquots, exhibiting the following sequence: ***X*** GA***X*** Gag gcg aaa gcc Gaa Agc ccu c**B**, were reacted again with amidites **1** to **10** separately. The aliquots were not mixed, but kept separate to obtain a unique residue at the 28th position of each of the ten pools. The ribozyme synthesis was then finished independently to yield ten random ribozymes pools. Each pool comprises a 3'-terminal inverted abasic residue **B**, followed by the sequence Gag gcg aaa gcc Gaa Agc ccu c, followed with one random position ***X*** in the 24th position corresponding to a mixture of amidites 1 to 10, followed by the sequence GA, followed one random position ***X*** in the 27th position corresponding to a mixture of amidites 1 to 10, followed by a fixed monomer ***F*** (one of the amidites **1** to **10**) in the 28th position and finally the 5'-terminal sequence aₛcₛaₛaₛa g a. This is represented by the sequence notation 5'- aₛcₛaₛaₛag a***FX*** GA***X*** Gag gcg aaa gcc Gaa Agc ccu c**B**-3', in which ***X*** are random positions and ***F*** is a unique fixed position. The total complexity of such a ribozyme library was 10³ or 1,000 members separated in 10 pools of 100 different ribozyme sequences each.

### EXAMPLE 17: Competitive coupling method (monomer mixing approach) for the preparation of the x₂₋₆ and X₃₀₋₃₅ "binding arms" ribozyme library

Synthesis of 5'-xₛxₛx xFF c***u***G A***u*** G Agg ccg uua ggc cGA AAF xxx x**B**-3' is described, with F being a defined 2'-*O*-methyl-ribonucleoside chosen among 2'-*O*-methyl-ribo-adenosine (mA), -guanosine (mG), -cytidine (mC), -uridine (mU) and x being an equal mixture of 2'-*O*-methyl-ribo-adenosine, -guanosine, -cytidine, -uridine.

The syntheses of this ribozyme library was performed with an ABI 394 DNA synthesizer (Applied Biosystems, Foster City, Calif.) using standard nucleic acid synthesis reagents and synthesis protocols, with the exception of an extended (7.5 min) coupling time for the ribonucleoside phosphoramidites (upper case) and 2'-amino-uridine phosphoramidite, ***u,*** (2.5 min) coupling time for the 2'*-O*-methyl-ribonucleoside phosphoramidites (lower case) and the 2'-*O*-methyl-ribonucleoside phosphoramidites mixture, n.

Sixty four (64) batches of 0.086 g aliquots of 3'-*O*-DMT-2'-deoxy-inverted abasic-Polystyrene **(B)** solid-support loaded at 29 µmol/g and equivalent to a 2.5 µmol scale synthesis were individually reacted with a 27:32:19:22 / v:v:v:v mixture, x, of mA:mC:mG:mU diluted in dry acetonitrile to 0.1 M as described in example 7. This synthesis cycle was repeated for a total of four times. The 64 aliquots were then grouped into four subsets of sixteen aliquots (Class 1) that were reacted with either mA, mG, mC, mU to synthesize the n6 position. This accomplished, the sequence: 5'- c***u***G A***u*** G Agg ccg uua ggc cGA AA was added onto the 6 position of the 64 aliquots constituting Class 1. Each subset of Class 1 was then divided into four subsets of four aliquots (Class 2) that were reacted with either mA, mG, mC, mU to synthesize the F30 position. Each subset of Class 2 was then divided into four subsets of one aliquot (Class 3) that were reacted with either mA, mG, mC, mU to synthesize the F31 position. Finally, the random sequence 5'-xₛxₛx x was added onto each of the 64 aliquots.

The ribozyme library yielded sixty four random ribozymes pools each having an equal mixture of the four 2'-*O*-methyl-nucleoside at the position x2 to 6 and x30 to 35, and a defined 2'-*O*-methyl-nucleoside chosen among mA, mC, mG, mU at the positions F6, F30 and F31. The total complexity of such a "binding arms" ribozyme library was 4¹¹ or 4,194,304 members separated in 64 pools of 65,536 different ribozyme sequences each.

### EXAMPLE 18: Competitive coupling method (monomer mixing approach) for the preparation of the position 15 to 18 "loop II" ribozyme library

Synthesis of 5' UCU CCA UCU GAU GAG GCC XXF XGG CCG AAA AUC CCU 3' is described, with F being a defined ribonucleoside chosen among adenosine (A), guanosine (G), cytidine (C), uridine (U) and X being an equal mixture of adenosine (A), guanosine (G), cytidine (C), uridine (U).

The syntheses of this ribozyme library was performed with an ABI 394 DNA synthesizer (Applied Biosystems, Foster City, Calif.) using standard nucleic acid synthesis reagents and synthesis protocols, with the exception of an extended (7.5 min) coupling time for the ribonucleoside phosphoramidites (A, G, C, U) and the ribonucleoside phosphoramidite mixture, X.

Four batches (4) of 2.5 µmol scale of GG CCG AAA AUC CCU sequence were synthesized on 0.085 g samples of 5'-O-DMT-2'-O-TBDMS-3'-succinyl-uridine-Polystyrene (U) solid-support loaded at 29.8 µmol/g. To synthesize the position X15, the four aliquots of solid-supports were individually reacted with a 30:26:24:20 / v:v:v:v mixture, X, of A:C:G:U diluted in dry acetonitrile to 0.1 M according to the optimized conditions for the DNA phosphoramidites mixed-base coupling as described in the DNA Synthesis Course Manual published by Perkin-Elmer-Applied Biosystem Division. (DNA Synthesis Course Manual : Evaluating and isolating synthetic oligonucleotides, the complete guide, p. 2-4, Alex Andrus, August 1995). The four aliquots of solid-supports were then individually reacted with either of the four ribonucleoside phosphoramidites (A, G, C, U) to create the F16 position. The position X17 and X18 were then added onto the F16 (either A, G, C or U) of the four aliquots of solid-supports by repeating twice the same procedure used for the position X15.

The synthesis of the ribozyme library was then ended by adding the sequence 5'-UCU CCA UCU GAU GAG GCC on the position X18 of each of the four subsets of the ribozyme library. The ribozyme library yielded four random ribozymes pools that each have an equal mixture of the four ribonucleoside (A, G, C and U) at the position X15, X17 and X18, and a discrete ribonucleoside chosen among A, C, G or U at the positions F16. The total complexity of such a loop II ribozyme library was 256 members separated in 4 pools of 64 different ribozyme sequences.

### Example 19: Arm-Combinatorial Library Screening For Bc1-2, K-ras and Urokinase plasminogen Activator (UPA)

*Substrate synthesis through in vitro transcription:* Run-off transcripts for Bcl-2 and Kras were prepared using linearized plasmids (975 and 796 nucleotides respectively). Transcripts for UPA were produced from a PCR generated DNA fragment containing a T7 promoter (400 nucleotides). Transcription was performed using the T7 Megascript transcription kit (Ambion, Inc.) with the following conditions: a 50ul reaction volume containing 7.5mM each of ATP, CTP, UTP, and GTP, 2mM guanosine, 5ul 10x T7 reaction buffer, 5ul T7 enzyme mix, and 0.5ug of linearized plasmid or PCR'd DNA template. The mixture was incubated at 37°C for 4 hours (6 hours for transcripts < 500 bases). Guanosine was added to the transcription reactions so that the final transcript could be efficiently 5'-end labeled without prior phosphatase treatment. Transcription volume was then increased to 200ul with buffer containing 50mM TRIS pH 7.5, 100mM KCl, and 2mM MgCl₂ and spin column purified over Bio-Gel P-60 (BioRad) equilibrated in the same buffer. 100ul of transcript was then applied to 750ul of packed resin. Spin column flow-through was used directly in a 5'-end labeling reaction as follows (100ul final volume): 82ul of P-60 spin column purified transcript, 10ul 10x polynucleotide kinase buffer, 4ul 10U/ul Polynucleotide Kinase (Boehringer/Mannheim) and 4ul 150uCi/ul Gamma-32P-ATP (NEN) were incubated together at 37°C for one hour. The reaction volume was increased to 200ul with buffer containing 50mM TRIS pH 7.5, 100mM KCl and 2mM MgCl₂ and the sample was then purified over Bio-Gel P-60 packed spin column as described above. Approximate specific activities of the 5'-end labeled transcripts were determined via BioScan and stored frozen at -20°C.

### Synthesis of Ribozyme pools:

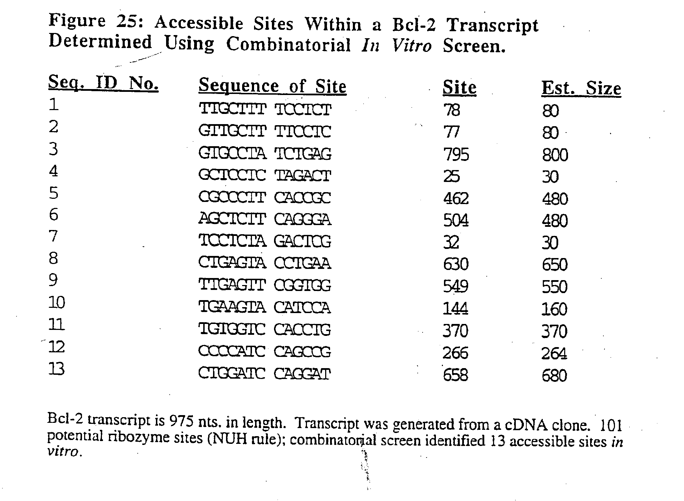

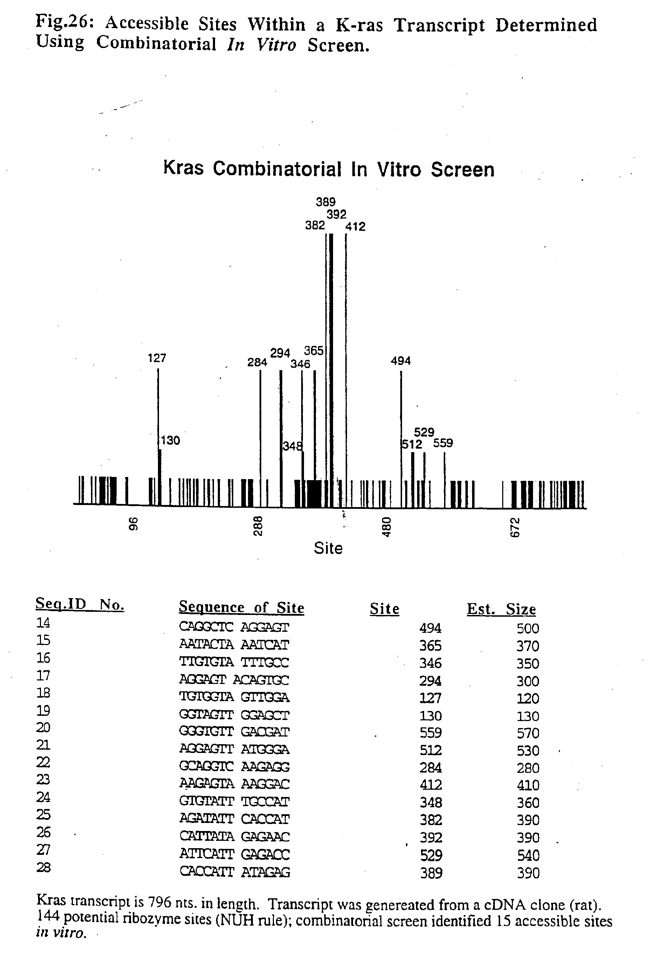

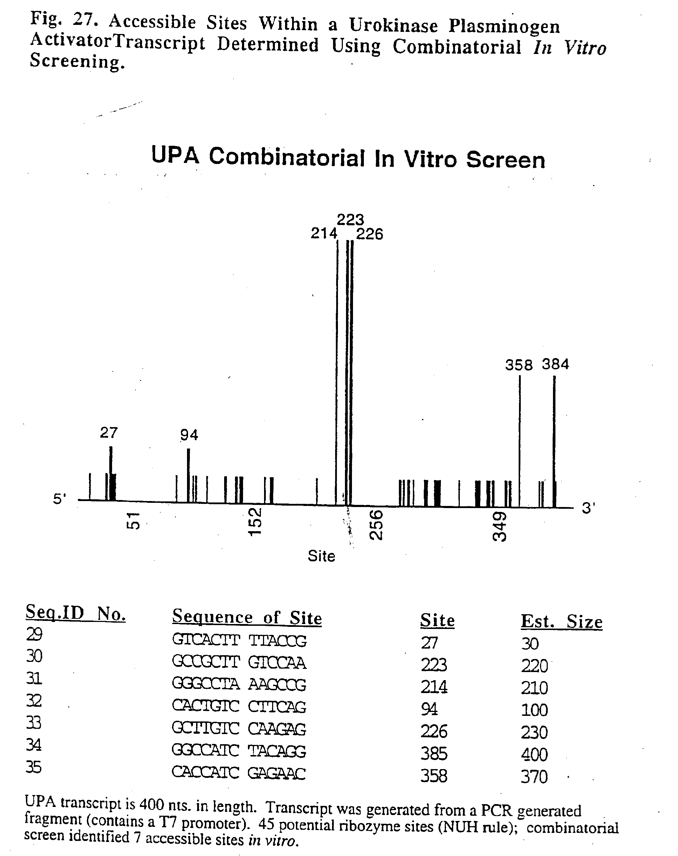

*In vitro ribozyme-transcript cleavage reactions:* Cleavage reactions were carried out as follows: 5'-end labeled transcript (∼2-4 x 10⁴ dpm/ul final) was incubated with 10uM ribozyme pool in 50mM TRIS pH 7.5, 50mM NaCl, 2mM MgCl₂ and 0.01% SDS for 24-48 hours at room temperature (∼22°C). An equal volume of gel loading dye (95% formamide, 0.01M EDTA, 0.0375% bromophenol blue, and 0.0375% xylene cyanol) was added to stop the reaction and the samples are heated to 95°C. Reactions (1-2 x 10⁵ dpm per lane) were run on a 5% denaturing polyacrylamide gel containing 7M urea and 1x TBE. Gels are dried and imaged using the PhosphorImager system (Molecular Dynamics). Ambion, Inc. RNA Century Marker Plus RNA standards body labeled in a T7 Megascript reaction as described above using 3ul of 10mCi/ml Alpha-³²P-ATP (BioRad) and 0.5ug Century RNA template and subsequently spin column purified over Bio-Gel P-6 (Bio-Rad) were used as a size reference on the gel. Cleavage product sizes were determined using the RNA standards which provided an approximate site of cleavage (est. Size in Figure). Because each of the ribozyme pools has three positions within the binding arms fixed, it is possible to identify all of the potential ribozyme sites that can potentially be cleaved by that pool. The estimated size of the cleavage product is therefore compared with the potential sites to identify the exact site of cleavage.
This protocol has been completed on three different transcripts: Bcl-2 (figure 25), Kras (figure 26), and UPA (figure 27). The data is summarized in the figures. All potential hammerhead ribozyme cleavage sites are indicated in the graph with a short vertical line. The actual sites identified are indicated in the graph. The size of the bar reflects the intensity of the cleavage product from the cleavage reaction. The combinatorial pool used to identify each site, the actual sequence of each site, the position of cleavage within the transcript (based on the known sequence), and the estimated size of the cleavage product (based on gel analysis) are listed.

### Example 20: Reduction of Bcl-2 mRNA using Optimized Ribozymes

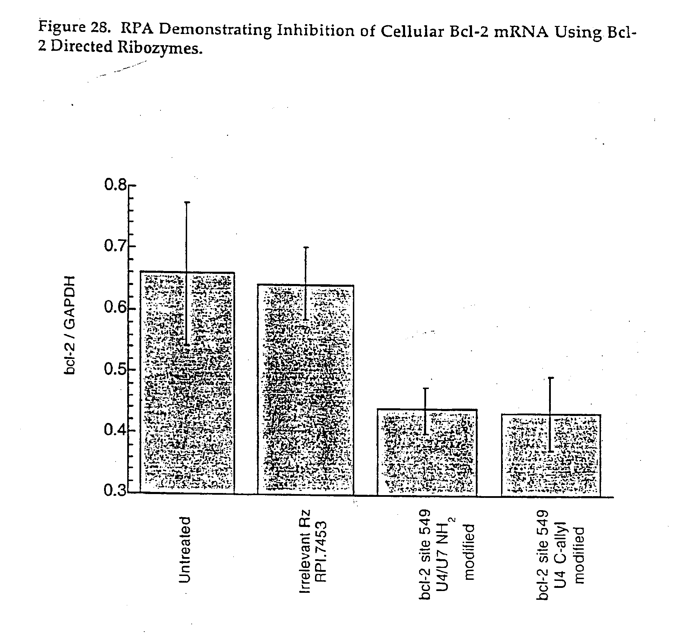

Two ribozymes targeted against the same site in the bc1-2 transcript (Seq.ID#9, figure 25) were synthesized, but the two ribozymes were stabilized using two different chemistries (U4/U7 amino and U4 c-allyl). Ribozymes (200 nM) were delivered using lipofectamine (7.2 mM) for 3 hours into MCF-7 cells (50% confluency). Cellular RNA was harvested 24 hours after delivery, analyzed by RNase protectection analysis (RPA) and normalized to GAPDH mRNA in triplicate samples. Both ribozymes gave a reduction in bcl-2 mRNA (see Figure 28). A ribozyme targeted against an irrelevant mRNA (c-myb) had no effect on the ratio of bc1-2 mRNA to GAPDH mRNA. All reduction of bc1-2 RNA was statistically significant with respect to untreated samples and samples treated with the irrelevant ribozyme.

### Example 21: Synthesis of purine nucleoside triphosphates: 2'-O-methyl-guanosine-5'-triphosphate

2'-*O*-methyl guanosine nucleoside (0.25 grams, 0.84 mmol) was dissolved in triethyl phosphate (5.0) ml by heating to 100C for 5 minutes. The resulting clear, colorless solution was cooled to 0 C using an ice bath under an argon atmosphere. Phosphorous oxychloride (1.8 eq., 0.141 ml) was then added to the reaction mixture with vigorous stirring. The reaction was monitored by HPLC, using a sodium perchlorate gradient. After 5 hours at 0 C, tributylamine (0.65 ml) was added followed by the addition of anhydrous acetonitrile (10.0 ml), and after 5 minutes (reequilibration to 0 C) tributylammonium pyrophosphate (4.0 eq., 1.53 g) was added. The reaction mixture was quenched with 20 ml of 2M TEAB after 15 minutes at 0 C (HPLC analysis with above conditions showed consumption of monophosphate at 10 minutes) then stirred overnight at room temperature, the mixture was evaporated *in vacuo* with methanol co-evaporation (4x) then diluted in 50 ml 0.05M TEAB. DEAE sephadex purification was used with a gradient of 0.05 to 0.6 M TEAB to obtain pure triphosphate (0.52 g, 66.0% yield) (elutes around 0.3M TEAB); the purity was confirmed by HPLC and NMR analysis.

### Example 22: Synthesis of Pyrimdine nucleoside triphosphates: 2'-O-methylthiomethyl-uridine-5'-triphosphate

2'-*O*-methylthiomethyl uridine nucleoside (0.27 grams, 1.0 mmol) was dissolved in triethyl phosphate (5.0 ml). The resulting clear, colorless solution was cooled to 0 C with an ice bath under an argon atmosphere. Phosphorus oxychloride (2.0 eq., 0.190 ml) was then added to the reaction mixture with vigorous stirring. Dimethylaminopyridine (DMAP, 0.2eq., 25 mg) was added, the solution warmed to room temperature and the reaction was monitored by HPLC, using a sodium perchlorate gradient. After 5 hours at 20 C, tributylamine (1.0 ml) was added followed by anhydrous acetonitrile (10.0 ml), and after 5 minutes tributylammonium pyrophosphate (4.0 eq., 1.8 g) was added. The reaction mixture was quenched with 20 ml of 2M TEAB after 15 minutes at 20 C (HPLC analysis with above conditions showed consumption of monophosphate at 10 minutes) then stirred overnight at room temperature. The mixture was evaporated *in vacuo* with methanol co-evaporation (4x) then diluted in 50 ml 0.05M TEAS. DEAE fast flow Sepharose purification with a gradient of 0.05 to 1.0 M TEAB was used to obtain pure triphosphate (0.40 g, 44% yield) (elutes around 0.3M TEAB) as determined by HPLC and NMR analysis.

### Example 23: Utilization of DMAP in Uridine 5'-Triphosphate Synthesis

The reactions were performed on 20 mg aliquots of nucleoside dissolved in 1 ml of triethyl phosphate and 19 ul of phosphorus oxychloride. The reactions were monitored at 40 minute intervals automatically by HPLC to generate yield-of-product curves at times up to 18 hours. A reverse phase column and ammonium acetate/ sodium acetate buffer system (50mM & 100mM respectively at pH 4.2) was used to separate the 5', 3', 2' monophosphates (the monophosphates elute in that order) from the 5'-triphosphate and the starting nucleoside. The data is shown in table VI. These conditions doubled the product yield and resulted in a 10-fold improvement in the reaction time to maximum yield (1200 minutes down to 120 minutes for a 90% yield). Selectivity for 5'-monophosphorylation was observed for all reactions. Subsequent triphosphorylation occurred in nearly quantitative yield.

### Materials Used in Bacteriophage T7 RNA Polymerase Reactions

BUFFER 1: Reagents are mixed together to form a 10X stock solution of buffer 1 (400 mM Tris-Cl (pH 8.1), 200 mM MgCl₂, 100 mM DTT, 50 mM spermidine, and 0.1% triton X-100. Prior to initiation of the polymerase reaction methanol, LiCl is added and the buffer is diluted such that the final reaction conditions for condition 1 consisted of : 40mM tris pH (8.1), 20mM MgCl₂, 10 mM DTT, 5 mM spermidine, 0.01% triton X-100, 10% methanol, and 1 mM LiCl.
BUFFER 2: Reagents are mixed together to form a 10X stock solution of buffer 2(400 mM Tris-Cl (pH 8.1), 200 mM MgCl₂, 100 mM DTT, 50 mM spermidine, and 0.1% triton X-100. Prior to initiation of the polymerase reaction PEG, LiCl is added and the buffer is diluted such that the final reaction conditions for buffer 2 consisted of: 40mM tris pH (8.1), 20mM MgCl₂, 10 mM DTT, 5 mM spermidine, 0.01% triton X-100, 4% PEG, and 1 mM LiCl.
BUFFER 3: Reagents are mixed together to form a 10X stock solution of buffer 3 (400 mM Tris-Cl (pH 8.0), 120 mM MgCl₂, 50 mM DTT, 10 mM spermidine and 0.02% triton X-100. Prior to initiation of the polymerase reaction PEG is added and the buffer is diluted such that the final reaction conditions for buffer 3 consisted of: 40mM tris pH (8.0), 12 mM MgCl₂, 5 mM DTT, 1 mM spermidine, 0.002% triton X-100, and 4% PEG.
BUFFER 4: Reagents are mixed together to form a 10X stock solution of buffer 4 (400 mM Tris-Cl (pH 8.0), 120 mM MgCl₂, 50 mM DTT, 10 mM spermidine and 0.02% triton X-100. Prior to initiation of the polymerase reaction PEG, methanol is added and the buffer is diluted such that the final reaction conditions for buffer 4 consisted of : 40mM tris pH (8.0), 12 mM MgCl₂, 5 mM DTT, 1 mM spermidine, 0.002% triton X-100, 10% methanol, and 4% PEG.
BUFFER 5: Reagents are mixed together to form a 10X stock solution of buffer 5 (400 mM Tris-Cl (pH 8.0), 120 mM MgCl₂, 50 mM DTT, 10 mM spermidine and 0.02% triton X-100. Prior to initiation of the polymerase reaction PEG, LiCl is added and the buffer is diluted such that the final reaction conditions for buffer 5 consisted of : 40mM tris pH (8.0), 12 mM MgCl₂, 5 mM DTT, 1 mM spermidine, 0.002% triton X-100, 1 mM LiCI and 4% PEG.
BUFFER 6: Reagents are mixed together to form a 10X stock solution of buffer 6 (400 mM Tris-Cl (pH 8.0), 120 mM MgCl₂, 50 mM DTT, 10 mM spermidine and 0.02% triton X-100. Prior to initiation of the polymerase reaction PEG, methanol is added and the buffer is diluted such that the final reaction conditions for buffer 6 consisted of : 40mM tris pH (8.0), 12 mM MgCl₂, 5 mM DTT, 1 mM spermidine, 0.002% triton X-100, 10% methanol, and 4% PEG.

### Example 24: Screening of Modified Nucleoside triphosphates with Mutant T7 RNA Polymerase

Each modified nucleotide triphosphate was individully tested in buffers 1 through 6 at two different temperatures (25 and 37°C). Buffers 1-6 tested at 25°C were designated conditions 1-6 and buffers 1-6 test at 37°C were designated conditions 7-12 (**table VII**). In each condition, Y639F mutant T7 polymerase (Sousa and Padilla, *Supra*) (0.3-2 mg/20 ml reaction), NTP's (2 mM each), DNA template (10 pmol), inorganic pyrophosphatase (5U/ml) and α-³²P NTP(0.8 mCi/pmol template) were combined and heated at the designated temperatures for 1-2 hours. The radiolabeled NTP used was different from the modified triphosphate being testing. The samples were resolved by polyacrylamide gel electrophoresis. Using a phosphorImager (Molecular Dynamics, Sunnyvale, CA), the amount of full-length transcript was quantified and compared with an all-RNA control reaction. The data is presented in **Table VIII;** results in each reaction is expressed as a percent compared to the all-ribonucleotide triphosphate (rNTP) control. The control was run with the mutant T7 polymerase using commercially available polymerase buffer (Boehringer Mannheim, Indianapolis, IN).

### Example 25: Incorporation of Modified NTP's using Wild-type T7 RNA polymerase

Bacteriophage T7 RNA polymerase was purchased from Boehringer Mannheim at 0.4 U/µL concentration. Applicant used the commercial buffer supplied with the enzyme and 0.2 µCi alpha-³²P NTP in a 50 µL reaction with nucleotides triphosphates at 2 mM each. The template was double-stranded PCR fragment, which was used in previous screens. Reactions were carried out at 37°C for 1 hour. 10 µL of the sample was run on a 7.5% analytical PAGE and bands were quantitated using a Phosphorlmager. Results are calculated as a comparison to an "all ribo" control (non-modified nucleoside triphosphates) and the results are in **Table IX.**

### Example 26: Incorporation of Multiple Modified Nucleoside triphosphates Into Oligonucleotides

Combinations of modified nucleoside triphosphates were tested with the transcription protocol described in example 9, to determine the rates of incorporation of two or more of these triphosphates. Incorporation 2'-Deoxy-2'-(L-histidine) amino uridine (2'-his-NH₂-UTP) was tested with unmodified cytidine nucleoside triphosphates, rATP and rGTP in reaction condition number 9. The data is presented as a percentage of incorporation of modified NTP's compared to the all rNTP control and is shown in **Table Xa.**

Two modified cytidines (2'-NH₂-CTP or 2'dCTP) were incorporated along with 2'-his-NH₂-UTP with identical efficiencies. 2'-his-NH₂-UTP and 2'-NH₂-CTP were then tested with various unmodified and modified adenosine triphosphates in the same buffer **(Table Xb).** The best modified adenosine triphosphate for incorporation with both 2'-his-NH2-UTP and 2'-NH₂-CTP was 2'-NH₂-DAPTP.

### EXAMPLE 27: Optimization of Reaction conditions for Incorporation of Modified Nucleotide Triphosphate

The combination of 2'-his-NH₂-UTP, 2'-NH₂-CTP, 2'-NH₂-DAP, and rGTP was tested in several reaction conditions **(Table XI)** using the incorporation protocol described in example 14. The results demonstrate that of the buffer conditions tested, incorporation of these modified nucleoside triphosphates occur in the presence of both methanol and LiCl.

### Example 28: Deprotection of Ribozyme in a 96 Well Plate

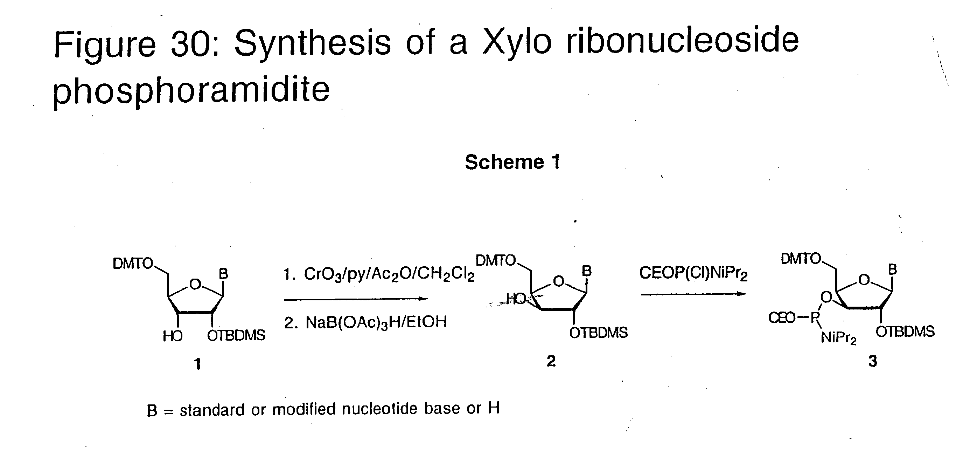

A ribozyme sequence (200nmole) was synthesized as described herein on a polystyrene solid support in a well of a 96 well plate. A 10:3:13 mixture (800 µL) of anhydrous methylamine (308µL), triethylamine (92µL) and dimethylsulfoxide (DMSO) (400 µL) was prepared of which half (400 µL) was added to the well and incubated at room temperature for 45 minutes. Following the reaction the solution was replaced with the remaining 400 µL and incubated as before. At the end of the reaction, the solid support was filtered off, all 800 µL of MA/TEA/DMSO solution was collected together and 100 µL of TEA.3HF was added. The reaction was then heated at 65°C for 15 minutes and then cooled to room temperature. The solution was then quenched with aqueous NH₄⁺HCO₃⁻ (1mL) (see Figure 30). HPLC chromatography of the reaction mixture afforded 32 O. D.u₂₆₀ₙₘ of which 46% was full length ribozyme.

### Example 29: Column Deprotection of Ribozyme

A ribozyme was synthesized using the column format as described herein. The polystyrene solid-support with protected oligoribonucleotide or modified oligoribonucleotide (200 nmole) was transferred into a glass vial equipped with a screw cap. A 10:3:13 mixture of anhydrous methylamine ( 308 µL), triethylamine (92 µL) and dimethylsulfoxide (DMSO) (400 µL) was added followed by vortexing of the glass vial. After allowing the reaction for 1.5 hours, the solid support was filtered off. 100 µL of TEA.3HF was added at room temperature to the vial and the reaction was mixed causing the solution to gel. The reaction was then heated at 65 °C for 15 minutes and then cooled to room temperature. The solution was then quenched with 1.5 M aqueous NH₄⁺HCO₃⁻ (1mL). HPLC chromatography of the reaction mixture afforded 32 O. D.u_{260 nm} of which 46% was full length ribozyme.

### Example 30: Column Deprotection of Ribozyme with anhydrous ethanolic methylamine

A ribozyme was synthesized using the column format as described herein. The polystyrene solid-support with protected oligoribonucleotide or modified oligoribonucleotide (200 nmole) was transferred into a glass vial equipped with a screw cap. A 1:1 mixture of anhydrous ethanolic methylamine (400 µL) and dimethylsulfoxide (DMSO) (400 µL) was added followed by vortexing of the glass vial. After allowing the reaction for 1.5 hours, the solid support was filtered off. 100 µL of TEA.3HF was added at room temperature to the vial and the reaction was mixed causing the solution to gel. The reaction was then heated at 65 °C for 15 minutes and then cooled to room temperature. The solution was then quenched with 1.5 M aqueous NH₄⁺HCO₃⁻ (1mL). HPLC chromatography of the reaction mixture afforded 32 O. D.u_{260 nm} of which 46% was full length ribozyme.

### Example 31. Large-scale One-Pot Deprotection of Ribozyme

A ribozyme was synthesized at the 0.5 mmol scale using the column format as described herein. The polystyrene solid-support (24 grs) with protected oligoribonucleotide or modified oligoribonucleotide (500 µmole) was transferred into a 1L Schott bottle equipped with a screw cap. A 1:1.3 mixture of anhydrous ethanolic methylamine ( 150 mL) and dimethylsulfoxide (DMSO) (200 mL) was added followed by vortexing (200 rpm) of the glass bottle for 1.5 hours. The reaction mixture was then frozen at -70 °C for 30 minutes. 50 mL of neat TEA.3HF was then added at room temperature to the reaction mixture and the reaction was placed in a shaking oven (200 rpm) where it was heated at 65 °C for 60 minutes and subsequently frozen at -70 °C for 30 minutes. The solution was then quenched with 1.5 M aqueous NH₄⁺HCO₃⁻ (200 mL). The reaction mixture was separated from the polystyrene solid-support by filtration on a sintered glass funnel (10-20 µm porosity). U.V. spectrophotometric quantification and HPLC chromatography of the reaction mixture afforded 160,000 O.D.u_{260 nm} of which 46.4% was full length ribozyme. After allowing the reaction for 1.5 hours, the solid support was filtered off

### Example 32: Antitumor and antimetastatic efficacy of ribozymes directed against the mRNA encoding the two VEGF receptor subtypes, flt-1 and flk-1 in the mouse Lewis lung-HM carcinoma model of primary tumor growth and metastasis

The Lewis lung carcinoma (LLC) model is a syngeneic mouse model of metastatic cancer commonly used for antitumor agent efficacy screening. According to Folkman (1995, *supra*), primary tumor growth and metastasis in this model is dependent upon VEGF-induced angiogenesis. Two variants of the LLC model exist. The low metastatic form involves the implantation of a tumor, usually subcutaneous, which sends micrometastases to the lungs whose growth is suppressed by the presence of the primary tumor. The highly metastatic (HM) form differs from the low metastatic variant in that the growth of metastases is not suppressed by the presence of the primary tumor. Thus, the HM form is a model in which it is possible to measure pharmacologic efficacy on both primary tumor growth and metastasis in the same mouse without excision of the primary tumor.

Applicant selected the highly metastatic variant of the Lewis lung model for antitumor/metastatic screening of ribozymes directed against VEGF receptor (*flt-1 and flk-1*) mRNA. These ribozymes have been shown to reduce VEGF binding and VEGF-stimulated proliferation in cultured MVEC's as well as VEGF-induced neovascularization of the rat cornea (Cushman et al., 1996, Angiogenesis Inhibitors and Other Novel Therapeutic Strategies for Ocular Diseases of Neovascularization, IBC Conference Abstract). Pharmacokinetically, Applicant has found that ribozymes distribute systemically following continuous i.v. infusion (*via* Alzet osmotic minipumps) at significant concentrations within' most tissues including subcutaneously implanted tumors. This study examines the antitumor/antimetastatic efficacy of *flt-1* and *flk-1* ribozymes continuously infused i.v. in the LLC-HM mouse model.

### Methods

### Ribozymes

The ribozymes used in this study were hammerhead ribozymes comprising a 4 base pair stem II, four phosphorothioate linkages at the 5'-end, a 2'-*C*-allyl substitution at position 4 (see Figure 1), and an inverted abasic nucleotide substitution at the 3'-end. The catalytically active and inactive ribozymes were RPI.4610/4611 (active/inactive) and RPI.4733/4734 directed against *flt-1* and *flk-1* messages, respectively. Ribozymes solutions were prepared in normal saline (USP).

Test solutions (ribozymes or saline control) were dispensed into Alzet® osmotic minipumps (Model # 1002--total volume capacity including excess = 200 µl) which dispense 0.5 µl/h at 37 °C when exposed to interstitial water. Pumps were either filled with normal saline (USP) or 167.0, 50.0, 16.7, 5.0, or 1.7 mg/ml ribozyme solutions. Prior to animal implantation, osmotic minipumps were placed in 37 °C sterile water for at least four hours to activate pumping.

### Tumor inoculation

All animal procedures in this study were performed in accordance with the National Research Council's Guide for the Care and Use of Laboratory Animals (1996), USDA regulations, and the policies and procedures of the RPI Institutional Animal Care and Use Committee. A total of 210 female C57BL/6J mice weighing between 20-25 g were used in this study. All animals were housed under 12 h on/12 h off light cycles and received *ad libitum* food and water.

Highly metastatic variant Lewis lung carcinoma (LLC-HM) tumors were propogated *in vivo* from an LLC-HM cell line. These tumors needed to be propogated *in vivo* because they can revert to the low metastatic phenotype in culture. LLC-HM cells were initially cultured in DMEM + 10% FCS + 1 % GPS. For *in vivo* propogation, 5 X 10 ⁶ cells were injected subcutaneously in mice. Tumors were allowed to grow for 25 days at which time animals were euthanized by CO₂ inhalation and lung macrometastases were counted. Animals with the most macrometastases (approximately 15-20) were selected for preparation of tumor breis and propogation. When tumors in animals selected for propogation reached a volume of approximately 1500 mm³, animals were euthanized by CO₂ inhalation and tumors were excised. Tumors were seived through a 100 µm pore size sterile nylon mesh. LLC-HM cells were resuspended in normal saline to a final concentration of 5 x 10⁶ viable cells/ml (*via* hemocytometer). Three days prior to ribozyme dosing, all animals were subcutaneously inoculated on the right flank with 5 x 10⁵ cells (in a volume of 100 µl).

### Ribozyme or saline dosing

Each ribozyme solution was prepared to deliver 100, 30, 10, 3, or 1 mg/kg/day in a volume of 12 ml. A total of 10 animals per dose or saline control group were surgically implanted on the left flank with osmotic minipumps pre-filled with the respective test solution three days following tumor inoculation. Pumps were attached to indwelling jugular vein catheters. The specifications for the model #1002 Alzet osmotic minipump show that they accurately deliver aqueous solutions at 0.5 µl/h for 14 days. Table III summarizes the experimental groups.

### Tumor volume and metastatic index quantitation

Beginning four days and ending 24 days days following tumor inoculation, the length and width of all primary tumors were measured every other day using microcalipers. Tumor volumes were calculated using the standard formula for an elipsoid volume, (L^{·}W²)/2. Tumor volumes were calculated in triplicate for each animal. A mean tumor volume was calculated for each animal. Group means and standard error of the group means were calculated from individual animal mean tumor volumes.

Twenty-five days following tumor inoculation, all animals were euthanized by CO₂ inhalation and lungs and primary tumors harvested. Lung macrometastases were counted under a dissecting microscope (2.5 X magnification). Lungs and primary tumors were also weighed on an analytical balance. Lung weights served as an index of total lung metastatic burden.

### Statistical analysis

For all treatment groups, group tumor volume means on day 18 (end of treatment) as well as means of primary tumor and lung weight and numbers of lung metastases were evaluated for normality and subjected to analysis of variance. Statistical differences between group means were evaluated using the Tukey-Kramer post-hoc test (alpha = 0.05). Comparisons with the control group (saline control) were made using the Dunnett's test (alpha = 0.05).

### Results

### Flt-1

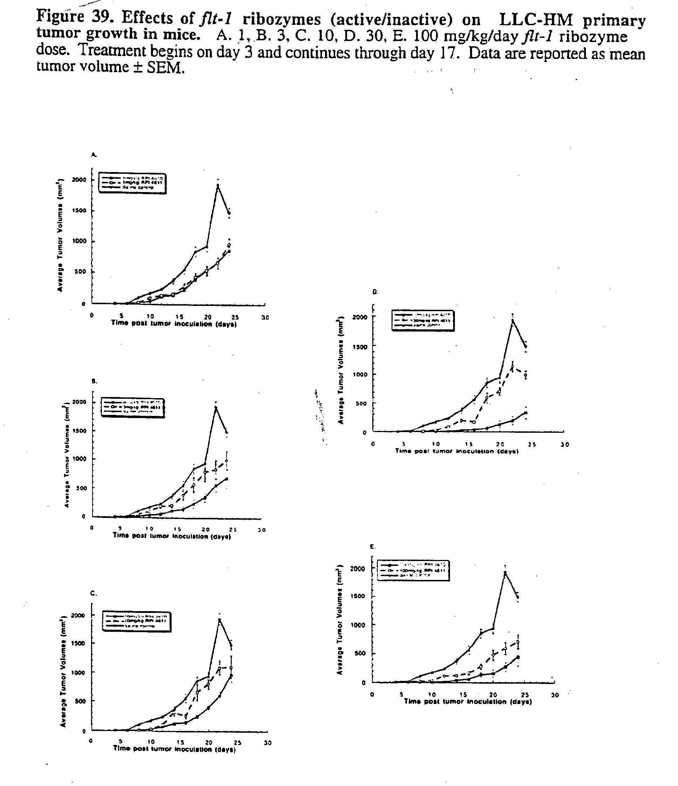

The effects of several doses of active and inactive *Flt-1* ribozymes (RPI.4610/4611, respectively) on primary LLC-HM tumor growth are summarized in Figure 39 (A-E). At the lowest dose (Figure 39A), both active and inactive reduce primary tumor growth similarly throughout the entire time course compared to saline controls. However, with increasing dose, active ribozyme reduces primary tumor growth to a greater extent than the inactive ribozyme, with the largest difference observed at 30 mg/kg/day (Figure 39D). The magnitude of the maximal reduction compared to saline was approximately four fold with the active ribozyme RPI.4610 at 30 mg/kg/day. It should be noted that this observed four fold reduction is still present at day 24 even though treatment ended 7 days earlier.

The growth curve data was subjected to exponential regression. The curve fits show that the tumor growth data fits an exponential curve with a high correlation coefficient (R>0.95). Thus, there appears to be no long lasting toxic effect on tumor growth. Since the calculated slope of the exponential curve at any point indicates the rate of tumor growth, it should be possible to compare rates of growth between treatments. Since the curve fits do not assume that the tumor growth starts from the same point (which is a correct assumption since the all tumors start with the same tumor cell inoculum concentration), an accurate calculation of the slope of the exponential curve is not possible since the curve fitting algorithm extrapolates a t = 0 tumor size which is then used to calculate the slope. In the analysis, the saline tumor size at t = 0 is much greater than the other treatment groups, thus comparisons with saline are not necessarily accurate. If the curve fit algorithm is restricted to the same tumor size, a dose-dependent reduction in the rate of tumor growth is observed with the active ribozyme. However, the curve fits show lower correlation coefficients in some cases.

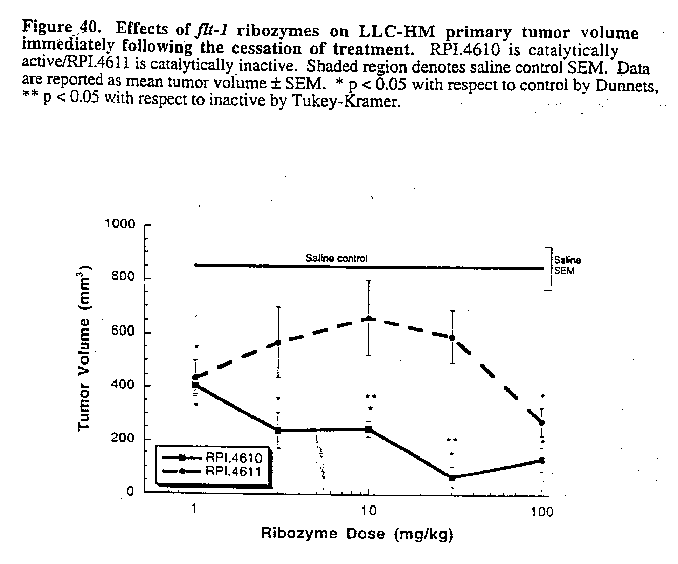

In order to see whether a the ribozyme treatments statistically reduce primary tumor growth, primary tumor volume measurements at each dose immediately following treatment (day 18) were compared (Figure 40). Active ribozyme RPI.4610 produced a statistically significant (p < 0.05) and dose-dependent reduction in primary tumor volume. Although the inactive ribozyme (RPI.4611) showed some reduction in primary tumor volume at the lowest and highest doses, there was no dose-dependent reduction observed. At doses between 3 and 30 mg/kg/day, the inactive ribozyme showed no significant reduction in primary tumor volume. There was a significant difference (p < 0.05) between active and inactive ribozymes (Tukey-Kramer test) at doses of 10 and 30 mg/kg/day.

Applicant has also observed that the active ribozyme RPI.4610 produced a significant reduction in primary tumor mass at all doses tested (1-100 mg/kg/day) 25 days following inoculation.

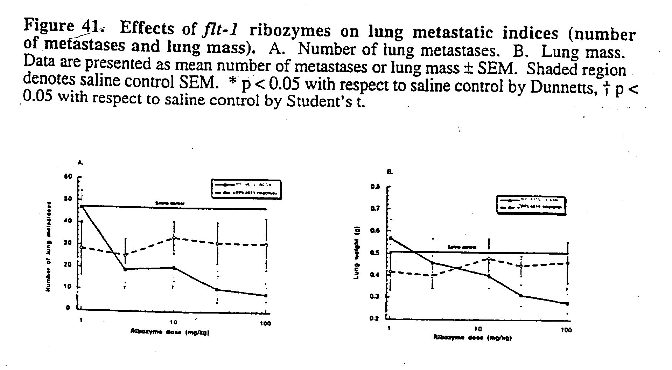

Figures 41 A and B illustrate that the active ribozyme reduced both the number of lung metastases and lung mass in a dose-dependent manner. The active *flt-1* ribozyme showed a significant reduction (p < 0.05 by Dunnetts) in the number of lung metastases at the 30 and 100 mg/kg/day doses compared to saline. There was also a significant difference between active and inactive ribozymes at these doses (p , 0.05 by Student's t). RPI.4610 reduced the lung weight to almost normal levels at the highest dose (100 mg/kg/day). There was no observable dose-related effect of the inactive ribozyme on either the number of lung metastases or lung weight. A significant reduction (p < 0.05, Student's t) in lung mass, an index of metastatic burden, was observed between saline and the active ribozyme. The lack of significance using more stringent statistical tests (Dunnet's or Tukey-Kramer), which take into account the variance within all groups, was due to high variability, especially in the inactive ribozyme group. However, since five doses were tested, it is possible to say that there is a dose-dependent trend in the reduction of lung metastases/lung weight.

### Example 33: Effects of flk-1 ribozymes (active/inactive) on LLC-HM primary tumor growth in mice.

The dose-related effects of active and inactive *flk-1* directed ribozymes (RPI.4733/4734, respectively) on primary LLCare shown in Figure 38 A-E.

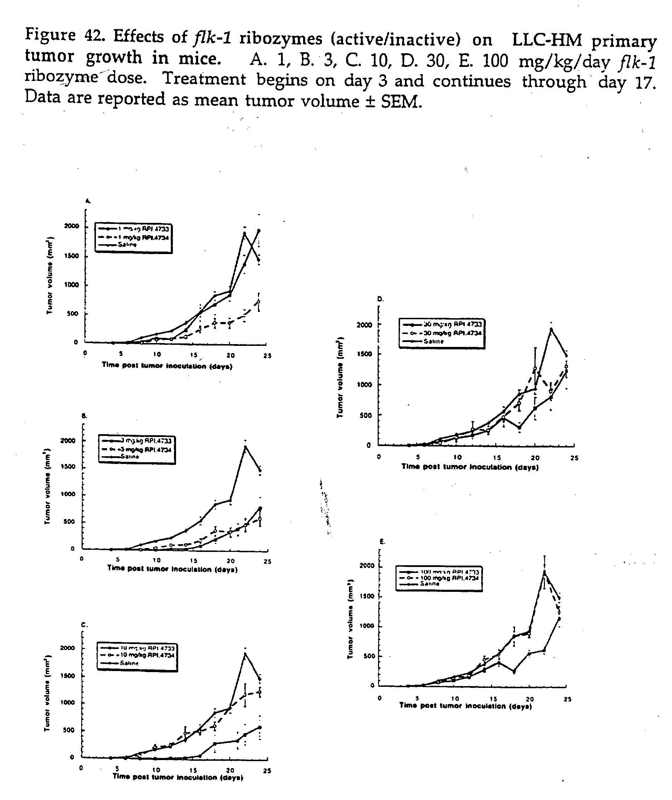

The dose-related effects of active and inactive *flk-1* directed ribozymes (RPI.4733/4734, respectively) on primary LLCare shown in Figure 42 A-E. At the lowest dose, there was no observable effect on primary tumor growth with the active *flk-1* ribozyme (Figure 42A). The inactive ribozyme showed a modest reduction in primary tumor growth. At higher doses (3-100 mg/kg/day, Figure 42B-E), the active *flk-1* ribozyme reduced primary tumor growth while the inactive ribozyme showed little, if any, antitumor efficacy over the dose range between 10 and 100 mg/kg/day (Figures 42C-E). The antitumor efficacy of both active and inactive *flk-1* ribozymes are similar at 3 mg/kg/day (Figure 42B).

As in the case of the *flt-1* ribozymes, tumor growth followed exponential growth kinetics. Again, since the t = 0 tumor size could not accurately be estimated by the curve fit program, it is not possible to calculate the slope of the exponential curve fits for the *flk-1* ribozymes.

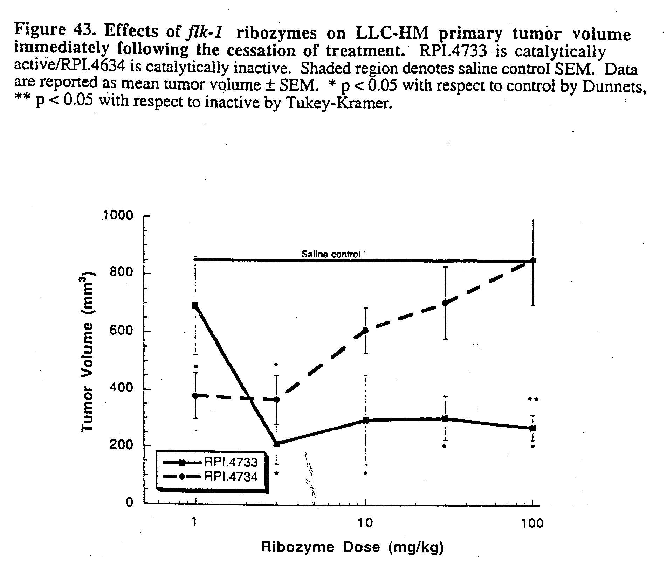

Immediately following the cessation of treatment (day 18), the active *flk-1* ribozyme showed a significant reduction in primary tumor volume from 3 to 100 mg/kg/day (Figure 43). The magnitude of the reduction is approximately four fold and appeared to be maximal at 3 mg/kg/day. The lowest dose had no significant effect on primary tumor volume. The inactive *flk-1* ribozyme had a significant antitumor effect at doses of 1 and 3 mg/kg/day; however, this effect disappeared between 10 and 100 mg/kg/day.

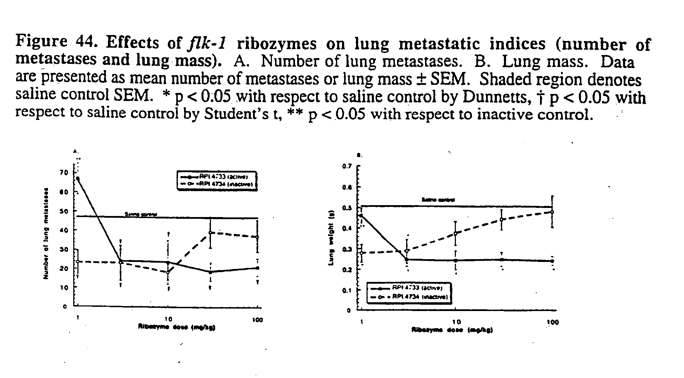

The antimetastatic effects of the *flk-1* ribozymes are illustrated in Figure 44 A and B. Although neither ribozyme showed a statistically significant effect on the number of lung metastases at any dose, it appears that the active *flk-1* ribozyme showed a significant reduction in lung mass over the dose range between 3 and 100 mg/kg/day.

Applicant has further observed that the lung mass was reduced to normal over the entire dose range. The inactive ribozyme reduced lung mass at 1 and 3 mg/kg/day (Figure 41C); however, this trend was not observed at higher doses (3-100 mg/kg/day).

### Example 34: Ribozyme-mediated decrease in vascularity of tumor

Three tumors from each of three treatment groups (saline controls, inactive RPI.4611 and active RPI.4610, 30 mg/kg/day dose only) were analyzed for vascularity using an immunohistochemical assay which stains endothelial cells for CD31 (PECAM). The vascularity was quantitated in a blinded fashion. From the raw data the average number of vessels per high magnification field (400X) were calculated. They are as follows: SALINE CONTROL = 24.1; RPI.4611 (Inactive) = 27.6; RPI.4610 (Active) = 16.0.

It is suggestive that ribozyme-specific antiangiogenic effect is exhibited by the active Flt-1 ribozyme in Lewis lung tumors. Thus, the mechanism of action for the observed reduction in the primary tumor volumes may be due to an antiangiogenic effect. Similar delivery strategies can be used to deliver c-raf ribozymes to treat a variety of diseases.

### Use of Ribozymes Targeting c-raf

Overexpression of the *c-raf* oncogene has been reported in a number of cancers (see above). Thus, inhibition of *c-raf* expression (for example using ribozymes) can reduce cell proliferation of a number of cancers, *in vitro* and *in vivo* and can reduce their proliferative potential. A cascade of MMP and serine proteinase expression is implicated in the acquisition of an invasive phenotype as well as in angiogenesis in tumors (MacDougall & Matrisian, 1995, *Cancer & Metastasis Reviews* 14, 351;Ritchlin & Winchester, 1989, *Springer Semin Immunopathol*., 11, 219).

A number of human diseases are characterized by the inappropriate proliferation of cells at sites of injury or damage to the normal tissue architecture. These diseases include restenosis, caused by the local proliferation of medial smooth muscle cells at sites of arterial wall disruption by surgery; psoriasis, caused by proliferation of keratinocytes at regions of endothelial cell damage in the skin, and various fibrosis, caused by the inappropriate replication of cells during wound healing processes. In certain inflammatory processes, cell proliferation may not be causative, yet it exacerbates the disease pathology. For example, in rheumatoid arthritis, synovial hyperplasia leads to accelerated cartilage damage due to secretion of proteases by the expanding population of synovial fibroblasts. Any number of these diseases and others which involve cellular proliferation or the loss of proliferative control, such as cancer, could be treated using ribozymes which inhibit the expression of the cellular *Raf* gene products. Alternatively, ribozyme inhibition of the cellular growth factor receptors could be used to inhibit downstream signalling pathways. The specific growth factors involved would depend upon the cell type indicated in the proliferative event.

Ribozymes, with their catalytic activity and increased site specificity (see above), are likely to represent a potent and safe therapeutic molecule for the treatment of cancer. In the present invention, ribozymes are shown to inhibit smooth muscle cell proliferation. From those practiced in the art, it is clear from the examples described, that the same ribozymes may be delivered in a similar fashion to cancer cells to block their proliferation.

### Diagnostic uses

Ribozymes of this invention may be used as diagnostic tools to examine genetic drift and mutations within diseased cells or to detect the presence of *c-raf* RNA in a cell. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules). Other in vitro uses of ribozymes of this invention are well known in the art, and include detection of the presence of mRNAs associated with *c-raf* related condition. Such RNA is detected by determining the presence of a cleavage product after treatment with a ribozyme using standard methodology.

In a specific example, ribozymes which can cleave only wild-type or mutant forms of the target RNA are used for the assay. The first ribozyme is used to identify wild-type RNA present in the sample and the second ribozyme will be used to identify mutant RNA in the sample. As reaction controls, synthetic substrates of both wild-type and mutant RNA will be cleaved by both ribozymes to demonstrate the relative ribozyme efficiencies in the reactions and the absence of cleavage of the "non-targeted" RNA species. The cleavage products from the synthetic substrates will also serve to generate size markers for the analysis of wild-type and mutant RNAs in the sample population. Thus each analysis will require two ribozymes, two substrates and one unknown sample which will be combined into six reactions. The presence of cleavage products will be determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (*i.e., c-raf*) is adequate to establish risk. If probes of comparable specific activity are used for both transcripts, then a qualitative comparison of RNA levels will be adequate and will decrease the cost of the initial diagnosis. Higher mutant form to wild-type ratios will be correlated with higher risk whether RNA levels are compared qualitatively or quantitatively.

### Additional Uses

Potential usefulness of sequence-specific nucleic acid catalysts of the instant invention might have many of the same applications for the study of RNA that DNA restriction endonucleases have for the study of DNA (Nathans *et al.,* 1975 *Ann. Rev. Biochem.* 44:273). For example, the pattern of restriction fragments could be used to establish sequence relationships between two related RNAs, and large RNAs could be specifically cleaved to fragments of a size more useful for study. The ability to engineer sequence specificity of the ribozyme is ideal for cleavage of RNAs of unknown sequence.

The use of NTP's described in this invention have several research and commercial applications. These modified nucleoside triphosphates can be used for *in vitro* selection (evolution) of oligonucleotides with novel functions. Examples of *in vitro* selection protocols are incorporated herein by reference (Joyce, 1989, *Gene,* 82, 83-87; Beaudry *et al.,* 1992, *Science 257,* 635-641; Joyce, 1992, *Scientific American* 267, 90-97; Breaker *et al.,* 1994, *TIBTECH* 12, 268; Bartel *et al*., 1993, *Science* 261:1411-1418; Szostak, 1993, *TIBS* 17, 89-93; Kumar *et al.,* 1995, *FASEB J., 9,* 1183; Breaker, 1996, *Curr. Op. Biotech.,* 7, 442).

Additionally, these modified nucleoside triphosphates can be employed to generate modified oligonucleotide combinatorial chemistry libraries. Several references for this technology exist (Brenner *et al.,* 1992, *PNAS* 89, 5381-5383, Eaton, 1997, *Curr. Opin. Chem. Biol.* **1**, 10-16) which are all incorporated herein by reference.

Nucleic acid molecules of the instant invention might have many of the same applications for the study of RNA that DNA restriction endonucleases have for the study of DNA (Nathans *et al*., 1975 *Ann. Rev. Biochem.* 44:273). For example, the pattern of restriction fragments could be used to establish sequence relationships between two related RNAs, and large RNAs could be specifically cleaved to fragments of a size more useful for study. The ability to engineer sequence specificity of the ribozyme is ideal for cleavage of RNAs of unknown sequence. Nucleic acid molecules (*e.g.,* ribozymes) of the invention can be used, for example, to target cleavage of virtually any RNA transcript (Zaug *et al.,* 324, *Nature* 429 1986 ; Cech, 260 *JAMA* 3030, 1988; and Jefferies *et al.,* 17 *Nucleic Acids Research* 1371, 1989). Such nucleic acids can be used as a therapeutic or to validate a therapeutic gene target and/or to determine the function of a gene in a biological system (Christoffersen, 1997, *Nature Biotech.* 15, 483).

Various ligands can be attached to oligonucleotides using the componds containing zylo modification for the purposes of cellular delivery, nuclease resistance, cellular trafficking and localization, chemical ligation of oligonucleotide fragments. Incorporation of one or more compounds of Formula II into a ribozyme may increase its effectiveness. Compounds of Formula II can be used as potential antiviral agents.

Other embodiments are within the following claims.

**TABLE I**

| **Characteristics of naturally occurring ribozymes** |
|---|
| **Group I Introns** |
| • Size: ∼150 to >1000 nucleotides. |
| • Requires a U in the target sequence immediately 5' of the cleavage site. |
| • Binds 4-6 nucleotides at the 5'-side of the cleavage site. |
| • Reaction mechanism: attack by the 3'-OH of guanosine to generate cleavage products with 3'-OH and 5'-guanosine. |
| • Additional protein cofactors required in some cases to help folding and maintainance of the active structure. |
| • Over 300 known members of this class. Found as an intervening sequence in *Tetrahymena thermophila* rRNA, fungal mitochondria, chloroplasts, phage T4, blue-green algae, and others. |
| • Major structural features largely established through phylogenetic comparisons, mutagenesis, and biochemical studies [¹,²]. |
| • Complete kinetic framework established for one ribozyme [³,⁴,⁵,⁶]. |
| • Studies of ribozyme folding and substrate docking underway [⁷,⁸,⁹]. |
| • Chemical modification investigation of important residues well established [¹⁰,¹¹]. |
| • The small (4-6 nt) binding site may make this ribozyme too non-specific for targeted RNA cleavage, however, the Tetrahymena group I intron has been used to repair a "defective" β-galactosidase message by the ligation of new β-galactosidase sequences onto the defective message [¹²]. |

| **RNAse P RNA (M1 RNA)** |
|---|
| • Size: ∼290 to 400 nucleotides. |
| • RNA portion of a ubiquitous ribonucleoprotein enzyme. |
| • Cleaves tRNA precursors to form mature tRNA [¹³]. |
| • Reaction mechanism: possible attack by M²⁺-OH to generate cleavage products with 3'-OH and 5'-phosphate. |
| • RNAse P is found throughout the prokaryotes and eukaryotes. The RNA subunit has been sequenced from bacteria, yeast, rodents, and primates. |
| • Recruitment of endogenous RNAse P for therapeutic applications is possible through hybridization of an External Guide Sequence (EGS) to the target RNA [¹⁴,¹⁵] |
| • Important phosphate and 2' OH contacts recently identified [¹⁶,¹⁷] |

| **Group II Introns** |
|---|
| • Size: >1000 nucleotides. |
| • Trans cleavage of target RNAs recently demonstrated [¹⁸,¹⁹]. |
| • Sequence requirements not fully determined. |
| • Reaction mechanism: 2'-OH of an internal adenosine generates cleavage products with 3'-OH and a "lariat" RNA containing a 3'-5' and a 2'-5' branch point. |
| • Only natural ribozyme with demonstrated participation in DNA cleavage [²⁰,²¹] in addition to RNA cleavage and ligation. |
| • Major structural features largely established through phylogenetic comparisons [²²]. |
| • Important 2' OH contacts beginning to be identified [²³] |
| • Kinetic framework under development [²⁴] |

| **Neurospora VS RNA** |
|---|
| • Size: ∼144 nucleotides. |
| • Trans cleavage of hairpin target RNAs recently demonstrated [²⁵]. |
| • Sequence requirements not fully determined. |
| • Reaction mechanism: attack by 2'-OH 5' to the scissile bond to generate cleavage products with 2',3'-cyclic phosphate and 5'-OH ends. |
| • Binding sites and structural requirements not fully determined. |
| • Only 1 known member of this class. Found in Neurospora VS RNA. |

| **Hammerhead Ribozyme** (see text for references) |
|---|
| • Size: ∼13 to 40 nucleotides. |
| • Requires the target sequence UH immediately 5' of the cleavage site. |
| • Binds a variable number nucleotides on both sides of the cleavage site. |
| • Reaction mechanism: attack by 2'-OH 5' to the scissile bond to generate cleavage products with 2',3'-cyclic phosphate and 5'-OH ends. |
| • 14 known members of this class. Found in a number of plant pathogens (virusoids) that use RNA as the infectious agent. |
| • Essential structural features largely defined, including 2 crystal structures [²⁶,²⁷] |
| • Minimal ligation activity demonstrated (for engineering through *in vitro* selection) [²⁸] |
| • Complete kinetic framework established for two or more ribozymes [²⁹]. |
| • Chemical modification investigation of important residues well established [³⁰]. |

| **Hairpin Ribozyme** |
|---|
| • Size: ∼50 nucleotides. |
| • Requires the target sequence GUC immediately 3' of the cleavage site. |
| • Binds 4-6 nucleotides at the 5'-side of the cleavage site and a variable number to the 3'-side of the cleavage site. |
| • Reaction mechanism: attack by 2'-OH 5' to the scissile bond to generate cleavage products with 2',3'-cyclic phosphate and 5'-OH ends. |
| • 3 known members of this class. Found in three plant pathogen (satellite RNAs of the tobacco ringspot virus, arabis mosaic virus and chicory yellow mottle virus) which uses RNA as the infectious agent. |
| • Essential structural features largely defined [³¹,³²,³³,³⁴] |
| • Ligation activity (in addition to cleavage activity) makes ribozyme amenable to engineering through *in vitro* selection [³⁵] |
| • Complete kinetic framework established for one ribozyme [³⁶]. |
| • Chemical modification investigation of important residues begun [³⁷,³⁸], |

| **Hepatitis Delta Virus (HDV) Ribozyme** |
|---|
| • Size: ∼60 nucleotides. |
| • Trans cleavage of target RNAs demonstrated [³⁹]. |
| • Binding sites and structural requirements not fully determined, although no sequences 5' of cleavage site are required. Folded ribozyme contains a pseudoknot structure [⁴⁰]. |
| • Reaction mechanism: attack by 2'-OH 5' to the scissile bond to generate cleavage products with 2',3'-cyclic phosphate and 5'-OH ends. |
| • Only 2 known members of this class. Found in human HDV. |
| • Circular form of HDV is active and shows increased nuclease stability [⁴¹] |

| |
|---|
| ¹. Michel, Francois; Westhof, Eric. Slippery substrates. Nat. Struct. Biol. (1994), 1(1), 5-7. |
| ². Lisacek, Frederique; Diaz, Yolande; Michel, Francois. Automatic identification of group I intron cores in genomic DNA sequences. J. Mol. Biol. (1994), 235(4), 1206-17. |
| ³. Herschlag, Daniel; Cech, Thomas R.. Catalysis of RNA cleavage by the Tetrahymena thermophila ribozyme. 1. Kinetic description of the reaction of an RNA substrate complementary to the active site. Biochemistry (1990), 29(44), 10159-71. |
| ⁴. Herschlag, Daniel; Cech, Thomas R.. Catalysis of RNA cleavage by the Tetrahymena thermophila ribozyme. 2. Kinetic description of the reaction of an RNA substrate that forms a mismatch at the active site. Biochemistry (1990), 29(44), 10172-80. |
| ⁵. Knitt, Deborah S.; Herschlag, Daniel. pH Dependencies of the Tetrahymena Ribozyme Reveal an Unconventional Origin of an Apparent pKa. Biochemistry (1996), 35(5), 1560-70. |
| ⁶. Bevilacqua, Philip C.; Sugimoto, Naoki; Turner, Douglas H.. A mechanistic framework for the second step of splicing catalyzed by the Tetrahymena ribozyme. Biochemistry (1996), 35(2), 648-58. |
| ⁷. Li, Yi; Bevilacqua, Philip C.; Mathews, David; Turner, Douglas H.. Thermodynamic and activation parameters for binding of a pyrene-labeled substrate by the Tetrahymena ribozyme: docking is not diffusion-controlled and is driven by a favorable entropy change. Biochemistry (1995), 34(44), 14394-9. |
| ⁸. Banerjee, Aloke Raj; Turner, Douglas H.. The time dependence of chemical modification reveals slow steps in the folding of a group I ribozyme. Biochemistry (1995), 34(19), 6504-12. |
| ⁹. Zarrinkar, Patrick P.; Williamson, James R.. The P9.1-P9.2 peripheral extension helps guide folding of the Tetrahymena ribozyme. Nucleic Acids Res. (1996), 24(5), 854-8. |
| ¹⁰. Strobel, Scott A.; Cech, Thomas R.. Minor groove recognition of the conserved G.cntdot.U pair at the Tetrahymena ribozyme reaction site. Science (Washington, D. C.) (1995), 267(5198), 675-9. |
| ¹¹. Strobel, Scott A.; Cech, Thomas R.. Exocyclic Amine of the Conserved G.cntdot.U Pair at the Cleavage Site of the Tetrahymena Ribozyme Contributes to 5'-Splice Site Selection and Transition State Stabilization. Biochemistry (1996), 35(4), 1201-11. |
| ¹². Sullenger, Bruce A.; Cech, Thomas R.. Ribozyme-mediated repair of defective mRNA by targeted trans-splicing. Nature (London) (1994), 371(6498), 619-22. |
| ¹³. Robertson, H.D.; Altman, S.; Smith, J.D. J. Biol. Chem., 247, 5243-5251 (1972). |
| ¹⁴. Forster, Anthony C.; Altman, Sidney. External guide sequences for an RNA enzyme. Science (Washington, D. C., 1883-) (1990), 249(4970), 783-6. |
| ¹⁵. Yuan, Y.; Hwang, E. S.; Altman, S. Targeted cleavage of mRNA by human RNase P. Proc. Natl. Acad. Sci. USA (1992) 89, 8006-10. |
| ¹⁶. Harris, Michael E.; Pace, Norman R.. Identification of phosphates involved in catalysis by the ribozyme RNase P RNA. RNA (1995), 1(2), 210-18. |
| ¹⁷. Pan, Tao; Loria, Andrew; Zhong, Kun. Probing of tertiary interactions in RNA: 2'-hydroxyl-base contacts between the RNase P RNA and pre-tRNA. Proc. Natl. Acad. Sci. U. S. A. (1995), 92(26), 12510-14. |
| ¹⁸ Pyle, Anna Marie; Green, Justin B.. Building a Kinetic Framework for Group II Intron Ribozyme Activity: Quantitation of Interdomain Binding and Reaction Rate. Biochemistry (1994), 33(9), 2716-25. |
| ¹⁹. Michels, William J. Jr.; Pyle, Anna Marie. Conversion of a Group II Intron into a New Multiple-Turnover Ribozyme that Selectively Cleaves Oligonucleotides: Elucidation of Reaction Mechanism and Structure/Function Relationships. Biochemistry (1995), 34(9), 2965-77. |
| ²⁰. Zimmerly, Steven; Guo, Huatao; Eskes, Robert; Yang, Jian; Perlman, Philip S.; Lambowitz, Alan M.. A group II intron RNA is a catalytic component of a DNA endonuclease involved in intron mobility. Cell (Cambridge, Mass.) (1995), 83(4), 529-38. |
| ²¹. Griffin, Edmund A., Jr.; Qin, Zhifeng; Michels, Williams J., Jr.; Pyle, Anna Marie. Group II intron ribozymes that cleave DNA and RNA linkages with similar efficiency, and lack contacts with substrate 2'-hydroxyl groups. Chem. Biol. (1995), 2(11), 761-70. |
| ²². Michel, Francois; Ferat, Jean Luc. Structure and activities of group II introns. Annu. Rev. Biochem. (1995), 64, 435-61. |
| ²³. Abramovitz, Dana L.; Friedman, Richard A.; Pyle, Anna Marie. Catalytic role of 2'-hydroxyl groups within a group II intron active site. Science (Washington, D. C.) (1996), 271(5254), 1410-13. |
| ²⁴. Daniels, Danette L.; Michels, William J., Jr.; Pyle, Anna Marie. Two competing pathways for self-splicing by group II introns: a quantitative analysis of in vitro reaction rates and products. J. Mol. Biol. (1996), 256(1), 31-49. |
| ²⁵. Guo, Hans C. T.; Collins, Richard A.. Efficient trans-cleavage of a stem-loop RNA substrate by a ribozyme derived from Neurospora VS RNA. EMBO J. (1995), 14(2), 368-76. |
| ²⁶. Scott, W.G., Finch, J.T., Aaron,K. The crystal structure of an all RNA hammerhead ribozyme:Aproposed mechanism for RNA catalytic cleavage. Cell, (1995), 81, 991-1002. |
| ²⁷. McKay, Structure and function of the hammerhead ribozyme: an unfinished story. RNA, (1996), 2, 395-403. |
| ²⁸. Long, D., Uhlenbeck, O., Hertel, K. Ligation with hammerhead ribozymes. US Patent No. 5,633,133. |
| ²⁹. Hertel, K.J., Herschlag, D., Uhlenbeck, O. A kinetic and thermodynamic framework for the hammerhead ribozyme reaction. Biochemistry, (1994) 33, 3374-3385.Beigelman, L., *et al*., Chemical modifications of hammerhead ribozymes. J. Biol. Chem., (1995) 270, 25702-25708. |
| ³⁰. Beigelman, L., *et al*., Chemical modifications of hammerhead ribozymes. J. Biol. Chem., (1995) 270, 25702-25708. |
| ³¹. Hampel, Arnold; Tritz, Richard; Hicks, Margaret; Cruz, Phillip. 'Hairpin' catalytic RNA model: evidence for helixes and sequence requirement for substrate RNA. Nucleic Acids Res. (1990), 18(2), 299-304. |
| ³². Chowrira, Bharat M.; Berzal-Herranz, Alfredo; Burke, John M.. Novel guanosine requirement for catalysis by the hairpin ribozyme. Nature (London) (1991), 354(6351), 320-2. |
| ³³. Berzal-Herranz, Alfredo; Joseph, Simpson; Chowrira, Bharat M.; Butcher, Samuel E.; Burke, John M.. Essential nucleotide sequences and secondary structure elements of the hairpin ribozyme. EMBO J. (1993), 12(6), 2567-73. |
| ³⁴. Joseph, Simpson; Berzal-Herranz, Alfredo; Chowrira, Bharat M.; Butcher, Samuel E.. Substrate selection rules for the hairpin ribozyme determined by in vitro selection, mutation, and analysis of mismatched substrates. Genes Dev. (1993), 7(1), 130-8. |
| ³⁵. Berzal-Herranz, Alfredo; Joseph, Simpson; Burke, John M.. In vitro selection of active hairpin ribozymes by sequential RNA-catalyzed cleavage and ligation reactions. Genes Dev. (1992), 6(1), 129-34. |
| ³⁶. Hegg, Lisa A.; Fedor, Martha J.. Kinetics and Thermodynamics of Intermolecular Catalysis by Hairpin Ribozymes. Biochemistry (1995), 34(48), 15813-28. |
| ³⁷. Grasby, Jane A.; Mersmann, Karin; Singh, Mohinder; Gait, Michael J.. Purine Functional Groups in Essential Residues of the Hairpin Ribozyme Required for Catalytic Cleavage of RNA. Biochemistry (1995), 34(12), 4068-76. |
| ³⁸. Schmidt, Sabine; Beigelman, Leonid; Karpeisky, Alexander; Usman, Nassim; Sorensen, Ulrik S.; Gait, Michael J.. Base and sugar requirements for RNA cleavage of essential nucleoside residues in internal loop B of the hairpin ribozyme: implications for secondary structure. Nucleic Acids Res. (1996), 24(4), 573-81. |
| ³⁹. Perrotta, Anne T.; Been, Michael D.. Cleavage of oligoribonucleotides by a ribozyme derived from the hepatitis. delta, virus RNA sequence. Biochemistry (1992), 31(1), 16-21. |
| ⁴⁰. Perrotta, Anne T.; Been, Michael D.. A pseudoknot-like structure required for efficient self-cleavage of hepatitis delta virus RNA. Nature (London) (1991), 350(6317), 434-6. |
| ⁴¹. Puttaraju, M.; Perrotta, Anne T.; Been, Michael D., A circular trans-acting hepatitis delta virus ribozyme. Nucleic Acids Res. (1993), 21(18), 4253-8. |

**Table II:**

| **2.5 µmol RNA Synthesis Cycle** | | | |
|---|---|---|---|
| **Reagent** | **Equivalents** | **Amount** | **Wait** **Time*** |
| Phosphoramidites | 6.5 | 163 µL | 2.5 |
| *S*-Ethyl Tetrazole | 23.8 | 238 µL | 2.5 |
| Acetic Anhydride | 100 | 233 µL | 5 sec |
| *N*-Methyl Imidazole | 186 | 233 µL | 5 sec |
| TCA | 83.2 | 1.73 mL | 21 sec |
| Iodine | 8.0 | 1.18 mL | 45 sec |
| Acetonitrile | NA | 6.67 mL | NA |

| | | | |
|---|---|---|---|
| * Wait time does not include contact time during delivery. | | | |

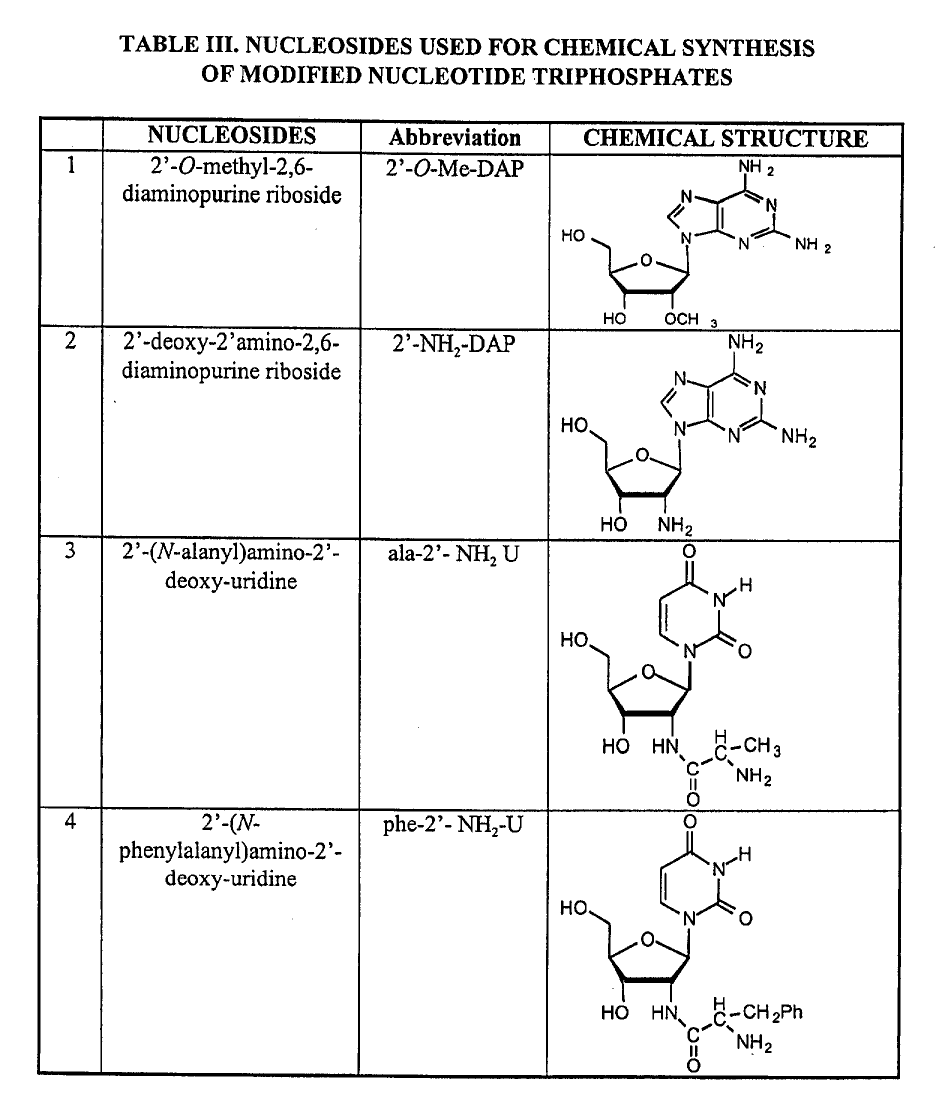

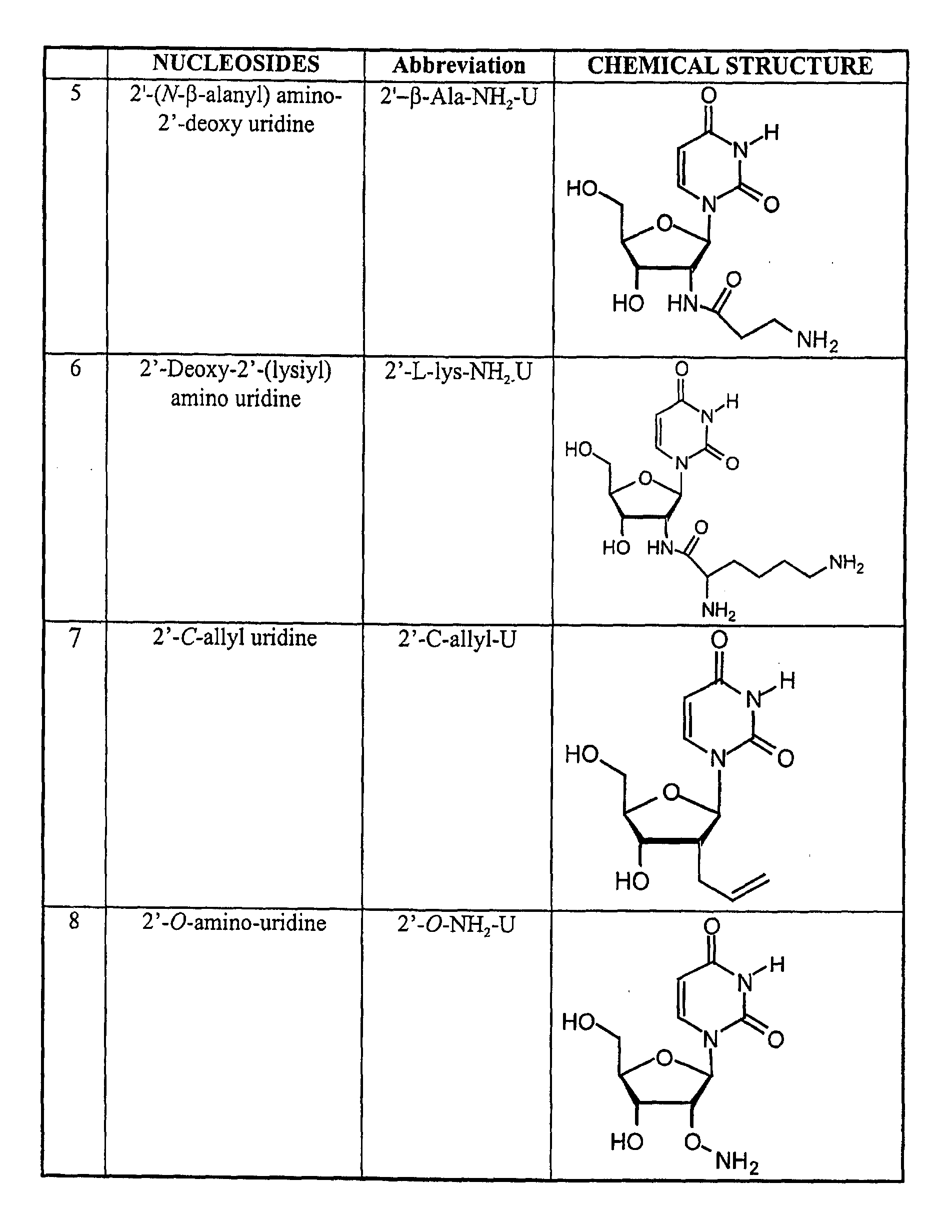

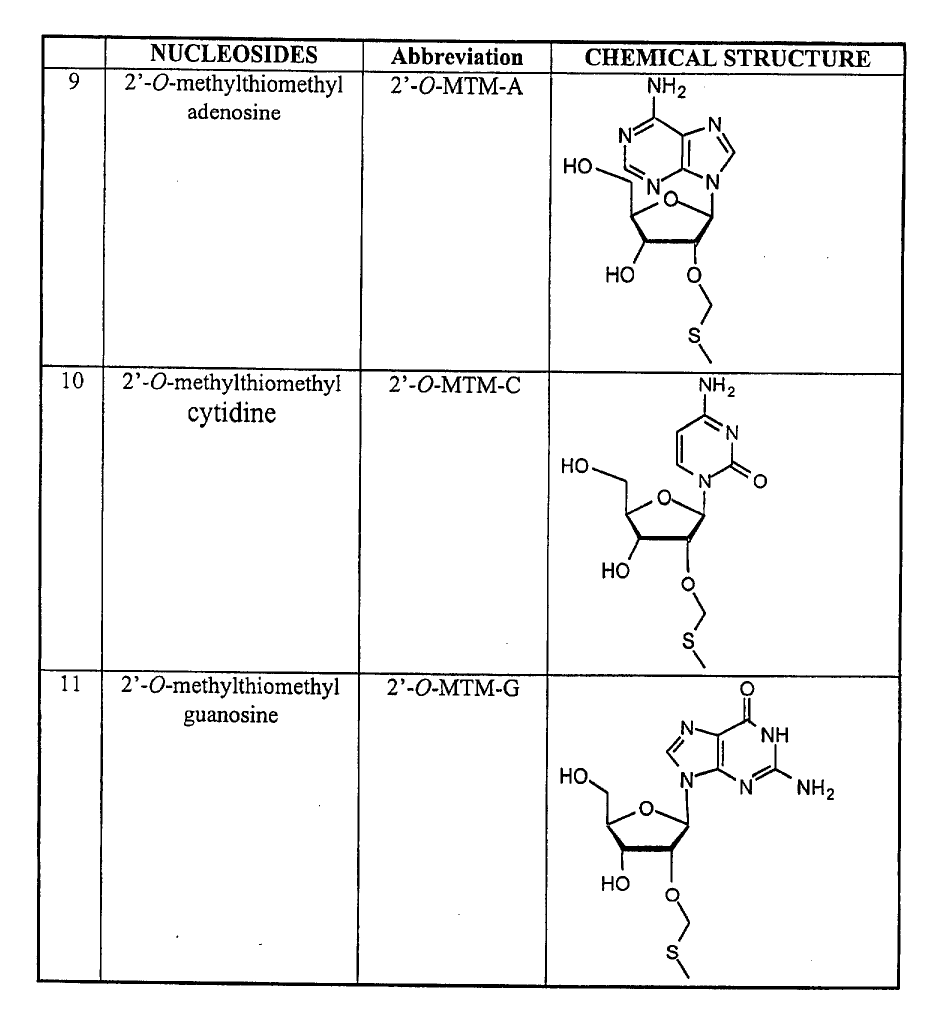

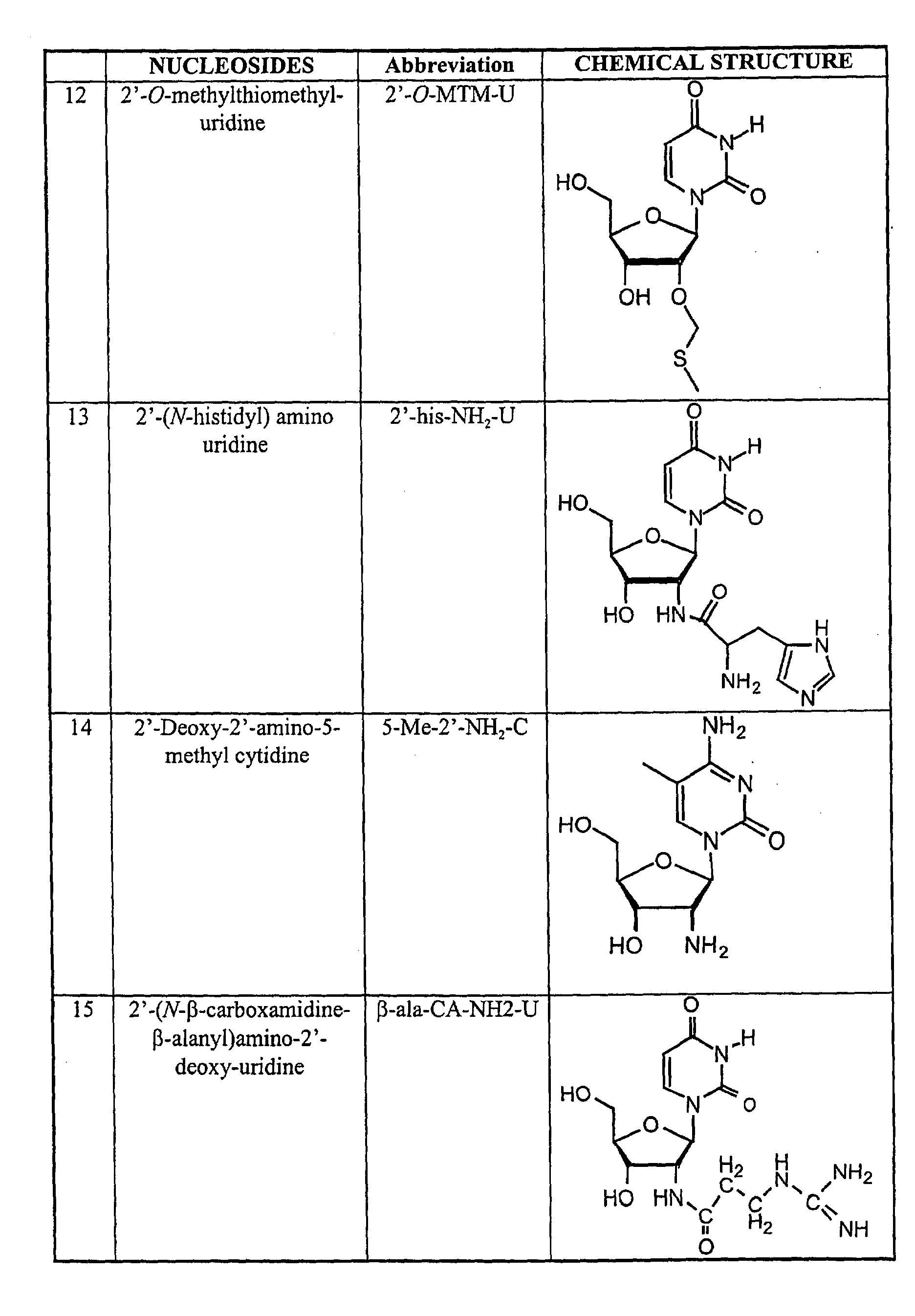

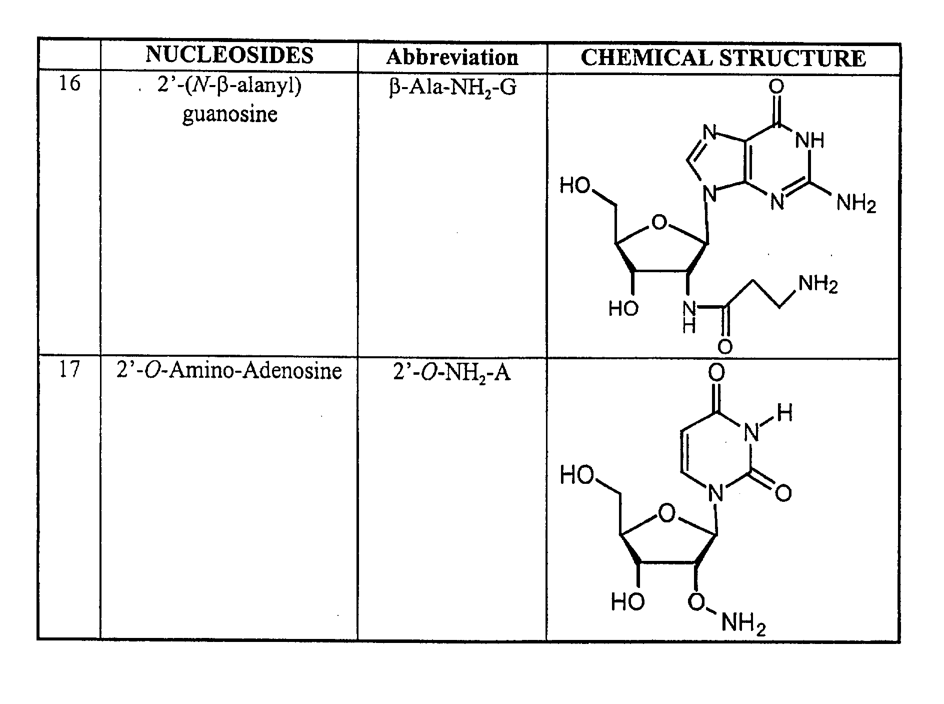

**Table VI.**

| **PHOSPHORYLATION OF URIDINE IN THE PRESENCE OF DMAP** | | | | | | | |
|---|---|---|---|---|---|---|---|
| 0 equiv. DMAP | | 0.2 equiv. DMAP | | 0.5 equiv. DMAP | | 1.0 equiv. DMAP | |
| Time (min) | Product % | Time (min) | Product % | Time (min) | Product % | Time (min) | Product % |
| 0 | 1 | 0 | 0 | 0 | 0 | 0 | 0 |
| 40 | 7 | 10 | 8 | 20 | 27 | 30 | 74 |
| 80 | 10 | 50 | 24 | 60 | 46 | 70 | 77 |
| 120 | 12 | 90 | 33 | 100 | 57 | **110** | **84** |
| 160 | 14 | 130 | 39 | 140 | 63 | 150 | 83 |
| 200 | 17 | 170 | 43 | 180 | 63 | 190 | 84 |
| 240 | 19 | 210 | 47 | 220 | 64 | 230 | 77 |
| 320 | 20 | 250 | 48 | 260 | 68 | 270 | 79 |
| **1130** | **48** | 290 | 49 | 300 | 64 | 310 | 77 |
| 1200 | 46 | 1140 | 68 | 1150 | 76 | 1160 | 72 |
| | | 1210 | 69 | 1220 | 76 | 1230 | 74 |

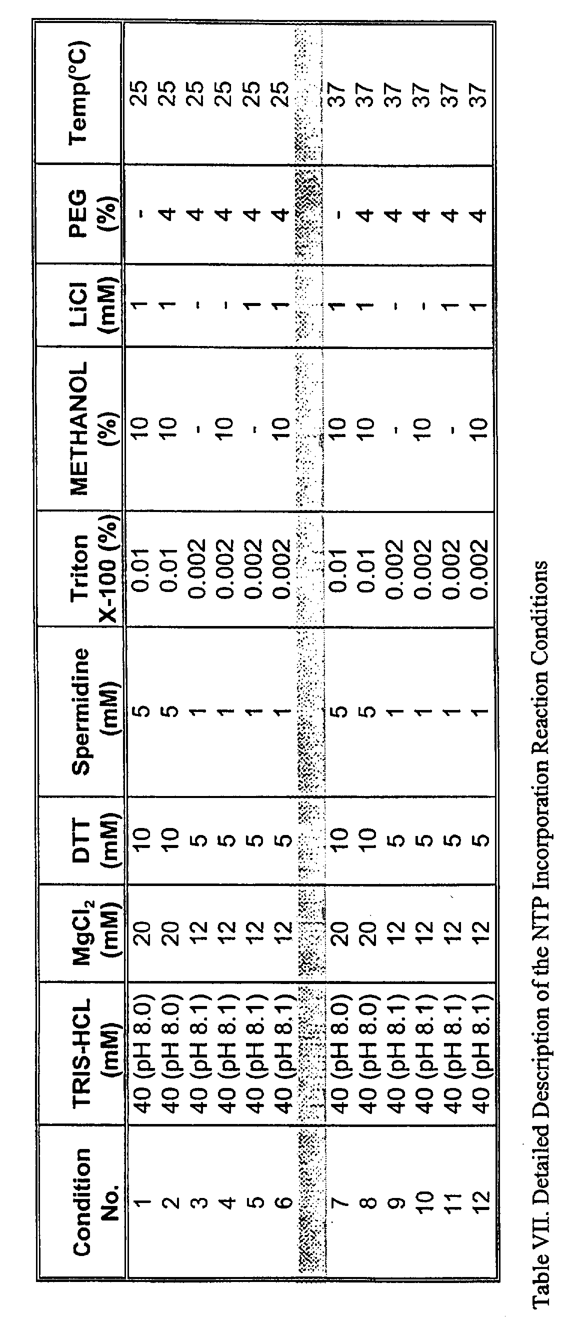

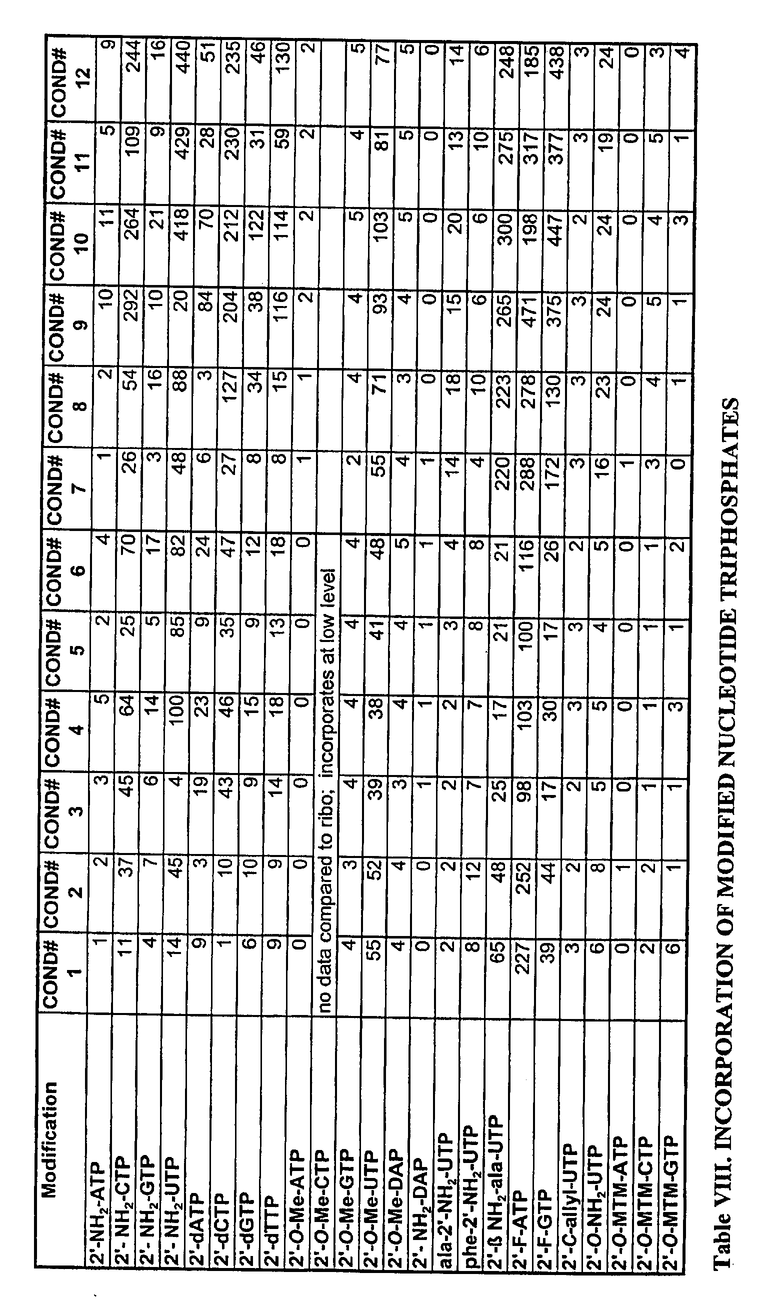

**Table IX:**

| **INCORPORATION OF MODIFIED** **NUCLEOTIDE TRIPHOSPHATES USING WILD TYPE** **BACTERIOPHAGE T7 POLYMERASE** | | |
|---|---|---|
| **Modification** | **label** | **% ribo control** |
| 2'-NH₂-GTP | ATP | 4% |
| 2'-dGTP | ATP | 3% |
| 2'-*O*-Me-GTP | ATP | 3% |
| 2'-F-GTP | ATP | 4% |
| 2'-*O*-MTM-GTP | ATP | 3% |
| 2'-NH₂-UTP | ATP | 39% |
| 2'-dTTP | ATP | 5% |
| 2'-*O*-Me-UTP | ATP | 3% |
| ala-2'-NH₂-UTP | ATP | 2% |
| phe-2'-NH₂-UTP | ATP | 1% |
| 2'-β-ala-NH₂₋UTP | ATP | 3% |
| 2'-*C*-allyl-UTP | ATP | 2% |
| 2'-*O*-NH₂-UTP | ATP | 1% |
| 2'-*O*-MTM-UTP | ATP | 64% |
| 2'-NH₂-ATP | GTP | 1% |
| 2'-*O*-MTM-ATP | GTP | 1% |
| 2'-NH₂-CTP | GTP | 59% |
| 2'-dCTP | GTP | 40% |

**Table Xa:**

| **Incorporation of 2'-his-UTP and Modified CTP's** | | |
|---|---|---|
| **modification** | **2'-his-UTP** | **rUTP** |
| CTP | 16.1 | 100 |
| 2'-amino-CTP | **9.5*** | 232.7 |
| 2'-deoxy-CTP | **9.6*** | 130.1 |
| 2'-OMe-CTP | 1.9 | 6.2 |
| 2'-MTM-CTP | 5.9 | 5.1 |
| | | |
| control | 1.2 | |

**Table Xb:**

| **Incorporation of 2'-his-UTP, 2-amino CTP, and Modified ATP's** | | |
|---|---|---|
| **modification** | **2'-his-UTP and 2'-amino-CTP** | **rUTP and rCTP** |
| ATP | 15.7 | 100 |
| 2'-amino-ATP | 2.4 | 28.9 |
| 2'-deoxy-ATP | 2.3 | 146.3 |
| 2'-OMe-ATP | 2.7 | 15 |
| 2'-F-ATP | 4 | 222.6 |
| 2'-MTM-ATP | 4.7 | 15.3 |
| 2'-OMe-DAP | 1.9 | 5.7 |
| 2'-amino-DAP | **8.9*** | 9.6 |

| | | |
|---|---|---|
| Numbers shown are a percentage of incorporation compared to the all-RNA control * -Bold number indicates best observed rate of modified nucleotide triphosphate incorporation | | |

**Table XI:**

| **INCORPORATION OF 2'-his-UTP, 2'-NH**_{**2**}**-CTP, 2'-NH**_{**2**}**-DAP,** **and rGTP USING VARIOUS REACTION CONDITIONS** | |
|---|---|
| **Conditions** | **compared to all rNTP** |
| 7 | 8.7* |
| 8 | 7* |
| 9 | 2.3 |
| 10 | 2.7 |
| 11 | 1.6 |
| 12 | 2.5 |

| | |
|---|---|
| Numbers shown are a percentage of incorporation compared to the all-RNA control * Two highest levels of incorporation contained both methanol and LiCl | |

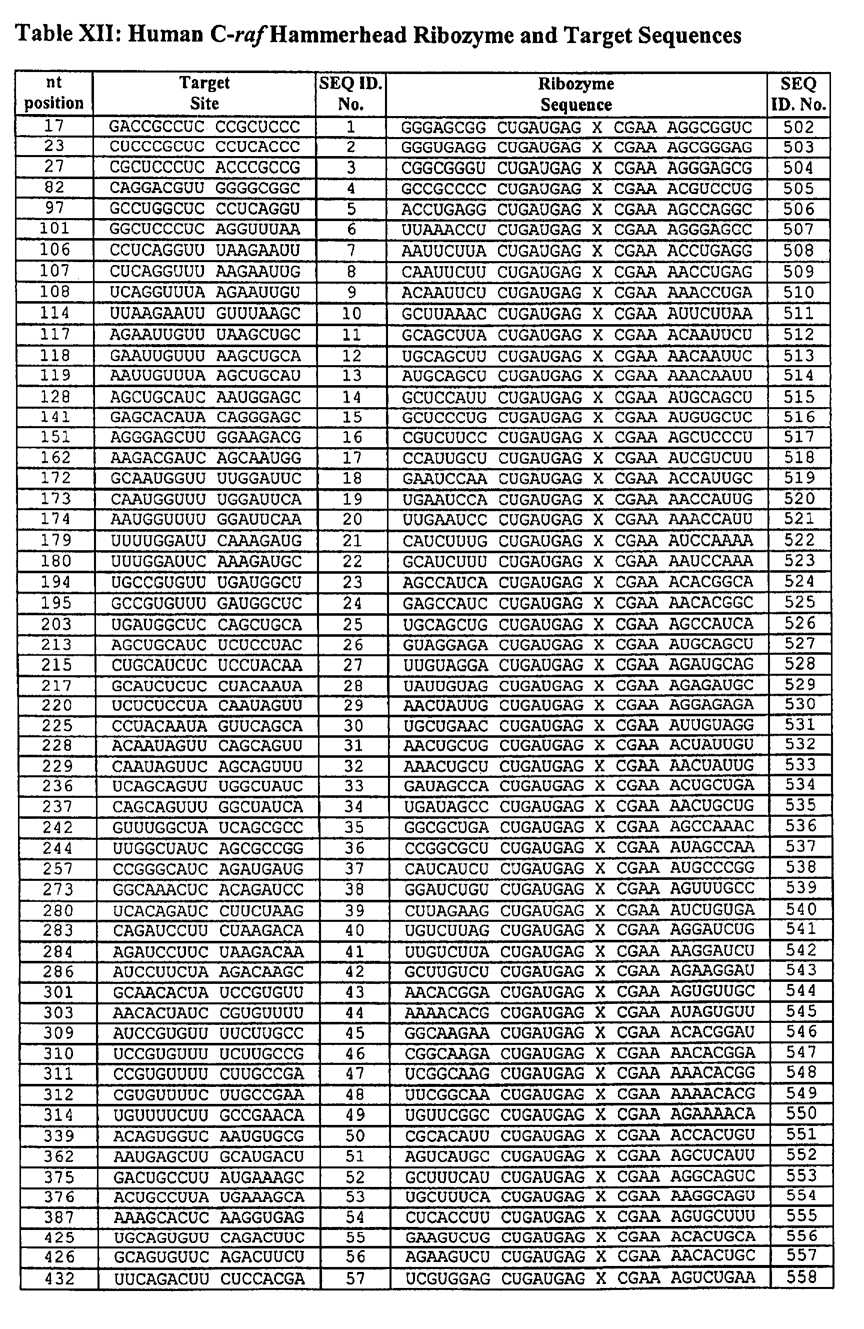

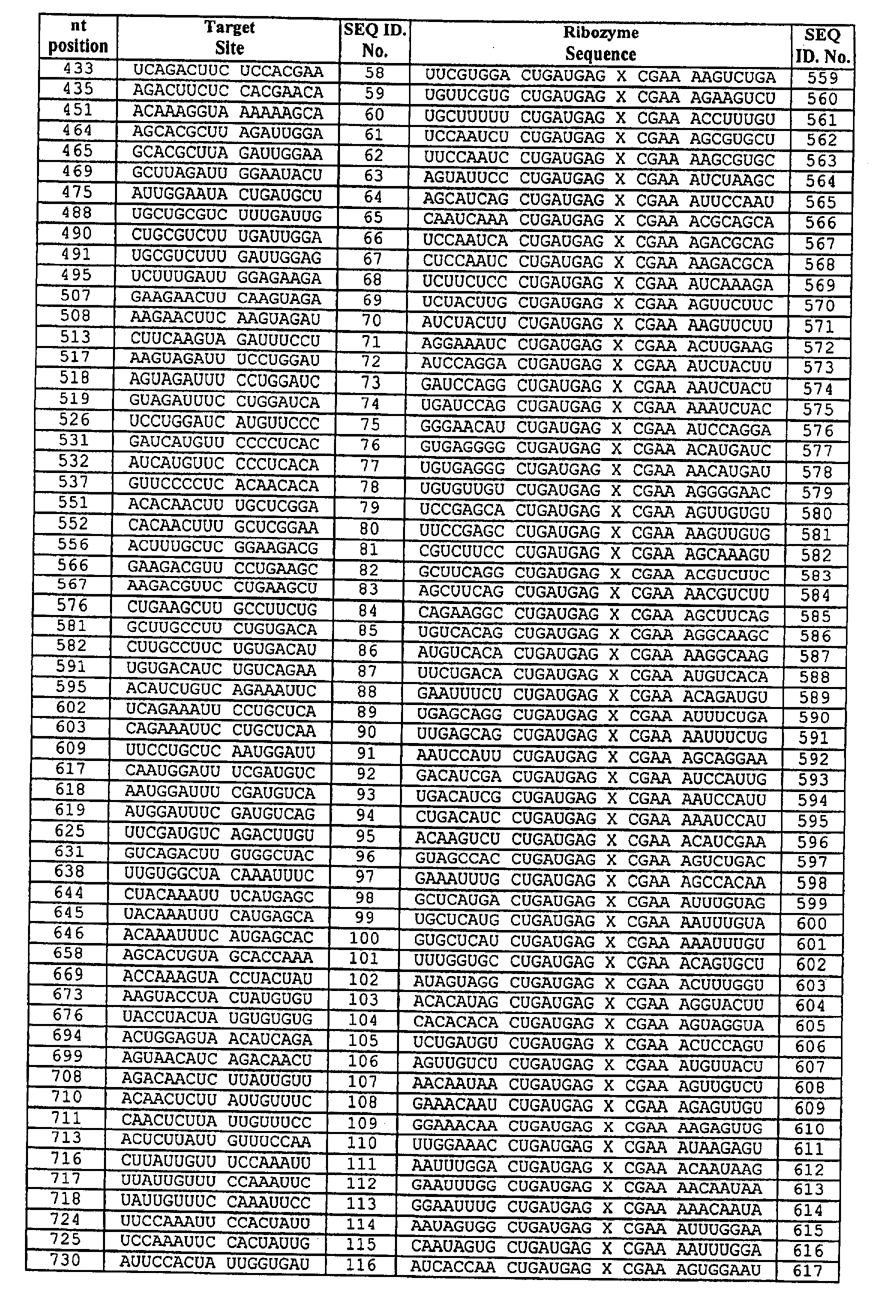

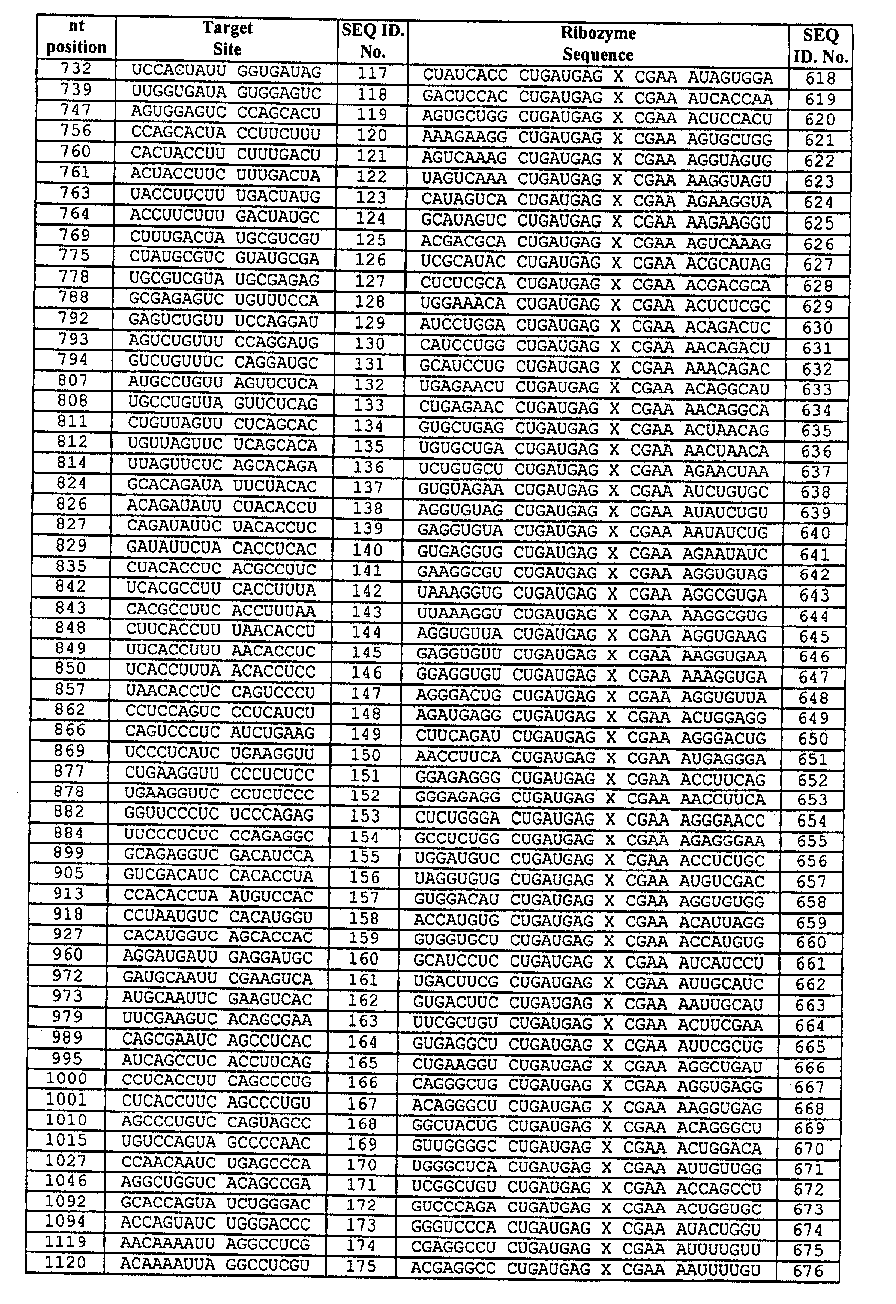

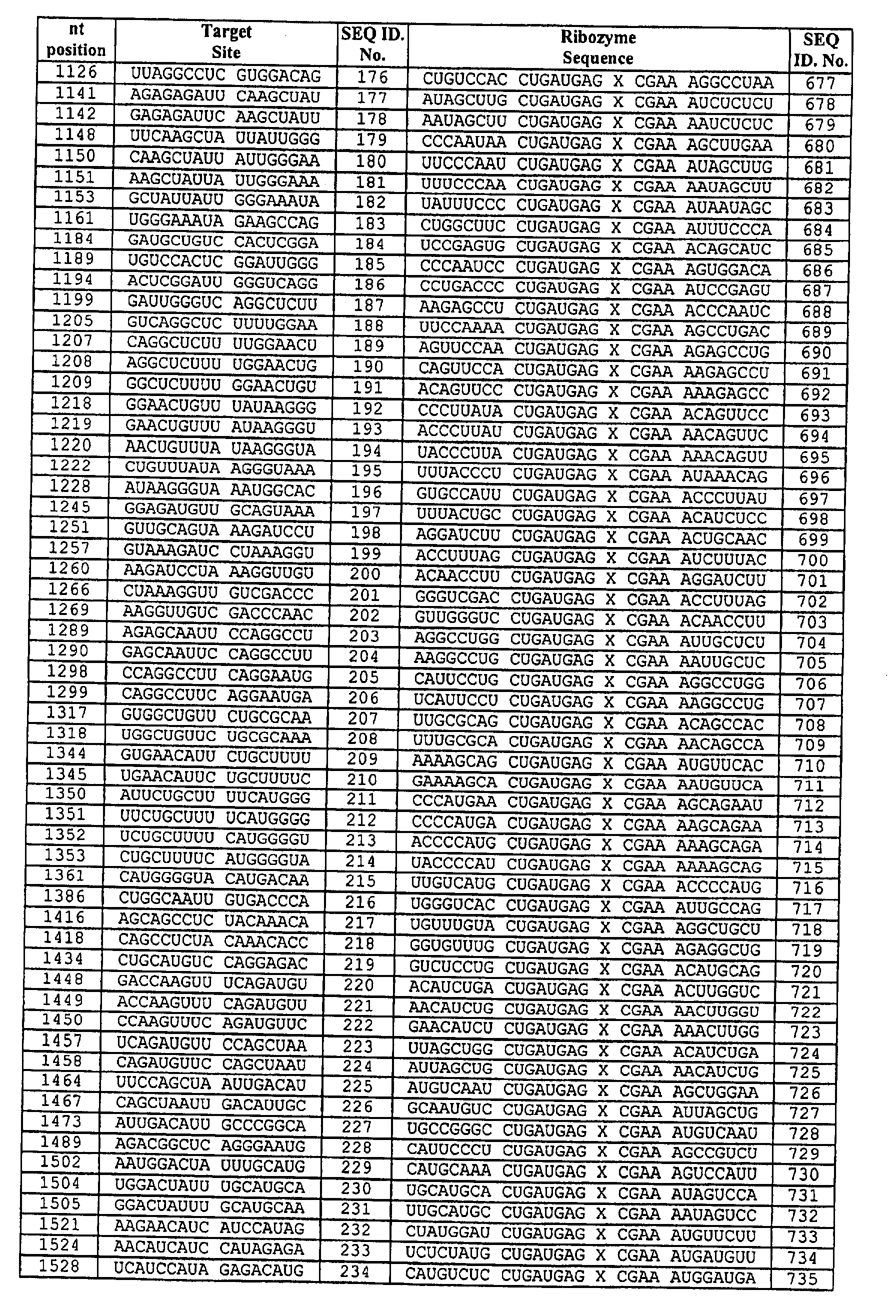

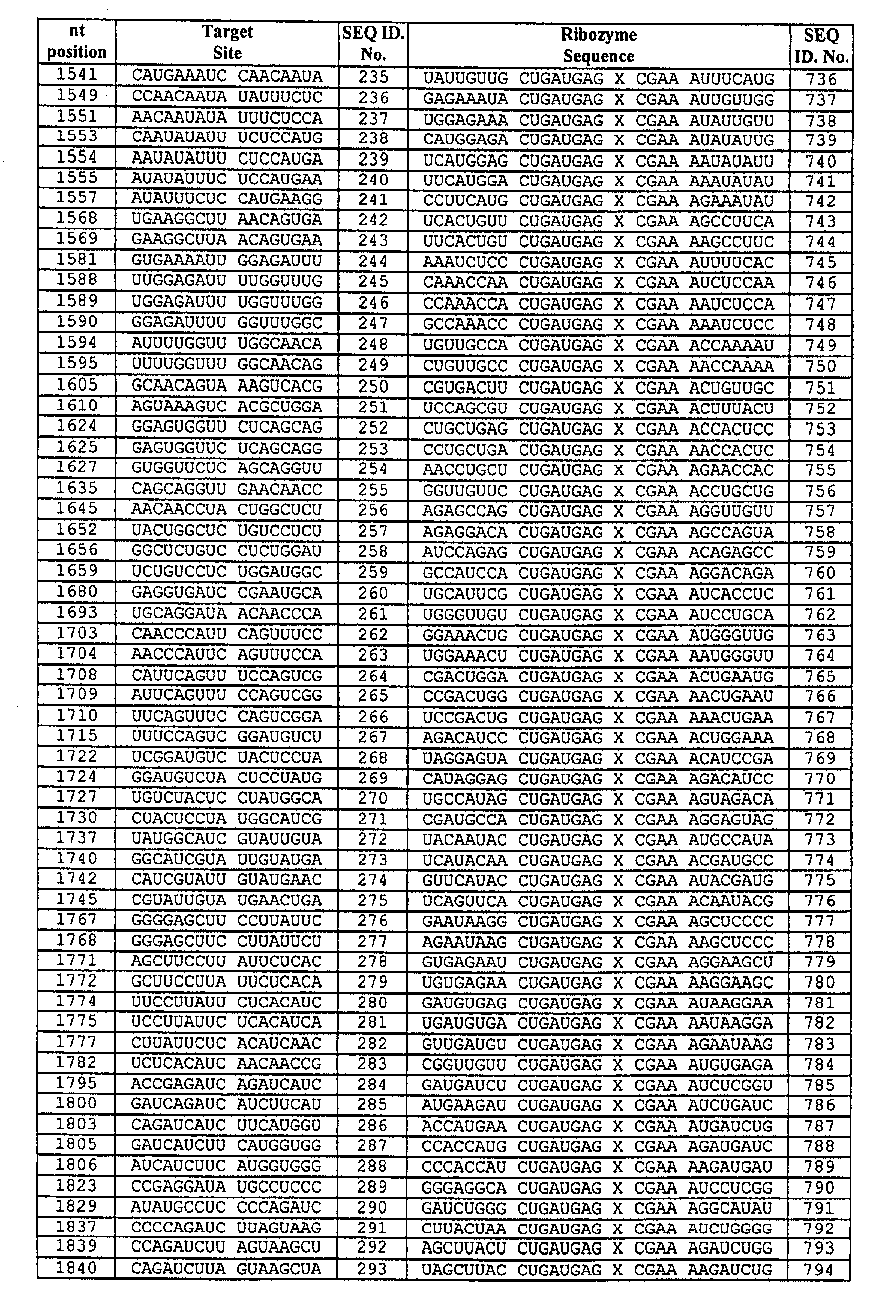

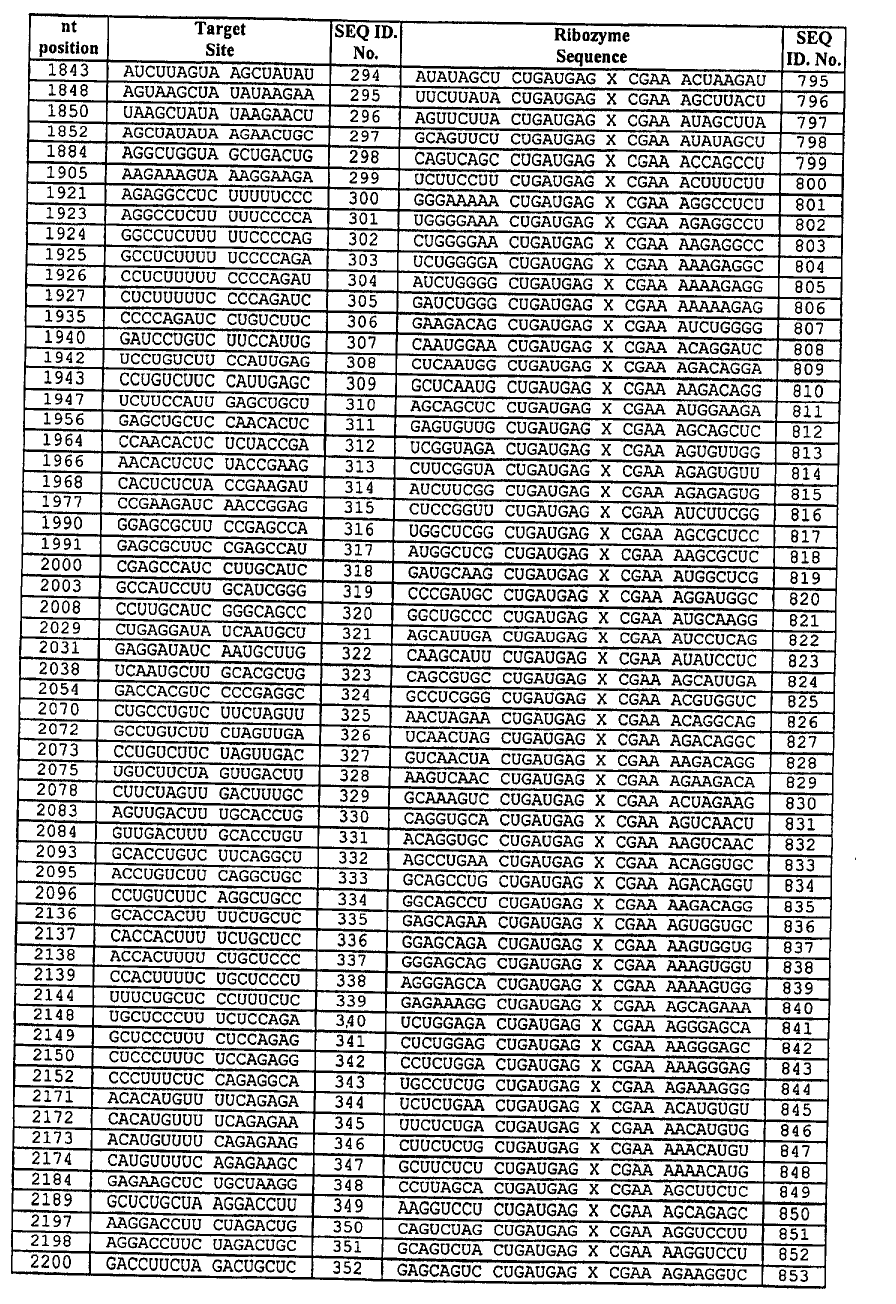

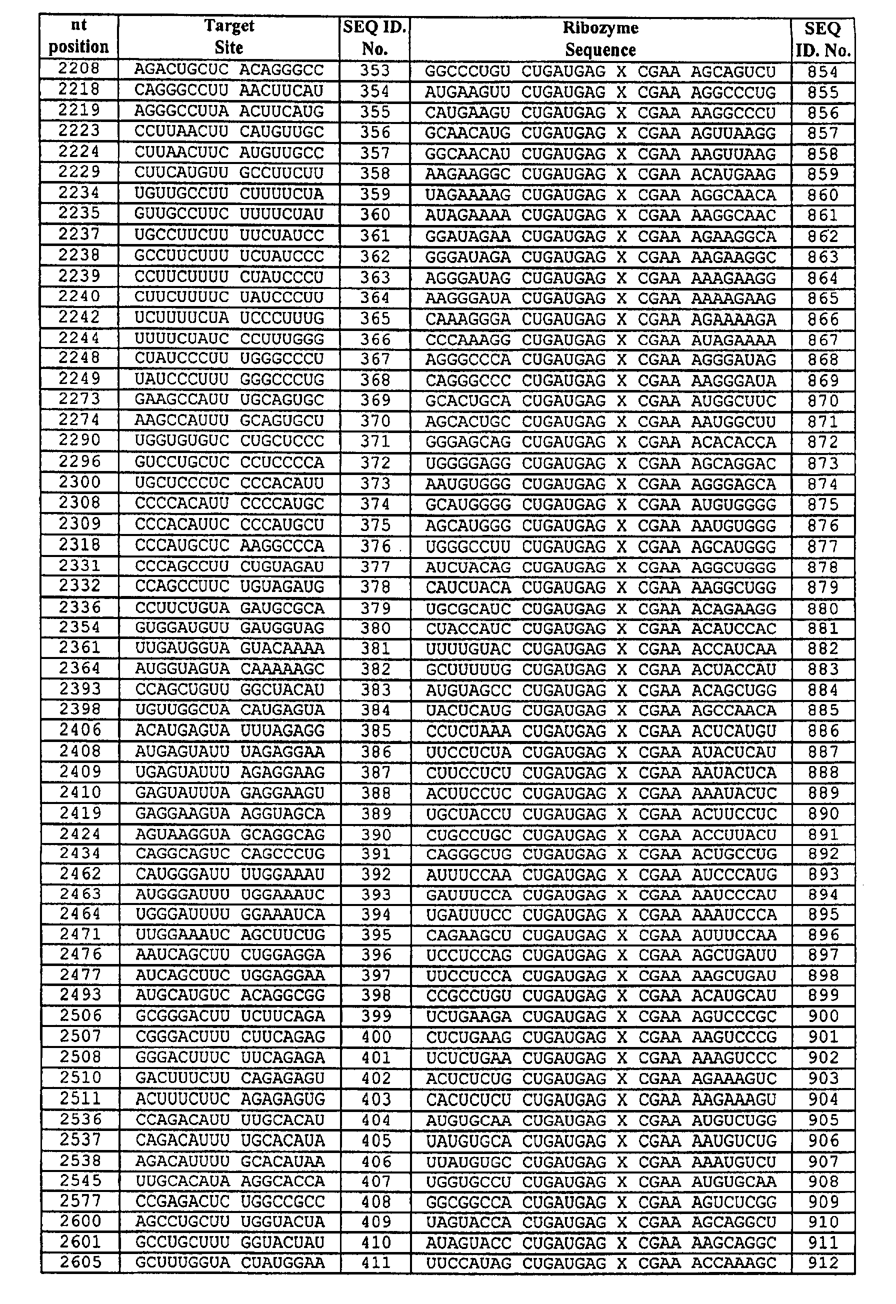

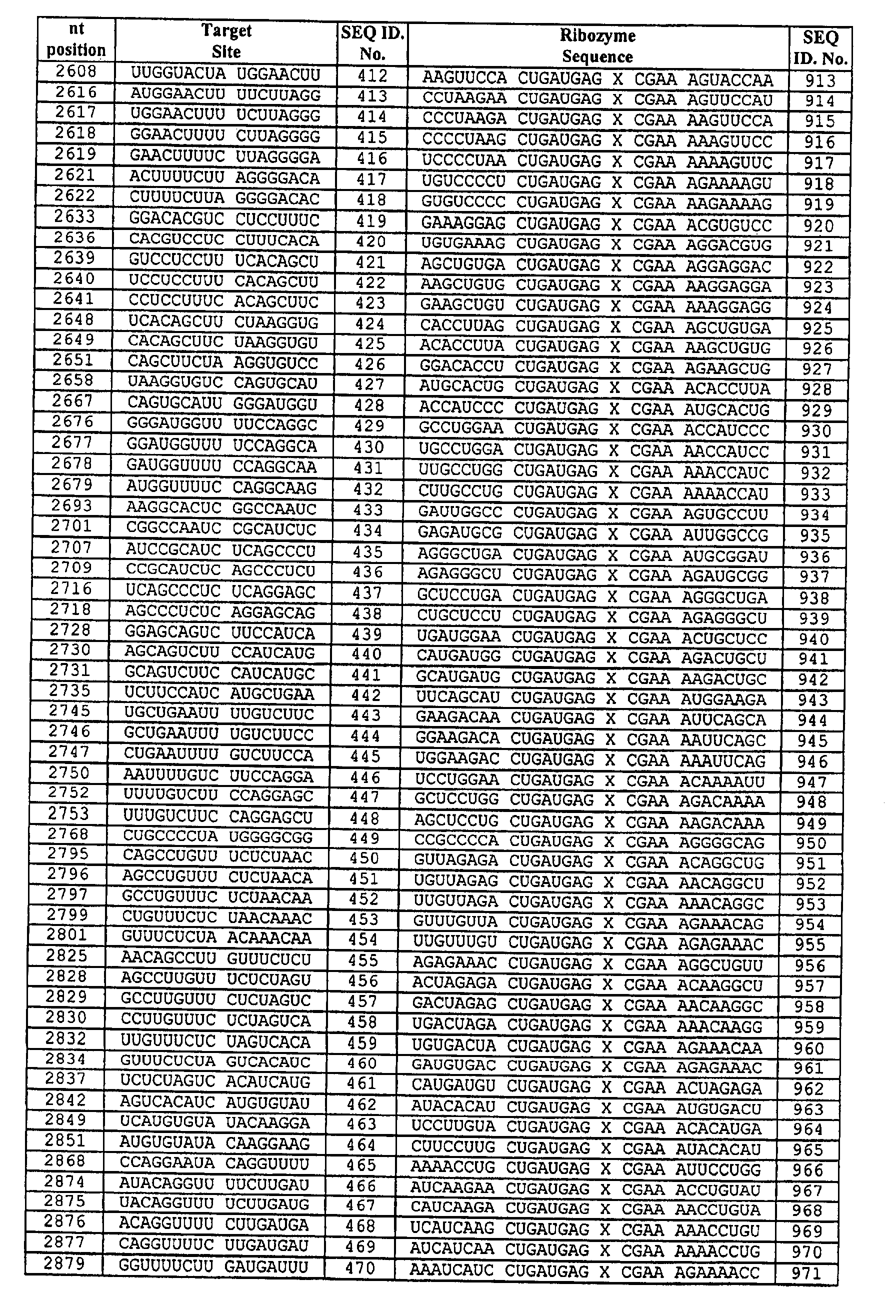

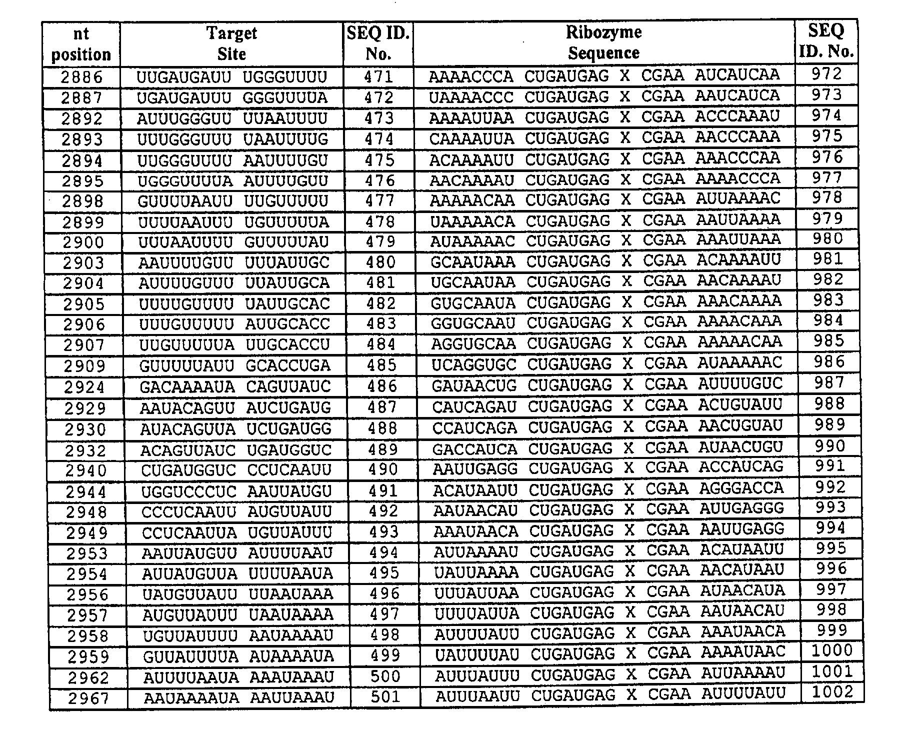

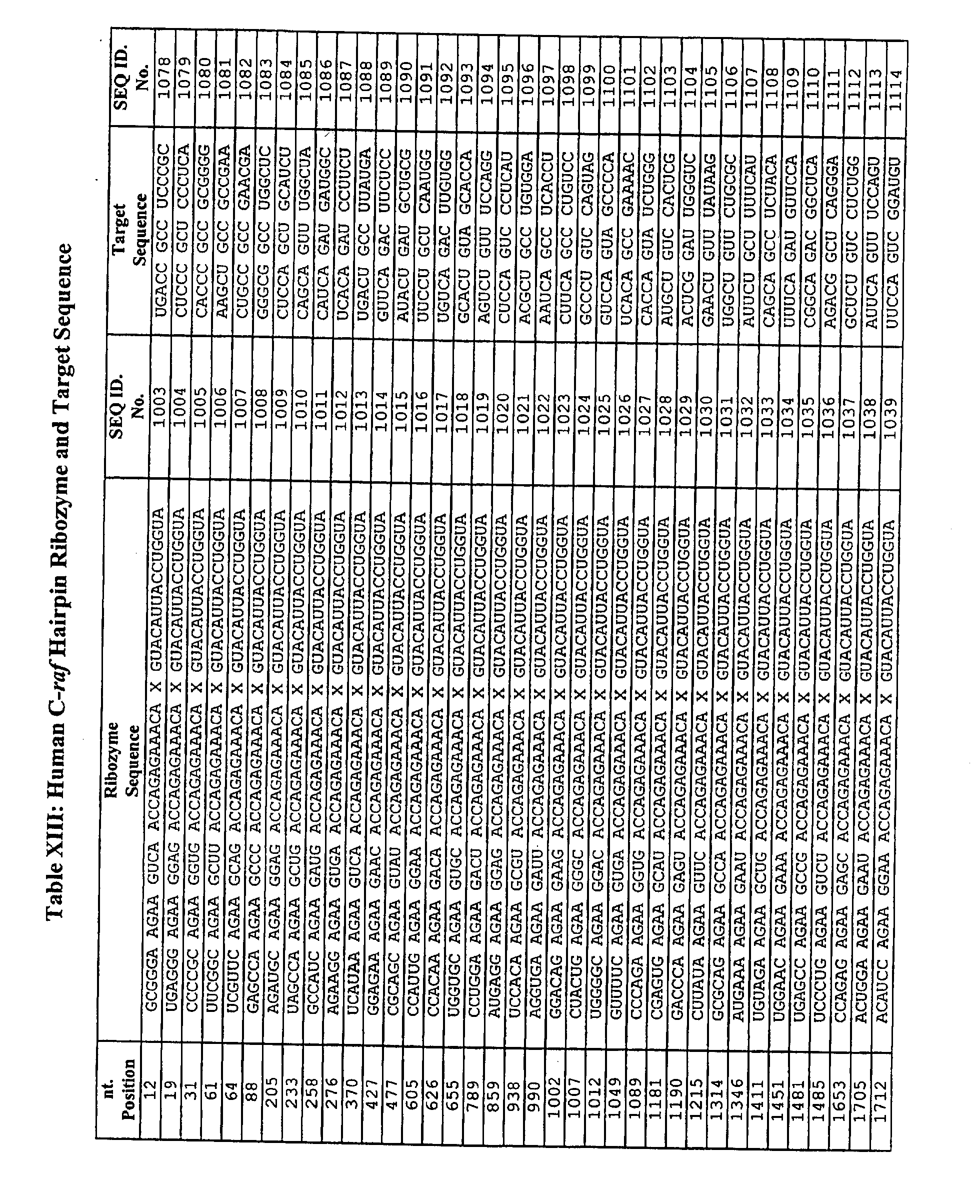

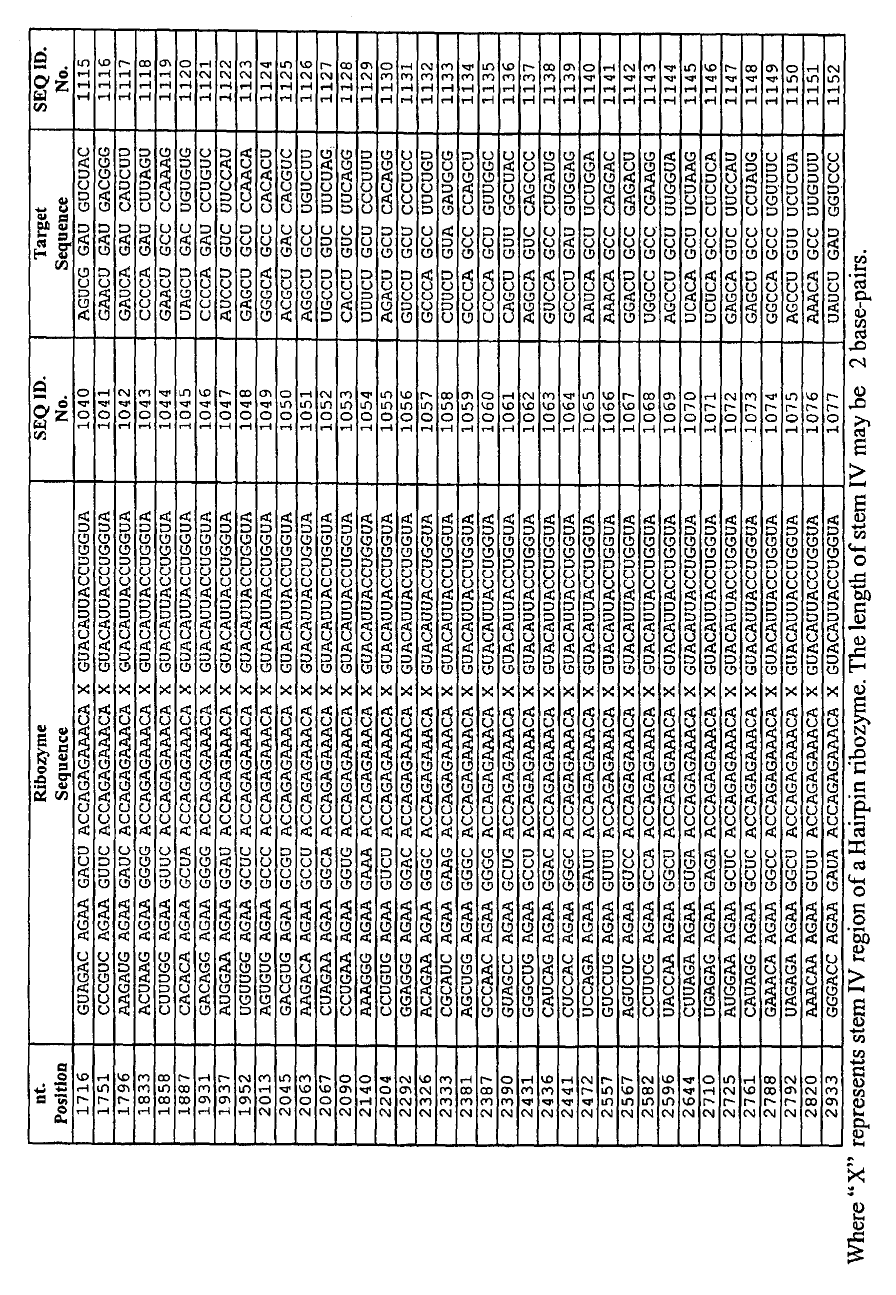

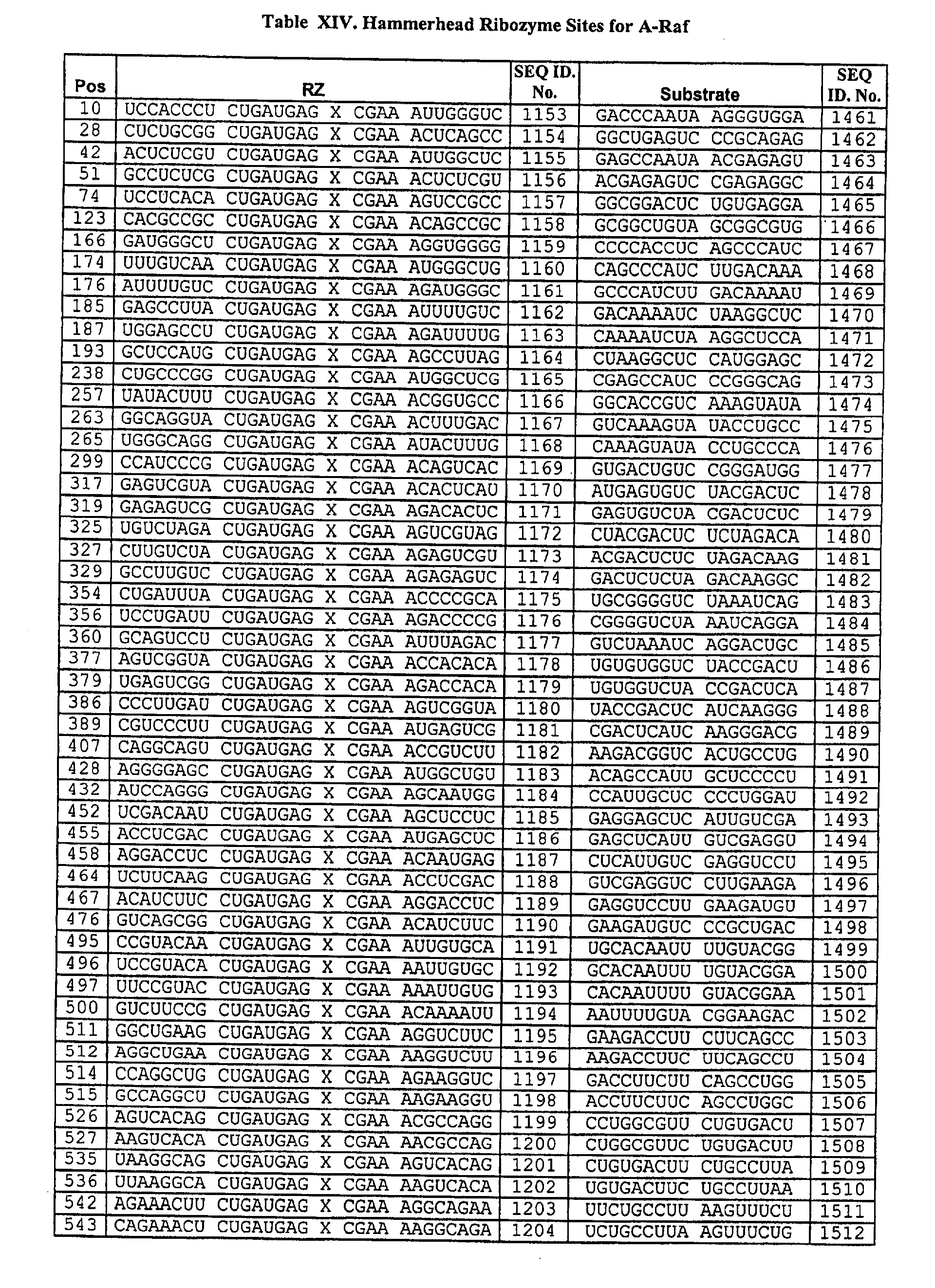

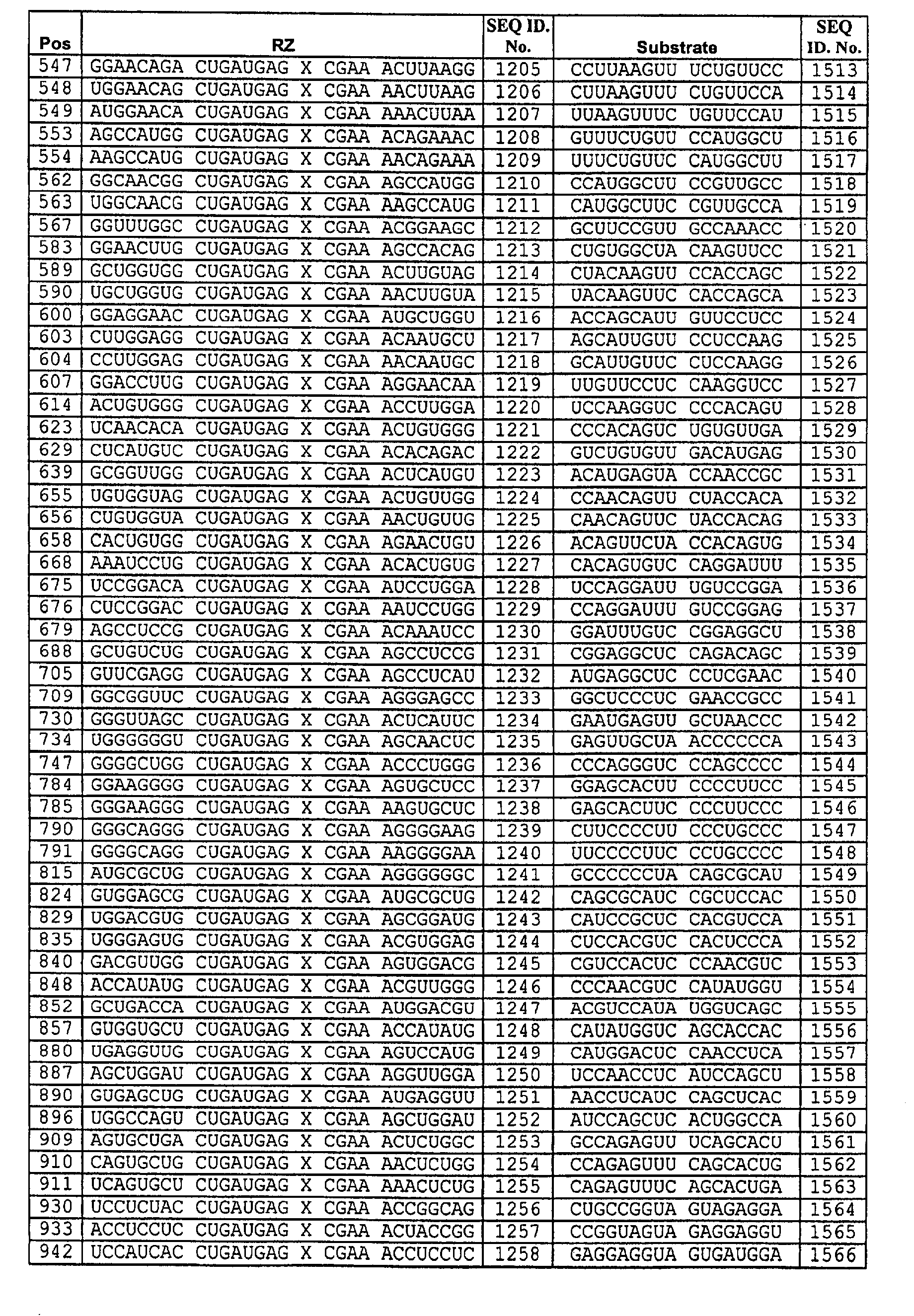

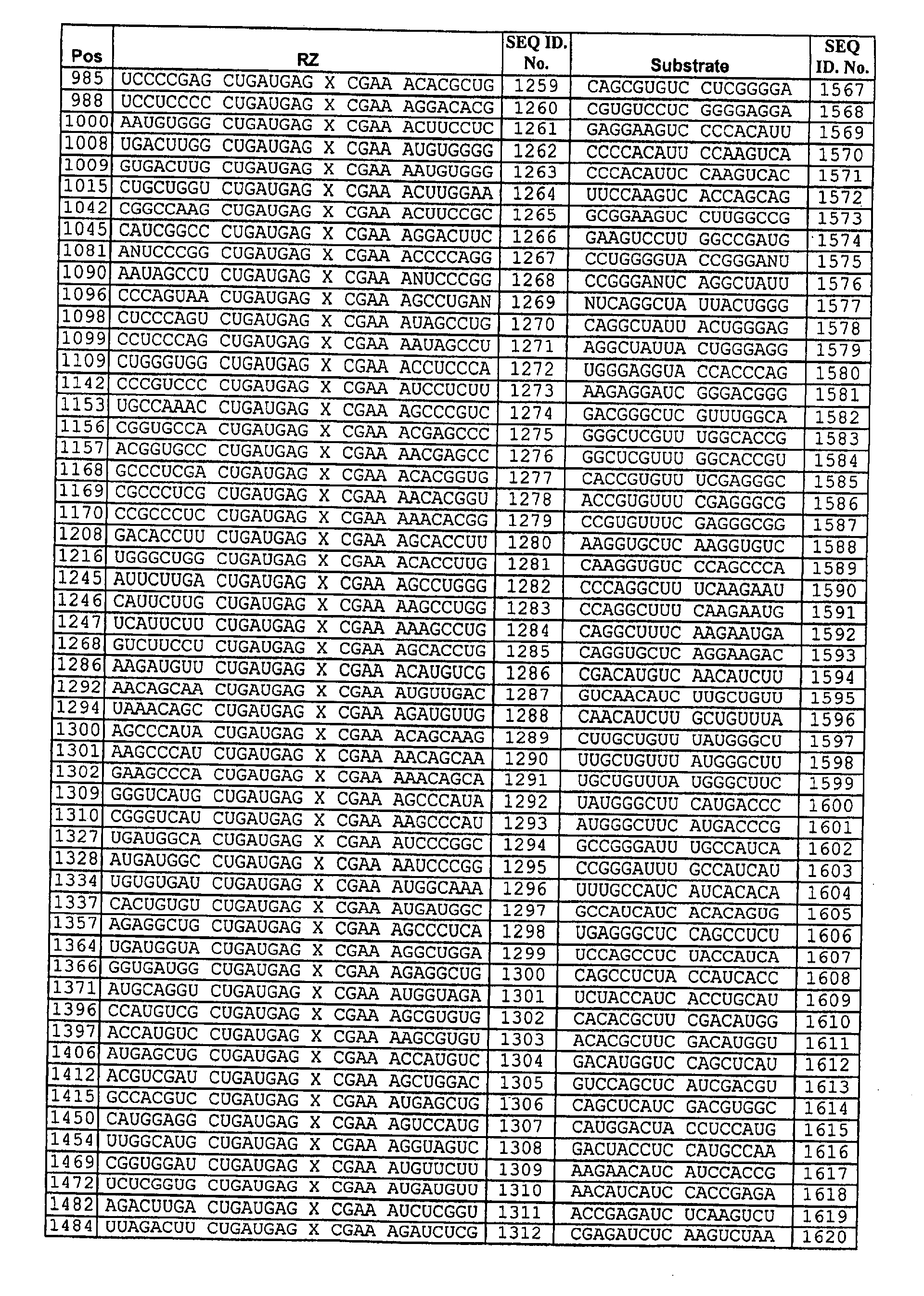

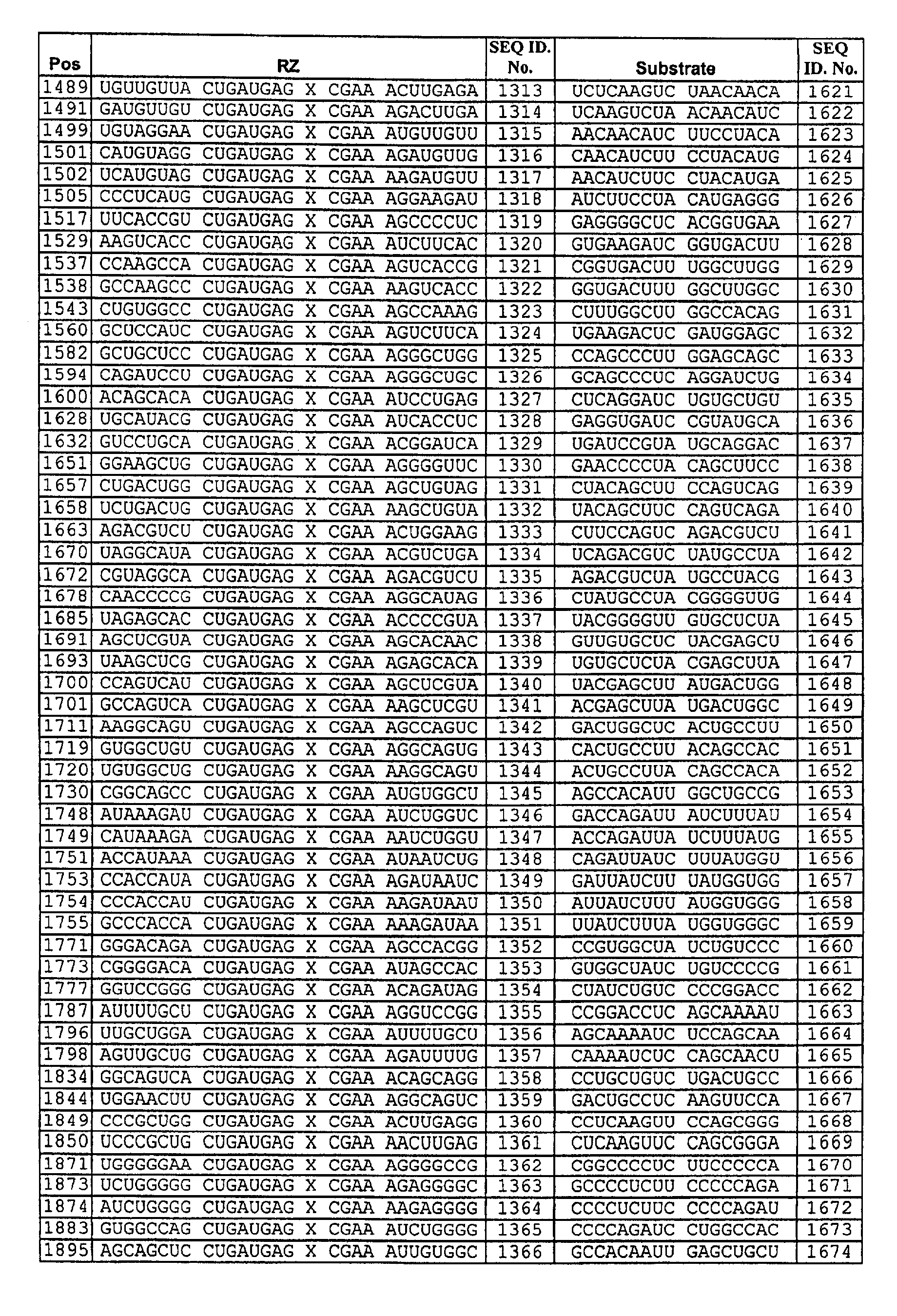

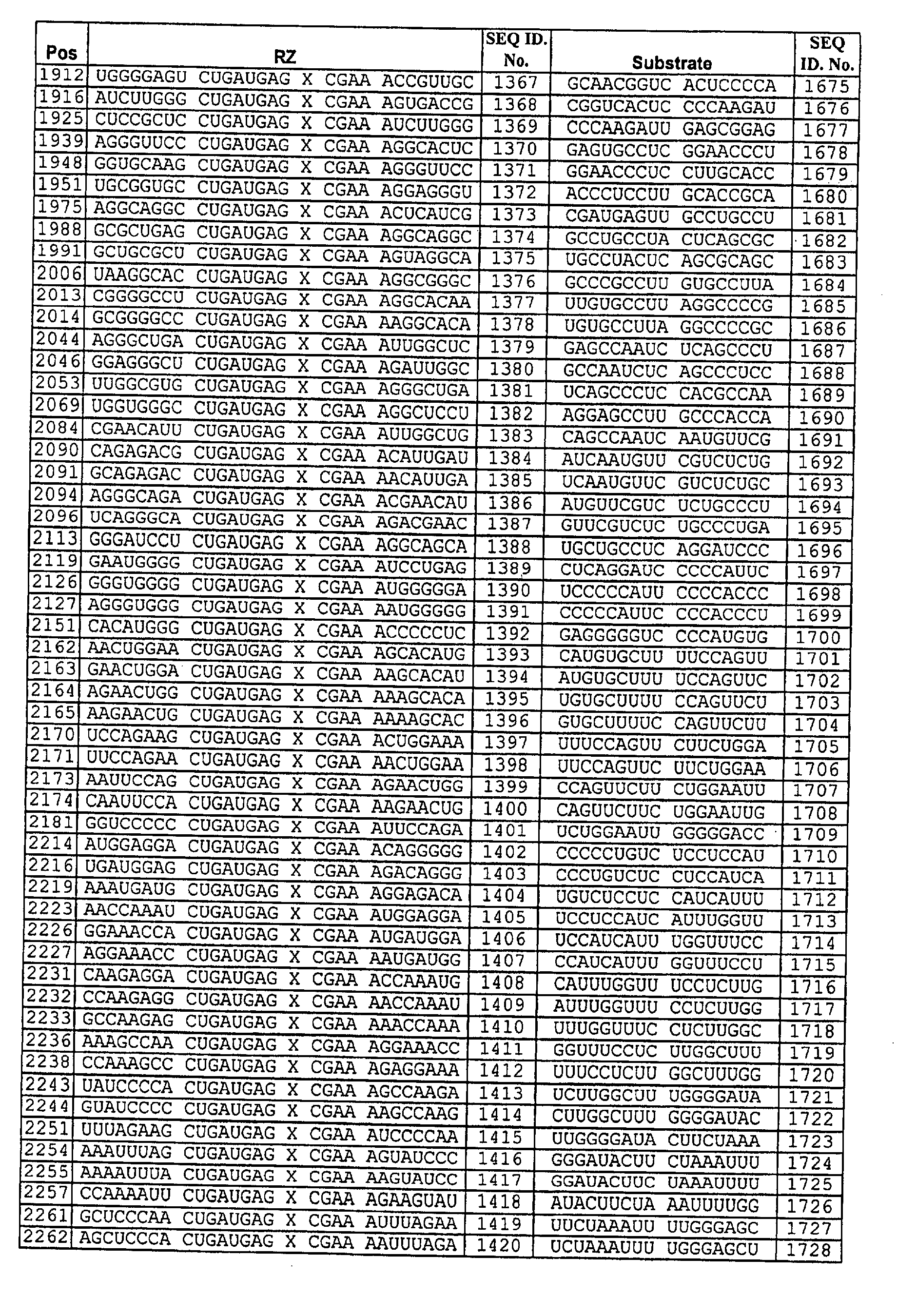

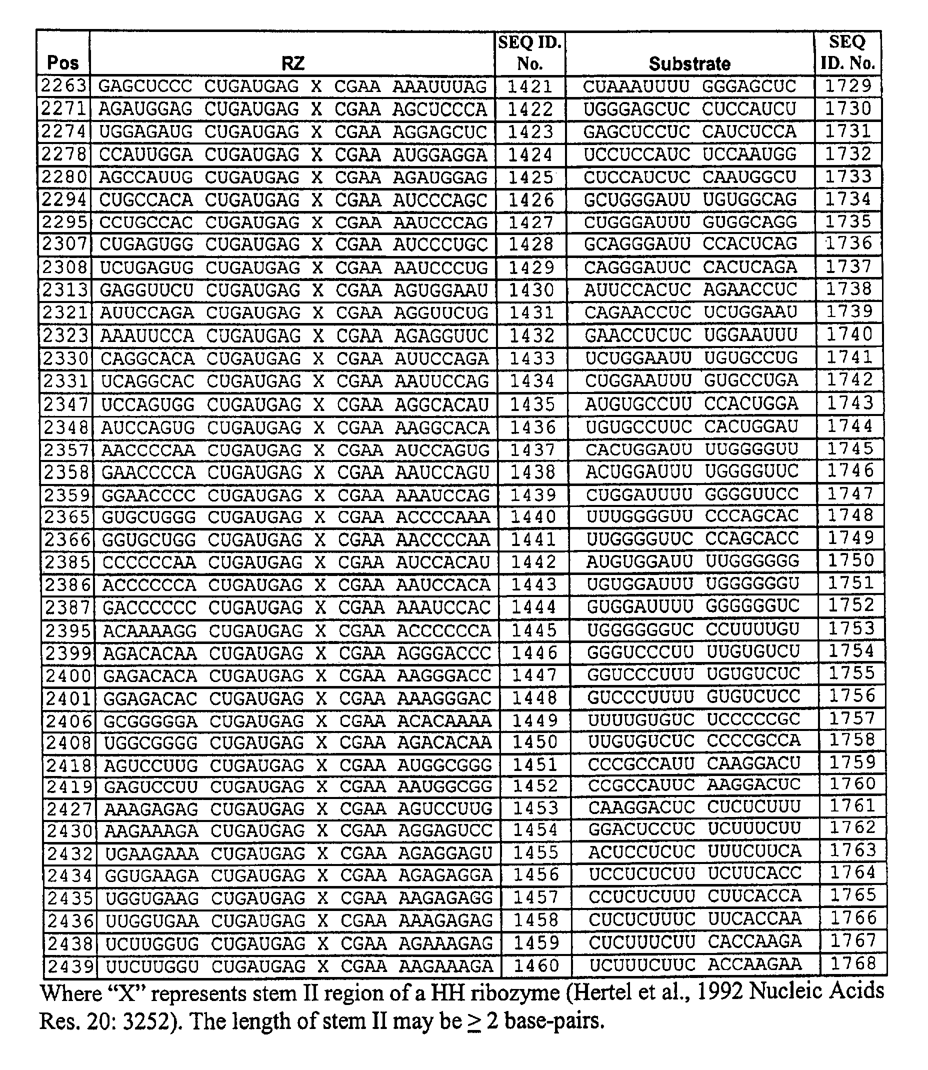

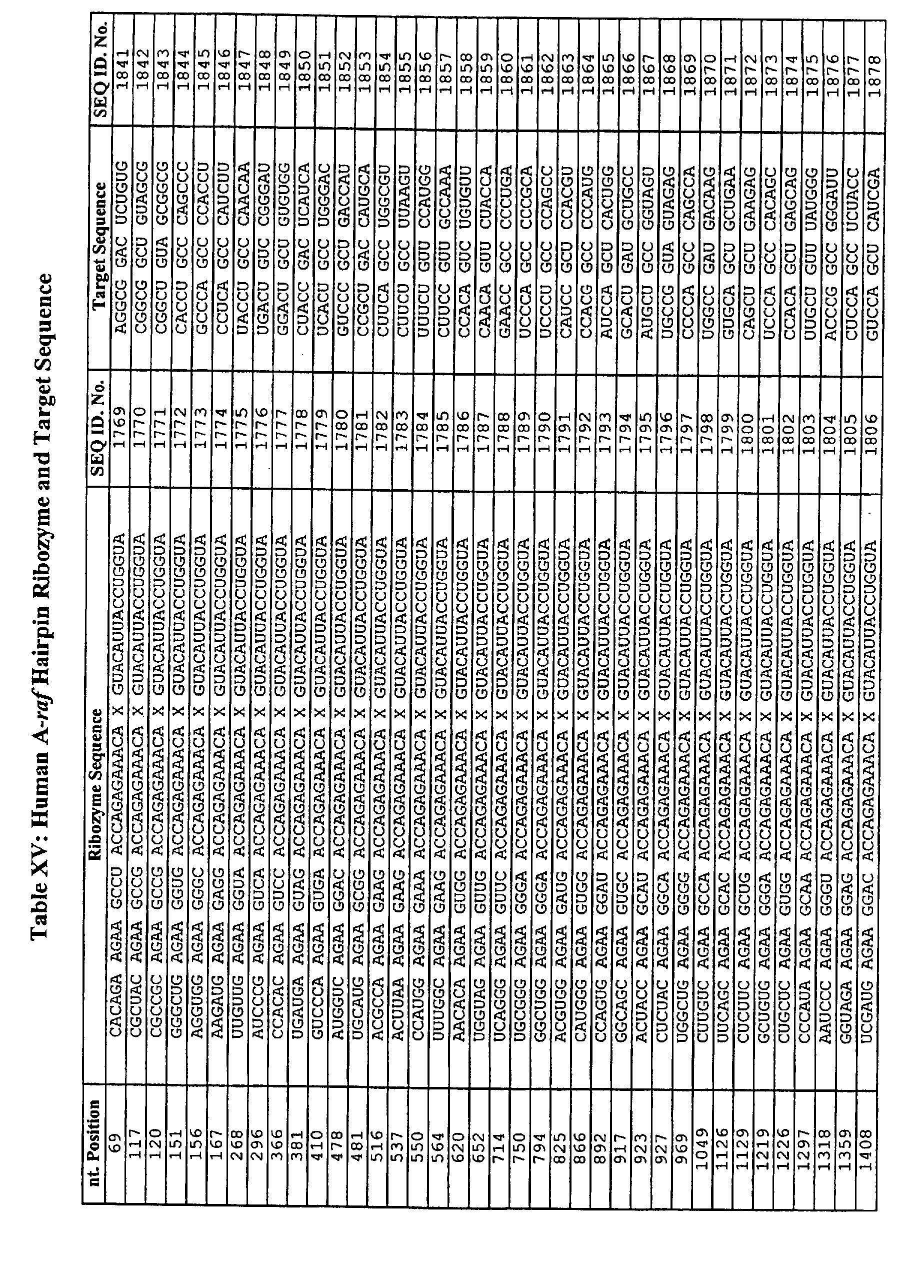

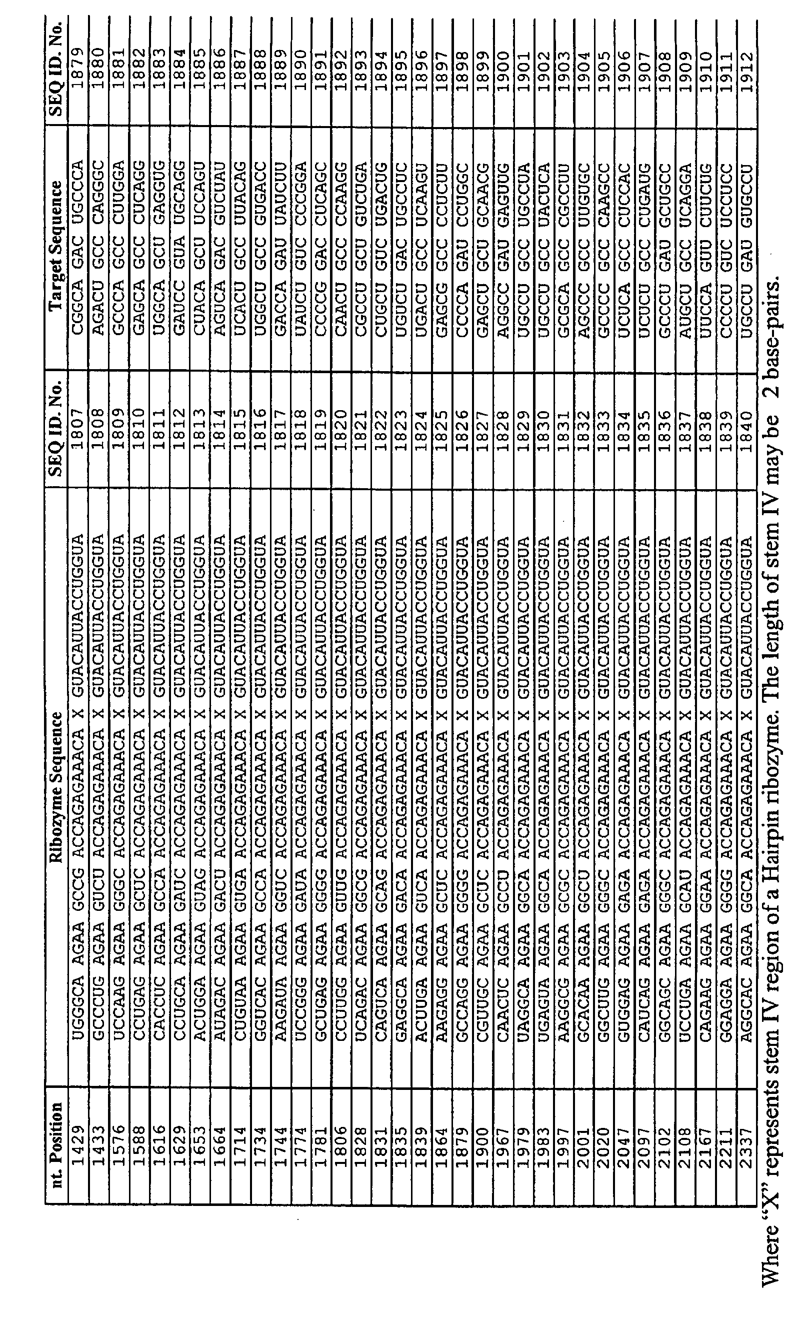

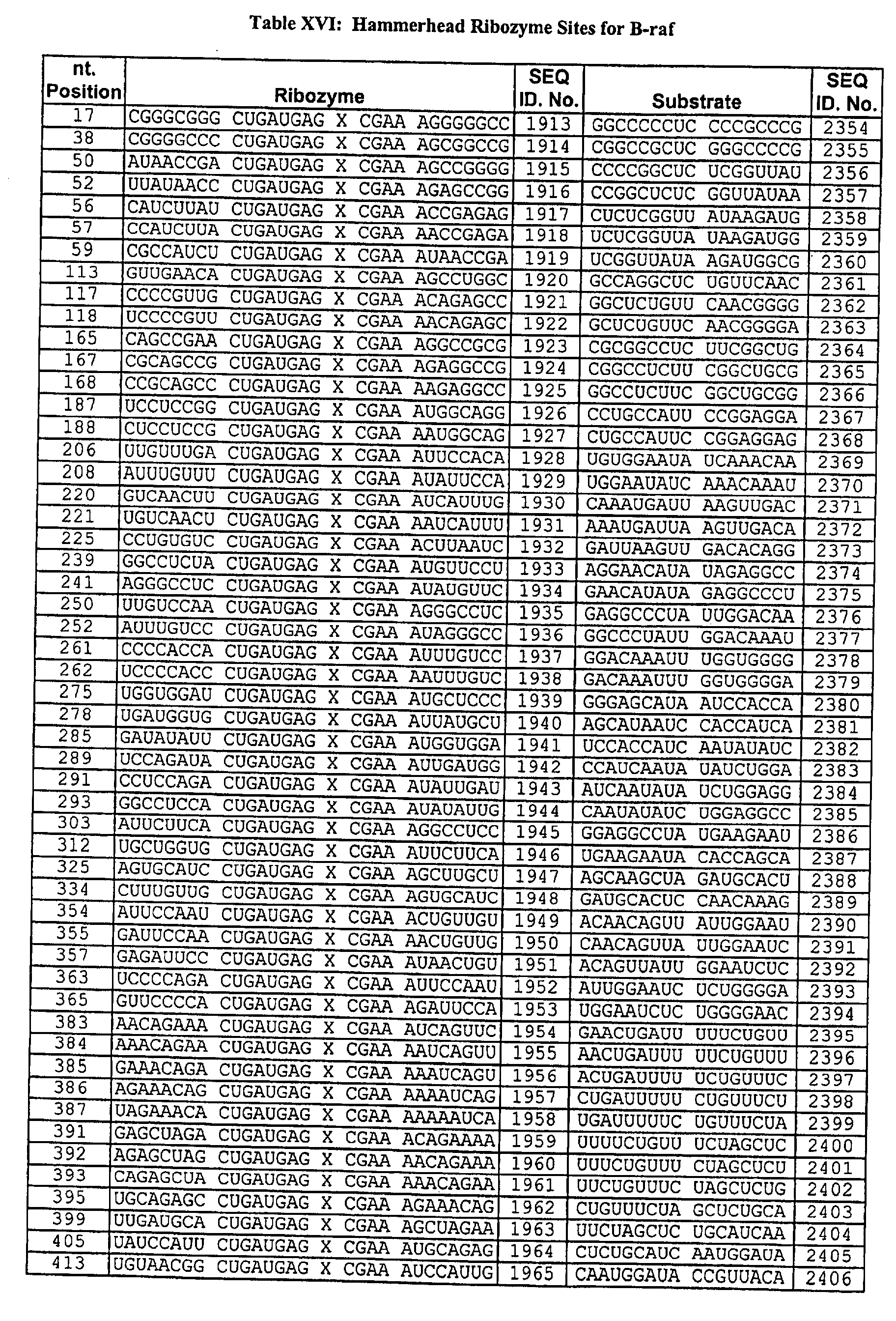

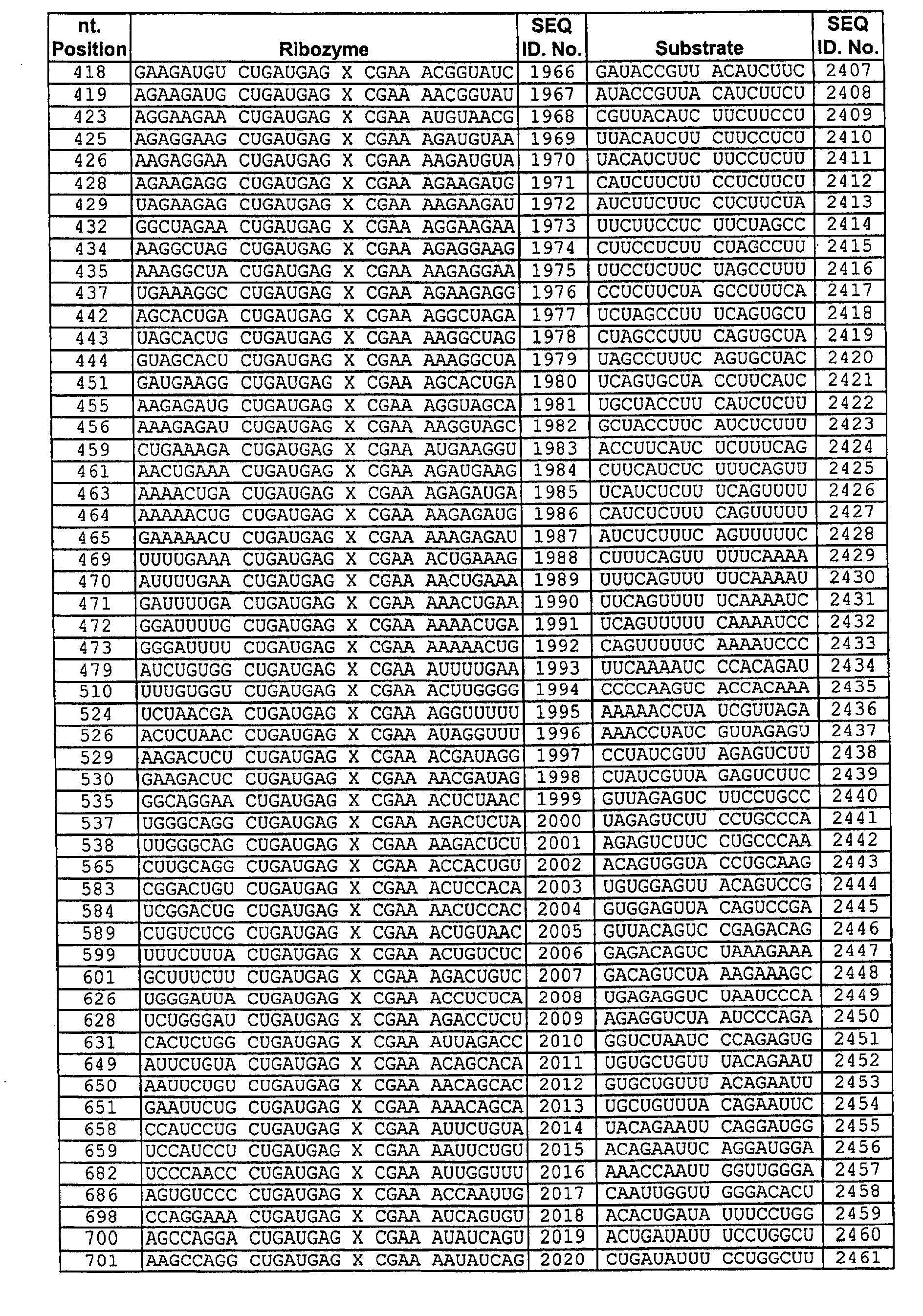

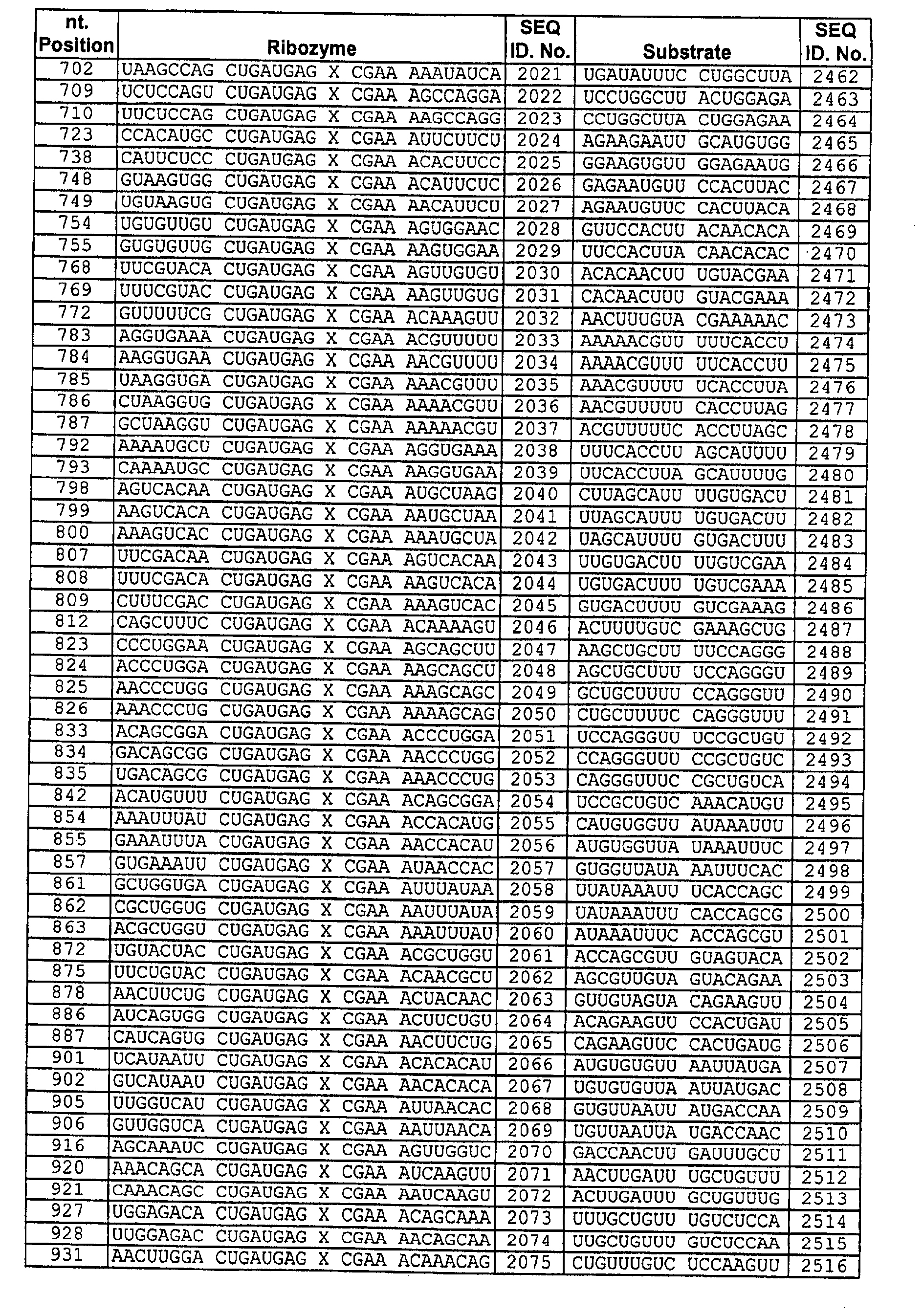

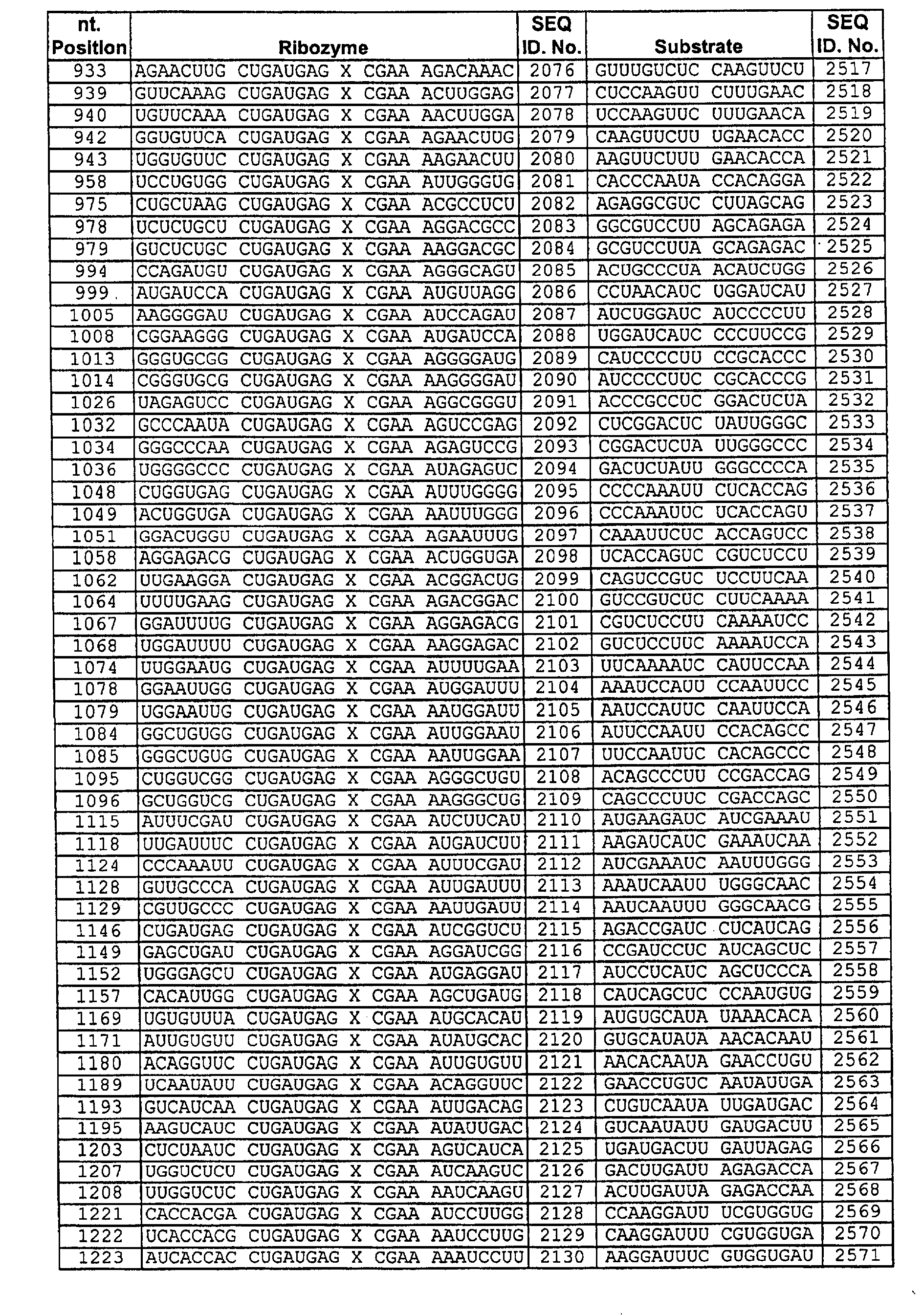

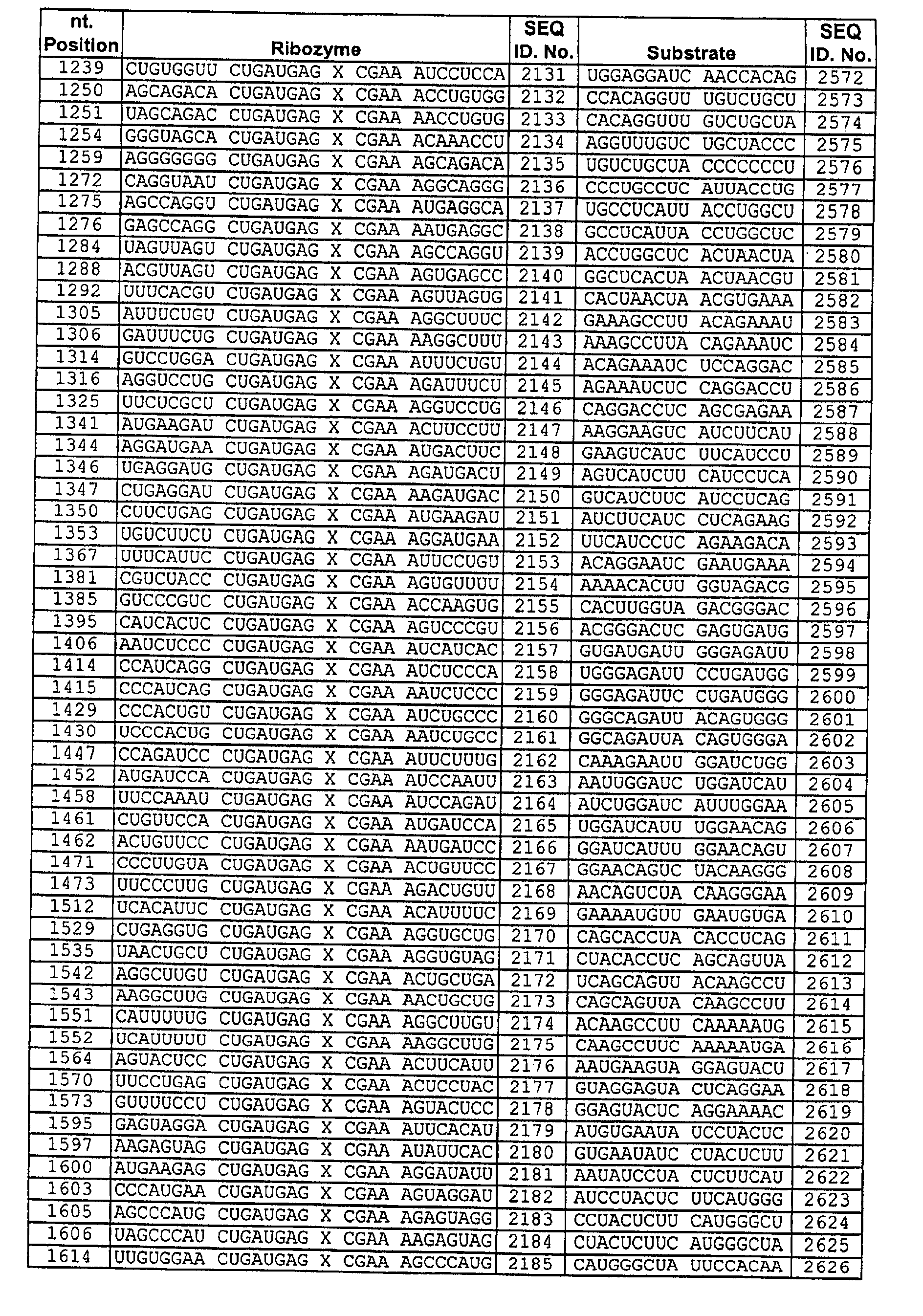

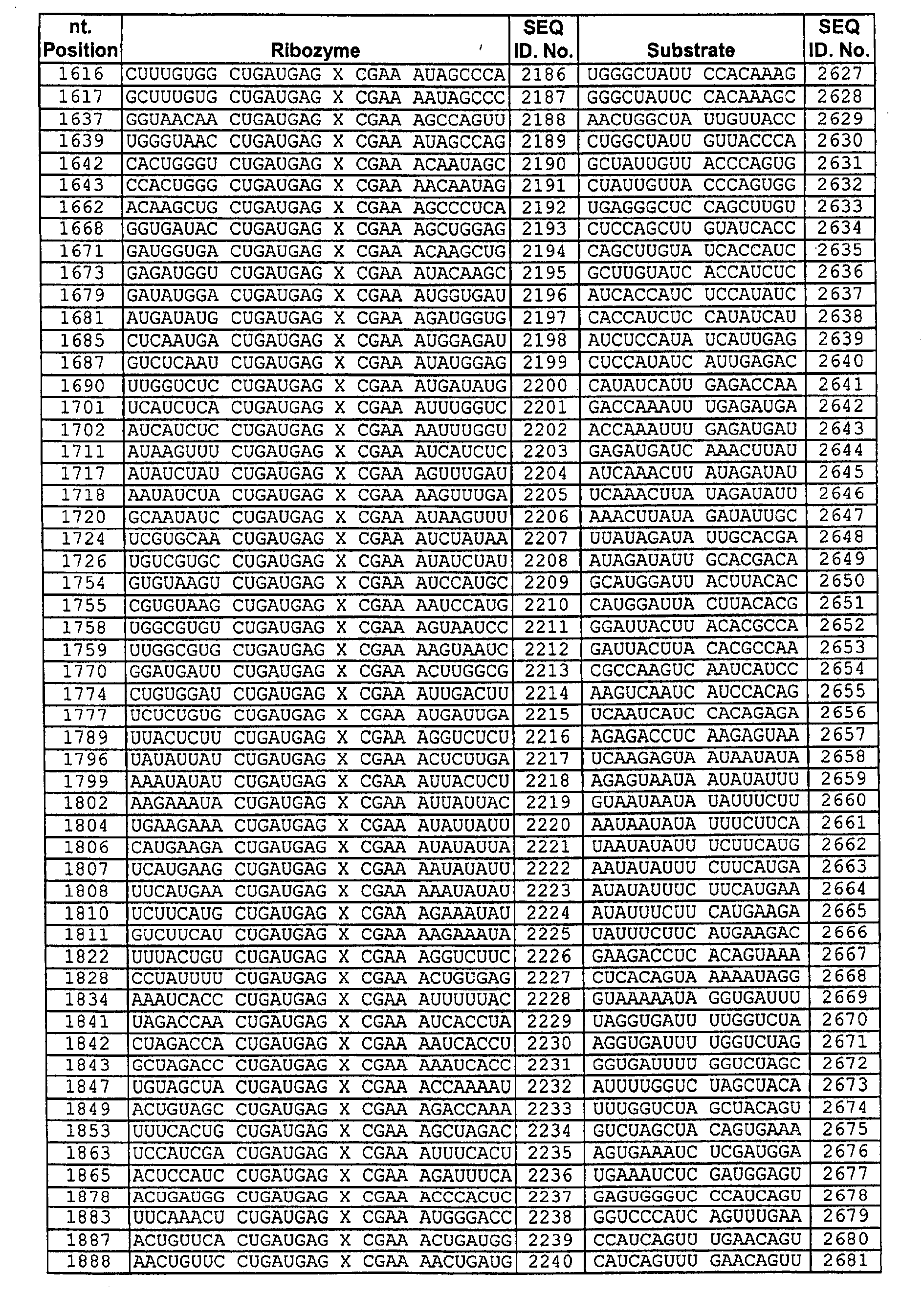

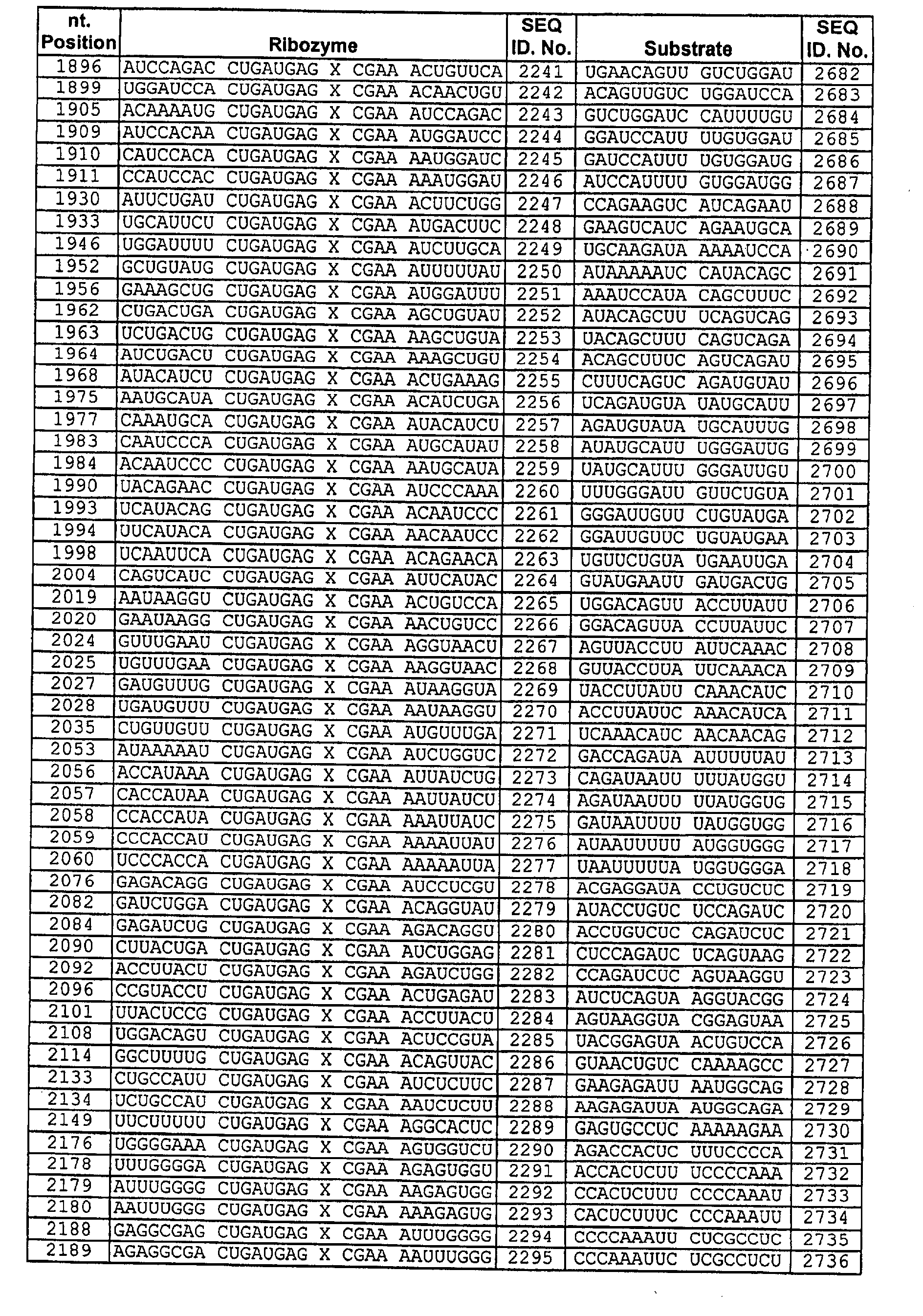

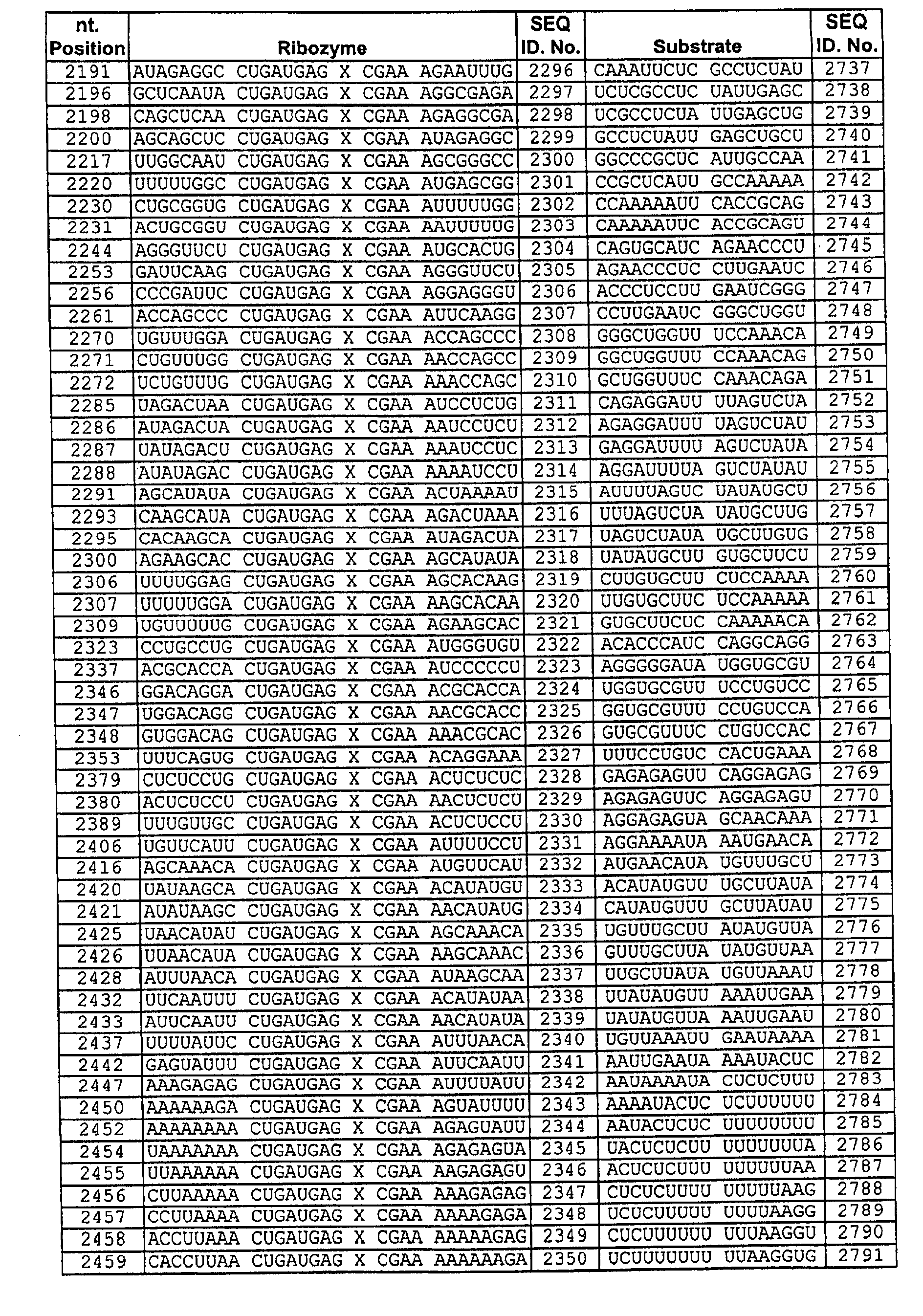

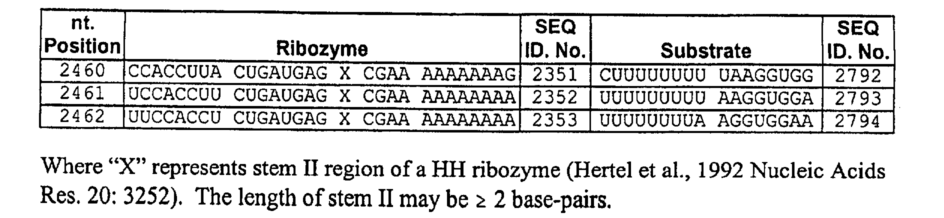

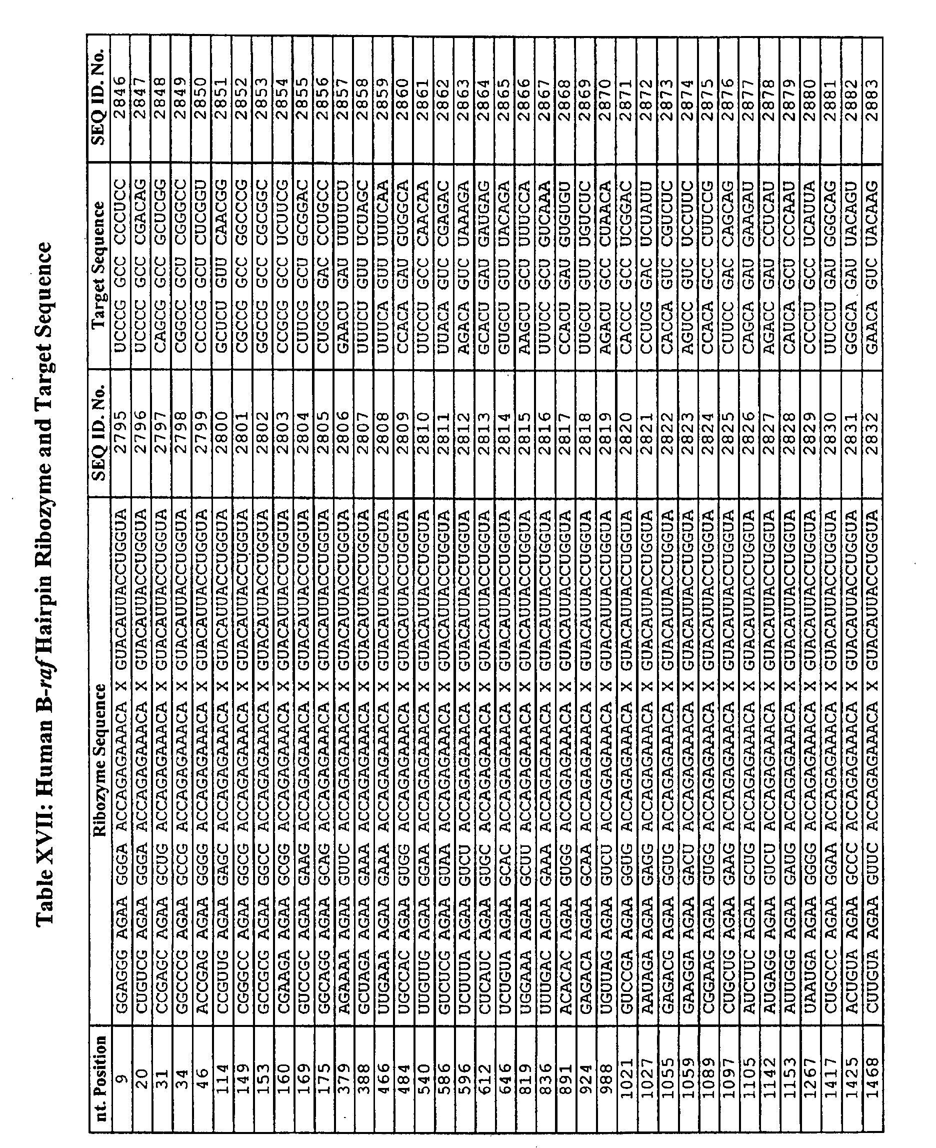

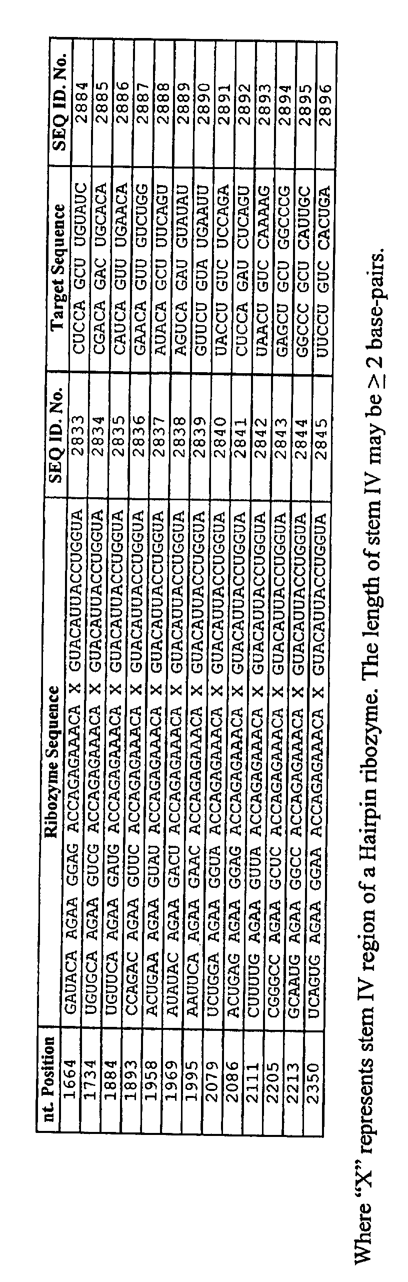

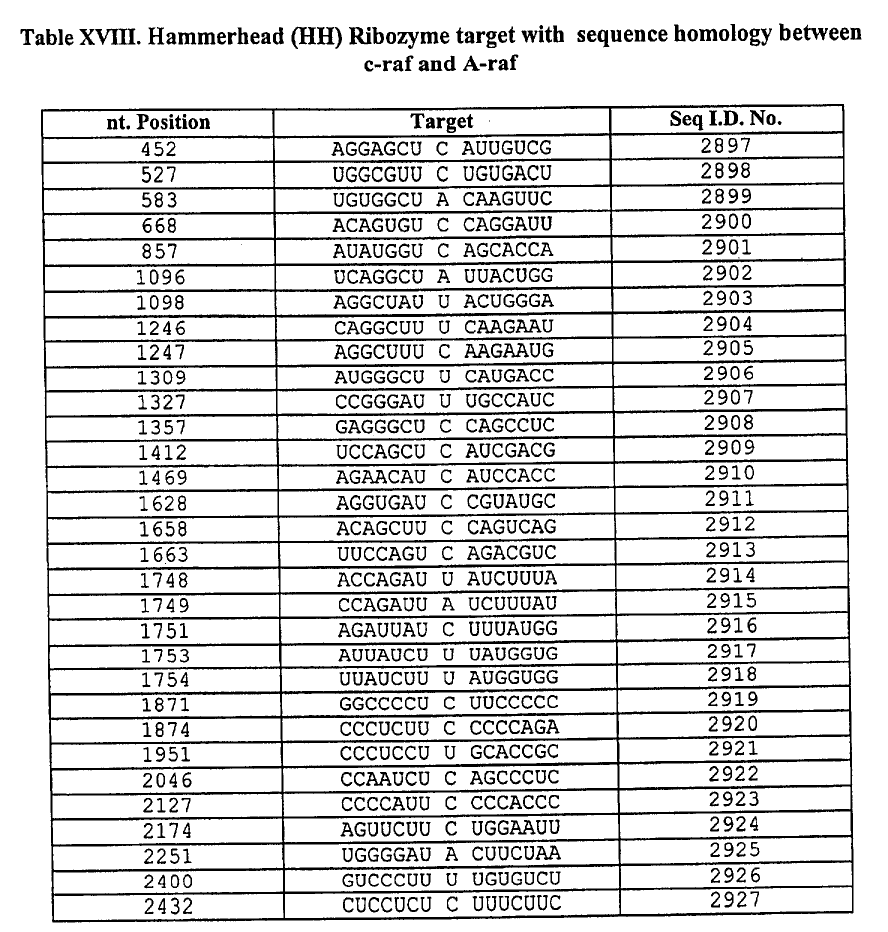

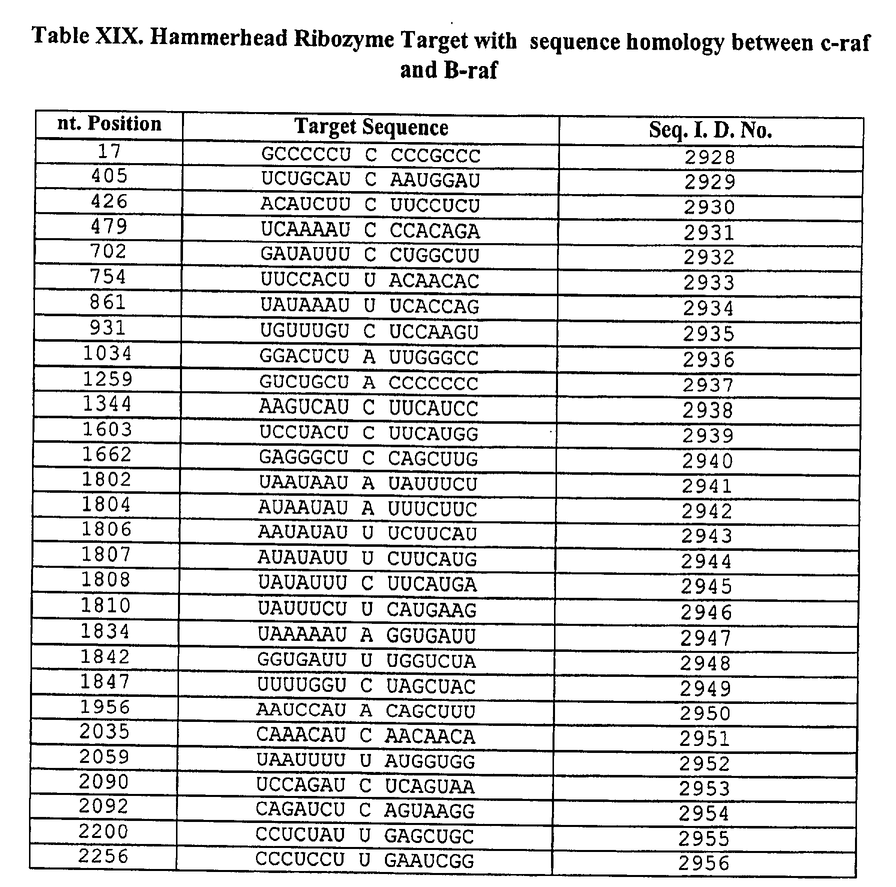

**Table XX.**

| **Experimental** **Group** | **Ribozyme** **Activity/Target** | **Dose** **(mg/kg/day)** | **Sample Size per** **dose** |
|---|---|---|---|
| RPI.4610 | Active/*flt-1* | 1, 3, 10, 30, 100 | 10 |
| RPI.4611 | Inactive/*flt-1* | 1, 3, 10, 30, 100 | 10 |
| RPI.4733 | Active/*flk-1* | 1, 3, 10, 30, 100 | 10 |
| RPI.4734 | Inactive/*flk-1* | 1, 3, 10, 30, 100 | 10 |
| Saline | NA | 12 µl/day | 10 |

## Claims

1. A hammerhead ribozyme which down regulates expression of *flt-1* gene, wherein said hammerhead comprises four phosphorothioate linkages at the 5'-end, a 2'-C-allyl substitution, and an inverted abasic nucleotide substitution at the 3'-end.

2. The hammerhead ribozyme of claim 1, wherein said hammerhead comprises a stem I and stem III sequence complimentary to *flt-1* mRNA.

3. The hammerhead ribozyme of claim 2, wherein said stem I is about 4 to about 10 nucleotides in length and said stem III is about 4 to about 10 nucleotides in length.

4. The hammerhead ribozyme of claim 2, wherein said stem I is about 7 nucleotides in length and said stem III is about 7 nucleotides in length.

5. A cell including the hammerhead ribozyme of claim 1.

6. The cell of claim 5, wherein said cell is a mammalian cell.

7. The cell of claim 6, wherein said cell is a human cell.

8. A medicament comprising the hammerhead ribozyme of claim 1 in a pharmaceutically acceptable carrier or diluent.

9. A method for treating tumor angiogenesis, comprising administering to a patient the medicament of claim 8 under conditions suitable for said treatment.
